# EUROPEAN PATENT APPLICATION

(11) **EP 2 135 611 A1**
(43) Date of publication of application: **23.12.2009**
(21) Application number: 09172321.3
(22) Date of filing: 01.03.2002
(51) Int. Cl.: A61K 31/57, C07J 1/00

(54) **Pregn-5-en-20-yne-3,7,17-triol derivatives for use in therapy**

(30) Priority: 01.03.2001 US 272624 P; 29.03.2001 US 820483; 10.09.2001 US 323016 P; 11.10.2001 US 328738 P; 01.11.2001 US 340054 P; 08.11.2001 US 338015 P; 20.12.2001 US 343523 P
(62) Divisional of application: 02709780.7
(71) Applicant: Hollis-Eden Pharmaceuticals Inc., San Diego, CA 92121-1980 (US)
(72) Inventor: Frincke, James, San Diego, CA 92192 (US)
(74) Representative: Huenges, Martin

(57) **Abstract**

The invention relates to the use of compounds to treat a number of conditions, such as thrombocytopenia, neutropenia or the delayed effects of radiation therapy. Compounds that can be used in the invention include methyl-2,3,4-trihydroxy-1-O-(7,17-dioxoandrost-5-ene-3β-yl)-β-D-glucopyranosiduronate.

## Description

### FIELD OF THE INVENTION

The invention relates to methods to make or use compounds, such as 16α-bromo-3β-hydroxy-5α-androstane-17-one (16α-bromoepiandrosterone or hereafter "BrEA") 3,7,16,17-tetrahydroxyandrost-5-ene, 3,7,16,17-tetrahydroxyandrostane, 3,17-dihydroxy-16-haloandrostane, methyl 2,3,4-trihydroxy-1-O-(7,17-dioxoandrost-5-ene-3β-yl)-β-D-glucopyranosiduronate and related compounds. The invention relates to the use of compounds to treat a number of conditions, such as thrombocytopenia and neutropenia.

### DESCRIPTION OF THE INVENTION

Mammalian immune responses to infections or other conditions are often characterized by responses mediated by different effector cell populations. In some situations, helper T cells designated Th1 in the murine system, facilitate immune effector functions that are typically dominated by cell-mediated responses. In other cases, helper T cells designated Th2 cells facilitate immune effector functions that are typically dominated by humoral responses. A vigorous Th1 response is usually desirable to help clear infections or to slow the progression of an infection. When a subject's immune response is biased to, or dominated by, a Th2-type response, the cytokines associated with the Th2 response tend to suppress the immune system's capacity to mount a vigorous Th1 response at the same time. The converse is also generally true. When mammalian immune responses begin to result in an increasing Th2 response, the Th1 response to the same condition tends to weaken. Insufficient Th1 responses may be associated with progression of some infections or other conditions, see, e.g., M. Clerici and G.M. Shearer, Immunol. Today 14:107-111, 1993; M. Clerici and G.M. Shearer, Immunol. Today 15:575-581, 1994. The invention provides compounds and compositions useful to enhance Th1 immune responses.

The use of 3β-hydroxyandrost-5-ene-17-one, 3β,17β-dihydroxyandrost-5-ene and other steroids to modulate immune functions or to stimulate myelopoiesis has been described, see, e.g., M.H. Whitnall et al., Int'l. J. Immunopharmacology 2000 22:1-14. U.S. patents 5,162,198, 5,206,008, 5,292,730, 5,407,684, 5,461,042, 5,461,768, 5,478,566, 5,585,371, 5,635,496, 5,641,766, 5,753,237, 5,837,269, 5,885,977 and 5,919,465, PCT publication nos. WO93/20696 and WO99/25333. I. Porsova-Dutoit et al., Physiological Res. 2000 49 (Suppl. 1):S43-S56, R.L. Jesse et al., Ann. N. Y. Acad. Sci. 1995 774:281-290 and U.S. patent 5,532,230, 5,811,418 and 5,846,963 discuss the capacity of 3β-hydroxyandrost-5-ene-17-one, its 3-sulfate derivative and other steroids to affect platelet and neutrophil aggregation or their adhesion to endothelial cells.

U.S. patents 5,532,230, 5,686,438, 5,753,640 and 5,811,418 and J. Bratt and M. Heimburger, Scand. J. Rheumatol. 1999 28:308-313 describe the capacity of compounds such as 3β,7β-dihydroxyandrost-5-ene-17-one, prednisolone, and 3β-hydroxyandrost-5-ene-17-one to limit tissue damage in ischemic tissues by inhibiting adhesion of cells such as neutrophils to endothelial cells or to treat pulmonary hypertension.

U.S. patent 5,859,000 describes the capacity of compounds such as 3β,7β-dihydroxyandrost-5-ene-17-one and 3β-hydroxyandrost-5-ene-17-one to reduce mast cell mediated allergic reactions.

U.S. patent 5,763,433 and PCT publication WO 96/35428 describe the capacity of compounds related to dehydroepiandrosterone and 16α-halodehydroepiandrosterone to modulate immune responses and to treat conditions certain immune related conditions such as systemic lupus erythematosus.

U.S. patents 5,925,630, 5,939,545 and 5,962,443 describe the capacity of 19-nor-pregnane steroids, 3α-hydroxy-5α-pregnan-20-one and related steroids to modulate certain neurological activities such as hypothalamic function and GABA receptor activity.

There is a current need for cost-effective pharmaceutical agents or treatment methods that are more effective in treating deficiencies of blood cells or reducing their symptoms. The present invention provides therapeutic agents and treatment methods to treat hemopoiesis deficiencies and disorders such as TP and NP. The agents and methods are thus useful to reduce one or more symptoms associated with any of these conditions. Also, the use of the invention agents and methods can be combined with one or more conventional treatments for these disorders.

Summary of invention embodiments. The invention provides for the use of a compound of formula 1 to prepare a medicament for the treatment of a blood cell deficiency in a subject wherein formula 1 has the structure

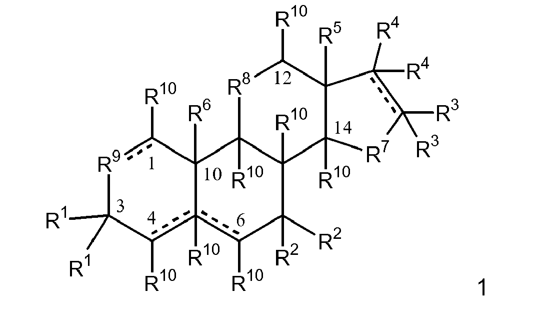

wherein, each R¹, R², R³, R⁴, R⁵, R⁶ and R¹⁰ independently are -H, -OH, -OR^{PR}, - SR^{PR}, -N(R^{PR})₂, -O-Si-(R¹³)₃, -CHO, -CHS, -CH=NH, -CN, -SCN, -NO₂, -OSO₃H, -OPO₃H, an ester, a thioester, a thionoester, a phosphoester, a phosphothioester, a phosphonoester, a phosphiniester, a sulfite ester, a sulfate ester, an amide, an amino acid, a peptide, an ether, a thioether, an acyl group, a thioacyl group, a carbonate, a carbamate, a halogen, an optionally substituted alkyl group, an optionally substituted alkenyl group, an optionally substituted alkynyl group, an optionally substituted aryl moiety, an optionally substituted heteroaryl moiety, an optionally substituted heterocycle, an optionally substituted monosaccharide, an optionally substituted oligosaccharide, a nucleoside, a nucleotide, an oligonucleotide, a polymer, or,

one or more of both R¹, R², R³ or R⁴ together comprise an independently selected spiro ring, or

one more of R¹, R², R³, R⁴, R⁵, R⁶ and R¹⁰ are =O, =S, =N-OH, =CH₂, or a spiro ring and the hydrogen atom or the second variable group that is bonded to the same carbon atom is absent, or,

one or more of two adjacent R¹-R⁶ and R¹⁰ comprise an independently selected acetal, thioacetal, ketal or thioketal moiety;

R⁷ is -C(R¹⁰)₂-, -C(R¹⁰)₂-C(R¹⁰)₂-, -C(R¹⁰)₂-C(R¹⁰)₂-C(R¹⁰)₂-, -C(R¹⁰)₂-O-C(R¹⁰)₂-, - C(R¹⁰)₂-S-C(R¹⁰)₂-, -C(R¹⁰)₂-NR^{PR}-C(R¹⁰)₂-, -O-, -O-C(R¹⁰)₂-, -S-, -S-C(R¹⁰)₂-, -NR^{PR}- or-NR^{PR}-C(R¹⁰)₂-;

R⁸ and R⁹ independently are -C(R¹⁰)₂-, -C(R¹⁰)₂-C(R¹⁰)₂-, -O-, -O-C(R¹⁰)₂-, -S-, -S-C(R¹⁰)₂-, -NR^{PR}- or -NR^{PR}-C(R¹⁰)₂-, or one or both of R⁸ or R⁹ independently are absent, leaving a 5-membered ring;

R¹³ independently is C₁₋₆ alkyl;

R^{PR} independently is -H or a protecting group;

The invention also provides for the use of a formula 1 compound to prepare a medicament for the treatment of a disease, condition or symptom that is caused by or associated with one or more of an autoimmune condition, an immune suppression condition, an infection, a cancer, a precancer, an inflammation condition, a neurological condition, a cardiovascular condition or a side-effect of radiation exposure or chemotherapy or a delayed adverse or unwanted effect of radiation exposure in a subject in need thereof. In some of these embodiments, the medicament is for administration to the subject or delivery to the subject's tissues beginning at least 1 day after the subject has been exposed to a dose of radiation that will cause or could potentially cause the one or more delayed adverse or unwanted effects of the radiation exposure or wherein the medicament is for administration to the subject or delivery to the subject's tissues beginning at least 1 day after the subject has been exposed to at least one subdose of a planned course of radiation exposures that will cause or could potentially cause the one or more delayed adverse effects or unwanted effects of the radiation exposure. In some of these embodiments 1, 2, 3, 4 or more R¹⁰ at the 1, 4, 6, 8, 9, 12 and 14 positions are not hydrogen. In other embodiments, they are all hydrogen atoms.

Invention embodiments also provide a method to modulate an immune or cellular response in a subject in need thereof comprising administering to the subject, or delivering to the subject's tissues, an effective amount of a compound of formula 1

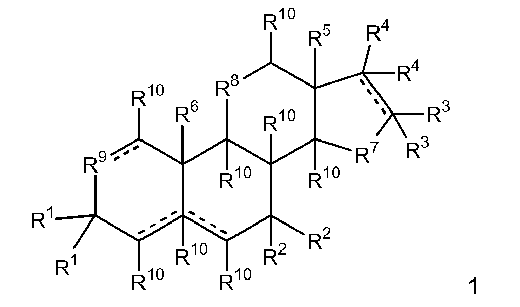

wherein,

each R¹, R², R³, R⁴, R⁵, R⁶ and R¹⁰ independently are -H, -OH, -OR^{PR}, -SR^{PR}, - N(R^{PR})₂, -O-Si-(R¹³)₃, -CHO, -CHS, -CH=NH, -CN, -SCN, -NO₂, -OSO₃H, -OPO₃H, an ester, a thioester, a thionoester, a phosphoester, a phosphothioester, a phosphonoester, a phosphiniester, a sulfite ester, a sulfate ester, an amide, an amino acid, a peptide, an ether, a thioether, an acyl group, a thioacyl group, a carbonate, a carbamate, a halogen, an optionally substituted alkyl group, an optionally substituted alkenyl group, an optionally substituted alkynyl group, an optionally substituted aryl moiety, an optionally substituted heteroaryl moiety, an optionally substituted heterocycle, an optionally substituted monosaccharide, an optionally substituted oligosaccharide, a nucleoside, a nucleotide, an oligonucleotide, a polymer, or,

one or more of both R¹, R², R³ or R⁴ together comprise an independently selected spiro ring, or

one more of R¹, R², R³, R⁴, R⁵, R⁶ and R¹⁰ independently are =O, =S, =N-OH, =CH₂, or a spiro ring, and the hydrogen atom or the second variable group that is bonded to the same carbon atom is absent, or,

one or more of two adjacent R¹-R⁶ and R¹⁰ comprise an independently selected acetal, thioacetal, ketal or thioketal moiety;

R⁷ is -C(R¹⁰)₂-, -C(R¹⁰)₂-C(R¹⁰)₂-, -C(R¹⁰)₂-C(R¹⁰)₂-C(R¹⁰)₂-, -C(R¹⁰)₂-O-C(R¹⁰)₂-, - C(R¹⁰)₂-S-C(R¹⁰)₂-, -C(R¹⁰)₂-NR^{PR}-C(R¹⁰)₂-, -O-, -O-C(R¹⁰)₂-, -S-, -S-C(R¹⁰)₂-, -NR^{PR}- or-NR^{PR}-C(R¹⁰)₂-;

R⁸ and R⁹ independently are -C(R¹⁰)₂-, -C(R¹⁰)₂-C(R¹⁰)₂-, -O-, -O-C(R¹⁰)₂-, -S-, -S-C(R¹⁰)₂-, -NR^{PR}- or -NR^{PR}-C(R¹⁰)₂-, or one or both of R⁸ or R⁹ independently are absent, leaving a 5-membered ring;

R¹³ independently is C₁₋₆ alkyl;

R^{PR} independently is -H or a protecting group;

Immune and cellular response modulation includes enhancing Th1 immune responses, reducing Th2 immune responses, reducing inflammation and enhancing hemopoiesis. These compounds are useful in treating the diseases, conditions and symptoms described herein.

Other embodiments include a method to enhance the expression of one or more cytokines or interleukins that facilitate Th1 or Tc1 immune responses in a subject or to reduce the expression of one or more cytokines or interleukins that facilitate Th2 or Tc2 immune response in a subject comprising administering to the subject an effective amount of of a formula 1 compound, whereby the subject's Th1 or Tc1 immune response is enhanced ot the subject's undesired Th2 or Tc2 immune response is reduced.

A further embodiment is a method to modulate a subject's innate immunity, Th1 immune responses, Tc1 immune responses, Th2 immune responses, Tc2 immune responses, or inflammation comprising administering an effective amount of a formula 1 compound to a subject or delivering the formula 1 compound to the subject's tissues.

The formula 1 compounds are useful to prepare a medicament for use in the treatment of one or more of the diseases, conditions or symptoms described herein.

Other embodiments are as described elsewhere in the specification including the numbered embodiments and the claims.

Definitions. As used herein and unless otherwise stated or implied by context, terms that are used herein have the meanings that are defined here. The descriptions of embodiments and examples that are described illustrate the invention and they are not intended to limit it in any way. Unless otherwise contraindicated or implied, e.g., by including mutually exclusive elements or options, in these definitions and throughout this specification, the terms "a" and "an" mean one or more and the term "or" means and/or.

An "invention formulation", "formulation" or the like means a composition that one can administer to a subject, e.g., human or animal, without further manipulations that change the ingredients or the ingredient proportions that are present. Formulations are suitable for human or veterinary applications and would typically have expected characteristics for the formulation, e.g., parenteral formulations for human use would usually be sterile.

An "invention composition", "composition" or the like means a composition, that is a formulation or that can be an intermediate one can use to make the formulations, i.e., a change(s) in an ingredient(s) or its amount(s) may be needed to make a formulation. Compositions may also comprise other types of materials, e.g., reagents for assays or cells that are optionally contacted with a formula 1 compound or mixtures of compounds. Thus, invention compositions include compositions where further processing may be required before it is a formulation, e.g., mixing or addition of a desired amount of an ingredient.

Phrases such as "administration of a compound of formula 1 ", "treatment with a formula 1 compound" or similar terms mean that the compound(s) is administered to, or delivered to, the subject or to the subject's tissues by one or more suitable methods, e.g., by an oral, topical, parenteral, buccal or sublingual route.

Expressions such as "a formula 1 compound(s)", "a formula 1 compound" and the like mean invention compositions or formulations where one or more than one formula 1 compound is present, e.g., in a composition, or is used in the disclosed method, typically 1, 2, 3 or 4, usually 1. Any reference to a "formula 1 compound", "one or more compounds of formula 1" or the like means that the formula 1 compound can have the formula 2 structure or any other structure disclosed herein that is within the definition of formula 1 compounds.

Reference to subject matter "as disclosed herein" such as a "therapeutic treatment or agent as disclosed herein", a "dosing protocol as disclosed herein" or a "clinical condition or symptom as disclosed herein" or the like means a treatment, agent, protocol, condition, symptom or the like that is described herein or in any reference that is cited herein.

An "excipient", "carrier", "pharmaceutically acceptable carrier" or similar terms mean one or more component(s) or ingredient(s) that is acceptable in the sense of being compatible with the other ingredients of invention compositions or formulations and not overly deleterious to the patient, animal, tissues or cells to which the formulation is to be administered.

As used here, "excipients" include liquids, such as benzyl benzoate, cottonseed oil, N,N-dimethylacetamide, a C₂₋₁₂ alcohol (e.g., ethanol), glycerol, peanut oil, a polyethylene glycol ("PEG"), vitamin E, poppyseed oil, propylene glycol, safflower oil, sesame oil, soybean oil and vegetable oil. Any solid excipient may be a fine powder or granulated. Excipients, as used herein may optionally exclude one or more excipient, e.g., chloroform, dioxane, vegetable oil, DMSO, other excipients or any combination of these. Excipients include one or more components typically used in the pharmaceutical formulation arts, e.g., one, two or more of fillers, binders, disintegrants, dispersants, preservatives, glidants and lubricants. Exemplary excipients include povidone, crospovidone, corn starch, carboxymethyl cellulose, hydroxypropyl methylcellulose, microcrystalline cellulose, gum arabic, polysorbate 80, butylparaben, propylparaben, methylparaben, BHA, EDTA, sodium lauryl sulfate, sodium chloride, potassium chloride, titanium dioxide, magnesium stearate, castor oil, olive oil, vegetable oil, buffering agents such as sodium hydroxide, monobasic sodium phosphate, dibasic sodium phosphate, potassium hydroxide, monobasic potassium phosphate, dibasic potassium phosphate, tribasic potassium phosphate, potassium carbonate, potassium bicarbonate, ammonium hydroxide, ammouium chloride, saccharides such as mannitol, glucose, fructose, sucrose or lactose any of which may be compressible or any of which may be spray dried.

A "subject" means a human or animal. Usually the animal is a mammal or vertebrate such as a primate, rodent, lagomorph, domestic animal or game animal. Primates include chimpanzees, cynomologous monkeys, spider monkeys, and macaques, e.g., *Rhesus* or *Pan*. Rodents and lagomorphs include mice, rats, woodchucks, ferrets, rabbits and hamsters. Domestic and game animals include cows, horses, pigs, sheep, deer, bison, buffalo, mink, felines, e.g., domestic cat, canines, e.g., dog, wolf and fox, avian species, e.g., chicken, turkey, emu and ostrich, and fish, e.g., trout, catfish and salmon. Subject includes any subset of the foregoing, e.g., all of the above, but excluding one or more groups or species such as humans, primates or rodents. Other subsets of subjects include subjects of a given species or group of species of varying ages, e.g., young humans, e.g., about 1 week of age to about 9 years of age, adolescent humans, e.g., about 10-19 years of age, adult humans, e.g., about 20-100 years of age, and mature adult or elderly humans, e.g., at least about 55 years of age, at least about 60 years of age, at least about 65 years of age or a range of ages such as about 60-100 years of age. Thus, as used herein, prevention or treatment of a disease, condition or symptom may include or exclude any subset of subjects that are grouped by age.

The terms "effective amount", "effective dose" or the like mean an amount of a formula 1 compound that is sufficient to elicit a desired response, e.g., restoration of normal immune responsiveness in an immunodeficient subject to which it is administered or to detectable modulation or amelioration of an immune or cellular parameter or a clinical condition or symptom. An effective amount may be a a single dose or two or more subdoses of a forlula 1 compound administered in one day, or it may be administered as multiple doses over a period of time, e.g., over 2 days to about 1 year.

References such as "a disease", "a disorder", "a condition", "a symptom' or "a complication" or the like mean that one or more disease, disorder, condition, symptom or complication may be treated.

Terms such as "use", "treat", "treatment", "address" or the like in the context of using the formula 1 compounds in the treatment methods or other methods disclosed herein mean that a formula 1 compound is administered to a subject, delivered to the subject's tissues or contacted with tissues, cells or cell free systems, e.g., as described herein or a reference cited herein. Typically such use or treatment results in detectable improvement in or amelioration of the condition or symptom being treated. Such amelioration may be transient, e.g., lasting for at least a few, e.g., about 1 to 24, hours or days, e.g., about 1-,7 days, or amelioration may be prolonged, e.g., lasting about 8 to about 60 days or more, or it may be permanent. A treatment may slow the progression or severity of a disease or symptom. A use or treatment may result in detectable modulation in a relevant immune parameter such as modulation of the level or activity of a target effector or suppressor immune cell population, interleukin, cytokine, chemokine, immunoglobulin or modulation of the level or activity of a relevant transcription factor, enzyme or cell biological activity. A treatment with the compounds may be used to prevent the onset of a disease, symptom or complication or to ameliorate a preexisting disease, condition, symptom or complication, or to facilitate elimination of a disease, condition, symptom or complication.

"Ameliorate", "amelioration", "improvement" or the like means a detectable improvement or a detectable change consistent with improvement occurs in a subject or in at least a minority of subjects, e.g., in at least about 2%, 5%, 10%, 15%, 20%, 25%, 30%, 40%, 50%, 60%, 70%, 75%, 80%, 85%, 90%, 95%, 98%, 100% or in a range about between any two of these values. Such improvement or change may be observed in treated subjects as compared to subjects not treated with a formula 1 compound, where the untreated subjects have, or are subject to developing, the same or similar disease, condition, symptom or the like. Amelioration of a disease, condition, symptom or assay parameter may be determined subjectively or objectively, e.g., self assessment by a subject(s), by a clinician's assessment or by conducting an appropriate assay or measurement, including, e.g., a quality of life assessment, a slowed progression of a disease(s) or condition(s), a reduced severity of a disease(s) or condition(s), or a suitable assay(s) for the level or activity(ies) of a biomolecule(s), cell(s) or by detection of cell migration within a subject. Amelioration may be transient, prolonged or permanent or it may be variable at relevant times during or after a formula 1 compound is administered to a subject or is used in an assay or other method described herein or a cited reference, e.g., within about 1 hour of the administration or use of a formula 1 compound to about 3, 6, 9 months or more after a subject(s) has received a formula 1 compound.

The "modulation" of, e.g., a symptom, level or biological activity of a molecule, replication of a pathogen, cellular response, cellular activity or the like, means that the cell, level or activity, or the like is detectably increased or decreased. Such increase or decrease may be observed in treated subjects as compared to subjects not treated with a formula 1 compound, where the untreated subjects have, or are subject to developing, the same or similar disease, condition, symptom or the like. Such increases or decreases may be at least about 2%, 5%, 10%, 15%, 20%, 25%, 30%, 40%, 50%, 60%, 70%, 75%, 80%, 85%, 90%, 95%, 98%, 100%, 150%, 200%, 250%, 300%, 400%, 500% or more or about within any range about between any two of these values. Modulation may be determined subjectively or objectively, e.g., by the subject's self assessment, by a clinician's assessment or by conducting an appropriate assay or measurement, including, e.g., quality of life assessments or suitable assays for the level or activity of molecules, cells or cell migration within a subject. Modulation may be transient, prolonged or permanent or it may be variable at relevant times during or after a formula 1 compound is administered to a subject or is used in an assay or other method described herein or a cited reference, e.g., within about 1 hour of the administration or use of a formula 1 compound to about 3, 6, 9 months or more after a subject(s) has received a formula 1 compound.

At various locations in the present disclosure, e.g., in any disclosed embodiments or in the claims, reference is made to compounds, compositions, formulations, or methods that comprise one or more specified components, elements or steps. Invention embodiments also specifically include those compounds, compositions, formulations or methods that consist of or that consist essentially of those specified components, elements or steps. The terms "comprising", "consist of" and "consist essentially of " have meanings as follows. Compositions or methods that "comprise" a component or step are open and they optionally include on those specified compositions or methods plus an additional component(s) or step(s). Similarly, compositions or methods that "consist of" a component or step would not include on those compositions or methods having appreciable amounts of an additional component(s) or an additional step(s). Compositions or methods that "consist essentially of" a component or step would optionally include on those compositions or methods having minor amounts of an additional component(s) or an additional step(s), but without significantly affecting the salient desired properties of the specified composition.

"Alkyl" as used here means linked normal, secondary, tertiary or cyclic carbon atoms, i.e., linear, branched, cyclic or any combination thereof. Alkyl moieties, as used herein, may be saturated, or unsaturated, i.e., the moiety may comprise one or more independently selected double bonds or triple bonds. Unsaturated alkyl moieties include moieties as described for alkenyl and alkynyl moieties described below. The number of carbon atoms in an alkyl group or moiety is 1 to about 50, e.g., about 1-30 or about 1-20, unless otherwise specified, e.g., C₁₋₈ alkyl means an alkyl moiety containing 1, 2, 3, 4, 5, 6, 7 or 8 carbon atoms. When an alkyl group is specified, species may include methyl, ethyl, 1-propyl (*n*-propyl), 2-propyl (*i*-propyl, -CH(CH₃)₂), 1-butyl (*n*-butyl), 2-methyl-1-propyl (*i*-butyl, - CH₂CH(CH₃)₂), 2-butyl (*s*-butyl, -CH(CH₃)CH₂CH₃ ), 2-methyl-2-propyl (*t*-butyl, -C(CH₃)₃), 1-pentyl (*n*-pentyl), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl, 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), cyclopropyl, cyclobutyl, cyclopentyl, cyclohexyl, cycloheptyl, cyclooctyl, - (CH₂)ₙ-(CHCH₃)ₘ-(CH₂)ₒ-CH₃ and -(CH₂)ₙ-(CHC₂H₅)ₘ-(CH₂)ₒ-CH₃ where n, m and o independently are 0, 1, 2, 3, 4, 5, 6, 7 or 8.

"Alkenyl" as used here means a moiety that comprises linked normal, secondary, tertiary or cyclic carbon atoms, i.e., linear, branched, cyclic or any combination thereof, that comprises one or more double bonds (e.g., -CH=CH-), e.g., 1, 2, 3, 4, 5, 6 or more, typically 1 or 2. The number of carbon atoms in an alkenyl group or moiety is 2 to about 50, e.g., about 2-30 or about 2-20, unless otherwise specified, e.g., C₂₋₈ alkenyl or C2-8 alkenyl means an alkenyl moiety containing 2, 3, 4, 5, 6, 7 or 8 carbon atoms. When an alkenyl group is specified, species may include vinyl, allyl, -(CH₂)ₙ-(CH=CH)-(CH₂)ₘ-CH₃, -(CH₂)ₙ-(CCH₃=CH)-(CH₂)ₘ-CH₃, -(CH₂)ₙ-(CH=CCH₃)-(CH₂)ₘ-CH₃ and -(CH₂)ₙ-(CH=CH)₀₋₁-(CH₂)ₘ-CH₂CH=CH₂, where n and m independently are 0, 1, 2, 3, 4, 5, 6, 7 or 8.

"Alkynyl" as used here means a moiety that comprises linked normal, secondary, tertiary or cyclic carbon atoms, i.e., linear, branched, cyclic or any combination thereof, that comprises one or more triple bonds (-C≡C-), e.g., 1, 2, 3, 4, 5, 6 or more, typically 1 or 2 triple bonds, optionally comprising 1, 2, 3, 4, 5, 6 or more double bonds, with the remaining bonds being single bonds. The number of carbon atoms in an alkenyl group or moiety is 2 to about 50, e.g., about 2-30 or about 2-20, unless otherwise specified, e.g., C₂₋₈ alkynyl or C2-8 alkynyl means an alkynyl moiety containing 2, 3, 4, 5, 6, 7 or 8 carbon atoms. When an alkynyl group is specified, groups and species may include -CCH, -CCCH₃, -CCCH₂CH₃, - CCC₃H₇, -CCCH₂C₃H₇, -(CH₂)ₙ-(C≡C)-(CH₂)ₘ-CH₃, and -(CH₂)ₙ-(C≡C)₀₋₁-(CH₂)ₘ-CH₂C≡CH, where n and m independently are 0, 1, 2, 3, 4, 5, 6, 7 or 8.

"Aryl" means phenyl or naphthyl.

"Substituted alkyl", "substituted alkenyl", "substituted heterocycle", "substituted aryl", "substituted monosaccharide" and the like mean an alkyl, alkenyl, heterocycle, aryl, monosaccharide or other group or moiety as defined herein that has a substituent(s) or that comprises a substituent(s) that replaces a hydrogen atom(s) and is bonded to a carbon atom(s) or a substituent(s) that interrupts a carbon atom chain. Substituted heterocycles may have a substituent bonded to a ring carbon or a ring heteroatom such as a nitrogen. Alkenyl and alkynyl groups that comprise a substituent(s), are optionally substituted at a carbon that is one or more methylene moiety removed from the double bond, e.g., separated by one, two, three or more independently selected -CH₂-, -CH(C₁₋₆ optionally substituted alkyl)-, - CH(C₁₋₆ optionally substituted alkenyl)-, -CH(C₁₋₆ optionally substituted alkynyl)-, - CH(optionally substituted heterocycle)-, -CH(optionally substituted aryl-optionally substituted alkyl)- or -CH(optionally substituted alkyl-optionally substituted aryl)- moieties.

"Heterocycle" or "heterocyclic" includes by way of example and not limitation the heterocycles described in Paquette, Leo A.; "Principles of Modern Heterocyclic Chemistry" (W. A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Volumes 13, 14, 16, 19, and 28; and J. Am. Chem. Soc. 1960, 82:5566. Heterocycles are typically bonded to moieties of which they are a part through a ring carbon atom, a ring nitrogen atom or a ring sulfur atom.

Examples of heterocycles include by way of example and not limitation pyridyl, thiazolyl, tetrahydrothiophenyl, sulfur oxidized tetrahydrothiophenyl, pyrimidinyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, piperidinyl, 4-piperidonyl, pyrrolidinyl, 2-pyrrolidonyl, pyrrolinyl, tetrahydrofuranyl, tetrahydroquinolinyl, tetrahydroisoquinolinyl, decahydroquinolinyl, octahydroisoquinolinyl, azocinyl, triazinyl, 6H-1,2,5-thiadiazinyl, 2H,6H-1,5,2dithiazinyl, thienyl, thianthrenyl, pyranyl, isobenzofuranyl, chromenyl, xanthenyl, phenoxathiinyl, 2H-pyrrolyl, isothiazolyl, isoxazolyl, pyrazinyl, pyridazinyl, indolizinyl, isoindolyl, 3H-indolyl, 1H-indazoly, purinyl, 4H-quinolizinyl, phthalazinyl, naphthyridinyl, quinoxalinyl, quinazolinyl, cinnolinyl, pteridinyl, 4aH-carbazolyl, carbazolyl, β-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl, indolinyl, isoindolinyl, quinuclidinyl, morpholinyl, oxazolidinyl, benzotriazolyl, benzisoxazolyl, oxindolyl, benzoxazolinyl, and isatinoyl.

By way of example and not limitation, carbon bonded heterocycles are bonded at position 2, 3, 4, 5, or 6 of a pyridine, position 3, 4, 5, or 6 of a pyridazine, position 2, 4, 5, or 6 of a pyrimidine, position 2, 3, 5, or 6 of a pyrazine, position 2, 3, 4, or 5 of a furan, tetrahydrofuran, thiofuran, thiophene, pyrrole or tetrahydropyrrole, position 2, 4, or 5 of an oxazole, imidazole or thiazole, position 3, 4, or 5 of an isoxazole, pyrazole, or isothiazole, position 2 or 3 of an aziridine, position 2, 3, or 4 of an azetidine, position 2, 3, 4, 5, 6, 7, or 8 of a quinoline or position 1, 3, 4, 5, 6, 7, or 8 of an isoquinoline. Still more typically, carbon bonded heterocycles include 2-pyridyl, 3-pyridyl, 4-pyridyl, 5-pyridyl, 6-pyridyl, 3-pyridazinyl, 4-pyridazinyl, 5-pyridazinyl, 6-pyridazinyl, 2-pyrimidinyl, 4-pyrimidinyl, 5--pyrimidinyl, 6-pyrimidinyl, 2-pyrazinyl, 3-pyrazinyl, 5-pyrazinyl, 6-pyrazinyl, 2-thiazolyl, 4-thiazolyl, or 5-thiazolyl.

By way of example and not limitation, nitrogen bonded heterocycles are bonded at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, position 2 of a isoindole, or isoindoline, position 4 of a morpholine, and position 9 of a carbazole, or β-carboline. Typically, nitrogen bonded heterocycles include 1-aziridyl, 1-azetedyl, 1-pyrrolyl, 1-imidazolyl, 1-pyrazolyl, and 1-piperidinyl.

"Heteroaryl" means an aromatic ring or two or more fused rings that contain one or more aromatic rings where the ring or fused rings comprise 1, 2, 3 or more heteroatoms, usually oxygen (-O-), nitrogen (-NX-) or sulfur (-S-) where X is -H, a protecting group or C₁₋₆ alkyl, usually -H. Examples are as described for heterocycle.

"Alcohol" as used herein means an alcohol that comprises a C₁₋₁₂ alkyl moiety substituted at a hydrogen atom with one hydroxyl group. Alcohols include methanol, ethanol, *n*-propanol, *i*-propanol, *n*-butanol, *i*-butanol, *s*-butanol, *t*-butanol, *n*-pentanol, *i*-pentanol, *n-*hexanol, cyclohexanol, *n*-heptanol, *n*-octanol, *n*-nonanol and *n*-decanol. The carbon atoms in alcohols can be straight, branched or cyclic. Alcohol includes any subset of the foregoing, e.g., C₂₋₄ alcohols (alcohols having 2, 3 or 4 carbon atoms).

"Halogen" means fluorine, chlorine, bromine or iodine.

"Protecting group" means a moiety that prevents the atom to which it is linked from participating in unwanted reactions. For example, for -OR^{PR}, R^{PR} may be hydrogen or a protecting group for the oxygen atom found in a hydroxyl, while for -C(O)-OR^{PR}, R^{PR} may be hydrogen or a carboxyl protecting group, for -SR^{PR}, R^{PR} may be hydrogen or a protecting group for sulfur in thiols for instance, and for -NHR^{PR} or -N(R^{PR})₂-, R^{PR} may be hydrogen or a nitrogen atom protecting group for primary or secondary amines. Hydroxyl, amine and other reactive groups are found in formula 1 compounds at, e.g., R¹ or R². These groups may require protection against reactions taking place elsewhere in the molecule. The protecting groups for oxygen, sulfur or nitrogen atoms are usually used to prevent unwanted reactions with electrophilic compounds, such as acylating used, e.g., in steroid chemistry.

"Ester" means a moiety that comprises a -C(O)-O- structure. Typically, esters as used here comprise an organic moiety containing about 1-50 carbon atoms (e.g., about 2-20 carbon atoms) and 0 to about 10 independently selected heteroatoms (e.g., O, S, N, P, Si), where the organic moiety is bonded to a formula 1 steroid nucleus at, e.g., R¹ or R² through the -C(O)-O- structure, e.g., organic moiety-C(O)-O-steroid or organic moiety-O-C(O)-steroid. The organic moiety usually comprises one or more of any of the organic groups described above, e.g., C₁₋₂₀ alkyl moieties, C₂₋₂₀ alkenyl moieties, C₂₋₂₀ alkynyl moieties, aryl moieties, C₂₋₉ heterocycles or substituted derivatives of any of these, e.g., comprising 1, 2, 3, 4 or more substituents, where each substituent is independently chosen. Exemplary substitutions for hydrogen or carbon atoms in these organic groups are as described above for substituted alkyl moieties and include 1, 2, 3, 4, 5, 6 or more, usually 1, 2, or 3 -O-, -S-, - NR^{PR}- (including -NH-), -C(O)-, -CHO, -CHS, -C=NH, -C(S), =O, =S, -N(R^{PR})₂ (including - NH₂), -C(O)OR^{PR} (including -C(O)OH), -OC(O)R^{PR} (including -O-C(O)-H), -OR^{PR} (including - OH), -SR^{PR} (including -SH), -NO₂, -CN, -SCN, -C₆H₅, -CH₂C₆H₅, -NHC(O)-, -C(O)NH-, - OC(O)-, -C(O)O-, -O-A8, -S-A8, -C(O)-A8, -OC(O)-A8, -C(O)O-A8, =N-, -N=, =N-OH, - OPO₃(R^{PR})₂, -OSO₃H₂ or halogen moieties or atoms, where each R^{PR} is -H, an independently selected protecting group or both R^{PR} together comprise a protecting group, and A8 is C₁₋₈ alkyl, C₂₋₈ alkenyl, C₂₋₈ alkynyl, C₁₋₄ alkyl-aryl (e.g., benzyl), aryl (e.g. phenyl) or C₀₋₄ alkyl-C₂₋₉ heterocycle. Substitutions are independently chosen. The organic moiety includes compounds defined by the R₄ variable. The organic moieties exclude obviously unstable moieties, e.g., -O-O-, except where such unstable moieties are transient species that one can use to make a compound with sufficient chemical stability for one or more of the uses described herein, including for synthesis of the formula 1 or other compounds. The substitutions listed above are typically substituents that one can use to replace one or more carbon atoms, e.g., -O- or -C(O)-, or one or more hydrogen atom, e.g., halogen, -NH₂ or - OH. Exemplary esters include one or more independently selected acetate, enanthate, propionate, isopropionate, cyclopropionate, isobutyrate, butyrate, valerate, caproate, isocaproate, hexanoate, heptanoate, octanoate, nonanoate, decanoate, undecanoate, phenylacetate or benzoate, which are typically hydroxyl esters.

"Amide" means an organic moiety as described for ester that comprises 1, 2, 3, 4 or more -C(O)-NR^{PR}- moieties, usually 1 or 2, where R^{PR} is -H or a protecting group, R^{PR} is usually H. In some embodiments, the -C(O)NR^{PR}- group is linked to the steroid nucleus at a variable group such as R¹-R⁶, R¹⁰, R¹⁵, R¹⁷ or R¹⁸, i.e., organic moiety-C(O)NR^{PR}-steroid or steroid-C(O)NR^{PR}-organic moiety. The organic moiety is as described above for esters.

"Ether" means an organic moiety as described for ester that comprises 1, 2, 3, 4 or more -O- moieties, usually 1 or 2. In some embodiments, the -O- group is linked to the steroid nucleus at a variable group such as R¹-R⁶, R¹⁰, R¹⁵, R¹⁷ or R¹⁸, e.g., organic moiety-O-steroid. The organic moiety is as described above for esters.

"Acyl group" means an organic moiety as described for ester that comprises 1, 2, 3, 4 or more -C(O)- groups. In some embodiments, the -C(O)- group is linked to the steroid nucleus at a variable group such as R¹-R⁶, R¹⁰, R¹⁵, R¹⁷ or R¹⁸, e.g., organic moiety-C(O)-steroid. The organic moiety is as described above for esters.

"Carbonate" means an organic moiety as described for ester that comprises 1, 2, 3, 4 or more -O-C(O)-O- structures. Typically, carbonate groups as used here comprise an organic moiety containing about 1-50 carbon atoms and 0 to about 10 heteroatoms (e.g., O, S, N, P, Si) linked to a formula 1 steroid nucleus at a variable group such as R¹-R⁶, R¹⁰, R¹⁵, R¹⁷ or R¹⁸ through the -O-C(O)-O- structure, e.g., organic moiety-O-C(O)-O-steroid. The organic moiety is as described above for esters.

"Carbamate" means an organic moiety as described for ester that comprises 1, 2, 3, 4 or more -O-C(O)NR^{PR}- structures where R^{PR} is -H, a protecting group or an organic moiety as described for ester. Typically, carbamate groups as used here comprise an organic moiety containing about 1-50 carbon atoms and 0 to about 10 heteroatoms (e.g., O, S, N, P, Si) linked to a formula 1 steroid nucleus at a variable group such as R¹-R⁶, R¹⁰, R¹⁵, R¹⁷ or R¹⁸ through the -O-C(O)-NR^{PR}- structure, e.g., organic moiety-O-C(O)-NR^{PR}-steroid or steroid-O-C(O)-NR^{PR}-organic moiety. The organic moiety is as described above for esters.

As used herein, "monosaccharide" means a polyhydroxy aldehyde or ketone having the empirical formula (CH₂O)ₙ where n is 3, 4, 5, 6 or 7. Monosaccharide includes open chain and closed chain forms, but will usually be closed chain forms. Monosaccharide includes hexofuranose and pentofuranose sugars such as 2'-deoxyribose, ribose, arabinose, xylose, their 2'-deoxy and 3'-deoxy derivatives and their 2',3'-dideoxy derivatives. Monosaccharide also includes the 2',3' dideoxydidehydro derivative of ribose.

Optionally substituted alkyl group, optionally substituted alkenyl group, optionally substituted alkynyl group, optionally substituted aryl moiety and optionally substituted heterocycle mean an alkyl, alkenyl, alkynyl, aryl or heterocycle moiety that contains an optional substitution(s). Such moieties include include C₁₋₂₀ alkyl moieties, C₂₋₂₀ alkenyl moieties, C₂₋₂₀ alkynyl moieties, aryl moieties, C₂₋₉ heterocycles or substituted derivatives of any of these. Typical substitutions for these organic groups are as described above for substituted alkyl moieties and include, e.g., 1, 2, 3, 4, 5, 6 or more, independently selected - O-, -S-, -NR^{PR}-, -C(O)-, -N(R^{PR})₂, -C(O)OR^{PR}, -OC(O)R^{PR}, -OR^{PR}, -SR^{PR}, -NO₂, -CN, - NHC(O)-, -C(O)NH-, -OC(O)-, -C(O)O-, -O-A8, -S-A8, -C(O)-A8, -OC(O)-A8, -C(O)O-A8, =N-, -N=, -OPO₂R^{PR}, -OSO₃H or halogen moieties or atoms, where R^{PR} independently is -H, a protecting group or both R^{PR} together are a protecting group and A8 is C₁₋₈ alkyl, C₁₋₈ alkenyl, C₁₋₈ alkynyl, C₁₋₄ alkyl-aryl (e.g., benzyl), aryl (e.g. phenyl) or C₁₋₄ alkyl-C₁₋₅ heterocycle. Substitutions are independently chosen. The organic moieties as described here, and for other any other moieties described herein, exclude obviously unstable moieties, e.g., -O-O-, except where such unstable moieties are transient species that one can use to make a compound with sufficient chemical stability for the one or more of the uses described herein.

Optionally substituted "monosaccharide" comprise any C3-C7 sugar, D-, L- or DL-configurations, e.g., erythrose, glycerol, ribose, deoxyribose, arabinose, glucose, mannose, galactose, fucose, mannose, glucosamine, N-acetylneuraminic acid, N-acetylglucosamine, N-acetylgalactosamine that is optionally substituted at one or more hydroxyl groups. Suitable substitutions are as described above for substituted alkyl moieties and include independently selected hydrogen, hydroxyl, protected hydroxyl, carboxyl, azido, cyano, -O-C₁₋₆ alkyl, -S-C₁₋₆ alkyl, -O-C₂₋₆ alkenyl, -S-C₂₋₆ alkenyl, optionally protected amine, optionally protected carboxyl, halogen, thiol or protected thiol. The linkage between the monosaccharide and the steroid is α or β.

Optionally substituted "oligosaccharide" comprises two, three, four or more of any C3-C7 sugars that are covalently linked to each other. The linked sugars may have D-, L- or DL-configurations. Suitable sugars and substitutions are as described for monosaccharides. The linkage between the oligosaccharide and the steroid is α or β, as are the linkages between the monosaccharides that comprise the oligosaccharide.

Nucleoside includes 3TC, AZT, D4T, ddl, ddC, G, A, U, C, T, dG, dA, dT and dC.

Polymer includes biocompatible organic polymers, e.g., PEGs and polyhydroxyalkyl polymers.

PEG means an ethylene glycol polymer that contains about 20 to about 2000000 linked monomers, typically about 50-1000 linked monomers, usually about 100-300. Polyethylene glycols include PEGs containing various numbers of linked monomers or having differing average molecular weights, e.g., PEG20, PEG30, PEG40, PEG60, PEG80, PEG100, PEG115, PEG200, PEG300, PEG400, PEG500, PEG600, PEG1000, PEG1500, PEG2000, PEG 3350, PEG4000, PEG4600, PEG5000, PEG6000, PEG8000, PEG11000, PEG12000, PEG2000000 and any mixtures thereof.

As used herein, position numbers that are given for the formula 1 compounds use the numbering convention for cholesterol.

Unless otherwise stated or implied by context, expressions of a percentage of a liquid ingredient, e.g., an excipient, in an invention composition or formulation mean the ingredient's percent by volume (v/v). Thus, 20% propylene glycol means 20% v/v propylene glycol is present in an invention composition or formulation. The amount of excipient indicated in invention compositions is not affected by the form used, e.g., NF or USP grade solvent or excipient. Thus, an invention composition that comprises about 30% polyethylene glycol 300 NF can instead comprise a USP counterpart, provided that other limitations, such as the amount of water present, are not exceeded.

As used herein, "innate immunity" refers to one or more components typically associated with nonspecific immune defense mechanisms in a subject. These components include the alternate complement pathway, e.g., Factor B, Factor D and properdin; NK cells, phagocytes (monocytes, macrophages), neutrophils, eosinophils, dendritic cells, fibrocytes; anti-microbial chemicals, e.g., one or more of defensins; physical barriers - skin, mucosal epithelium; or certain interleukins, chemokines, cytokines, lung or alveolar macrophage respiratory burst activity or a lung surfactant protein such as surfactant protein A or surfactant protein D. Innate immunity plays a role in resistance to intracellular parasite infections, e.g., white blood cell infection, a liver infection, and other infections, e.g., lymph node infections. Detectable enhancement of innate immunity mechanism by formula 1 compounds or method described herein can also enhance phagolysosome fusion or movement, which some pathogens, e.g., intracellular bacteria such as mycobacteria, or *Listeria* inhibit.

Terms such as "immune disregulation", "immune disregulation condition", "unwanted immune response" and the like mean that a subject has or is subject to developing an immune response that is not desirable or is suboptimal for the subject's condition. Such disregulation or unwanted responses can arise from various clinical conditions or diseases or as a result of treatment of such conditions or diseases, e.g., inflammation, autoimmunity, organ or tissue transplant rejection (e.g., allograft, xenograft), infections, cancers, chemotherapy treatments, trauma, allergy conditions or in conditions where a subject mounts a Th1, Tc1, Th2 or Tc2 immune response that is considered to be pathogenic, ineffective, insufficient or suboptimal. Immune disregulation conditions are as described herein or in the cited references.

Terms such as "cellular response", "cellular activity", biological response", "biological activity" and the like mean a response or activity that is detectably modulated in response to the presence of a formula 1 compound. Such responses or activities can be direct effects or indirect effects on one or more cellular activities or on the expression or level of one or more molecules that the affected cell(s) bind, sequester, synthesize or respond to. Such responses or activities include a detectable change in the synthesis or level of one or more cytokines, growth factors, transcription factors (including receptors and their cofactors), enzymes, Th1- or Th2-associated antibody subtype responses or the like. Typically, the cytokines, growth factors, transcription factors, enzymes or antibodies that are modulated are involved in the amelioration of a pathological condition or in the establishment, maintenance or progression of a pathological condition.

As used herein, references to CD molecules, specific immune cell subsets, immune responses and the like, generally use nomenclature that applies to molecules, cells or the like that are found in humans. Analogs or counterparts of such molecules, cells or the like in other species may have a differing nomenclature, but are included in this invention. A description of the nomenclature and function of various CD molecules and immune cell subsets are as found in the scientific literature. References to Th0, Th1 or Th2 cells and references to Th1 or Th2 immune responses in the context of human patients refers to the human counterparts of the murine Th0, Th1 or Th2 immune cells or responses. For reviews see, e.g., A.K. Abbas et al., editors, Cellular and Molecular Immunology, W.B. Saunders Company, third edition, 1997, ISBN 0-7216-4024-9, pages 4-469, and I. Kimber and M.K. Selgrade, editors, T Lymphocyte Subpopulations in Immunotoxicology, John Wiley & Sons Ltd., 1998, ISBN 0-471-97194-4, pages 1-53.

"Immunosuppressive molecule" means molecules such as cyclosporin, cyclohexamide, mitomycin C, adriamycin, taxol and amphotericin B. These molecules tend to have toxicities toward the immune system and are directly or indirectly immunosuppressive, e.g., they are toxic to dividing cells, they inhibit proliferation of immune cell precursors or they can downregulate an otherwise desired or improved immune response or condition.

"Steroid receptor" means a gene product, typically a protein monomer or dimer that can bind to a ligand, e.g., a natural steroid, a steroid analog, or another ligand such as a formula 1 compound or a metabolic precursor thereof or a metabolite thereof, a lipid, e.g., a prostaglandin, or the like. Steroid receptors include orphan steroid receptors. Orphan steroid receptors are proteins for which the natural ligand or biological function is at least partially unknown. As used here, steroid receptors include homodimers, e.g., SXR and (CARβ)₂, and heterodimers, e.g., PXR-CARβ or RXR-CARβ. Steroid receptors also include isoforms, e.g., PXR.1 and PXR.2 for the PXR receptor, and homologs of the steroid receptors, e.g., the homolog of CARβ known as MB67. Isoforms are typically generated by different splicing pathways for a nuclear RNA from one gene, while homologs are typically a distinct copy of a steroid receptor gene, where the gene copy encodes only relatively small differences compared to the reference steroid receptor gene product. Such differences are most often found in areas other than the dimerization region and the steroid binding region of the steroid receptor's structure. Typically isoforms and homologs bind the same or similar ligands as the reference gene product or steroid receptor. Steroid receptors may be of human or animal origin, e.g., obtained from cells, tissues or cDNA expression libraries derived from cells or tissues of any primate, rodent (including murine), avian, ovine, bovine, equine, canine or feline species or any of the species or any species within any group (e.g., Family or Genus) of species mentioned elsewhere herein or in any reference cited herein. Modulation of steroid receptors by formula 1 compounds can arise from (1) their direct interaction with a steroid receptor or a cofactor thereof or (2) indirect effects such as (A) detectably increased or decreased synthesis or level of the steroiud receptor or (B) generation of a signal or stimulus that leads to detectable modulation of one or more biological activities of the receptor, e.g., detectable inhibition of steroid receptor mediated gene transcription or detectable enhancement of steroid receptor mediated gene transcription.

An "agonist" or an "antagonist" is a compound or composition that respectively, either detectably increases or decreases the activity of a receptor, an enzyme or another biological molecule, which can lead to increased or decreased transcription of a regulated gene or to another measurable effect. The increase or decrease in a receptor's or enzyme's activity will be an increase or a decrease of at least about 5%, 10%, 20%, 30%, 40%, 50%, 60%, 70%, 80%, 90%, 95% or a range about between any two of these values, for one or more measurable activities. Receptors, their accessory factors and associated transcription factors can modulate transcription of their target gene(s) by detectably enhancing transcription or decreasing it. Biological activities of receptors may also include modulating biological responses such as signal transduction within a cell or ion flux, e.g., sodium, potassium or calcium, across cell or organelle membranes.

Terms such as "biologically active metabolite" and the like mean derivatives of the formula 1 compounds that retain a detectable level, e.g., at least about 10%, at least about 20%, at least about 30% or at least about 50%, of at least one desired activity of the parent compound, e.g., antiinflammatory activity or stimulation of a desired immune response. Determination of a desired activity is accomplished essentially as described herein. Such metabolites can be generated in the gastrointestinal tract, in blood or in one or more subject tissues. Such metabolites are detected using standard analytical methods, e.g., GC-MS analysis of an optionally radiolabeled formula 1 compound and its metabolites, in blood, urine or other biological samples after it is administered to a subject by one or more routes as disclosed herein. Terms such as "metabolic precursor" of formula 1 compounds and the like can include compounds that generate a detectable level of the formula 1 compound or a detectable level, e.g., at least about 10%, at least about 20%, at least about 30% or at least about 50%, of at least one desired activity of the formula 1 compound. Determination of a desired activity is accomplished essentially as described herein. Conversion of metabolic precursors can occur in the gastrointestinal tract, in blood or in one or more subject tissues.

"Amino acid" means an amino acid moiety that comprises any naturally-occurring or synthetic amino acid residue, i.e., any moiety comprising at least one carboxyl and at least one amino residue directly linked by one, two three or more carbon atoms, typically one (α) carbon atom. The nature and identity of the intervening structure located between the carboxyl and amino groups can have a variety of structures including those described herein. Typically, amino acids linked to the steroid through the amine group have sufficient conformation and length to be capable of autocatalytic hydrolysis of the amino acid-steroid bond and release of the steroid. This can occur when the free carboxyl is generated *in vivo* by deesterification, deamidation or peptidolytic cleavage of the precursor containing a linkage between the amino acid's amine group and the steroid. Hydrolysis of the bond between an amino acid's carboxyl or amino group and the steroid can also occur by chemical or enzymatic activity, e.g., esterase cleavage or non-enzymatic hydrolysis.

Peptide means one, 2, 3 or more of the two or more amino acids as defined above are bonded together, usually by an amide bond. Variable groups in the formula 1 compounds such as R¹-R¹⁰ can comprise a peptide. Typically the amino acids are linked through normal peptide bonds, e.g., -CO-NH-, between adjacent amino acid residues. Peptides comprise dipeptides (dimers), tripeptides (trimers), short peptides of 4, 5, 6, 8, 10 or 15 residues, and longer peptides or proteins having about 100 or more residues. Formula 1 compounds that comprise a peptide can be used as immunogens, prodrugs or as synthetic precursors for other steroid derivatives. In one embodiment, the peptide will contain a peptidolytic enzyme cleavage site at the peptide bond linking the first residue and the next residue distal to the steroid residue. Such cleavage sites are optionally flanked by enzymatic recognition structures, e.g. particular residues recognized by a hydrolytic enzyme, e.g., a peptidase located in the serum or in cells.

Peptidolytic enzymes are well known, and in particular include carboxypeptidases. Carboxypeptidases digest polypeptides by removing C-terminal residues, and are specific in many instances for particular C-terminal sequences. Such enzymes and their substrate requirements in general are well known. For example, a dipeptide having a given pair of residues and a free carboxyl terminus is covalently bonded through its α-amino group to the steroid nucleus. It is expected that the peptide will be cleaved by the appropriate dipeptidase, protease or by chemical hydrolysis, leaving the carboxyl of the proximal amino acid residue to autocatalytically cleave the amidate bond.

Salts of formula 1 compounds. Invention embodiments include salts and complexes of formula 1 compounds, including pharmaceutically acceptable or salts that are relatively non-toxic. Some of the formula 1 compounds have one or more moieties that carry at least a partial positive or negative charge in aqueous solutions, typically at a pH of about 4-10, that can participate in forming a salt, a complex, a composition with partial salt and partial complex properties or other noncovalent interactions, all of which we refer to as a "salt(s)". Salts are usually biologically compatible or pharmaceutically acceptable or non-toxic, particularly for mammalian cells. Salts that are biologically toxic are optionally used with synthetic intermediates of formula 1 compounds. When a water-soluble composition is desired, monovalent salts are usually used.

Metal salts typically are prepared by reacting the metal hydroxide with a compound of this invention. Examples of metal salts that are optionally prepared in this way are salts containing Li⁺, Na⁺, and K⁺. A less soluble metal salt can be precipitated from the solution of a more soluble salt by adding a suitable metal compound. Invention salts may be formed from acid addition of certain organic acids, such as organic carboxylic acids, and inorganic acids, such as alkylsulfonic acids or hydrogen halide acids, to acidic or basic centers on formula 1 compounds, such as basic centers on the invention pyrimidine base analogs. Metal salts include ones containing Na⁺, Li⁺, K⁺, Ca⁺⁺ or Mg⁺⁺. Other metal salts may contain aluminum, barium, strontium, cadmium, bismuth, arsenic or zinc ion.

Salt(s) of formula 1 compounds may comprise a combination of appropriate cations such as alkali and alkaline earth metal ions or ammonium and quaternary ammonium ions with the acid anion moiety of the phosphoric acid or phosphonic acid group, which may be present in invention polymers or monomers.

Salts are produced by standard methods, including dissolving free base in an aqueous, aqueous-alcohol or aqueous-organic solution containing the selected acid, optionally followed by evaporating the solution. The free base is reacted in an organic solution containing the acid, in which case the salt usually separates directly or one can concentrate the solution.

Suitable amine salts include amines having sufficient basicity to form a stable salt, usually amines of low toxicity including trialkyl amines (tripropylamine, triethylamine, trimethylamine), procaine, dibenzylamine, N-benzyl-betaphenethylamine, ephenamine, N,N'-dibenzylethylenediamine, N-ethylpiperidine, benzylamine and dicyclohexylamine.

Salts include organic sulfonic acid or organic carboxylic acid salts, made for example by addition of the acids to basic centers, typically amines. Exemplary sulfonic acids include C₆₋₁₆ aryl sulfonic acids, C₆₋₁₆ heteroaryl sulfonic acids and C₁₋₁₆ alkyl sulfonic acids such as phenyl sulfonic acid, α-naphthalene sulfonic acid, β-naphthalene sulfonic acid, (S)-camphorsulfonic acid, methyl (CH₃SO₃H), ethyl (C₂H₅SO₃H), *n*-propyl, *i*-propyl, *n*-butyl, *s*-butyl, *i*-butyl, *t*-butyl, pentyl and hexyl sulfonic acids. Exemplary organic carboxylic acids include C₁₋₁₆ alkyl, C₆₋₁₆ aryl carboxylic acids and C₄₋₁₆ heteroaryl carboxylic acids such as acetic, glycolic, lactic, pyruvic, malonic, glutaric, tartaric, citric, fumaric, succinic, malic, maleic, oxalic, hydroxymaleic, benzoic, hydroxybenzoic, phenylacetic, cinnamic, salicylic, nicotinic and 2-phenoxybenzoic.

Invention salts include those made from inorganic acids, e.g., HF, HCl, HBr, Hl, H₂SO₄, H₃PO₄, Na₂CO₃, K₂CO₃, CaCO₃, MgCO₃ and NaClO₃. Salts also include the formula 1 compound salts with one or more amino acids. Many amino acids are suitable, especially the naturally-occurring amino acids found as protein components, although the amino acid typically is one bearing a side chain with a basic or acidic group, e.g., lysine, arginine, histidine or glutamic acid, or a neutral group such as glycine, serine, threonine, alanine, isoleucine, or leucine.

The invention compositions include formula 1 compounds, their hydrates and the compounds in their un-ionized, as well as zwitterionic form.

Stereoisomers. The formula 1 compounds include enriched or resolved optical isomers at any or all asymmetric atoms as are apparent from the depictions. Both racemic and diasteromeric mixtures, as well as the individual optical isomers can be isolated or synthesized so as to be substantially free of their enantiomeric or diastereomeric partners, and these are all within the scope of the invention. Chiral centers may be found in formula 1 compounds at, for example, one or more of R¹, R², R³, R⁴ or R¹⁰.

One or more of the following methods are used to prepare the enantiomerically enriched or pure isomers herein. The methods are listed in approximately their order of preference, i.e., one ordinarily should employ stereospecific synthesis from chiral precursors before chromatographic resolution before spontaneous crystallization.

Stereospecific synthesis is described in the examples. Methods of this type conveniently are used when the appropriate chiral starting material is available and reaction steps are chosen do not result in undesired racemization at chiral sites. One advantage of stereospecific synthesis is that it does not produce undesired enantiomers that must be removed from the final product, thereby lowering overall synthetic yield. In general, those skilled in the art would understand what starting materials and reaction conditions should be used to obtain the desired enantiomerically enriched or pure isomers by stereospecific synthesis.

One method of general utility is chromatographic resolution of enantiomers on chiral chromatography resins. Not all of these columns are likely to be effective with every racemic mixture. However, those skilled in the art understand that a certain amount of routine screening may be required to identify the most effective stationary phase. When using such columns it is desirable to employ embodiments of the compounds of this invention in which the charges are not neutralized, e.g., where acidic functionalities such as carboxyl are not esterified or amidated.

Another method entails converting the enantiomers in the mixture to diasteriomers with chiral auxiliaries and then separating the conjugates by ordinary column chromatography. This is a very suitable method, particularly when the embodiment contains free carboxyl, amino or hydroxyl that will form a salt or covalent bond to a chiral auxiliary. Chirally pure amino acids, organic acids or organosulfonic acids are all worthwhile exploring as chiral auxiliaries, all of which are well known in the art. Salts with such auxiliaries can be formed, or they can be covalently (but reversibly) bonded to the functional group. For example, pure D or L amino acids can be used to amidate the carboxyl group of invention embodiments that comprise a carboxyl group and then separated by chromatography.

Enzymatic resolution is another method of potential value. In such methods one prepares covalent derivatives of the enantiomers in the racemic mixture, generally lower alkyl esters (for example of carboxyl), and then exposes the derivative to enzymatic cleavage, generally hydrolysis. For this method to be successful an enzyme must be chosen that is capable of stereospecific cleavage, so it is frequently necessary to routinely screen several enzymes. If esters are to be cleaved, then one selects a group of esterases, phosphatases, and lipases and determines their activity on the derivative. Typical esterases are from liver, pancreas or other animal organs, and include porcine liver esterase.

If the enatiomeric mixture separates from solution or a melt as a conglomerate, i.e., a mixture of enantiomerically pure crystals, then the crystals can be mechanically separated, thereby producing the enantiomerically enriched preparation. This method, however, is not practical for large-scale preparations and is of limited value for true racemic compounds.

Asymmetric synthesis is another technique for achieving enantiomeric enrichment. For example, a chiral protecting group is reacted with the group to be protected and the reaction mixture allowed to equilibrate. If the reaction is enantiomerically specific then the product will be enriched in that enantiomer.

Further guidance in the separation of enantiomeric mixtures can be found, by way of example and not limitation, in "Enantiomers, Racemates, and resolutions", Jean Jacques, Andre Collet, and Samuel H. Wilen (Krieger Publishing Company, Malabar, FL, 1991, ISBN 0-89464-618-4): Part 2, Resolution of Enantiomer Mixture, pages 217-435; more particularly, section 4, Resolution by Direct Crystallization, pages 217-251, section 5, Formation and Separation of Diastereomers, pages 251-369, section 6, Crystallization-Induced Asymmetric Transformations, pages 369-378, and section 7, Experimental Aspects and Art of Resolutions, pages 378-435; still more particularly, section 5.1.4, Resolution of Alcohols, Transformation of Alcohols into Salt-Forming Derivatives, pages 263-266, section 5.2.3, Covalent Derivatives of Alcohols, Thiols, and Phenols, pages 332-335, section 5.1.1, Resolution of Acids, pages 257-259, section 5.1.2, Resolution of Bases, pages 259-260, section 5.1.3, Resolution of Amino Acids, page 261-263, section 5.2.1, Covalent Derivatives of Acids, page 329, section 5.2.2, Covalent derivatives of Amines, pages 330-331, section 5.2.4, Covalent Derivatives of Aldehydes, Ketones, and Sulfoxides, pages 335-339, and section 5.2.7, Chromatographic Behavior of Covalent Diastereomers, pages 348-354.

Specific embodiments of formula 1 compounds. Other embodiments include compounds, compositions and formulations where one or more variable groups that are bonded to the formula 1 compounds, e.g., one or more of R¹-R⁶, R¹⁰, R¹⁵, R¹⁷ and R¹⁸ comprise an amino acid or a peptide, e.g., R¹, R² or R⁴ comprises an amino acid or a peptide, R³ is a halogen and R⁵ and R⁶ are both -CH₃.

In the formula 1 compounds, each R⁴ is independently selected. In some embodiments one R⁴ is hydrogen and the other is another moiety. In other embodiments, both R⁴ are independently selected moieties other than hydrogen, e.g., a C1 to C20 organic moiety.

R¹-R⁶, R¹⁰, R¹⁵, R¹⁷ and R¹⁸ include moieties, e.g., esters, thioesters, thionoesters, carbonates, amino acids, peptides and/or carbamates, that are chemically and/or enzymatically hydrolyzable, often under physiological conditions. Such moieties are independently chosen. Typically these moieties will give rise to -OH, -SH or -NH₂ at the R¹-R⁶ positions of the steroid nucleus. Embodiments of formula 1 compounds include ones where (1) one of R¹, R² and R⁴ is a hydrolyzable moiety (e.g., ester, thioester, thionoester, carbonate, amino acid, peptide or carbamate), the other two of R¹, R² and R⁴ are -H, R³ is not hydrogen and R⁵ and R⁶ are both -CH₃, (2) two of R¹, R² and R⁴ are hydrolyzable moieties (e.g., independently chosen esters, thioesters, thionoesters, carbonates, amino acids, peptides and/or carbamates), the other of R¹, R² and R⁴ is -H, R³ is not hydrogen and R⁵ and R⁶ are both -CH₃, (3) R¹, R² and R⁴ are hydrolyzable moieties, R³ is not hydrogen and R⁵ and R⁶ are both -CH₃. In these embodiments, the R³ group is typically in the β-configuration and the R¹, R² and R⁴-R⁶ groups are typically in the α-configuration.

In other embodiments, one or more of R¹-R⁶, R¹⁰, R¹⁵, R¹⁷ and R¹⁸, usually one, comprises an amino acid or a peptide, while the remaining groups are independently selected from the moieties defined herein. In these embodiments, the peptides are typically dimers (dipeptides) or trimers (tripeptides). For example one of R¹, R² or R⁴ comprises an amino acid, the remaining of R¹, R² or R⁴ independently comprise -OH, =O, an ester, a carbonate or a carbamate, while R³ is a halogen, hydroxyl or an ester and R⁵ and R⁶ independently are -H, -(CH₂)ₙ-CH₃, -(CH₂)ₙ-CH₂OH, or -(CH₂)ₙ-CH₂F, -(CH₂)₂₋₄-O-(CH₂)₂₋₄-CH₃, where n is 0, 1, 2, 3, 4, 5, 6, 7 or 8 often 0, 1, or 2, usually 0. Typically the ester, carbonate or carbamate are hydrolyzable under physiological conditions.

Embodiments of formula 1 compounds include ones wherein one, two, three, four or more of the variable groups that are bonded to the steroid rings, e.g., R¹-R⁶ or R¹⁰, comprise a moiety that can hydrolyze or metabolize to, e.g., a -H, -OH, =O, -SH, =S, - COOH, -NH₂, -CH₂OH, -CH₂SH, -C(O)-C1-C6 alkyl-OH, -C(O)-C1-C6 alkyl-SH, -C(S)-C1-C6 alkyl-OH, -C(O)-C1-C6 alkyl or-C(O)-NH₂ atom or group.

Invention embodiments include a composition comprising (1) a compound of formula 1 or 2 and one or more nonaqueous liquid excipients, wherein the composition comprises less than about 3% v/v water and (2) a compound of formula 1 or 2 and one or more solid excipients.

Invention embodiments include one or more compounds of formula 1 or formula 2 including formula 1 compounds having the structure

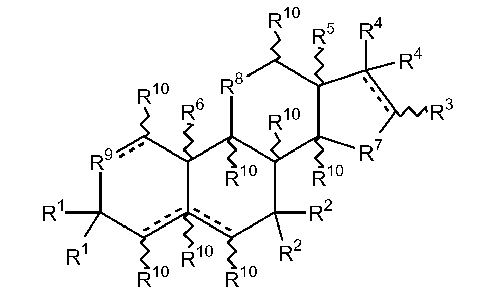

wherein,

R¹, R², R³, R⁴, R⁵, R⁶ and R¹⁰ independently are -H, -OR^{PR}, -SR^{PR}, -N(R^{PR})₂, -O-Si-(R¹³)₃, -CHO, -CHS, -SCN, -CH=NH, -CN, -NO₂, -OSO₃H, -OPO₃H, an ester, a thioester, a thionoester, a phosphoester, a phosphothioester, a phosphonoester, a phosphiniester, a sulfite ester, a sulfate ester, an amide, an amino acid, a peptide, an ether, a thioether, an acyl group, a thioacyl group, a carbonate, a carbamate, a thioacetal, a halogen, an optionally substituted alkyl group, an optionally substituted alkenyl group, an optionally substituted alkynyl group, an optionally substituted aryl moiety, an optionally substituted heteroaryl moiety, an optionally substituted monosaccharide, an optionally substituted oligosaccharide, a nucleoside, a nucleotide, an oligonucleotide, a polymer, or,

one, two or more of R¹, R², R³, R⁴, R⁵, R⁶ and R¹⁰ independently are =O, =S, =N-OH, =CH₂ or a spiro ring and the hydrogen atom that is bonded to the same carbon atom is absent;

R⁷ is -C(R¹⁰)₂-, -C(R¹⁰)₂-C(R¹⁰)₂-, -C(R¹⁰)₂-C(R¹⁰)₂-C(R¹⁰)₂-, -C(R¹⁰)₂-O-C(R¹⁰)₂-, - C(R¹⁰)₂-S-C(R¹⁰)₂-, -C(R¹⁰)₂-NR^{PR}-C(R¹⁰)₂-, -O-, -O-C(R¹⁰)₂-, -S-, -S-C(R¹⁰)₂-, -NR^{PR}-, -NR^{PR}-C(R¹⁰)₂-, -CHR¹⁰-, -CHR¹⁰-CHR¹⁰-, -CHR¹⁰-CHR¹⁰-CHR¹⁰-, -CHR¹⁰-O-CHR¹⁰-, -CHR¹⁰-S-CHR¹⁰-, -CHR¹⁰-NR^{PR}-CHR¹⁰-, -O-, -O-CHR¹⁰-, -S-, -S-CHR¹⁰-, -NR^{PR}- or -NR^{PR}-CHR¹⁰-;

R⁸ and R⁹ independently are -C(R¹⁰)₂-, -C(R¹⁰)₂-C(R¹⁰)₂-, -O-, -O-C(R¹⁰)₂-, -S-, -S-C(R¹⁰)₂-, -NR^{PR}-, -NR^{PR}-C(R¹⁰)₂-, -CHR¹⁰-, -CHR¹⁰-CHR¹⁰-, -O-, -O-CHR¹⁰-, -S-, -S-CHR¹⁰-, - NR^{PR}- or -NR^{PR}-CHR¹⁰-, or one or both of R⁸ or R⁹ independently are absent, leaving a 5-membered ring;

R⁸ and R⁹ independently are -CHR¹⁰-, -CHR¹⁰-CHR¹⁰-, -O-, -O-CHR¹⁰-, -S-, -S-CHR¹⁰-, -NR^{PR}- or-NR^{PR}-CHR¹⁰-, or R⁸ or R⁹ independently is absent, leaving a 5-membered ring;

R¹³ independently is C₁₋₆ alkyl;

R^{PR} independently is -H or a protecting group for, e.g., an O, N or S atom;

Embodiments of formula 1 compounds include or exclude any subset of compounds within the definition of formula 1, provided that at least one compound remains. For example, a subset of formula 1 compounds that are may be included, for example in the invention nonaqueous formulations and in the invention intermittent dosing protocols and immune modulation methods, are formula 1 compounds where R² is hydroxyl, or a group that can hydrolyze or metabolize to hydroxyl or thiol, in either configuration and R⁵ and R⁶ are methyl in the α-configuration. A subset compounds that are optionally excluded from formula 1 compounds comprises one or all compounds that are disclosed in one or more prior art references or publications, e.g., one or more compounds that are disclosed in one or more of the references cited herein, especially for those compounds that can render any claim or embodiment unpatentable for novelty, obviousness and/or inventive step reasons.

Exemplary embodiments of species and genera of formula 1 compounds are named as described below.

Group 1. Exemplary embodiments include the formula 1 compounds named according to the compound structure designations given in Tables A and B below. Each compound named in Table B is depicted as a compound having formula B

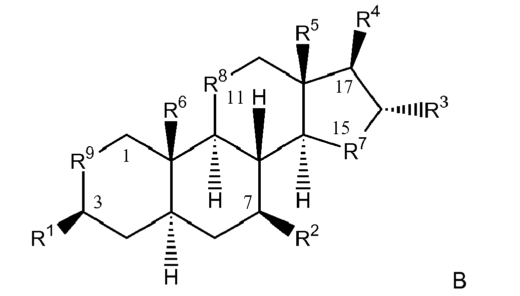

where R⁵ and R⁶ are both -CH₃, there is no double bond at the 1-2-, 4-5- or 5-6-positions, one R⁴ is hydrogen, R⁷, R⁸ and R⁹ are all -CH₂- and R¹, R², R³ and R⁴ are the substituents designated in Table A. The compounds named according to Tables A and B are referred to as "group 1" compounds.

Compounds named in Table B are named by numbers assigned to R¹, R², R³ and R⁴ according to the following compound naming convention, R¹.R².R³.R⁴, based on the numbered chemical substituents depicted in Table A. Each Table A number specifies a different structure for each of R¹, R², R³ and R⁴. When R¹, R², R³ or R⁴ is a divalent moiety, e.g., =O, the hydrogen at the corresponding position is absent. Thus, the group 1 compound named 1.2.1.1 is a formula B structure with a β-hydroxyl bonded to carbons at the 3- and 7-positions (the variable groups R¹ and R² respectively), an α-bromine bonded to carbon 16 (the variable group R³) and double bonded oxygen (=O) at carbon 17 (the variable group R⁴), i.e., 1.2.1.1 has the structure shown below.

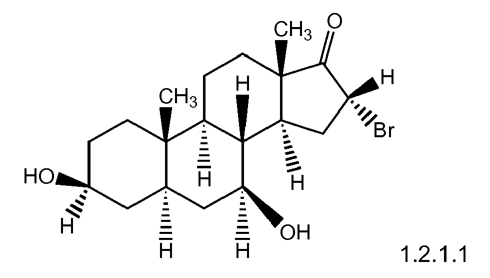

**TABLE A**

| | |
|---|---|
| R¹ | R² |
| 1 -OH | 1 -H |
| 2 =O | 2 -OH |
| 3 -SH | 3 =O |
| 4 =S | 4 -CH₃ |
| 5 -O-CH₃ | 5 -OCH₃ |
| 6 -O-S(O)(O)-O⁻Na⁺ | 6 -OC₂H₅ |
| 7 -O-S(O)(O)-OC₂H₅ | 7 -OCH₂CH₂CH₃ |
| 8 -CH₃ | 8 -OCH₂CH₂CH₂CH₃ |
| 9 -H | 9 -Cl |
| 10 -OC(O)C(CH₃)₃ | 10 -Br |
| | |
| R³ | R⁴ |
| 1 -Br | 1 =O |
| 2 -Cl | 2 -OH |
| 3 -I | 3 -H |
| 4 -F | 4 -F |
| 5 -H | 5 -Cl |
| 6 -OH | 6 -Br |
| 7 =O | 7 -I |
| 8 -O-C(O)-CH₃ | 8 -O-C(O)-CH₃ |
| 9 -O-C(O)-CH₂CH₃ | 9 -O-C(O)-CH₂CH₃ |
| 10 -O-C(O)-CH₂CH₂CH₃ | 10 -O-C(O)-CH₂CH₂CH₃ |

**TABLE B**

| |
|---|
| 1.1.1.1, 1.1.1.2, 1.1.1.3, 1.1.1.4, 1.1.1.5, 1.1.1.6, 1.1.1.7, 1.1.1.8, 1.1.1.9, 1.1.1.10, 1.1.2.1, 1.1.2.2, 1.1.2.3, |
| 1.1.2.4, 1.1.2.5, 1.1.2.6, 1.1.2.7, 1.1.2.8, 1.1.2.9, 1.1.2.10, 1.1.3.1, 1.1.3.2, 1.1.3.3, 1.1.3.4, 1.1.3.5, 1.1.3.6, |
| 1.1.3.7, 1.1.3.8, 1.1.3.9, 1.1.3.10, 1.1.4.1, 1.1.4.2, 1.1.4.3, 1.1.4.4, 1.1.4.5, 1.1.4.6, 1.1.4.7, 1.1.4.8, 1.1.4.9, |
| 1.1.4.10, 1.1.5.1, 1.1.5.2, 1.1.5.3, 1.1.5.4, 1.1.5.5, 1.1.5.6, 1.1.5.7, 1.1.5.8, 1.1.5.9, 1.1.5.10, 1.1.6.1, 1.1.6.2, |
| 1.1.6.3, 1.1.6.4, 1.1.6.5, 1.1.6.6, 1.1.6.7, 1.1.6.8, 1.1.6.9, 1.1.6.10, 1.1.7.1, 1.1.7.2, 1.1.7.3, 1.1.7.4, 1.1.7.5, |
| 1.1.7.6, 1.1.7.7, 1.1.7.8, 1.1.7.9, 1.1.7.10, 1.1.8.1, 1.1.8.2, 1.1.8.3, 1.1.8.4, 1.1.8.5, 1.1.8.6, 1.1.8.7, 1.1.8.8, |
| 1.1.8.9, 1.1.8.10, 1.1.9.1, 1.1.9.2, 1.1.9.3, 1.1.9.4, 1.1.9.5, 1.1.9.6, 1.1.9.7, 1.1.9.8, 1.1.9.9, 1.1.9.10, 1.1.10.1, |
| 1.1.10.2, 1.1.10.3, 1.1.10.4, 1.1.10.5, 1.1.10.6, 1.1.10.7, 1.1.10.8, 1.1.10.9, 1.1.10.10, 1.2.1.1, 1.2.1.2, 1.2.1.3, |
| 1.2.1.4, 1.2.1.5, 1.2.1.6, 1.2.1.7, 1.2.1.8, 1.2.1.9, 1.2.1.10, 1.2.2.1, 1.2.2.2, 1.2.2.3, 1.2.2.4, 1.2.2.5, 1.2.2.6, |
| 1.2.2.7, 1.2.2.8, 1.2.2.9, 1.2.2.10, 1.2.3.1, 1.2.3.2, 1.2.3.3, 1.2.3.4, 1.2.3.5, 1.2.3.6, 1.2.3.7, 1.2.3.8, 1.2.3.9, |
| 1.2.3.10, 1.2.4.1, 1.2.4.2, 1.2.4.3, 1.2.4.4, 1.2.4.5, 1.2.4.6, 1.2.4.7, 1.2.4.8, 1.2.4.9, 1.2.4.10, 1.2.5.1, 1.2.5.2, |
| 1.2.5.3, 1.2.5.4, 1.2.5.5, 1.2.5.6, 1.2.5.7, 1.2.5.8, 1.2.5.9, 1.2.5.10, 1.2.6.1, 1.2.6.2, 1.2.6.3, 1.2.6.4, 1.2.6.5, |
| 1.2.6.6, 1.2.6.7, 1.2.6.8, 1.2.6.9, 1.2.6.10, 1.2.7.1, 1.2.7.2, 1.2.7.3, 1.2.7.4, 1.2.7.5, 1.2.7.6, 1.2.7.7, 1.2.7.8, |
| 1.2.7.9, 1.2.7.10, 1.2.8.1, 1.2.8.2, 1.2.8.3, 1.2.8.4, 1.2.8.5, 1.2.8.6, 1.2.8.7, 1.2.8.8, 1.2.8.9, 1.2.8.10, 1.2.9.1, |
| 1.2.9.2, 1.2.9.3, 1.2.9.4, 1.2.9.5, 1.2.9.6, 1.2.9.7, 1.2.9.8, 1.2.9.9, 1.2.9.10, 1.2.10.1, 1.2.10.2, 1.2.10.3, 1.2.10.4, |
| 1.2.10.5, 1.2.10.6, 1.2.10.7, 1.2.10.8, 1.2.10.9, 1.2.10.10, 1.3.1.1, 1.3.1.2, 1.3.1.3, 1.3.1.4, 1.3.1.5, 1.3.1.6, |
| 1.3.1.7, 1.3.1.8, 1.3.1.9, 1.3.1.10, 1.3.2.1, 1.3.2.2, 1.3.2.3, 1.3.2.4, 1.3.2.5, 1.3.2.6, 1.3.2.7, 1.3.2.8, 1.3.2.9, |
| 1.3.2.10, 1.3.3.1, 1.3.3.2, 1.3.3.3, 1.3.3.4, 1.3.3.5, 1.3.3.6, 1.3.3.7, 1.3.3.8, 1.3.3.9, 1.3.3.10, 1.3.4.1, 1.3.4.2, |
| 1.3.4.3, 1.3.4.4, 1.3.4.5, 1.3.4.6, 1.3.4.7, 1.3.4.8, 1.3.4.9, 1.3.4.10, 1.3.5.1, 1.3.5.2, 1.3.5.3, 1.3.5.4, 1.3.5.5, |
| 1.3.5.6, 1.3.5.7, 1.3.5.8, 1.3.5.9, 1.3.5.10, 1.3.6.1, 1.3.6.2, 1.3.6.3, 1.3.6.4, 1.3.6.5, 1.3.6.6, 1.3.6.7, 1.3.6.8, |
| 1.3.6.9, 1.3.6.10, 1.3.7.1, 1.3.7.2, 1.3.7.3, 1.3.7.4, 1.3.7.5, 1.3.7.6, 1.3.7.7, 1.3.7.8, 1.3.7.9, 1.3.7.10, 1.3.8.1, |
| 1.3.8.2, 1.3.8.3, 1.3.8.4, 1.3.8.5, 1.3.8.6, 1.3.8.7, 1.3.8.8, 1.3.8.9, 1.3.8.10, 1.3.9.1, 1.3.9.2, 1.3.9.3, 1.3.9.4, |
| 1.3.9.5, 1.3.9.6, 1.3.9.7, 1.3.9.8, 1.3.9.9, 1.3.9.10, 1.3.10.1, 1.3.10.2, 1.3.10.3, 1.3.10.4, 1.3.10.5, 1.3.10.6, |
| 1.3.10.7, 1.3.10.8, 1.3.10.9, 1.3.10.10, 1.4.1.1, 1.4.1.2, 1.4.1.3, 1.4.1.4, 1.4.1.5, 1.4.1.6, 1.4.1.7, 1.4.1.8, 1.4.1.9, |
| 1.4.1.10, 1.4.2.1, 1.4.2.2, 1.4.2.3, 1.4.2.4, 1.4.2.5, 1.4.2.6, 1.4.2.7, 1.4.2.8, 1.4.2.9, 1.4.2.10, 1.4.3.1, 1.4.3.2, |
| 1.4.3.3, 1.4.3.4, 1.4.3.5, 1.4.3.6, 1.4.3.7, 1.4.3.8, 1.4.3.9, 1.4.3.10, 1.4.4.1, 1.4.4.2, 1.4.4.3, 1.4.4.4, 1.4.4.5, |
| 1.4.4.6, 1.4.4.7, 1.4.4.8, 1.4.4.9, 1.4.4.10, 1.4.5.1, 1.4.5.2, 1.4.5.3, 1.4.5.4, 1.4.5.5, 1.4.5.6, 1.4.5.7, 1.4.5.8, |
| 1.4.5.9, 1.4.5.10, 1.4.6.1, 1.4.6.2, 1.4.6.3, 1.4.6.4, 1.4.6.5, 1.4.6.6, 1.4.6.7, 1.4.6.8, 1.4.6.9, 1.4.6.10, 1.4.7.1, |
| 1.4.7.2, 1.4.7.3, 1.4.7.4, 1.4.7.5, 1.4.7.6, 1.4.7.7, 1.4.7.8, 1.4.7.9, 1.4.7.10, 1.4.8.1, 1.4.8.2, 1.4.8.3, 1.4.8.4, |
| 1.4.8.5, 1.4.8.6, 1.4.8.7, 1.4.8.8, 1.4.8.9, 1.4.8.10, 1.4.9.1, 1.4.9.2, 1.4.9.3, 1.4.9.4, 1.4.9.5, 1.4.9.6, 1.4.9.7, |
| 1.4.9.8, 1.4.9.9, 1.4.9.10, 1.4.10.1, 1.4.10.2, 1.4.10.3, 1.4.10.4, 1.4.10.5, 1.4.10.6, 1.4.10.7, 1.4.10.8, 1.4.10.9, |
| 1.4.10.10, 1.5.1.1, 1.5.1.2, 1.5.1.3, 1.5.1.4, 1.5.1.5, 1.5.1.6, 1.5.1.7, 1.5.1.8, 1.5.1.9, 1.5.1.10, 1.5.2.1, 1.5.2.2, |
| 1.5.2.3, 1.5.2.4, 1.5.2.5, 1.5.2.6, 1.5.2.7, 1.5.2.8, 1.5.2.9, 1.5.2.10, 1.5.3.1, 1.5.3.2, 1.5.3.3, 1.5.3.4, 1.5.3.5, |
| 1.5.3.6, 1.5.3.7, 1.5.3.8, 1.5.3.9, 1.5.3.10, 1.5.4.1, 1.5.4.2, 1.5.4.3, 1.5.4.4, 1.5.4.5, 1.5.4.6, 1.5.4.7, 1.5.4.8, |
| 1.5.4.9, 1.5.4.10, 1.5.5.1, 1.5.5.2, 1.5.5.3, 1.5.5.4, 1.5.5.5, 1.5.5.6, 1.5.5.7, 1.5.5.8, 1.5.5.9, 1.5.5.10, 1.5.6.1, |
| 1.5.6.2, 1.5.6.3, 1.5.6.4, 1.5.6.5, 1.5.6.6, 1.5.6.7, 1.5.6.8, 1.5.6.9, 1.5.6.10, 1.5.7.1, 1.5.7.2, 1.5.7.3, 1.5.7.4, |
| 1.5.7.5, 1.5.7.6, 1.5.7.7, 1.5.7.8, 1.5.7.9, 1.5.7.10, 1.5.8.1, 1.5.8.2, 1.5.8.3, 1.5.8.4, 1.5.8.5, 1.5.8.6, 1.5.8.7, |
| 1.5.8.8, 1.5.8.9, 1.5.8.10, 1.5.9.1, 1.5.9.2, 1.5.9.3, 1.5.9.4, 1.5.9.5, 1.5.9.6, 1.5.9.7, 1.5.9.8, 1.5.9.9, 1.5.9.10, |
| 1.5.10.1, 1.5.10.2, 1.5.10.3, 1.5.10.4, 1.5.10.5, 1.5.10.6, 1.5.10.7, 1.5.10.8, 1.5.10.9, 1.5.10.10, 1.6.1.1, 1.6.1.2, |
| 1.6.1.3, 1.6.1.4, 1.6.1.5, 1.6.1.6, 1.6.1.7, 1.6.1.8, 1.6.1.9, 1.6.1.10, 1.6.2.1, 1.6.2.2, 1.6.2.3, 1.6.2.4, 1.6.2.5, |
| 1.6.2.6, 1.6.2.7, 1.6.2.8, 1.6.2.9, 1.6.2.10, 1.6.3.1, 1.6.3.2, 1.6.3.3, 1.6.3.4, 1.6.3.5, 1.6.3.6, 1.6.3.7, 1.6.3.8, |
| 1.6.3.9, 1.6.3.10, 1.6.4.1, 1.6.4.2, 1.6.4.3, 1.6.4.4, 1.6.4.5, 1.6.4.6, 1.6.4.7, 1.6.4.8, 1.6.4.9, 1.6.4.10, 1.6.5.1, |
| 1.6.5.2, 1.6.5.3, 1.6.5.4, 1.6.5.5, 1.6.5.6, 1.6.5.7, 1.6.5.8, 1.6.5.9, 1.6.5.10, 1.6.6.1, 1.6.6.2, 1.6.6.3, 1.6.6.4, |
| 1.6.6.5, 1.6.6.6, 1.6.6.7, 1.6.6.8, 1.6.6.9, 1.6.6.10, 1.6.7.1, 1.6.7.2, 1.6.7.3, 1.6.7.4, 1.6.7.5, 1.6.7.6, 1.6.7.7, |
| 1.6.7.8, 1.6.7.9, 1.6.7.10, 1.6.8.1, 1.6.8.2, 1.6.8.3, 1.6.8.4, 1.6.8.5, 1.6.8.6, 1.6.8.7, 1.6.8.8, 1.6.8.9, 1.6.8.10, |
| 1.6.9.1, 1.6.9.2, 1.6.9.3, 1.6.9.4, 1.6.9.5, 1.6.9.6, 1.6.9.7, 1.6.9.8, 1.6.9.9, 1.6.9.10, 1.6.10.1, 1.6.10.2, 1.6.10.3, |
| 1.6.10.4, 1.6.10.5, 1.6.10.6, 1.6.10.7, 1.6.10.8, 1.6.10.9, 1.6.10.10, 1.7.1.1, 1.7.1.2, 1.7.1.3, 1.7.1.4, 1.7.1.5, |
| 1.7.1.6, 1.7.1.7, 1.7.1.8, 1.7.1.9, 1.7.1.10, 1.7.2.1, 1.7.2.2, 1.7.2.3, 1.7.2.4, 1.7.2.5, 1.7.2.6, 1.7.2.7, 1.7.2.8, |
| 1.7.2.9, 1.7.2.10, 1.7.3.1, 1.7.3.2, 1.7.3.3, 1.7.3.4, 1.7.3.5, 1.7.3.6, 1.7.3.7, 1.7.3.8, 1.7.3.9, 1.7.3.10, 1.7.4.1, |
| 1.7.4.2, 1.7.4.3, 1.7.4.4, 1.7.4.5, 1.7.4.6, 1.7.4.7, 1.7.4.8, 1.7.4.9, 1.7.4.10, 1.7.5.1, 1.7.5.2, 1.7.5.3, 1.7.5.4, |
| 1.7.5.5, 1.7.5.6, 1.7.5.7, 1.7.5.8, 1.7.5.9, 1.7.5.10, 1.7.6.1, 1.7.6.2, 1.7.6.3, 1.7.6.4, 1.7.6.5, 1.7.6.6, 1.7.6.7, |
| 1.7.6.8, 1.7.6.9, 1.7.6.10, 1.7.7.1, 1.7.7.2, 1.7.7.3, 1.7.7.4, 1.7.7.5, 1.7.7.6, 1.7.7.7, 1.7.7.8, 1.7.7.9, 1.7.7.10, |
| 1.7.8.1, 1.7.8.2, 1.7.8.3, 1.7.8.4, 1.7.8.5, 1.7.8.6, 1.7.8.7, 1.7.8.8, 1.7.8.9, 1.7.8.10, 1.7.9.1, 1.7.9.2, 1.7.9.3, |
| 1.7.9.4, 1.7.9.5, 1.7.9.6, 1.7.9.7, 1.7.9.8, 1.7.9.9, 1.7.9.10, 1.7.10.1, 1.7.10.2, 1.7.10.3, 1.7.10.4, 1.7.10.5, |
| 1.7.10.6, 1.7.10.7, 1.7.10.8, 1.7.10.9, 1.7.10.10, 1.8.1.1, 1.8.1.2, 1.8.1.3, 1.8.1.4, 1.8.1.5, 1.8.1.6, 1.8.1.7, 1.8.1.8, |
| 1.8.1.9, 1.8.1.10, 1.8.2.1, 1.8.2.2, 1.8.2.3, 1.8.2.4, 1.8.2.5, 1.8.2.6, 1.8.2.7, 1.8.2.8, 1.8.2.9, 1.8.2.10, 1.8.3.1, |
| 1.8.3.2, 1.8.3.3, 1.8.3.4, 1.8.3.5, 1.8.3.6, 1.8.3.7, 1.8.3.8, 1.8.3.9, 1.8.3.10, 1.8.4.1, 1.8.4.2, 1.8.4.3, 1.8.4.4, |
| 1.8.4.5, 1.8.4.6, 1.8.4.7, 1.8.4.8, 1.8.4.9, 1.8.4.10, 1.8.5.1, 1.8.5.2, 1.8.5.3, 1.8.5.4, 1.8.5.5, 1.8.5.6, 1.8.5.7, |
| 1.8.5.8, 1.8.5.9, 1.8.5.10, 1.8.6.1, 1.8.6.2, 1.8.6.3, 1.8.6.4, 1.8.6.5, 1.8.6.6, 1.8.6.7, 1.8.6.8, 1.8.6.9, 1.8.6.10, |
| 1.8.7.1, 1.8.7.2, 1.8.7.3, 1.8.7.4, 1.8.7.5, 1.8.7.6, 1.8.7.7, 1.8.7.8, 1.8.7.9, 1.8.7.10, 1.8.8.1, 1.8.8.2, 1.8.8.3, |
| 1.8.8.4, 1.8.8.5, 1.8.8.6, 1.8.8.7, 1.8.8.8, 1.8.8.9, 1.8.8.10, 1.8.9.1, 1.8.9.2, 1.8.9.3, 1.8.9.4, 1.8.9.5, 1.8.9.6, |
| 1.8.9.7, 1.8.9.8, 1.8.9.9, 1.8.9.10, 1.8.10.1, 1.8.10.2, 1.8.10.3, 1.8.10.4, 1.8.10.5, 1.8.10.6, 1.8.10.7, 1.8.10.8, |
| 1.8.10.9, 1.8.10.10, 1.9.1.1, 1.9.1.2, 1.9.1.3, 1.9.1.4, 1.9.1.5, 1.9.1.6, 1.9.1.7, 1.9.1.8, 1.9.1.9, 1.9.1.10, 1.9.2.1, |
| 1.9.2.2, 1.9.2.3, 1.9.2.4, 1.9.2.5, 1.9.2.6, 1.9.2.7, 1.9.2.8, 1.9.2.9, 1.9.2.10, 1.9.3.1, 1.9.3.2, 1.9.3.3, 1.9.3.4, |
| 1.9.3.5, 1.9.3.6, 1.9.3.7, 1.9.3.8, 1.9.3.9, 1.9.3.10, 1.9.4.1, 1.9.4.2, 1.9.4.3, 1.9.4.4, 1.9.4.5, 1.9.4.6, 1.9.4.7, |
| 1.9.4.8, 1.9.4.9, 1.9.4.10, 1.9.5.1, 1.9.5.2, 1.9.5.3, 1.9.5.4, 1.9.5.5, 1.9.5.6, 1.9.5.7, 1.9.5.8, 1.9.5.9, 1.9.5.10, |
| 1.9.6.1, 1.9.6.2, 1.9.6.3, 1.9.6.4, 1.9.6.5, 1.9.6.6, 1.9.6.7, 1.9.6.8, 1.9.6.9, 1.9.6.10, 1.9.7.1, 1.9.7.2, 1.9.7.3, |
| 1.9.7.4, 1.9.7.5, 1.9.7.6, 1.9.7.7, 1.9.7.8, 1.9.7.9, 1.9.7.10, 1.9.8.1, 1.9.8.2, 1.9.8.3, 1.9.8.4, 1.9.8.5, 1.9.8.6, |
| 1.9.8.7, 1.9.8.8, 1.9.8.9, 1.9.8.10, 1.9.9.1, 1.9.9.2, 1.9.9.3, 1.9.9.4, 1.9.9.5, 1.9.9.6, 1.9.9.7, 1.9.9.8, 1.9.9.9, |
| 1.9.9.10, 1.9.10.1, 1.9.10.2, 1.9.10.3, 1.9.10.4, 1.9.10.5, 1.9.10.6, 1.9.10.7, 1.9.10.8, 1.9.10.9, 1.9.10.10, |
| 1.10.1.1, 1.10.1.2, 1.10.1.3, 1.10.1.4, 1.10.1.5, 1.10.1.6, 1.10.1.7, 1.10.1.8, 1.10.1.9, 1.10.1.10, 1.10.2.1, |
| 1.10.2.2, 1.10.2.3, 1.10.2.4, 1.10.2.5, 1.10.2.6, 1.10.2.7, 1.10.2.8, 1.10.2.9, 1.10.2.10, 1.10.3.1, 1.10.3.2, |
| 1.10.3.3, 1.10.3.4, 1.10.3.5, 1.10.3.6, 1.10.3.7, 1.10.3.8, 1.10.3.9, 1.10.3.10, 1.10.4.1, 1.10.4.2, 1.10.4.3, |
| 1.10.4.4, 1.10.4.5, 1.10.4.6, 1.10.4.7, 1.10.4.8, 1.10.4.9, 1.10.4.10, 1.10.5.1, 1.10.5.2, 1.10.5.3, 1.10.5.4, |
| 1.10.5.5, 1.10.5.6, 1.10.5.7, 1.10.5.8, 1.10.5.9, 1.10.5.10, 1.10.6.1, 1.10.6.2, 1.10.6.3, 1.10.6.4, 1.10.6.5, |
| 1.10.6.6, 1.10.6.7, 1.10.6.8, 1.10.6.9, 1.10.6.10, 1.10.7.1, 1.10.7.2, 1.10.7.3, 1.10.7.4, 1.10.7.5, 1.10.7.6, |
| 1.10.7.7, 1.10.7.8, 1.10.7.9, 1.10.7.10, 1.10.8.1, 1.10.8.2, 1.10.8.3, 1.10.8.4, 1.10.8.5, 1.10.8.6, 1.10.8.7, |
| 1.10.8.8, 1.10.8.9, 1.10.8.10, 1.10.9.1, 1.10.9.2, 1.10.9.3, 1.10.9.4, 1.10.9.5, 1.10.9.6, 1.10.9.7, 1.10.9.8, |
| 1.10.9.9, 1.10.9.10, 1.10.10.1, 1.10.10.2, 1.10.10.3, 1.10.10.4, 1.10.10.5, 1.10.10.6, 1.10.10.7, 1.10.10.8, |
| 1.10.10.9, 1.10.10.10, 2.1.1.1, 2.1.1.2, 2.1.1.3, 2.1.1.4, 2.1.1.5, 2.1.1.6, 2.1.1.7, 2.1.1.8, 2.1.1.9, 2.1.1.10, 2.1.2.1, |
| 2.1.2.2, 2.1.2.3, 2.1.2.4, 2.1.2.5, 2.1.2.6, 2.1.2.7, 2.1.2.8, 2.1.2.9, 2.1.2.10, 2.1.3.1, 2.1.3.2, 2.1.3.3, 2.1.3.4, |
| 2.1.3.5, 2.1.3.6, 2.1.3.7, 2.1.3.8, 2.1.3.9, 2.1.3.10, 2.1.4.1, 2.1.4.2, 2.1.4.3, 2.1.4.4, 2.1.4.5, 2.1.4.6, 2.1.4.7, |
| 2.1.4.8, 2.1.4.9, 2.1.4.10, 2.1.5.1, 2.1.5.2, 2.1.5.3, 2.1.5.4, 2.1.5.5, 2.1.5.6, 2.1.5.7, 2.1.5.8, 2.1.5.9, 2.1.5.10, |
| 2.1.6.1, 2.1.6.2, 2.1.6.3, 2.1.6.4, 2.1.6.5, 2.1.6.6, 2.1.6.7, 2.1.6.8, 2.1.6.9, 2.1.6.10, 2.1.7.1, 2.1.7.2, 2.1.7.3, |
| 2.1.7.4, 2.1.7.5, 2.1.7.6, 2.1.7.7, 2.1.7.8, 2.1.7.9, 2.1.7.10, 2.1.8.1, 2.1.8.2, 2.1.8.3, 2.1.8.4, 2.1.8.5, 2.1.8.6, |
| 2.1.8.7, 2.1.8.8, 2.1.8.9, 2.1.8.10, 2.1.9.1, 2.1.9.2, 2.1.9.3, 2.1.9.4, 2.1.9.5, 2.1.9.6, 2.1.9.7, 2.1.9.8, 2.1.9.9, |
| 2.1.9.10, 2.1.10.1, 2.1.10.2, 2.1.10.3, 2.1.10.4, 2.1.10.5, 2.1.10.6, 2.1.10.7, 2.1.10.8, 2.1.10.9, 2.1.10.10, 2.2.1.1, |
| 2.2.1.2, 2.2.1.3, 2.2.1.4, 2.2.1.5, 2.2.1.6, 2.2.1.7, 2.2.1.8, 2.2.1.9, 2.2.1.10, 2.2.2.1, 2.2.2.2, 2.2.2.3, 2.2.2.4, |
| 2.2.2.5, 2.2.2.6, 2.2.2.7, 2.2.2.8, 2.2.2.9, 2.2.2.10, 2.2.3.1, 2.2.3.2, 2.2.3.3, 2.2.3.4, 2.2.3.5, 2.2.3.6, 2.2.3.7, |
| 2.2.3.8, 2.2.3.9, 2.2.3.10, 2.2.4.1, 2.2.4.2, 2.2.4.3, 2.2.4.4, 2.2.4.5, 2.2.4.6, 2.2.4.7, 2.2.4.8, 2.2.4.9, 2.2.4.10, |
| 2.2.5.1, 2.2.5.2, 2.2.5.3, 2.2.5.4, 2.2.5.5, 2.2.5.6, 2.2.5.7, 2.2.5.8, 2.2.5.9, 2.2.5.10, 2.2.6.1, 2.2.6.2, 2.2.6.3, |
| 2.2.6.4, 2.2.6.5, 2.2.6.6, 2.2.6.7, 2.2.6.8, 2.2.6.9, 2.2.6.10, 2.2.7.1, 2.2.7.2, 2.2.7.3, 2.2.7.4, 2.2.7.5, 2.2.7.6, |
| 2.2.7.7, 2.2.7.8, 2.2.7.9, 2.2.7.10, 2.2.8.1, 2.2.8.2, 2.2.8.3, 2.2.8.4, 2.2.8.5, 2.2.8.6, 2.2.8.7, 2.2.8.8, 2.2.8.9, |
| 2.2.8.10, 2.2.9.1, 2.2.9.2, 2.2.9.3, 2.2.9.4, 2.2.9.5, 2.2.9.6, 2.2.9.7, 2.2.9.8, 2.2.9.9, 2.2.9.10, 2.2.10.1, 2.2.10.2, |
| 2.2.10.3, 2.2.10.4, 2.2.10.5, 2.2.10.6, 2.2.10.7, 2.2.10.8, 2.2.10.9, 2.2.10.10, 2.3.1.1, 2.3.1.2, 2.3.1.3, 2.3.1.4, |
| 2.3.1.5, 2.3.1.6, 2.3.1.7, 2.3.1.8, 2.3.1.9, 2.3.1.10, 2.3.2.1, 2.3.2.2, 2.3.2.3, 2.3.2.4, 2.3.2.5, 2.3.2.6, 2.3.2.7, |
| 2.3.2.8, 2.3.2.9, 2.3.2.10, 2.3.3.1, 2.3.3.2, 2.3.3.3, 2.3.3.4, 2.3.3.5, 2.3.3.6, 2.3.3.7, 2.3.3.8, 2.3.3.9, 2.3.3.10, |
| 2.3.4.1, 2.3.4.2, 2.3.4.3, 2.3.4.4, 2.3.4.5, 2.3.4.6, 2.3.4.7, 2.3.4.8, 2.3.4.9, 2.3.4.10, 2.3.5.1, 2.3.5.2, 2.3.5.3, |
| 2.3.5.4, 2.3.5.5, 2.3.5.6, 2.3.5.7, 2.3.5.8, 2.3.5.9, 2.3.5.10, 2.3.6.1, 2.3.6.2, 2.3.6.3, 2.3.6.4, 2.3.6.5, 2.3.6.6, |
| 2.3.6.7, 2.3.6.8, 2.3.6.9, 2.3.6.10, 2.3.7.1, 2.3.7.2, 2.3.7.3, 2.3.7.4, 2.3.7.5, 2.3.7.6, 2.3.7.7, 2.3.7.8, 2.3.7.9, |
| 2.3.7.10, 2.3.8.1, 2.3.8.2, 2.3.8.3, 2.3.8.4, 2.3.8.5, 2.3.8.6, 2.3.8.7, 2.3.8.8, 2.3.8.9, 2.3.8.10, 2.3.9.1, 2.3.9.2, |
| 2.3.9.3, 2.3.9.4, 2.3.9.5, 2.3.9.6, 2.3.9.7, 2.3.9.8, 2.3.9.9, 2.3.9.10, 2.3.10.1, 2.3.10.2, 2.3.10.3, 2.3.10.4, 2.3.10.5, |
| 2.3.10.6, 2.3.10.7, 2.3.10.8, 2.3.10.9, 2.3.10.10, 2.4.1.1, 2.4.1.2, 2.4.1.3, 2.4.1.4, 2.4.1.5, 2.4.1.6, 2.4.1.7, 2.4.1.8, |
| 2.4.1.9, 2.4.1.10, 2.4.2.1, 2.4.2.2, 2.4.2.3, 2.4.2.4, 2.4.2.5, 2.4.2.6, 2.4.2.7, 2.4.2.8, 2.4.2.9, 2.4.2.10, 2.4.3.1, |
| 2.4.3.2, 2.4.3.3, 2.4.3.4, 2.4.3.5, 2.4.3.6, 2.4.3.7, 2.4.3.8, 2.4.3.9, 2.4.3.10, 2.4.4.1, 2.4.4.2, 2.4.4.3, 2.4.4.4, |
| 2.4.4.5, 2.4.4.6, 2.4.4.7, 2.4.4.8, 2.4.4.9, 2.4.4.10, 2.4.5.1, 2.4.5.2, 2.4.5.3, 2.4.5.4, 2.4.5.5, 2.4.5.6, 2.4.5.7, |
| 2.4.5.8, 2.4.5.9, 2.4.5.10, 2.4.6.1, 2.4.6.2, 2.4.6.3, 2.4.6.4, 2.4.6.5, 2.4.6.6, 2.4.6.7, 2.4.6.8, 2.4.6.9, 2.4.6.10, |
| 2.4.7.1, 2.4.7.2, 2.4.7.3, 2.4.7.4, 2.4.7.5, 2.4.7.6, 2.4.7.7, 2.4.7.8, 2.4.7.9, 2.4.7.10, 2.4.8.1, 2.4.8.2, 2.4.8.3, |
| 2.4.8.4, 2.4.8.5, 2.4.8.6, 2.4.8.7, 2.4.8.8, 2.4.8.9, 2.4.8.10, 2.4.9.1, 2.4.9.2, 2.4.9.3, 2.4.9.4, 2.4.9.5, 2.4.9.6, |
| 2.4.9.7, 2.4.9.8, 2.4.9.9, 2.4.9.10, 2.4.10.1, 2.4.10.2, 2.4.10.3, 2.4.10.4, 2.4.10.5, 2.4.10.6, 2.4.10.7, 2.4.10.8, |
| 2.4.10.9, 2.4.10.10, 2.5.1.1, 2.5.1.2, 2.5.1.3, 2.5.1.4, 2.5.1.5, 2.5.1.6, 2.5.1.7, 2.5.1.8, 2.5.1.9, 2.5.1.10, 2.5.2.1, |
| 2.5.2.2, 2.5.2.3, 2.5.2.4, 2.5.2.5, 2.5.2.6, 2.5.2.7, 2.5.2.8, 2.5.2.9, 2.5.2.10, 2.5.3.1, 2.5.3.2, 2.5.3.3, 2.5.3.4, |
| 2.5.3.5, 2.5.3.6, 2.5.3.7, 2.5.3.8, 2.5.3.9, 2.5.3.10, 2.5.4.1, 2.5.4.2, 2.5.4.3, 2.5.4.4, 2.5.4.5, 2.5.4.6, 2.5.4.7, |
| 2.5.4.8, 2.5.4.9, 2.5.4.10, 2.5.5.1, 2.5.5.2, 2.5.5.3, 2.5.5.4, 2.5.5.5, 2.5.5.6, 2.5.5.7, 2.5.5.8, 2.5.5.9, 2.5.5.10, |
| 2.5.6.1, 2.5.6.2, 2.5.6.3, 2.5.6.4, 2.5.6.5, 2.5.6.6, 2.5.6.7, 2.5.6.8, 2.5.6.9, 2.5.6.10, 2.5.7.1, 2.5.7.2, 2.5.7.3, |
| 2.5.7.4, 2.5.7.5, 2.5.7.6, 2.5.7.7, 2.5.7.8, 2.5.7.9, 2.5.7.10, 2.5.8.1, 2.5.8.2, 2.5.8.3, 2.5.8.4, 2.5.8.5, 2.5.8.6, |
| 2.5.8.7, 2.5.8.8, 2.5.8.9, 2.5.8.10, 2.5.9.1, 2.5.9.2, 2.5.9.3, 2.5.9.4, 2.5.9.5, 2.5.9.6, 2.5.9.7, 2.5.9.8, 2.5.9.9, |
| 2.5.9.10, 2.5.10.1, 2.5.10.2, 2.5.10.3, 2.5.10.4, 2.5.10.5, 2.5.10.6, 2.5.10.7, 2.5.10.8, 2.5.10.9, 2.5.10.10, 2.6.1.1, |
| 2.6.1.2, 2.6.1.3, 2.6.1.4, 2.6.1.5, 2.6.1.6, 2.6.1.7, 2.6.1.8, 2.6.1.9, 2.6.1.10, 2.6.2.1, 2.6.2.2, 2.6.2.3, 2.6.2.4, |
| 2.6.2.5, 2.6.2.6, 2.6.2.7, 2.6.2.8, 2.6.2.9, 2.6.2.10, 2.6.3.1, 2.6.3.2, 2.6.3.3, 2.6.3.4, 2.6.3.5, 2.6.3.6, 2.6.3.7, |
| 2.6.3.8, 2.6.3.9, 2.6.3.10, 2.6.4.1, 2.6.4.2, 2.6.4.3, 2.6.4.4, 2.6.4.5, 2.6.4.6, 2.6.4.7, 2.6.4.8, 2.6.4.9, 2.6.4.10, |
| 2.6.5.1, 2.6.5.2, 2.6.5.3, 2.6.5.4, 2.6.5.5, 2.6.5.6, 2.6.5.7, 2.6.5.8, 2.6.5.9, 2.6.5.10, 2.6.6.1, 2.6.6.2, 2.6.6.3, |
| 2.6.6.4, 2.6.6.5, 2.6.6.6, 2.6.6.7, 2.6.6.8, 2.6.6.9, 2.6.6.10, 2.6.7.1, 2.6.7.2, 2.6.7.3, 2.6.7.4, 2.6.7.5, 2.6.7.6, |
| 2.6.7.7, 2.6.7.8, 2.6.7.9, 2.6.7.10, 2.6.8.1, 2.6.8.2, 2.6.8.3, 2.6.8.4, 2.6.8.5, 2.6.8.6, 2.6.8.7, 2.6.8.8, 2.6.8.9, |
| 2.6.8.10, 2.6.9.1, 2.6.9.2, 2.6.9.3, 2.6.9.4, 2.6.9.5, 2.6.9.6, 2.6.9.7, 2.6.9.8, 2.6.9.9, 2.6.9.10, 2.6.10.1, 2.6.10.2, |
| 2.6.10.3, 2.6.10.4, 2.6.10.5, 2.6.10.6, 2.6.10.7, 2.6.10.8, 2.6.10.9, 2.6.10.10, 2.7.1.1, 2.7.1.2, 2.7.1.3, 2.7.1.4, |
| 2.7.1.5, 2.7.1.6, 2.7.1.7, 2.7.1.8, 2.7.1.9, 2.7.1.10, 2.7.2.1, 2.7.2.2, 2.7.2.3, 2.7.2.4, 2.7.2.5, 2.7.2.6, 2.7.2.7, |
| 2.7.2.8, 2.7.2.9, 2.7.2.10, 2.7.3.1, 2.7.3.2, 2.7.3.3, 2.7.3.4, 2.7.3.5, 2.7.3.6, 2.7.3.7, 2.7.3.8, 2.7.3.9, 2.7.3.10, |
| 2.7.4.1, 2.7.4.2, 2.7.4.3, 2.7.4.4, 2.7.4.5, 2.7.4.6, 2.7.4.7, 2.7.4.8, 2.7.4.9, 2.7.4.10, 2.7.5.1, 2.7.5.2, 2.7.5.3, |
| 2.7.5.4, 2.7.5.5, 2.7.5.6, 2.7.5.7, 2.7.5.8, 2.7.5.9, 2.7.5.10, 2.7.6.1, 2.7.6.2, 2.7.6.3, 2.7.6.4, 2.7.6.5, 2.7.6.6, |
| 2.7.6.7, 2.7.6.8, 2.7.6.9, 2.7.6.10, 2.7.7.1, 2.7.7.2, 2.7.7.3, 2.7.7.4, 2.7.7.5, 2.7.7.6, 2.7.7.7, 2.7.7.8, 2.7.7.9, |
| 2.7.7.10, 2.7.8.1, 2.7.8.2, 2.7.8.3, 2.7.8.4, 2.7.8.5, 2.7.8.6, 2.7.8.7, 2.7.8.8, 2.7.8.9, 2.7.8.10, 2.7.9.1, 2.7.9.2, |
| 2.7.9.3, 2.7.9.4, 2.7.9.5, 2.7.9.6, 2.7.9.7, 2.7.9.8, 2.7.9.9, 2.7.9.10, 2.7.10.1, 2.7.10.2, 2.7.10.3, 2.7.10.4, 2.7.10.5, |
| 2.7.10.6, 2.7.10.7, 2.7.10.8, 2.7.10.9, 2.7.10.10, 2.8.1.1, 2.8.1.2, 2.8.1.3, 2.8.1.4, 2.8.1.5, 2.8.1.6, 2.8.1.7, 2.8.1.8, |
| 2.8.1.9, 2.8.1.10, 2.8.2.1, 2.8.2.2, 2.8.2.3, 2.8.2.4, 2.8.2.5, 2.8.2.6, 2.8.2.7, 2.8.2.8, 2.8.2.9, 2.8.2.10, 2.8.3.1, |
| 2.8.3.2, 2.8.3.3, 2.8.3.4, 2.8.3.5, 2.8.3.6, 2.8.3.7, 2.8.3.8, 2.8.3.9, 2.8.3.10, 2.8.4.1, 2.8.4.2, 2.8.4.3, 2.8.4.4, |
| 2.8.4.5, 2.8.4.6, 2.8.4.7, 2.8.4.8, 2.8.4.9, 2.8.4.10, 2.8.5.1, 2.8.5.2, 2.8.5.3, 2.8.5.4, 2.8.5.5, 2.8.5.6, 2.8.5.7, |
| 2.8.5.8, 2.8.5.9, 2.8.5.10, 2.8.6.1, 2.8.6.2, 2.8.6.3, 2.8.6.4, 2.8.6.5, 2.8.6.6, 2.8.6.7, 2.8.6.8, 2.8.6.9, 2.8.6.10, |
| 2.8.7.1, 2.8.7.2, 2.8.7.3, 2.8.7.4, 2.8.7.5, 2.8.7.6, 2.8.7.7, 2.8.7.8, 2.8.7.9, 2.8.7.10, 2.8.8.1, 2.8.8.2, 2.8.8.3, |
| 2.8.8.4, 2.8.8.5, 2.8.8.6, 2.8.8.7, 2.8.8.8, 2.8.8.9, 2.8.8.10, 2.8.9.1, 2.8.9.2, 2.8.9.3, 2.8.9.4, 2.8.9.5, 2.8.9.6, |
| 2.8.9.7, 2.8.9.8, 2.8.9.9, 2.8.9.10, 2.8.10.1, 2.8.10.2, 2.8.10.3, 2.8.10.4, 2.8.10.5, 2.8.10.6, 2.8.10.7, 2.8.10.8, |
| 2.8.10.9, 2.8.10.10, 2.9.1.1, 2.9.1.2, 2.9.1.3, 2.9.1.4, 2.9.1.5, 2.9.1.6, 2.9.1.7, 2.9.1.8, 2.9.1.9, 2.9.1.10, 2.9.2.1, |
| 2.9.2.2, 2.9.2.3, 2.9.2.4, 2.9.2.5, 2.9.2.6, 2.9.2.7, 2.9.2.8, 2.9.2.9, 2.9.2.10, 2.9.3.1, 2.9.3.2, 2.9.3.3, 2.9.3.4, |
| 2.9.3.5, 2.9.3.6, 2.9.3.7, 2.9.3.8, 2.9.3.9, 2.9.3.10, 2.9.4.1, 2.9.4.2, 2.9.4.3, 2.9.4.4, 2.9.4.5, 2.9.4.6, 2.9.4.7, |
| 2.9.4.8, 2.9.4.9, 2.9.4.10, 2.9.5.1, 2.9.5.2, 2.9.5.3, 2.9.5.4, 2.9.5.5, 2.9.5.6, 2.9.5.7, 2.9.5.8, 2.9.5.9, 2.9.5.10, |
| 2.9.6.1, 2.9.6.2, 2.9.6.3, 2.9.6.4, 2.9.6.5, 2.9.6.6, 2.9.6.7, 2.9.6.8, 2.9.6.9, 2.9.6.10, 2.9.7.1, 2.9.7.2, 2.9.7.3, |
| 2.9.7.4, 2.9.7.5, 2.9.7.6, 2.9.7.7, 2.9.7.8, 2.9.7.9, 2.9.7.10, 2.9.8.1, 2.9.8.2, 2.9.8.3, 2.9.8.4, 2.9.8.5, 2.9.8.6, |
| 2.9.8.7, 2.9.8.8, 2.9.8.9, 2.9.8.10, 2.9.9.1, 2.9.9.2, 2.9.9.3, 2.9.9.4, 2.9.9.5, 2.9.9.6, 2.9.9.7, 2.9.9.8, 2.9.9.9, |
| 2.9.9.10, 2.9.10.1, 2.9.10.2, 2.9.10.3, 2.9.10.4, 2.9.10.5, 2.9.10.6, 2.9.10.7, 2.9.10.8, 2.9.10.9, 2.9.10.10, |
| 2.10.1.1, 2.10.1.2, 2.10.1.3, 2.10.1.4, 2.10.1.5, 2.10.1.6, 2.10.1.7, 2.10.1.8, 2.10.1.9, 2.10.1.10, 2.10.2.1, |
| 2.10.2.2, 2.10.2.3, 2.10.2.4, 2.10.2.5, 2.10.2.6, 2.10.2.7, 2.10.2.8, 2.10.2.9, 2.10.2.10, 2.10.3.1, 2.10.3.2, |
| 2.10.3.3, 2.10.3.4, 2.10.3.5, 2.10.3.6, 2.10.3.7, 2.10.3.8, 2.10.3.9, 2.10.3.10, 2.10.4.1, 2.10.4.2, 2.10.4.3, |
| 2.10.4.4, 2.10.4.5, 2.10.4.6, 2.10.4.7, 2.10.4.8, 2.10.4.9, 2.10.4.10, 2.10.5.1, 2.10.5.2, 2.10.5.3, 2.10.5.4, |
| 2.10.5.5, 2.10.5.6, 2.10.5.7, 2.10.5.8, 2.10.5.9, 2.10.5.10, 2.10.6.1, 2.10.6.2, 2.10.6.3, 2.10.6.4, 2.10.6.5, |
| 2.10.6.6, 2.10.6.7, 2.10.6.8, 2.10.6.9, 2.10.6.10, 2.10.7.1, 2.10.7.2, 2.10.7.3, 2.10.7.4, 2.10.7.5, 2.10.7.6, |
| 2.10.7.7, 2.10.7.8, 2.10.7.9, 2.10.7.10, 2.10.8.1, 2.10.8.2, 2.10.8.3, 2.10.8.4, 2.10.8.5, 2.10.8.6, 2.10.8.7, |
| 2.10.8.8, 2.10.8.9, 2.10.8.10, 2.10.9.1, 2.10.9.2, 2.10.9.3, 2.10.9.4, 2.10.9.5, 2.10.9.6, 2.10.9.7, 2.10.9.8, |
| 2.10.9.9, 2.10.9.10, 2.10.10.1, 2.10.10.2, 2.10.10.3, 2.10.10.4, 2.10.10.5, 2.10.10.6, 2.10.10.7, 2.10.10.8, |
| 2.10.10.9, 2.10.10.10, 3.1.1.1, 3.1.1.2, 3.1.1.3, 3.1.1.4, 3.1.1.5, 3.1.1.6, 3.1.1.7, 3.1.1.8, 3.1.1.9, 3.1.1.10, 3.1.2.1, |
| 3.1.2.2, 3.1.2.3, 3.1.2.4, 3.1.2.5, 3.1.2.6, 3.1.2.7, 3.1.2.8, 3.1.2.9, 3.1.2.10, 3.1.3.1, 3.1.3.2, 3.1.3.3, 3.1.3.4, |
| 3.1.3.5, 3.1.3.6, 3.1.3.7, 3.1.3.8, 3.1.3.9, 3.1.3.10, 3.1.4.1, 3.1.4.2, 3.1.4.3, 3.1.4.4, 3.1.4.5, 3.1.4.6, 3.1.4.7, |
| 3.1.4.8, 3.1.4.9, 3.1.4.10, 3.1.5.1, 3.1.5.2, 3.1.5.3, 3.1.5.4, 3.1.5.5, 3.1.5.6, 3.1.5.7, 3.1.5.8, 3.1.5.9, 3.1.5.10, |
| 3.1.6.1, 3.1.6.2, 3.1.6.3, 3.1.6.4, 3.1.6.5, 3.1.6.6, 3.1.6.7, 3.1.6.8, 3.1.6.9, 3.1.6.10, 3.1.7.1, 3.1.7.2, 3.1.7.3, |
| 3.1.7.4, 3.1.7.5, 3.1.7.6, 3.1.7.7, 3.1.7.8, 3.1.7.9, 3.1.7.10, 3.1.8.1, 3.1.8.2, 3.1.8.3, 3.1.8.4, 3.1.8.5, 3.1.8.6, |
| 3.1.8.7, 3.1.8.8, 3.1.8.9, 3.1.8.10, 3.1.9.1, 3.1.9.2, 3.1.9.3, 3.1.9.4, 3.1.9.5, 3.1.9.6, 3.1.9.7, 3.1.9.8, 3.1.9.9, |
| 3.1.9.10, 3.1.10.1, 3.1.10.2, 3.1.10.3, 3.1.10.4, 3.1.10.5, 3.1.10.6, 3.1.10.7, 3.1.10.8, 3.1.10.9, 3.1.10.10, 3.2.1.1, |
| 3.2.1.2, 3.2.1.3, 3.2.1.4, 3.2.1.5, 3.2.1.6, 3.2.1.7, 3.2.1.8, 3.2.1.9, 3.2.1.10, 3.2.2.1, 3.2.2.2, 3.2.2.3, 3.2.2.4, |
| 3.2.2.5, 3.2.2.6, 3.2.2.7, 3.2.2.8, 3.2.2.9, 3.2.2.10, 3.2.3.1, 3.2.3.2, 3.2.3.3, 3.2.3.4, 3.2.3.5, 3.2.3.6, 3.2.3.7, |
| 3.2.3.8, 3.2.3.9, 3.2.3.10, 3.2.4.1, 3.2.4.2, 3.2.4.3, 3.2.4.4, 3.2.4.5, 3.2.4.6, 3.2.4.7, 3.2.4.8, 3.2.4.9, 3.2.4.10, |
| 3.2.5.1, 3.2.5.2, 3.2.5.3, 3.2.5.4, 3.2.5.5, 3.2.5.6, 3.2.5.7, 3.2.5.8, 3.2.5.9, 3.2.5.10, 3.2.6.1, 3.2.6.2, 3.2.6.3, |
| 3.2.6.4, 3.2.6.5, 3.2.6.6, 3.2.6.7, 3.2.6.8, 3.2.6.9, 3.2.6.10, 3.2.7.1, 3.2.7.2, 3.2.7.3, 3.2.7.4, 3.2.7.5, 3.2.7.6, |
| 3.2.7.7, 3.2.7.8, 3.2.7.9, 3.2.7.10, 3.2.8.1, 3.2.8.2, 3.2.8.3, 3.2.8.4, 3.2.8.5, 3.2.8.6, 3.2.8.7, 3.2.8.8, 3.2.8.9, |
| 3.2.8.10, 3.2.9.1, 3.2.9.2, 3.2.9.3, 3.2.9.4, 3.2.9.5, 3.2.9.6, 3.2.9.7, 3.2.9.8, 3.2.9.9, 3.2.9.10, 3.2.10.1, 3.2.10.2, |
| 3.2.10.3, 3.2.10.4, 3.2.10.5, 3.2.10.6, 3.2.10.7, 3.2.10.8, 3.2.10.9, 3.2.10.10, 3.3.1.1, 3.3.1.2, 3.3.1.3, 3.3.1.4, |
| 3.3.1.5, 3.3.1.6, 3.3.1.7, 3.3.1.8, 3.3.1.9, 3.3.1.10, 3.3.2.1, 3.3.2.2, 3.3.2.3, 3.3.2.4, 3.3.2.5, 3.3.2.6, 3.3.2.7, |
| 3.3.2.8, 3.3.2.9, 3.3.2.10, 3.3.3.1, 3.3.3.2, 3.3.3.3, 3.3.3.4, 3.3.3.5, 3.3.3.6, 3.3.3.7, 3.3.3.8, 3.3.3.9, 3.3.3.10, |
| 3.3.4.1, 3.3.4.2, 3.3.4.3, 3.3.4.4, 3.3.4.5, 3.3.4.6, 3.3.4.7, 3.3.4.8, 3.3.4.9, 3.3.4.10, 3.3.5.1, 3.3.5.2, 3.3.5.3, |
| 3.3.5.4, 3.3.5.5, 3.3.5.6, 3.3.5.7, 3.3.5.8, 3.3.5.9, 3.3.5.10, 3.3.6.1, 3.3.6.2, 3.3.6.3, 3.3.6.4, 3.3.6.5, 3.3.6.6, |
| 3.3.6.7, 3.3.6.8, 3.3.6.9, 3.3.6.10, 3.3.7.1, 3.3.7.2, 3.3.7.3, 3.3.7.4, 3.3.7.5, 3.3.7.6, 3.3.7.7, 3.3.7.8, 3.3.7.9, |
| 3.3.7.10, 3.3.8.1, 3.3.8.2, 3.3.8.3, 3.3.8.4, 3.3.8.5, 3.3.8.6, 3.3.8.7, 3.3.8.8, 3.3.8.9, 3.3.8.10, 3.3.9.1, 3.3.9.2, |
| 3.3.9.3, 3.3.9.4, 3.3.9.5, 3.3.9.6, 3.3.9.7, 3.3.9.8, 3.3.9.9, 3.3.9.10, 3.3.10.1, 3.3.10.2, 3.3.10.3, 3.3.10.4, 3.3.10.5, |
| 3.3.10.6, 3.3.10.7, 3.3.10.8, 3.3.10.9, 3.3.10.10, 3.4.1.1, 3.4.1.2, 3.4.1.3, 3.4.1.4, 3.4.1.5, 3.4.1.6, 3.4.1.7, 3.4.1.8, |
| 3.4.1.9, 3.4.1.10, 3.4.2.1, 3.4.2.2, 3.4.2.3, 3.4.2.4, 3.4.2.5, 3.4.2.6, 3.4.2.7, 3.4.2.8, 3.4.2.9, 3.4.2.10, 3.4.3.1, |
| 3.4.3.2, 3.4.3.3, 3.4.3.4, 3.4.3.5, 3.4.3.6, 3.4.3.7, 3.4.3.8, 3.4.3.9, 3.4.3.10, 3.4.4.1, 3.4.4.2, 3.4.4.3, 3.4.4.4, |
| 3.4.4.5, 3.4.4.6, 3.4.4.7, 3.4.4.8, 3.4.4.9, 3.4.4.10, 3.4.5.1, 3.4.5.2, 3.4.5.3, 3.4.5.4, 3.4.5.5, 3.4.5.6, 3.4.5.7, |
| 3.4.5.8, 3.4.5.9, 3.4.5.10, 3.4.6.1, 3.4.6.2, 3.4.6.3, 3.4.6.4, 3.4.6.5, 3.4.6.6, 3.4.6.7, 3.4.6.8, 3.4.6.9, 3.4.6.10, |
| 3.4.7.1, 3.4.7.2, 3.4.7.3, 3.4.7.4, 3.4.7.5, 3.4.7.6, 3.4.7.7, 3.4.7.8, 3.4.7.9, 3.4.7.10, 3.4.8.1, 3.4.8.2, 3.4.8.3, |
| 3.4.8.4, 3.4.8.5, 3.4.8.6, 3.4.8.7, 3.4.8.8, 3.4.8.9, 3.4.8.10, 3.4.9.1, 3.4.9.2, 3.4.9.3, 3.4.9.4, 3.4.9.5, 3.4.9.6, |
| 3.4.9.7, 3.4.9.8, 3.4.9.9, 3.4.9.10, 3.4.10.1, 3.4.10.2, 3.4.10.3, 3.4.10.4, 3.4.10.5, 3.4.10.6, 3.4.10.7, 3.4.10.8, |
| 3.4.10.9, 3.4.10.10, 3.5.1.1, 3.5.1.2, 3.5.1.3, 3.5.1.4, 3.5.1.5, 3.5.1.6, 3.5.1.7, 3.5.1.8, 3.5.1.9, 3.5.1.10, 3.5.2.1, |
| 3.5.2.2, 3.5.2.3, 3.5.2.4, 3.5.2.5, 3.5.2.6, 3.5.2.7, 3.5.2.8, 3.5.2.9, 3.5.2.10, 3.5.3.1, 3.5.3.2, 3.5.3.3, 3.5.3.4, |
| 3.5.3.5, 3.5.3.6, 3.5.3.7, 3.5.3.8, 3.5.3.9, 3.5.3.10, 3.5.4.1, 3.5.4.2, 3.5.4.3, 3.5.4.4, 3.5.4.5, 3.5.4.6, 3.5.4.7, |
| 3.5.4.8, 3.5.4.9, 3.5.4.10, 3.5.5.1, 3.5.5.2, 3.5.5.3, 3.5.5.4, 3.5.5.5, 3.5.5.6, 3.5.5.7, 3.5.5.8, 3.5.5.9, 3.5.5.10, |
| 3.5.6.1, 3.5.6.2, 3.5.6.3, 3.5.6.4, 3.5.6.5, 3.5.6.6, 3.5.6.7, 3.5.6.8, 3.5.6.9, 3.5.6.10, 3.5.7.1, 3.5.7.2, 3.5.7.3, |
| 3.5.7.4, 3.5.7.5, 3.5.7.6, 3.5.7.7, 3.5.7.8, 3.5.7.9, 3.5.7.10, 3.5.8.1, 3.5.8.2, 3.5.8.3, 3.5.8.4, 3.5.8.5, 3.5.8.6, |
| 3.5.8.7, 3.5.8.8, 3.5.8.9, 3.5.8.10, 3.5.9.1, 3.5.9.2, 3.5.9.3, 3.5.9.4, 3.5.9.5, 3.5.9.6, 3.5.9.7, 3.5.9.8, 3.5.9.9, |
| 3.5.9.10, 3.5.10.1, 3.5.10.2, 3.5.10.3, 3.5.10.4, 3.5.10.5, 3.5.10.6, 3.5.10.7, 3.5.10.8, 3.5.10.9, 3.5.10.10, 3.6.1.1, |
| 3.6.1.2, 3.6.1.3, 3.6.1.4, 3.6.1.5, 3.6.1.6, 3.6.1.7, 3.6.1.8, 3.6.1.9, 3.6.1.10, 3.6.2.1, 3.6.2.2, 3.6.2.3, 3.6.2.4, |
| 3.6.2.5, 3.6.2.6, 3.6.2.7, 3.6.2.8, 3.6.2.9, 3.6.2.10, 3.6.3.1, 3.6.3.2, 3.6.3.3, 3.6.3.4, 3.6.3.5, 3.6.3.6, 3.6.3.7, |
| 3.6.3.8, 3.6.3.9, 3.6.3.10, 3.6.4.1, 3.6.4.2, 3.6.4.3, 3.6.4.4, 3.6.4.5, 3.6.4.6, 3.6.4.7, 3.6.4.8, 3.6.4.9, 3.6.4.10, |
| 3.6.5.1, 3.6.5.2, 3.6.5.3, 3.6.5.4, 3.6.5.5, 3.6.5.6, 3.6.5.7, 3.6.5.8, 3.6.5.9, 3.6.5.10, 3.6.6.1, 3.6.6.2, 3.6.6.3, |
| 3.6.6.4, 3.6.6.5, 3.6.6.6, 3.6.6.7, 3.6.6.8, 3.6.6.9, 3.6.6.10, 3.6.7.1, 3.6.7.2, 3.6.7.3, 3.6.7.4, 3.6.7.5, 3.6.7.6, |
| 3.6.7.7, 3.6.7.8, 3.6.7.9, 3.6.7.10, 3.6.8.1, 3.6.8.2, 3.6.8.3, 3.6.8.4, 3.6.8.5, 3.6.8.6, 3.6.8.7, 3.6.8.8, 3.6.8.9, |
| 3.6.8.10, 3.6.9.1, 3.6.9.2, 3.6.9.3, 3.6.9.4, 3.6.9.5, 3.6.9.6, 3.6.9.7, 3.6.9.8, 3.6.9.9, 3.6.9.10, 3.6.10.1, 3.6.10.2, |
| 3.6.10.3, 3.6.10.4, 3.6.10.5, 3.6.10.6, 3.6.10.7, 3.6.10.8, 3.6.10.9, 3.6.10.10, 3.7.1.1, 3.7.1.2, 3.7.1.3, 3.7.1.4, |
| 3.7.1.5, 3.7.1.6, 3.7.1.7, 3.7.1.8, 3.7.1.9, 3.7.1.10, 3.7.2.1, 3.7.2.2, 3.7.2.3, 3.7.2.4, 3.7.2.5, 3.7.2.6, 3.7.2.7, |
| 3.7.2.8, 3.7.2.9, 3.7.2.10, 3.7.3.1, 3.7.3.2, 3.7.3.3, 3.7.3.4, 3.7.3.5, 3.7.3.6, 3.7.3.7, 3.7.3.8, 3.7.3.9, 3.7.3.10, |
| 3.7.4.1, 3.7.4.2, 3.7.4.3, 3.7.4.4, 3.7.4.5, 3.7.4.6, 3.7.4.7, 3.7.4.8, 3.7.4.9, 3.7.4.10, 3.7.5.1, 3.7.5.2, 3.7.5.3, |
| 3.7.5.4, 3.7.5.5, 3.7.5.6, 3.7.5.7, 3.7.5.8, 3.7.5.9, 3.7.5.10, 3.7.6.1, 3.7.6.2, 3.7.6.3, 3.7.6.4, 3.7.6.5, 3.7.6.6, |
| 3.7.6.7, 3.7.6.8, 3.7.6.9, 3.7.6.10, 3.7.7.1, 3.7.7.2, 3.7.7.3, 3.7.7.4, 3.7.7.5, 3.7.7.6, 3.7.7.7, 3.7.7.8, 3.7.7.9, |
| 3.7.7.10, 3.7.8.1, 3.7.8.2, 3.7.8.3, 3.7.8.4, 3.7.8.5, 3.7.8.6, 3.7.8.7, 3.7.8.8, 3.7.8.9, 3.7.8.10, 3.7.9.1, 3.7.9.2, |
| 3.7.9.3, 3.7.9.4, 3.7.9.5, 3.7.9.6, 3.7.9.7, 3.7.9.8, 3.7.9.9, 3.7.9.10, 3.7.10.1, 3.7.10.2, 3.7.10.3, 3.7.10.4, 3.7.10.5, |
| 3.7.10.6, 3.7.10.7, 3.7.10.8, 3.7.10.9, 3.7.10.10, 3.8.1.1, 3.8.1.2, 3.8.1.3, 3.8.1.4, 3.8.1.5, 3.8.1.6, 3.8.1.7, 3.8.1.8, |
| 3.8.1.9, 3.8.1.10, 3.8.2.1, 3.8.2.2, 3.8.2.3, 3.8.2.4, 3.8.2.5, 3.8.2.6, 3.8.2.7, 3.8.2.8, 3.8.2.9, 3.8.2.10, 3.8.3.1, |
| 3.8.3.2, 3.8.3.3, 3.8.3.4, 3.8.3.5, 3.8.3.6, 3.8.3.7, 3.8.3.8, 3.8.3.9, 3.8.3.10, 3.8.4.1, 3.8.4.2, 3.8.4.3, 3.8.4.4, |
| 3.8.4.5, 3.8.4.6, 3.8.4.7, 3.8.4.8, 3.8.4.9, 3.8.4.10, 3.8.5.1, 3.8.5.2, 3.8.5.3, 3.8.5.4, 3.8.5.5, 3.8.5.6, 3.8.5.7, |
| 3.8.5.8, 3.8.5.9, 3.8.5.10, 3.8.6.1, 3.8.6.2, 3.8.6.3, 3.8.6.4, 3.8.6.5, 3.8.6.6, 3.8.6.7, 3.8.6.8, 3.8.6.9, 3.8.6.10, |
| 3.8.7.1, 3.8.7.2, 3.8.7.3, 3.8.7.4, 3.8.7.5, 3.8.7.6, 3.8.7.7, 3.8.7.8, 3.8.7.9, 3.8.7.10, 3.8.8.1, 3.8.8.2, 3.8.8.3, |
| 3.8.8.4, 3.8.8.5, 3.8.8.6, 3.8.8.7, 3.8.8.8, 3.8.8.9, 3.8.8.10, 3.8.9.1, 3.8.9.2, 3.8.9.3, 3.8.9.4, 3.8.9.5, 3.8.9.6, |
| 3.8.9.7, 3.8.9.8, 3.8.9.9, 3.8.9.10, 3.8.10.1, 3.8.10.2, 3.8.10.3, 3.8.10.4, 3.8.10.5, 3.8.10.6, 3.8.10.7, 3.8.10.8, |
| 3.8.10.9, 3.8.10.10, 3.9.1.1, 3.9.1.2, 3.9.1.3, 3.9.1.4, 3.9.1.5, 3.9.1.6, 3.9.1.7, 3.9.1.8, 3.9.1.9, 3.9.1.10, 3.9.2.1, |
| 3.9.2.2, 3.9.2.3, 3.9.2.4, 3.9.2.5, 3.9.2.6, 3.9.2.7, 3.9.2.8, 3.9.2.9, 3.9.2.10, 3.9.3.1, 3.9.3.2, 3.9.3.3, 3.9.3.4, |
| 3.9.3.5, 3.9.3.6, 3.9.3.7, 3.9.3.8, 3.9.3.9, 3.9.3.10, 3.9.4.1, 3.9.4.2, 3.9.4.3, 3.9.4.4, 3.9.4.5, 3.9.4.6, 3.9.4.7, |
| 3.9.4.8, 3.9.4.9, 3.9.4.10, 3.9.5.1, 3.9.5.2, 3.9.5.3, 3.9.5.4, 3.9.5.5, 3.9.5.6, 3.9.5.7, 3.9.5.8, 3.9.5.9, 3.9.5.10, |
| 3.9.6.1, 3.9.6.2, 3.9.6.3, 3.9.6.4, 3.9.6.5, 3.9.6.6, 3.9.6.7, 3.9.6.8, 3.9.6.9, 3.9.6.10, 3.9.7.1, 3.9.7.2, 3.9.7.3, |
| 3.9.7.4, 3.9.7.5, 3.9.7.6, 3.9.7.7, 3.9.7.8, 3.9.7.9, 3.9.7.10, 3.9.8.1, 3.9.8.2, 3.9.8.3, 3.9.8.4, 3.9.8.5, 3.9.8.6, |
| 3.9.8.7, 3.9.8.8, 3.9.8.9, 3.9.8.10, 3.9.9.1, 3.9.9.2, 3.9.9.3, 3.9.9.4, 3.9.9.5, 3.9.9.6, 3.9.9.7, 3.9.9.8, 3.9.9.9, |
| 3.9.9.10, 3.9.10.1, 3.9.10.2, 3.9.10.3, 3.9.10.4, 3.9.10.5, 3.9.10.6, 3.9.10.7, 3.9.10.8, 3.9.10.9, 3.9.10.10, |
| 3.10.1.1, 3.10.1.2, 3.10.1.3, 3.10.1.4, 3.10.1.5, 3.10.1.6, 3.10.1.7, 3.10.1.8, 3.10.1.9, 3.10.1.10, 3.10.2.1, |
| 3.10.2.2, 3.10.2.3, 3.10.2.4, 3.10.2.5, 3.10.2.6, 3.10.2.7, 3.10.2.8, 3.10.2.9, 3.10.2.10, 3.10.3.1, 3.10.3.2, |
| 3.10.3.3, 3.10.3.4, 3.10.3.5, 3.10.3.6, 3.10.3.7, 3.10.3.8, 3.10.3.9, 3.10.3.10, 3.10.4.1, 3.10.4.2, 3.10.4.3, |
| 3.10.4.4, 3.10.4.5, 3.10.4.6, 3.10.4.7, 3.10.4.8, 3.10.4.9, 3.10.4.10, 3.10.5.1, 3.10.5.2, 3.10.5.3, 3.10.5.4, |
| 3.10.5.5, 3.10.5.6, 3.10.5.7, 3.10.5.8, 3.10.5.9, 3.10.5.10, 3.10.6.1, 3.10.6.2, 3.10.6.3, 3.10.6.4, 3.10.6.5, |
| 3.10.6.6, 3.10.6.7, 3.10.6.8, 3.10.6.9, 3.10.6.10, 3.10.7.1, 3.10.7.2, 3.10.7.3, 3.10.7.4, 3.10.7.5, 3.10.7.6, |
| 3.10.7.7, 3.10.7.8, 3.10.7.9, 3.10.7.10, 3.10.8.1, 3.10.8.2, 3.10.8.3, 3.10.8.4, 3.10.8.5, 3.10.8.6, 3.10.8.7, |
| 3.10.8.8, 3.10.8.9, 3.10.8.10, 3.10.9.1, 3.10.9.2, 3.10.9.3, 3.10.9.4, 3.10.9.5, 3.10.9.6, 3.10.9.7, 3.10.9.8, |
| 3.10.9.9, 3.10.9.10, 3.10.10.1, 3.10.10.2, 3.10.10.3, 3.10.10.4, 3.10.10.5, 3.10.10.6, 3.10.10.7, 3.10.10.8, |
| 3.10.10.9, 3.10.10.10, 4.1.1.1, 4.1.1.2, 4.1.1.3, 4.1.1.4, 4.1.1.5, 4.1.1.6, 4.1.1.7, 4.1.1.8, 4.1.1.9, 4.1.1.10, 4.1.2.1, |
| 4.1.2.2, 4.1.2.3, 4.1.2.4, 4.1.2.5, 4.1.2.6, 4.1.2.7, 4.1.2.8, 4.1.2.9, 4.1.2.10, 4.1.3.1, 4.1.3.2, 4.1.3.3, 4.1.3.4, |
| 4.1.3.5, 4.1.3.6, 4.1.3.7, 4.1.3.8, 4.1.3.9, 4.1.3.10, 4.1.4.1, 4.1.4.2, 4.1.4.3, 4.1.4.4, 4.1.4.5, 4.1.4.6, 4.1.4.7, |
| 4.1.4.8, 4.1.4.9, 4.1.4.10, 4.1.5.1, 4.1.5.2, 4.1.5.3, 4.1.5.4, 4.1.5.5, 4.1.5.6, 4.1.5.7, 4.1.5.8, 4.1.5.9, 4.1.5.10, |
| 4.1.6.1, 4.1.6.2, 4.1.6.3, 4.1.6.4, 4.1.6.5, 4.1.6.6, 4.1.6.7, 4.1.6.8, 4.1.6.9, 4.1.6.10, 4.1.7.1, 4.1.7.2, 4.1.7.3, |
| 4.1.7.4, 4.1.7.5, 4.1.7.6, 4.1.7.7, 4.1.7.8, 4.1.7.9, 4.1.7.10, 4.1.8.1, 4.1.8.2, 4.1.8.3, 4.1.8.4, 4.1.8.5, 4.1.8.6, |
| 4.1.8.7, 4.1.8.8, 4.1.8.9, 4.1.8.10, 4.1.9.1, 4.1.9.2, 4.1.9.3, 4.1.9.4, 4.1.9.5, 4.1.9.6, 4.1.9.7, 4.1.9.8, 4.1.9.9, |
| 4.1.9.10, 4.1.10.1, 4.1.10.2, 4.1.10.3, 4.1.10.4, 4.1.10.5, 4.1.10.6, 4.1.10.7, 4.1.10.8, 4.1.10.9, 4.1.10.10, 4.2.1.1, |
| 4.2.1.2, 4.2.1.3, 4.2.1.4, 4.2.1.5, 4.2.1.6, 4.2.1.7, 4.2.1.8, 4.2.1.9, 4.2.1.10, 4.2.2.1, 4.2.2.2, 4.2.2.3, 4.2.2.4, |
| 4.2.2.5, 4.2.2.6, 4.2.2.7, 4.2.2.8, 4.2.2.9, 4.2.2.10, 4.2.3.1, 4.2.3.2, 4.2.3.3, 4.2.3.4, 4.2.3.5, 4.2.3.6, 4.2.3.7, |
| 4.2.3.8, 4.2.3.9, 4.2.3.10, 4.2.4.1, 4.2.4.2, 4.2.4.3, 4.2.4.4, 4.2.4.5, 4.2.4.6, 4.2.4.7, 4.2.4.8, 4.2.4.9, 4.2.4.10, |
| 4.2.5.1, 4.2.5.2, 4.2.5.3, 4.2.5.4, 4.2.5.5, 4.2.5.6, 4.2.5.7, 4.2.5.8, 4.2.5.9, 4.2.5.10, 4.2.6.1, 4.2.6.2, 4.2.6.3, |
| 4.2.6.4, 4.2.6.5, 4.2.6.6, 4.2.6.7, 4.2.6.8, 4.2.6.9, 4.2.6.10, 4.2.7.1, 4.2.7.2, 4.2.7.3, 4.2.7.4, 4.2.7.5, 4.2.7.6, |
| 4.2.7.7, 4.2.7.8, 4.2.7.9, 4.2.7.10, 4.2.8.1, 4.2.8.2, 4.2.8.3, 4.2.8.4, 4.2.8.5, 4.2.8.6, 4.2.8.7, 4.2.8.8, 4.2.8.9, |
| 4.2.8.10, 4.2.9.1, 4.2.9.2, 4.2.9.3, 4.2.9.4, 4.2.9.5, 4.2.9.6, 4.2.9.7, 4.2.9.8, 4.2.9.9, 4.2.9.10, 4.2.10.1, 4.2.10.2, |
| 4.2.10.3, 4.2.10.4, 4.2.10.5, 4.2.10.6, 4.2.10.7, 4.2.10.8, 4.2.10.9, 4.2.10.10, 4.3.1.1, 4.3.1.2, 4.3.1.3, 4.3.1.4, |
| 4.3.1.5, 4.3.1.6, 4.3.1.7, 4.3.1.8, 4.3.1.9, 4.3.1.10, 4.3.2.1, 4.3.2.2, 4.3.2.3, 4.3.2.4, 4.3.2.5, 4.3.2.6, 4.3.2.7, |
| 4.3.2.8, 4.3.2.9, 4.3.2.10, 4.3.3.1, 4.3.3.2, 4.3.3.3, 4.3.3.4, 4.3.3.5, 4.3.3.6, 4.3.3.7, 4.3.3.8, 4.3.3.9, 4.3.3.10, |
| 4.3.4.1, 4.3.4.2, 4.3.4.3, 4.3.4.4, 4.3.4.5, 4.3.4.6, 4.3.4.7, 4.3.4.8, 4.3.4.9, 4.3.4.10, 4.3.5.1, 4.3.5.2, 4.3.5.3, |
| 4.3.5.4, 4.3.5.5, 4.3.5.6, 4.3.5.7, 4.3.5.8, 4.3.5.9, 4.3.5.10, 4.3.6.1, 4.3.6.2, 4.3.6.3, 4.3.6.4, 4.3.6.5, 4.3.6.6, |
| 4.3.6.7, 4.3.6.8, 4.3.6.9, 4.3.6.10, 4.3.7.1, 4.3.7.2, 4.3.7.3, 4.3.7.4, 4.3.7.5, 4.3.7.6, 4.3.7.7, 4.3.7.8, 4.3.7.9, |
| 4.3.7.10, 4.3.8.1, 4.3.8.2, 4.3.8.3, 4.3.8.4, 4.3.8.5, 4.3.8.6, 4.3.8.7, 4.3.8.8, 4.3.8.9, 4.3.8.10, 4.3.9.1, 4.3.9.2, |
| 4.3.9.3, 4.3.9.4, 4.3.9.5, 4.3.9.6, 4.3.9.7, 4.3.9.8, 4.3.9.9, 4.3.9.10, 4.3.10.1, 4.3.10.2, 4.3.10.3, 4.3.10.4, 4.3.10.5, |
| 4.3.10.6, 4.3.10.7, 4.3.10.8, 4.3.10.9, 4.3.10.10, 4.4.1.1, 4.4.1.2, 4.4.1.3, 4.4.1.4, 4.4.1.5, 4.4.1.6, 4.4.1.7, 4.4.1.8, |
| 4.4.1.9, 4.4.1.10, 4.4.2.1, 4.4.2.2, 4.4.2.3, 4.4.2.4, 4.4.2.5, 4.4.2.6, 4.4.2.7, 4.4.2.8, 4.4.2.9, 4.4.2.10, 4.4.3.1, |
| 4.4.3.2, 4.4.3.3, 4.4.3.4, 4.4.3.5, 4.4.3.6, 4.4.3.7, 4.4.3.8, 4.4.3.9, 4.4.3.10, 4.4.4.1, 4.4.4.2, 4.4.4.3, 4.4.4.4, |
| 4.4.4.5, 4.4.4.6, 4.4.4.7, 4.4.4.8, 4.4.4.9, 4.4.4.10, 4.4.5.1, 4.4.5.2, 4.4.5.3, 4.4.5.4, 4.4.5.5, 4.4.5.6, 4.4.5.7, |
| 4.4.5.8, 4.4.5.9, 4.4.5.10, 4.4.6.1, 4.4.6.2, 4.4.6.3, 4.4.6.4, 4.4.6.5, 4.4.6.6, 4.4.6.7, 4.4.6.8, 4.4.6.9, 4.4.6.10, |
| 4.4.7.1, 4.4.7.2, 4.4.7.3, 4.4.7.4, 4.4.7.5, 4.4.7.6, 4.4.7.7, 4.4.7.8, 4.4.7.9, 4.4.7.10, 4.4.8.1, 4.4.8.2, 4.4.8.3, |
| 4.4.8.4, 4.4.8.5, 4.4.8.6, 4.4.8.7, 4.4.8.8, 4.4.8.9, 4.4.8.10, 4.4.9.1, 4.4.9.2, 4.4.9.3, 4.4.9.4, 4.4.9.5, 4.4.9.6, |
| 4.4.9.7, 4.4.9.8, 4.4.9.9, 4.4.9.10, 4.4.10.1, 4.4.10.2, 4.4.10.3, 4.4.10.4, 4.4.10.5, 4.4.10.6, 4.4.10.7, 4.4.10.8, |
| 4.4.10.9, 4.4.10.10, 4.5.1.1, 4.5.1.2, 4.5.1.3, 4.5.1.4, 4.5.1.5, 4.5.1.6, 4.5.1.7, 4.5.1.8, 4.5.1.9, 4.5.1.10, 4.5.2.1, |
| 4.5.2.2, 4.5.2.3, 4.5.2.4, 4.5.2.5, 4.5.2.6, 4.5.2.7, 4.5.2.8, 4.5.2.9, 4.5.2.10, 4.5.3.1, 4.5.3.2, 4.5.3.3, 4.5.3.4, |
| 4.5.3.5, 4.5.3.6, 4.5.3.7, 4.5.3.8, 4.5.3.9, 4.5.3.10, 4.5.4.1, 4.5.4.2, 4.5.4.3, 4.5.4.4, 4.5.4.5, 4.5.4.6, 4.5.4.7, |
| 4.5.4.8, 4.5.4.9, 4.5.4.10, 4.5.5.1, 4.5.5.2, 4.5.5.3, 4.5.5.4, 4.5.5.5, 4.5.5.6, 4.5.5.7, 4.5.5.8, 4.5.5.9, 4.5.5.10, |
| 4.5.6.1, 4.5.6.2, 4.5.6.3, 4.5.6.4, 4.5.6.5, 4.5.6.6, 4.5.6.7, 4.5.6.8, 4.5.6.9, 4.5.6.10, 4.5.7.1, 4.5.7.2, 4.5.7.3, |
| 4.5.7.4, 4.5.7.5, 4.5.7.6, 4.5.7.7, 4.5.7.8, 4.5.7.9, 4.5.7.10, 4.5.8.1, 4.5.8.2, 4.5.8.3, 4.5.8.4, 4.5.8.5, 4.5.8.6, |
| 4.5.8.7, 4.5.8.8, 4.5.8.9, 4.5.8.10, 4.5.9.1, 4.5.9.2, 4.5.9.3, 4.5.9.4, 4.5.9.5, 4.5.9.6, 4.5.9.7, 4.5.9.8, 4.5.9.9, |
| 4.5.9.10, 4.5.10.1, 4.5.10.2, 4.5.10.3, 4.5.10.4, 4.5.10.5, 4.5.10.6, 4.5.10.7, 4.5.10.8, 4.5.10.9, 4.5.10.10, 4.6.1.1, |
| 4.6.1.2, 4.6.1.3, 4.6.1.4, 4.6.1.5, 4.6.1.6, 4.6.1.7, 4.6.1.8, 4.6.1.9, 4.6.1.10, 4.6.2.1, 4.6.2.2, 4.6.2.3, 4.6.2.4, |
| 4.6.2.5, 4.6.2.6, 4.6.2.7, 4.6.2.8, 4.6.2.9, 4.6.2.10, 4.6.3.1, 4.6.3.2, 4.6.3.3, 4.6.3.4, 4.6.3.5, 4.6.3.6, 4.6.3.7, |
| 4.6.3.8, 4.6.3.9, 4.6.3.10, 4.6.4.1, 4.6.4.2, 4.6.4.3, 4.6.4.4, 4.6.4.5, 4.6.4.6, 4.6.4.7, 4.6.4.8, 4.6.4.9, 4.6.4.10, |
| 4.6.5.1, 4.6.5.2, 4.6.5.3, 4.6.5.4, 4.6.5.5, 4.6.5.6, 4.6.5.7, 4.6.5.8, 4.6.5.9, 4.6.5.10, 4.6.6.1, 4.6.6.2, 4.6.6.3, |
| 4.6.6.4, 4.6.6.5, 4.6.6.6, 4.6.6.7, 4.6.6.8, 4.6.6.9, 4.6.6.10, 4.6.7.1, 4.6.7.2, 4.6.7.3, 4.6.7.4, 4.6.7.5, 4.6.7.6, |
| 4.6.7.7, 4.6.7.8, 4.6.7.9, 4.6.7.10, 4.6.8.1, 4.6.8.2, 4.6.8.3, 4.6.8.4, 4.6.8.5, 4.6.8.6, 4.6.8.7, 4.6.8.8, 4.6.8.9, |
| 4.6.8.10, 4.6.9.1, 4.6.9.2, 4.6.9.3, 4.6.9.4, 4.6.9.5, 4.6.9.6, 4.6.9.7, 4.6.9.8, 4.6.9.9, 4.6.9.10, 4.6.10.1, 4.6.10.2, |
| 4.6.10.3, 4.6.10.4, 4.6.10.5, 4.6.10.6, 4.6.10.7, 4.6.10.8, 4.6.10.9, 4.6.10.10, 4.7.1.1, 4.7.1.2, 4.7.1.3, 4.7.1.4, |
| 4.7.1.5, 4.7.1.6, 4.7.1.7, 4.7.1.8, 4.7.1.9, 4.7.1.10, 4.7.2.1, 4.7.2.2, 4.7.2.3, 4.7.2.4, 4.7.2.5, 4.7.2.6, 4.7.2.7, |
| 4.7.2.8, 4.7.2.9, 4.7.2.10, 4.7.3.1, 4.7.3.2, 4.7.3.3, 4.7.3.4, 4.7.3.5, 4.7.3.6, 4.7.3.7, 4.7.3.8, 4.7.3.9, 4.7.3.10, |
| 4.7.4.1, 4.7.4.2, 4.7.4.3, 4.7.4.4, 4.7.4.5, 4.7.4.6, 4.7.4.7, 4.7.4.8, 4.7.4.9, 4.7.4.10, 4.7.5.1, 4.7.5.2, 4.7.5.3, |
| 4.7.5.4, 4.7.5.5, 4.7.5.6, 4.7.5.7, 4.7.5.8, 4.7.5.9, 4.7.5.10, 4.7.6.1, 4.7.6.2, 4.7.6.3, 4.7.6.4, 4.7.6.5, 4.7.6.6, |
| 4.7.6.7, 4.7.6.8, 4.7.6.9, 4.7.6.10, 4.7.7.1, 4.7.7.2, 4.7.7.3, 4.7.7.4, 4.7.7.5, 4.7.7.6, 4.7.7.7, 4.7.7.8, 4.7.7.9, |
| 4.7.7.10, 4.7.8.1, 4.7.8.2, 4.7.8.3, 4.7.8.4, 4.7.8.5, 4.7.8.6, 4.7.8.7, 4.7.8.8, 4.7.8.9, 4.7.8.10, 4.7.9.1, 4.7.9.2, |
| 4.7.9.3, 4.7.9.4, 4.7.9.5, 4.7.9.6, 4.7.9.7, 4.7.9.8, 4.7.9.9, 4.7.9.10, 4.7.10.1, 4.7.10.2, 4.7.10.3, 4.7.10.4, 4.7.10.5, |
| 4.7.10.6, 4.7.10.7, 4.7.10.8, 4.7.10.9, 4.7.10.10, 4.8.1.1, 4.8.1.2, 4.8.1.3, 4.8.1.4, 4.8.1.5, 4.8.1.6, 4.8.1.7, 4.8.1.8, |
| 4.8.1.9, 4.8.1.10, 4.8.2.1, 4.8.2.2, 4.8.2.3, 4.8.2.4, 4.8.2.5, 4.8.2.6, 4.8.2.7, 4.8.2.8, 4.8.2.9, 4.8.2.10, 4.8.3.1, |
| 4.8.3.2, 4.8.3.3, 4.8.3.4, 4.8.3.5, 4.8.3.6, 4.8.3.7, 4.8.3.8, 4.8.3.9, 4.8.3.10, 4.8.4.1, 4.8.4.2, 4.8.4.3, 4.8.4.4, |
| 4.8.4.5, 4.8.4.6, 4.8.4.7, 4.8.4.8, 4.8.4.9, 4.8.4.10, 4.8.5.1, 4.8.5.2, 4.8.5.3, 4.8.5.4, 4.8.5.5, 4.8.5.6, 4.8.5.7, |
| 4.8.5.8, 4.8.5.9, 4.8.5.10, 4.8.6.1, 4.8.6.2, 4.8.6.3, 4.8.6.4, 4.8.6.5, 4.8.6.6, 4.8.6.7, 4.8.6.8, 4.8.6.9, 4.8.6.10, |
| 4.8.7.1, 4.8.7.2, 4.8.7.3, 4.8.7.4, 4.8.7.5, 4.8.7.6, 4.8.7.7, 4.8.7.8, 4.8.7.9, 4.8.7.10, 4.8.8.1, 4.8.8.2, 4.8.8.3, |
| 4.8.8.4, 4.8.8.5, 4.8.8.6, 4.8.8.7, 4.8.8.8, 4.8.8.9, 4.8.8.10, 4.8.9.1, 4.8.9.2, 4.8.9.3, 4.8.9.4, 4.8.9.5, 4.8.9.6, |
| 4.8.9.7, 4.8.9.8, 4.8.9.9, 4.8.9.10, 4.8.10.1, 4.8.10.2, 4.8.10.3, 4.8.10.4, 4.8.10.5, 4.8.10.6, 4.8.10.7, 4.8.10.8, |
| 4.8.10.9, 4.8.10.10, 4.9.1.1, 4.9.1.2, 4.9.1.3, 4.9.1.4, 4.9.1.5, 4.9.1.6, 4.9.1.7, 4.9.1.8, 4.9.1.9, 4.9.1.10, 4.9.2.1, |
| 4.9.2.2, 4.9.2.3, 4.9.2.4, 4.9.2.5, 4.9.2.6, 4.9.2.7, 4.9.2.8, 4.9.2.9, 4.9.2.10, 4.9.3.1, 4.9.3.2, 4.9.3.3, 4.9.3.4, |
| 4.9.3.5, 4.9.3.6, 4.9.3.7, 4.9.3.8, 4.9.3.9, 4.9.3.10, 4.9.4.1, 4.9.4.2, 4.9.4.3, 4.9.4.4, 4.9.4.5, 4.9.4.6, 4.9.4.7, |
| 4.9.4.8, 4.9.4.9, 4.9.4.10, 4.9.5.1, 4.9.5.2, 4.9.5.3, 4.9.5.4, 4.9.5.5, 4.9.5.6, 4.9.5.7, 4.9.5.8, 4.9.5.9, 4.9.5.10, |
| 4.9.6.1, 4.9.6.2, 4.9.6.3, 4.9.6.4, 4.9.6.5, 4.9.6.6, 4.9.6.7, 4.9.6.8, 4.9.6.9, 4.9.6.10, 4.9.7.1, 4.9.7.2, 4.9.7.3, |
| 4.9.7.4, 4.9.7.5, 4.9.7.6, 4.9.7.7, 4.9.7.8, 4.9.7.9, 4.9.7.10, 4.9.8.1, 4.9.8.2, 4.9.8.3, 4.9.8.4, 4.9.8.5, 4.9.8.6, |
| 4.9.8.7, 4.9.8.8, 4.9.8.9, 4.9.8.10, 4.9.9.1, 4.9.9.2, 4.9.9.3, 4.9.9.4, 4.9.9.5, 4.9.9.6, 4.9.9.7, 4.9.9.8, 4.9.9.9, |
| 4.9.9.10, 4.9.10.1, 4.9.10.2, 4.9.10.3, 4.9.10.4, 4.9.10.5, 4.9.10.6, 4.9.10.7, 4.9.10.8, 4.9.10.9, 4.9.10.10, |
| 4.10.1.1, 4.10.1.2, 4.10.1.3, 4.10.1.4, 4.10.1.5, 4.10.1.6, 4.10.1.7, 4.10.1.8, 4.10.1.9, 4.10.1.10, 4.10.2.1, |
| 4.10.2.2, 4.10.2.3, 4.10.2.4, 4.10.2.5, 4.10.2.6, 4.10.2.7, 4.10.2.8, 4.10.2.9, 4.10.2.10, 4.10.3.1, 4.10.3.2, |
| 4.10.3.3, 4.10.3.4, 4.10.3.5, 4.10.3.6, 4.10.3.7, 4.10.3.8, 4.10.3.9, 4.10.3.10, 4.10.4.1, 4.10.4.2, 4.10.4.3, |
| 4.10.4.4, 4.10.4.5, 4.10.4.6, 4.10.4.7, 4.10.4.8, 4.10.4.9, 4.10.4.10, 4.10.5.1, 4.10.5.2, 4.10.5.3, 4.10.5.4, |
| 4.10.5.5, 4.10.5.6, 4.10.5.7, 4.10.5.8, 4.10.5.9, 4.10.5.10, 4.10.6.1, 4.10.6.2, 4.10.6.3, 4.10.6.4, 4.10.6.5, |
| 4.10.6.6, 4.10.6.7, 4.10.6.8, 4.10.6.9, 4.10.6.10, 4.10.7.1, 4.10.7.2, 4.10.7.3, 4.10.7.4, 4.10.7.5, 4.10.7.6, |
| 4.10.7.7, 4.10.7.8, 4.10.7.9, 4.10.7.10, 4.10.8.1, 4.10.8.2, 4.10.8.3, 4.10.8.4, 4.10.8.5, 4.10.8.6, 4.10.8.7, |
| 4.10.8.8, 4.10.8.9, 4.10.8.10, 4.10.9.1, 4.10.9.2, 4.10.9.3, 4.10.9.4, 4.10.9.5, 4.10.9.6, 4.10.9.7, 4.10.9.8, |
| 4.10.9.9, 4.10.9.10, 4.10.10.1, 4.10.10.2, 4.10.10.3, 4.10.10.4, 4.10.10.5, 4.10.10.6, 4.10.10.7, 4.10.10.8, |
| 4.10.10.9, 4.10.10.10, 5.1.1.1, 5.1.1.2, 5.1.1.3, 5.1.1.4, 5.1.1.5, 5.1.1.6, 5.1.1.7, 5.1.1.8, 5.1.1.9, 5.1.1.10, 5.1.2.1, |
| 5.1.2.2, 5.1.2.3, 5.1.2.4, 5.1.2.5, 5.1.2.6, 5.1.2.7, 5.1.2.8, 5.1.2.9, 5.1.2.10, 5.1.3.1, 5.1.3.2, 5.1.3.3, 5.1.3.4, |
| 5.1.3.5, 5.1.3.6, 5.1.3.7, 5.1.3.8, 5.1.3.9, 5.1.3.10, 5.1.4.1, 5.1.4.2, 5.1.4.3, 5.1.4.4, 5.1.4.5, 5.1.4.6, 5.1.4.7, |
| 5.1.4.8, 5.1.4.9, 5.1.4.10, 5.1.5.1, 5.1.5.2, 5.1.5.3, 5.1.5.4, 5.1.5.5, 5.1.5.6, 5.1.5.7, 5.1.5.8, 5.1.5.9, 5.1.5.10, |
| 5.1.6.1, 5.1.6.2, 5.1.6.3, 5.1.6.4, 5.1.6.5, 5.1.6.6, 5.1.6.7, 5.1.6.8, 5.1.6.9, 5.1.6.10, 5.1.7.1, 5.1.7.2, 5.1.7.3, |
| 5.1.7.4, 5.1.7.5, 5.1.7.6, 5.1.7.7, 5.1.7.8, 5.1.7.9, 5.1.7.10, 5.1.8.1, 5.1.8.2, 5.1.8.3, 5.1.8.4, 5.1.8.5, 5.1.8.6, |
| 5.1.8.7, 5.1.8.8, 5.1.8.9, 5.1.8.10, 5.1.9.1, 5.1.9.2, 5.1.9.3, 5.1.9.4, 5.1.9.5, 5.1.9.6, 5.1.9.7, 5.1.9.8, 5.1.9.9, |
| 5.1.9.10, 5.1.10.1, 5.1.10.2, 5.1.10.3, 5.1.10.4, 5.1.10.5, 5.1.10.6, 5.1.10.7, 5.1.10.8, 5.1.10.9, 5.1.10.10, 5.2.1.1, |
| 5.2.1.2, 5.2.1.3, 5.2.1.4, 5.2.1.5, 5.2.1.6, 5.2.1.7, 5.2.1.8, 5.2.1.9, 5.2.1.10, 5.2.2.1, 5.2.2.2, 5.2.2.3, 5.2.2.4, |
| 5.2.2.5, 5.2.2.6, 5.2.2.7, 5.2.2.8, 5.2.2.9, 5.2.2.10, 5.2.3.1, 5.2.3.2, 5.2.3.3, 5.2.3.4, 5.2.3.5, 5.2.3.6, 5.2.3.7, |
| 5.2.3.8, 5.2.3.9, 5.2.3.10, 5.2.4.1, 5.2.4.2, 5.2.4.3, 5.2.4.4, 5.2.4.5, 5.2.4.6, 5.2.4.7, 5.2.4.8, 5.2.4.9, 5.2.4.10, |
| 5.2.5.1, 5.2.5.2, 5.2.5.3, 5.2.5.4, 5.2.5.5, 5.2.5.6, 5.2.5.7, 5.2.5.8, 5.2.5.9, 5.2.5.10, 5.2.6.1, 5.2.6.2, 5.2.6.3, |
| 5.2.6.4, 5.2.6.5, 5.2.6.6, 5.2.6.7, 5.2.6.8, 5.2.6.9, 5.2.6.10, 5.2.7.1, 5.2.7.2, 5.2.7.3, 5.2.7.4, 5.2.7.5, 5.2.7.6, |
| 5.2.7.7, 5.2.7.8, 5.2.7.9, 5.2.7.10, 5.2.8.1, 5.2.8.2, 5.2.8.3, 5.2.8.4, 5.2.8.5, 5.2.8.6, 5.2.8.7, 5.2.8.8, 5.2.8.9, |
| 5.2.8.10, 5.2.9.1, 5.2.9.2, 5.2.9.3, 5.2.9.4, 5.2.9.5, 5.2.9.6, 5.2.9.7, 5.2.9.8, 5.2.9.9, 5.2.9.10, 5.2.10.1, 5.2.10.2, |
| 5.2.10.3, 5.2.10.4, 5.2.10.5, 5.2.10.6, 5.2.10.7, 5.2.10.8, 5.2.10.9, 5.2.10.10, 5.3.1.1, 5.3.1.2, 5.3.1.3, 5.3.1.4, |
| 5.3.1.5, 5.3.1.6, 5.3.1.7, 5.3.1.8, 5.3.1.9, 5.3.1.10, 5.3.2.1, 5.3.2.2, 5.3.2.3, 5.3.2.4, 5.3.2.5, 5.3.2.6, 5.3.2.7, |
| 5.3.2.8, 5.3.2.9, 5.3.2.10, 5.3.3.1, 5.3.3.2, 5.3.3.3, 5.3.3.4, 5.3.3.5, 5.3.3.6, 5.3.3.7, 5.3.3.8, 5.3.3.9, 5.3.3.10, |
| 5.3.4.1, 5.3.4.2, 5.3.4.3, 5.3.4.4, 5.3.4.5, 5.3.4.6, 5.3.4.7, 5.3.4.8, 5.3.4.9, 5.3.4.10, 5.3.5.1, 5.3.5.2, 5.3.5.3, |
| 5.3.5.4, 5.3.5.5, 5.3.5.6, 5.3.5.7, 5.3.5.8, 5.3.5.9, 5.3.5.10, 5.3.6.1, 5.3.6.2, 5.3.6.3, 5.3.6.4, 5.3.6.5, 5.3.6.6, |
| 5.3.6.7, 5.3.6.8, 5.3.6.9, 5.3.6.10, 5.3.7.1, 5.3.7.2, 5.3.7.3, 5.3.7.4, 5.3.7.5, 5.3.7.6, 5.3.7.7, 5.3.7.8, 5.3.7.9, |
| 5.3.7.10, 5.3.8.1, 5.3.8.2, 5.3.8.3, 5.3.8.4, 5.3.8.5, 5.3.8.6, 5.3.8.7, 5.3.8.8, 5.3.8.9, 5.3.8.10, 5.3.9.1, 5.3.9.2, |
| 5.3.9.3, 5.3.9.4, 5.3.9.5, 5.3.9.6, 5.3.9.7, 5.3.9.8, 5.3.9.9, 5.3.9.10, 5.3.10.1, 5.3.10.2, 5.3.10.3, 5.3.10.4, 5.3.10.5, |
| 5.3.10.6, 5.3.10.7, 5.3.10.8, 5.3.10.9, 5.3.10.10, 5.4.1.1, 5.4.1.2, 5.4.1.3, 5.4.1.4, 5.4.1.5, 5.4.1.6, 5.4.1.7, 5.4.1.8, |
| 5.4.1.9, 5.4.1.10, 5.4.2.1, 5.4.2.2, 5.4.2.3, 5.4.2.4, 5.4.2.5, 5.4.2.6, 5.4.2.7, 5.4.2.8, 5.4.2.9, 5.4.2.10, 5.4.3.1, |
| 5.4.3.2, 5.4.3.3, 5.4.3.4, 5.4.3.5, 5.4.3.6, 5.4.3.7, 5.4.3.8, 5.4.3.9, 5.4.3.10, 5.4.4.1, 5.4.4.2, 5.4.4.3, 5.4.4.4, |
| 5.4.4.5, 5.4.4.6, 5.4.4.7, 5.4.4.8, 5.4.4.9, 5.4.4.10, 5.4.5.1, 5.4.5.2, 5.4.5.3, 5.4.5.4, 5.4.5.5, 5.4.5.6, 5.4.5.7, |
| 5.4.5.8, 5.4.5.9, 5.4.5.10, 5.4.6.1, 5.4.6.2, 5.4.6.3, 5.4.6.4, 5.4.6.5, 5.4.6.6, 5.4.6.7, 5.4.6.8, 5.4.6.9, 5.4.6.10, |
| 5.4.7.1, 5.4.7.2, 5.4.7.3, 5.4.7.4, 5.4.7.5, 5.4.7.6, 5.4.7.7, 5.4.7.8, 5.4.7.9, 5.4.7.10, 5.4.8.1, 5.4.8.2, 5.4.8.3, |
| 5.4.8.4, 5.4.8.5, 5.4.8.6, 5.4.8.7, 5.4.8.8, 5.4.8.9, 5.4.8.10, 5.4.9.1, 5.4.9.2, 5.4.9.3, 5.4.9.4, 5.4.9.5, 5.4.9.6, |
| 5.4.9.7, 5.4.9.8, 5.4.9.9, 5.4.9.10, 5.4.10.1, 5.4.10.2, 5.4.10.3, 5.4.10.4, 5.4.10.5, 5.4.10.6, 5.4.10.7, 5.4.10.8, |
| 5.4.10.9, 5.4.10.10, 5.5.1.1, 5.5.1.2, 5.5.1.3, 5.5.1.4, 5.5.1.5, 5.5.1.6, 5.5.1.7, 5.5.1.8, 5.5.1.9, 5.5.1.10, 5.5.2.1, |
| 5.5.2.2, 5.5.2.3, 5.5.2.4, 5.5.2.5, 5.5.2.6, 5.5.2.7, 5.5.2.8, 5.5.2.9, 5.5.2.10, 5.5.3.1, 5.5.3.2, 5.5.3.3, 5.5.3.4, |
| 5.5.3.5, 5.5.3.6, 5.5.3.7, 5.5.3.8, 5.5.3.9, 5.5.3.10, 5.5.4.1, 5.5.4.2, 5.5.4.3, 5.5.4.4, 5.5.4.5, 5.5.4.6, 5.5.4.7, |
| 5.5.4.8, 5.5.4.9, 5.5.4.10, 5.5.5.1, 5.5.5.2, 5.5.5.3, 5.5.5.4, 5.5.5.5, 5.5.5.6, 5.5.5.7, 5.5.5.8, 5.5.5.9, 5.5.5.10, |
| 5.5.6.1, 5.5.6.2, 5.5.6.3, 5.5.6.4, 5.5.6.5, 5.5.6.6, 5.5.6.7, 5.5.6.8, 5.5.6.9, 5.5.6.10, 5.5.7.1, 5.5.7.2, 5.5.7.3, |
| 5.5.7.4, 5.5.7.5, 5.5.7.6, 5.5.7.7, 5.5.7.8, 5.5.7.9, 5.5.7.10, 5.5.8.1, 5.5.8.2, 5.5.8.3, 5.5.8.4, 5.5.8.5, 5.5.8.6, |
| 5.5.8.7, 5.5.8.8, 5.5.8.9, 5.5.8.10, 5.5.9.1, 5.5.9.2, 5.5.9.3, 5.5.9.4, 5.5.9.5, 5.5.9.6, 5.5.9.7, 5.5.9.8, 5.5.9.9, |
| 5.5.9.10, 5.5.10.1, 5.5.10.2, 5.5.10.3, 5.5.10.4, 5.5.10.5, 5.5.10.6, 5.5.10.7, 5.5.10.8, 5.5.10.9, 5.5.10.10, 5.6.1.1, |
| 5.6.1.2, 5.6.1.3, 5.6.1.4, 5.6.1.5, 5.6.1.6, 5.6.1.7, 5.6.1.8, 5.6.1.9, 5.6.1.10, 5.6.2.1, 5.6.2.2, 5.6.2.3, 5.6.2.4, |
| 5.6.2.5, 5.6.2.6, 5.6.2.7, 5.6.2.8, 5.6.2.9, 5.6.2.10, 5.6.3.1, 5.6.3.2, 5.6.3.3, 5.6.3.4, 5.6.3.5, 5.6.3.6, 5.6.3.7, |
| 5.6.3.8, 5.6.3.9, 5.6.3.10, 5.6.4.1, 5.6.4.2, 5.6.4.3, 5.6.4.4, 5.6.4.5, 5.6.4.6, 5.6.4.7, 5.6.4.8, 5.6.4.9, 5.6.4.10, |
| 5.6.5.1, 5.6.5.2, 5.6.5.3, 5.6.5.4, 5.6.5.5, 5.6.5.6, 5.6.5.7, 5.6.5.8, 5.6.5.9, 5.6.5.10, 5.6.6.1, 5.6.6.2, 5.6.6.3, |
| 5.6.6.4, 5.6.6.5, 5.6.6.6, 5.6.6.7, 5.6.6.8, 5.6.6.9, 5.6.6.10, 5.6.7.1, 5.6.7.2, 5.6.7.3, 5.6.7.4, 5.6.7.5, 5.6.7.6, |
| 5.6.7.7, 5.6.7.8, 5.6.7.9, 5.6.7.10, 5.6.8.1, 5.6.8.2, 5.6.8.3, 5.6.8.4, 5.6.8.5, 5.6.8.6, 5.6.8.7, 5.6.8.8, 5.6.8.9, |
| 5.6.8.10, 5.6.9.1, 5.6.9.2, 5.6.9.3, 5.6.9.4, 5.6.9.5, 5.6.9.6, 5.6.9.7, 5.6.9.8, 5.6.9.9, 5.6.9.10, 5.6.10.1, 5.6.10.2, |
| 5.6.10.3, 5.6.10.4, 5.6.10.5, 5.6.10.6, 5.6.10.7, 5.6.10.8, 5.6.10.9, 5.6.10.10, 5.7.1.1, 5.7.1.2, 5.7.1.3, 5.7.1.4, |
| 5.7.1.5, 5.7.1.6, 5.7.1.7, 5.7.1.8, 5.7.1.9, 5.7.1.10, 5.7.2.1, 5.7.2.2, 5.7.2.3, 5.7.2.4, 5.7.2.5, 5.7.2.6, 5.7.2.7, |
| 5.7.2.8, 5.7.2.9, 5.7.2.10, 5.7.3.1, 5.7.3.2, 5.7.3.3, 5.7.3.4, 5.7.3.5, 5.7.3.6, 5.7.3.7, 5.7.3.8, 5.7.3.9, 5.7.3.10, |
| 5.7.4.1, 5.7.4.2, 5.7.4.3, 5.7.4.4, 5.7.4.5, 5.7.4.6, 5.7.4.7, 5.7.4.8, 5.7.4.9, 5.7.4.10, 5.7.5.1, 5.7.5.2, 5.7.5.3, |
| 5.7.5.4, 5.7.5.5, 5.7.5.6, 5.7.5.7, 5.7.5.8, 5.7.5.9, 5.7.5.10, 5.7.6.1, 5.7.6.2, 5.7.6.3, 5.7.6.4, 5.7.6.5, 5.7.6.6, |
| 5.7.6.7, 5.7.6.8, 5.7.6.9, 5.7.6.10, 5.7.7.1, 5.7.7.2, 5.7.7.3, 5.7.7.4, 5.7.7.5, 5.7.7.6, 5.7.7.7, 5.7.7.8, 5.7.7.9, |
| 5.7.7.10, 5.7.8.1, 5.7.8.2, 5.7.8.3, 5.7.8.4, 5.7.8.5, 5.7.8.6, 5.7.8.7, 5.7.8.8, 5.7.8.9, 5.7.8.10, 5.7.9.1, 5.7.9.2, |
| 5.7.9.3, 5.7.9.4, 5.7.9.5, 5.7.9.6, 5.7.9.7, 5.7.9.8, 5.7.9.9, 5.7.9.10, 5.7.10.1, 5.7.10.2, 5.7.10.3, 5.7.10.4, 5.7.10.5, |
| 5.7.10.6, 5.7.10.7, 5.7.10.8, 5.7.10.9, 5.7.10.10 ,5.8.1.1, 5.8.1.2, 5.8.1.3, 5.8.1.4, 5.8.1.5, 5.8.1.6, 5.8.1.7, 5.8.1.8, |
| 5.8.1.9, 5.8.1.10, 5.8.2.1, 5.8.2.2, 5.8.2.3, 5.8.2.4, 5.8.2.5, 5.8.2.6, 5.8.2.7, 5.8.2.8, 5.8.2.9, 5.8.2.10, 5.8.3.1, |
| 5.8.3.2, 5.8.3.3, 5.8.3.4, 5.8.3.5, 5.8.3.6, 5.8.3.7, 5.8.3.8, 5.8.3.9, 5.8.3.10, 5.8.4.1, 5.8.4.2, 5.8.4.3, 5.8.4.4, |
| 5.8.4.5, 5.8.4.6, 5.8.4.7, 5.8.4.8, 5.8.4.9, 5.8.4.10, 5.8.5.1, 5.8.5.2, 5.8.5.3, 5.8.5.4, 5.8.5.5, 5.8.5.6, 5.8.5.7, |
| 5.8.5.8, 5.8.5.9, 5.8.5.10, 5.8.6.1, 5.8.6.2, 5.8.6.3, 5.8.6.4, 5.8.6.5, 5.8.6.6, 5.8.6.7, 5.8.6.8, 5.8.6.9, 5.8.6.10, |
| 5.8.7.1, 5.8.7.2, 5.8.7.3, 5.8.7.4, 5.8.7.5, 5.8.7.6, 5.8.7.7, 5.8.7.8, 5.8.7.9, 5.8.7.10, 5.8.8.1, 5.8.8.2, 5.8.8.3, |
| 5.8.8.4, 5.8.8.5, 5.8.8.6, 5.8.8.7, 5.8.8.8, 5.8.8.9, 5.8.8.10, 5.8.9.1, 5.8.9.2, 5.8.9.3, 5.8.9.4, 5.8.9.5, 5.8.9.6, |
| 5.8.9.7, 5.8.9.8, 5.8.9.9, 5.8.9.10, 5.8.10.1, 5.8.10.2, 5.8.10.3, 5.8.10.4, 5.8.10.5, 5.8.10.6, 5.8.10.7, 5.8.10.8, |
| 5.8.10.9, 5.8.10.10, 5.9.1.1, 5.9.1.2, 5.9.1.3, 5.9.1.4, 5.9.1.5, 5.9.1.6, 5.9.1.7, 5.9.1.8, 5.9.1.9, 5.9.1.10, 5.9.2.1, |
| 5.9.2.2, 5.9.2.3, 5.9.2.4, 5.9.2.5, 5.9.2.6, 5.9.2.7, 5.9.2.8, 5.9.2.9, 5.9.2.10, 5.9.3.1, 5.9.3.2, 5.9.3.3, 5.9.3.4, |
| 5.9.3.5, 5.9.3.6, 5.9.3.7, 5.9.3.8, 5.9.3.9, 5.9.3.10, 5.9.4.1, 5.9.4.2, 5.9.4.3, 5.9.4.4, 5.9.4.5, 5.9.4.6, 5.9.4.7, |
| 5.9.4.8, 5.9.4.9, 5.9.4.10, 5.9.5.1, 5.9.5.2, 5.9.5.3, 5.9.5.4, 5.9.5.5, 5.9.5.6, 5.9.5.7, 5.9.5.8, 5.9.5.9, 5.9.5.10, |
| 5.9.6.1, 5.9.6.2, 5.9.6.3, 5.9.6.4, 5.9.6.5, 5.9.6.6, 5.9.6.7, 5.9.6.8, 5.9.6.9, 5.9.6.10, 5.9.7.1, 5.9.7.2, 5.9.7.3, |
| 5.9.7.4, 5.9.7.5, 5.9.7.6, 5.9.7.7, 5.9.7.8, 5.9.7.9, 5.9.7.10, 5.9.8.1, 5.9.8.2, 5.9.8.3, 5.9.8.4, 5.9.8.5, 5.9.8.6, |
| 5.9.8.7, 5.9.8.8, 5.9.8.9, 5.9.8.10, 5.9.9.1, 5.9.9.2, 5.9.9.3, 5.9.9.4, 5.9.9.5, 5.9.9.6, 5.9.9.7, 5.9.9.8, 5.9.9.9, |
| 5.9.9.10, 5.9.10.1, 5.9.10.2, 5.9.10.3, 5.9.10.4, 5.9.10.5, 5.9.10.6, 5.9.10.7, 5.9.10.8, 5.9.10.9, 5.9.10.10, |
| 5.10.1.1, 5.10.1.2, 5.10.1.3, 5.10.1.4, 5.10.1.5, 5.10.1.6, 5.10.1.7, 5.10.1.8, 5.10.1.9, 5.10.1.10, 5.10.2.1, |
| 5.10.2.2, 5.10.2.3, 5.10.2.4, 5.10.2.5, 5.10.2.6, 5.10.2.7, 5.10.2.8, 5.10.2.9, 5.10.2.10, 5.10.3.1, 5.10.3.2, |
| 5.10.3.3, 5.10.3.4, 5.10.3.5, 5.10.3.6, 5.10.3.7, 5.10.3.8, 5.10.3.9, 5.10.3.10, 5.10.4.1, 5.10.4.2, 5.10.4.3, |
| 5.10.4.4, 5.10.4.5, 5.10.4.6, 5.10.4.7, 5.10.4.8, 5.10.4.9, 5.10.4.10, 5.10.5.1, 5.10.5.2, 5.10.5.3, 5.10.5.4, |
| 5.10.5.5, 5.10.5.6, 5.10.5.7, 5.10.5.8, 5.10.5.9, 5.10.5.10, 5.10.6.1, 5.10.6.2, 5.10.6.3, 5.10.6.4, 5.10.6.5, |
| 5.10.6.6, 5.10.6.7, 5.10.6.8, 5.10.6.9, 5.10.6.10, 5.10.7.1, 5.10.7.2, 5.10.7.3, 5.10.7.4, 5.10.7.5, 5.10.7.6, |
| 5.10.7.7, 5.10.7.8, 5.10.7.9, 5.10.7.10, 5.10.8.1, 5.10.8.2, 5.10.8.3, 5.10.8.4, 5.10.8.5, 5.10.8.6, 5.10.8.7, |
| 5.10.8.8, 5.10.8.9, 5.10.8.10, 5.10.9.1, 5.10.9.2, 5.10.9.3, 5.10.9.4, 5.10.9.5, 5.10.9.6, 5.10.9.7, 5.10.9.8, |
| 5.10.9.9, 5.10.9.10, 5.10.10.1, 5.10.10.2, 5.10.10.3, 5.10.10.4, 5.10.10.5, 5.10.10.6, 5.10.10.7, 5.10.10.8, |
| 5.10.10.9, 5.10.10.10, 6.1.1.1, 6.1.1.2, 6.1.1.3, 6.1.1.4, 6.1.1.5, 6.1.1.6, 6.1.1.7, 6.1.1.8, 6.1.1.9, 6.1.1.10, 6.1.2.1, |
| 6.1.2.2, 6.1.2.3, 6.1.2.4, 6.1.2.5, 6.1.2.6, 6.1.2.7, 6.1.2.8, 6.1.2.9, 6.1.2.10, 6.1.3.1, 6.1.3.2, 6.1.3.3, 6.1.3.4, |
| 6.1.3.5, 6.1.3.6, 6.1.3.7, 6.1.3.8, 6.1.3.9, 6.1.3.10, 6.1.4.1, 6.1.4.2, 6.1.4.3, 6.1.4.4, 6.1.4.5, 6.1.4.6, 6.1.4.7, |
| 6.1.4.8, 6.1.4.9, 6.1.4.10, 6.1.5.1, 6.1.5.2, 6.1.5.3, 6.1.5.4, 6.1.5.5, 6.1.5.6, 6.1.5.7, 6.1.5.8, 6.1.5.9, 6.1.5.10, |
| 6.1.6.1, 6.1.6.2, 6.1.6.3, 6.1.6.4, 6.1.6.5, 6.1.6.6, 6.1.6.7, 6.1.6.8, 6.1.6.9, 6.1.6.10, 6.1.7.1, 6.1.7.2, 6.1.7.3, |
| 6.1.7.4, 6.1.7.5, 6.1.7.6, 6.1.7.7, 6.1.7.8, 6.1.7.9, 6.1.7.10, 6.1.8.1, 6.1.8.2, 6.1.8.3, 6.1.8.4, 6.1.8.5, 6.1.8.6, |
| 6.1.8.7, 6.1.8.8, 6.1.8.9, 6.1.8.10, 6.1.9.1, 6.1.9.2, 6.1.9.3, 6.1.9.4, 6.1.9.5, 6.1.9.6, 6.1.9.7, 6.1.9.8, 6.1.9.9, |
| 6.1.9.10, 6.1.10.1, 6.1.10.2, 6.1.10.3, 6.1.10.4, 6.1.10.5, 6.1.10.6, 6.1.10.7, 6.1.10.8, 6.1.10.9, 6.1.10.10, 6.2.1.1, |
| 6.2.1.2, 6.2.1.3, 6.2.1.4, 6.2.1.5, 6.2.1.6, 6.2.1.7, 6.2.1.8, 6.2.1.9, 6.2.1.10, 6.2.2.1, 6.2.2.2, 6.2.2.3, 6.2.2.4, |
| 6.2.2.5, 6.2.2.6, 6.2.2.7, 6.2.2.8, 6.2.2.9, 6.2.2.10, 6.2.3.1, 6.2.3.2, 6.2.3.3, 6.2.3.4, 6.2.3.5, 6.2.3.6, 6.2.3.7, |
| 6.2.3.8, 6.2.3.9, 6.2.3.10, 6.2.4.1, 6.2.4.2, 6.2.4.3, 6.2.4.4, 6.2.4.5, 6.2.4.6, 6.2.4.7, 6.2.4.8, 6.2.4.9, 6.2.4.10, |
| 6.2.5.1, 6.2.5.2, 6.2.5.3, 6.2.5.4, 6.2.5.5, 6.2.5.6, 6.2.5.7, 6.2.5.8, 6.2.5.9, 6.2.5.10, 6.2.6.1, 6.2.6.2, 6.2.6.3, |
| 6.2.6.4, 6.2.6.5, 6.2.6.6, 6.2.6.7, 6.2.6.8, 6.2.6.9, 6.2.6.10, 6.2.7.1, 6.2.7.2, 6.2.7.3, 6.2.7.4, 6.2.7.5, 6.2.7.6, |
| 6.2.7.7, 6.2.7.8, 6.2.7.9, 6.2.7.10, 6.2.8.1, 6.2.8.2, 6.2.8.3, 6.2.8.4, 6.2.8.5, 6.2.8.6, 6.2.8.7, 6.2.8.8, 6.2.8.9, |
| 6.2.8.10, 6.2.9.1, 6.2.9.2, 6.2.9.3, 6.2.9.4, 6.2.9.5, 6.2.9.6, 6.2.9.7, 6.2.9.8, 6.2.9.9, 6.2.9.10, 6.2.10.1, 6.2.10.2, |
| 6.2.10.3, 6.2.10.4, 6.2.10.5, 6.2.10.6, 6.2.10.7, 6.2.10.8, 6.2.10.9, 6.2.10.10, 6.3.1.1, 6.3.1.2, 6.3.1.3, 6.3.1.4, |
| 6.3.1.5, 6.3.1.6, 6.3.1.7, 6.3.1.8, 6.3.1.9, 6.3.1.10, 6.3.2.1, 6.3.2.2, 6.3.2.3, 6.3.2.4, 6.3.2.5, 6.3.2.6, 6.3.2.7, |
| 6.3.2.8, 6.3.2.9, 6.3.2.10, 6.3.3.1, 6.3.3.2, 6.3.3.3, 6.3.3.4, 6.3.3.5, 6.3.3.6, 6.3.3.7, 6.3.3.8, 6.3.3.9, 6.3.3.10, |
| 6.3.4.1, 6.3.4.2, 6.3.4.3, 6.3.4.4, 6.3.4.5, 6.3.4.6, 6.3.4.7, 6.3.4.8, 6.3.4.9, 6.3.4.10, 6.3.5.1, 6.3.5.2, 6.3.5.3, |
| 6.3.5.4, 6.3.5.5, 6.3.5.6, 6.3.5.7, 6.3.5.8, 6.3.5.9, 6.3.5.10, 6.3.6.1, 6.3.6.2, 6.3.6.3, 6.3.6.4, 6.3.6.5, 6.3.6.6, |
| 6.3.6.7, 6.3.6.8, 6.3.6.9, 6.3.6.10, 6.3.7.1, 6.3.7.2, 6.3.7.3, 6.3.7.4, 6.3.7.5, 6.3.7.6, 6.3.7.7, 6.3.7.8, 6.3.7.9, |
| 6.3.7.10, 6.3.8.1, 6.3.8.2, 6.3.8.3, 6.3.8.4, 6.3.8.5, 6.3.8.6, 6.3.8.7, 6.3.8.8, 6.3.8.9, 6.3.8.10, 6.3.9.1, 6.3.9.2, |
| 6.3.9.3, 6.3.9.4, 6.3.9.5, 6.3.9.6, 6.3.9.7, 6.3.9.8, 6.3.9.9, 6.3.9.10, 6.3.10.1, 6.3.10.2, 6.3.10.3, 6.3.10.4, 6.3.10.5, |
| 6.3.10.6, 6.3.10.7, 6.3.10.8, 6.3.10.9, 6.3.10.10, 6.4.1.1, 6.4.1.2, 6.4.1.3, 6.4.1.4, 6.4.1.5, 6.4.1.6, 6.4.1.7, 6.4.1.8, |
| 6.4.1.9, 6.4.1.10, 6.4.2.1, 6.4.2.2, 6.4.2.3, 6.4.2.4, 6.4.2.5, 6.4.2.6, 6.4.2.7, 6.4.2.8, 6.4.2.9, 6.4.2.10, 6.4.3.1, |
| 6.4.3.2, 6.4.3.3, 6.4.3.4, 6.4.3.5, 6.4.3.6, 6.4.3.7, 6.4.3.8, 6.4.3.9, 6.4.3.10, 6.4.4.1, 6.4.4.2, 6.4.4.3, 6.4.4.4, |
| 6.4.4.5, 6.4.4.6, 6.4.4.7, 6.4.4.8, 6.4.4.9, 6.4.4.10, 6.4.5.1, 6.4.5.2, 6.4.5.3, 6.4.5.4, 6.4.5.5, 6.4.5.6, 6.4.5.7, |
| 6.4.5.8, 6.4.5.9, 6.4.5.10, 6.4.6.1, 6.4.6.2, 6.4.6.3, 6.4.6.4, 6.4.6.5, 6.4.6.6, 6.4.6.7, 6.4.6.8, 6.4.6.9, 6.4.6.10, |
| 6.4.7.1, 6.4.7.2, 6.4.7.3, 6.4.7.4, 6.4.7.5, 6.4.7.6, 6.4.7.7, 6.4.7.8, 6.4.7.9, 6.4.7.10, 6.4.8.1, 6.4.8.2, 6.4.8.3, |
| 6.4.8.4, 6.4.8.5, 6.4.8.6, 6.4.8.7, 6.4.8.8, 6.4.8.9, 6.4.8.10, 6.4.9.1, 6.4.9.2, 6.4.9.3, 6.4.9.4, 6.4.9.5, 6.4.9.6, |
| 6.4.9.7, 6.4.9.8, 6.4.9.9, 6.4.9.10, 6.4.10.1, 6.4.10.2, 6.4.10.3, 6.4.10.4, 6.4.10.5, 6.4.10.6, 6.4.10.7, 6.4.10.8, |
| 6.4.10.9, 6.4.10.10, 6.5.1.1, 6.5.1.2, 6.5.1.3, 6.5.1.4, 6.5.1.5, 6.5.1.6, 6.5.1.7, 6.5.1.8, 6.5.1.9, 6.5.1.10, 6.5.2.1, |
| 6.5.2.2, 6.5.2.3, 6.5.2.4, 6.5.2.5, 6.5.2.6, 6.5.2.7, 6.5.2.8, 6.5.2.9, 6.5.2.10, 6.5.3.1, 6.5.3.2, 6.5.3.3, 6.5.3.4, |
| 6.5.3.5, 6.5.3.6, 6.5.3.7, 6.5.3.8, 6.5.3.9, 6.5.3.10, 6.5.4.1, 6.5.4.2, 6.5.4.3, 6.5.4.4, 6.5.4.5, 6.5.4.6, 6.5.4.7, |
| 6.5.4.8, 6.5.4.9, 6.5.4.10, 6.5.5.1, 6.5.5.2, 6.5.5.3, 6.5.5.4, 6.5.5.5, 6.5.5.6, 6.5.5.7, 6.5.5.8, 6.5.5.9, 6.5.5.10, |
| 6.5.6.1, 6.5.6.2, 6.5.6.3, 6.5.6.4, 6.5.6.5, 6.5.6.6, 6.5.6.7, 6.5.6.8, 6.5.6.9, 6.5.6.10, 6.5.7.1, 6.5.7.2, 6.5.7.3, |
| 6.5.7.4, 6.5.7.5, 6.5.7.6, 6.5.7.7, 6.5.7.8, 6.5.7.9, 6.5.7.10, 6.5.8.1, 6.5.8.2, 6.5.8.3, 6.5.8.4, 6.5.8.5, 6.5.8.6, |
| 6.5.8.7, 6.5.8.8, 6.5.8.9, 6.5.8.10, 6.5.9.1, 6.5.9.2, 6.5.9.3, 6.5.9.4, 6.5.9.5, 6.5.9.6, 6.5.9.7, 6.5.9.8, 6.5.9.9, |
| 6.5.9.10, 6.5.10.1, 6.5.10.2, 6.5.10.3, 6.5.10.4, 6.5.10.5, 6.5.10.6, 6.5.10.7, 6.5.10.8, 6.5.10.9, 6.5.10.10, 6.6.1.1, |
| 6.6.1.2, 6.6.1.3, 6.6.1.4, 6.6.1.5, 6.6.1.6, 6.6.1.7, 6.6.1.8, 6.6.1.9, 6.6.1.10, 6.6.2.1, 6.6.2.2, 6.6.2.3, 6.6.2.4, |
| 6.6.2.5, 6.6.2.6, 6.6.2.7, 6.6.2.8, 6.6.2.9, 6.6.2.10, 6.6.3.1, 6.6.3.2, 6.6.3.3, 6.6.3.4, 6.6.3.5, 6.6.3.6, 6.6.3.7, |
| 6.6.3.8, 6.6.3.9, 6.6.3.10, 6.6.4.1, 6.6.4.2, 6.6.4.3, 6.6.4.4, 6.6.4.5, 6.6.4.6, 6.6.4.7, 6.6.4.8, 6.6.4.9, 6.6.4.10, |
| 6.6.5.1, 6.6.5.2, 6.6.5.3, 6.6.5.4, 6.6.5.5, 6.6.5.6, 6.6.5.7, 6.6.5.8, 6.6.5.9, 6.6.5.10, 6.6.6.1, 6.6.6.2, 6.6.6.3, |
| 6.6.6.4, 6.6.6.5, 6.6.6.6, 6.6.6.7, 6.6.6.8, 6.6.6.9, 6.6.6.10, 6.6.7.1, 6.6.7.2, 6.6.7.3, 6.6.7.4, 6.6.7.5, 6.6.7.6, |
| 6.6.7.7, 6.6.7.8, 6.6.7.9, 6.6.7.10, 6.6.8.1, 6.6.8.2, 6.6.8.3, 6.6.8.4, 6.6.8.5, 6.6.8.6, 6.6.8.7, 6.6.8.8, 6.6.8.9, |
| 6.6.8.10, 6.6.9.1, 6.6.9.2, 6.6.9.3, 6.6.9.4, 6.6.9.5, 6.6.9.6, 6.6.9.7, 6.6.9.8, 6.6.9.9, 6.6.9.10, 6.6.10.1, 6.6.10.2, |
| 6.6.10.3, 6.6.10.4, 6.6.10.5, 6.6.10.6, 6.6.10.7, 6.6.10.8, 6.6.10.9, 6.6.10.10, 6.7.1.1, 6.7.1.2, 6.7.1.3, 6.7.1.4, |
| 6.7.1.5, 6.7.1.6, 6.7.1.7, 6.7.1.8, 6.7.1.9, 6.7.1.10, 6.7.2.1, 6.7.2.2, 6.7.2.3, 6.7.2.4, 6.7.2.5, 6.7.2.6, 6.7.2.7, |
| 6.7.2.8, 6.7.2.9, 6.7.2.10, 6.7.3.1, 6.7.3.2, 6.7.3.3, 6.7.3.4, 6.7.3.5, 6.7.3.6, 6.7.3.7, 6.7.3.8, 6.7.3.9, 6.7.3.10, |
| 6.7.4.1, 6.7.4.2, 6.7.4.3, 6.7.4.4, 6.7.4.5, 6.7.4.6, 6.7.4.7, 6.7.4.8, 6.7.4.9, 6.7.4.10, 6.7.5.1, 6.7.5.2, 6.7.5.3, |
| 6.7.5.4, 6.7.5.5, 6.7.5.6, 6.7.5.7, 6.7.5.8, 6.7.5.9, 6.7.5.10, 6.7.6.1, 6.7.6.2, 6.7.6.3, 6.7.6.4, 6.7.6.5, 6.7.6.6, |
| 6.7.6.7, 6.7.6.8, 6.7.6.9, 6.7.6.10, 6.7.7.1, 6.7.7.2, 6.7.7.3, 6.7.7.4, 6.7.7.5, 6.7.7.6, 6.7.7.7, 6.7.7.8, 6.7.7.9, |
| 6.7.7.10, 6.7.8.1, 6.7.8.2, 6.7.8.3, 6.7.8.4, 6.7.8.5, 6.7.8.6, 6.7.8.7, 6.7.8.8, 6.7.8.9, 6.7.8.10, 6.7.9.1, 6.7.9.2, |
| 6.7.9.3, 6.7.9.4, 6.7.9.5, 6.7.9.6, 6.7.9.7, 6.7.9.8, 6.7.9.9, 6.7.9.10, 6.7.10.1, 6.7.10.2, 6.7.10.3, 6.7.10.4, 6.7.10.5, |
| 6.7.10.6, 6.7.10.7, 6.7.10.8, 6.7.10.9, 6.7.10.10, 6.8.1.1, 6.8.1.2, 6.8.1.3, 6.8.1.4, 6.8.1.5, 6.8.1.6, 6.8.1.7, 6.8.1.8, |
| 6.8.1.9, 6.8.1.10, 6.8.2.1, 6.8.2.2, 6.8.2.3, 6.8.2.4, 6.8.2.5, 6.8.2.6, 6.8.2.7, 6.8.2.8, 6.8.2.9, 6.8.2.10, 6.8.3.1, |
| 6.8.3.2, 6.8.3.3, 6.8.3.4, 6.8.3.5, 6.8.3.6, 6.8.3.7, 6.8.3.8, 6.8.3.9, 6.8.3.10, 6.8.4.1, 6.8.4.2, 6.8.4.3, 6.8.4.4, |
| 6.8.4.5, 6.8.4.6, 6.8.4.7, 6.8.4.8, 6.8.4.9, 6.8.4.10, 6.8.5.1, 6.8.5.2, 6.8.5.3, 6.8.5.4, 6.8.5.5, 6.8.5.6, 6.8.5.7, |
| 6.8.5.8, 6.8.5.9, 6.8.5.10, 6.8.6.1, 6.8.6.2, 6.8.6.3, 6.8.6.4, 6.8.6.5, 6.8.6.6, 6.8.6.7, 6.8.6.8, 6.8.6.9, 6.8.6.10, |
| 6.8.7.1, 6.8.7.2, 6.8.7.3, 6.8.7.4, 6.8.7.5, 6.8.7.6, 6.8.7.7, 6.8.7.8, 6.8.7.9, 6.8.7.10, 6.8.8.1, 6.8.8.2, 6.8.8.3, |
| 6.8.8.4, 6.8.8.5, 6.8.8.6, 6.8.8.7, 6.8.8.8, 6.8.8.9, 6.8.8.10, 6.8.9.1, 6.8.9.2, 6.8.9.3, 6.8.9.4, 6.8.9.5, 6.8.9.6, |
| 6.8.9.7, 6.8.9.8, 6.8.9.9, 6.8.9.10, 6.8.10.1, 6.8.10.2, 6.8.10.3, 6.8.10.4, 6.8.10.5, 6.8.10.6, 6.8.10.7, 6.8.10.8, |
| 6.8.10.9, 6.8.10.10, 6.9.1.1, 6.9.1.2, 6.9.1.3, 6.9.1.4, 6.9.1.5, 6.9.1.6, 6.9.1.7, 6.9.1.8, 6.9.1.9, 6.9.1.10, 6.9.2.1, |
| 6.9.2.2, 6.9.2.3, 6.9.2.4, 6.9.2.5, 6.9.2.6, 6.9.2.7, 6.9.2.8, 6.9.2.9, 6.9.2.10, 6.9.3.1, 6.9.3.2, 6.9.3.3, 6.9.3.4, |
| 6.9.3.5, 6.9.3.6, 6.9.3.7, 6.9.3.8, 6.9.3.9, 6.9.3.10, 6.9.4.1, 6.9.4.2, 6.9.4.3, 6.9.4.4, 6.9.4.5, 6.9.4.6, 6.9.4.7, |
| 6.9.4.8, 6.9.4.9, 6.9.4.10, 6.9.5.1, 6.9.5.2, 6.9.5.3, 6.9.5.4, 6.9.5.5, 6.9.5.6, 6.9.5.7, 6.9.5.8, 6.9.5.9, 6.9.5.10, |
| 6.9.6.1, 6.9.6.2, 6.9.6.3, 6.9.6.4, 6.9.6.5, 6.9.6.6, 6.9.6.7, 6.9.6.8, 6.9.6.9, 6.9.6.10, 6.9.7.1, 6.9.7.2, 6.9.7.3, |
| 6.9.7.4, 6.9.7.5, 6.9.7.6, 6.9.7.7, 6.9.7.8, 6.9.7.9, 6.9.7.10, 6.9.8.1, 6.9.8.2, 6.9.8.3, 6.9.8.4, 6.9.8.5, 6.9.8.6, |
| 6.9.8.7, 6.9.8.8, 6.9.8.9, 6.9.8.10, 6.9.9.1, 6.9.9.2, 6.9.9.3, 6.9.9.4, 6.9.9.5, 6.9.9.6, 6.9.9.7, 6.9.9.8, 6.9.9.9, |
| 6.9.9.10, 6.9.10.1, 6.9.10.2, 6.9.10.3, 6.9.10.4, 6.9.10.5, 6.9.10.6, 6.9.10.7, 6.9.10.8, 6.9.10.9, 6.9.10.10, |
| 6.10.1.1, 6.10.1.2, 6.10.1.3, 6.10.1.4, 6.10.1.5, 6.10.1.6, 6.10.1.7, 6.10.1.8, 6.10.1.9, 6.10.1.10, 6.10.2.1, |
| 6.10.2.2, 6.10.2.3, 6.10.2.4, 6.10.2.5, 6.10.2.6, 6.10.2.7, 6.10.2.8, 6.10.2.9, 6.10.2.10, 6.10.3.1, 6.10.3.2, |
| 6.10.3.3, 6.10.3.4, 6.10.3.5, 6.10.3.6, 6.10.3.7, 6.10.3.8, 6.10.3.9, 6.10.3.10, 6.10.4.1, 6.10.4.2, 6.10.4.3, |
| 6.10.4.4, 6.10.4.5, 6.10.4.6, 6.10.4.7, 6.10.4.8, 6.10.4.9, 6.10.4.10, 6.10.5.1, 6.10.5.2, 6.10.5.3, 6.10.5.4, |
| 6.10.5.5, 6.10.5.6, 6.10.5.7, 6.10.5.8, 6.10.5.9, 6.10.5.10, 6.10.6.1, 6.10.6.2, 6.10.6.3, 6.10.6.4, 6.10.6.5, |
| 6.10.6.6, 6.10.6.7, 6.10.6.8, 6.10.6.9, 6.10.6.10, 6.10.7.1, 6.10.7.2, 6.10.7.3, 6.10.7.4, 6.10.7.5, 6.10.7.6, |
| 6.10.7.7, 6.10.7.8, 6.10.7.9, 6.10.7.10, 6.10.8.1, 6.10.8.2, 6.10.8.3, 6.10.8.4, 6.10.8.5, 6.10.8.6, 6.10.8.7, |
| 6.1 0.8.8, 6.1 0.8.9, 6.1 0.8.1 0, 6.1 0.9.1, 6.1 0.9.2, 6.10.9.3, 6.10.9.4, 6.10.9.5, 6.10.9.6, 6.10.9.7, 6.10.9.8, |
| 6.10.9.9, 6.10.9.10, 6.10.10.1, 6.10.10.2, 6.10.10.3, 6.10.10.4, 6.10.10.5, 6.10.10.6, 6.10.10.7, 6.10.10.8, |
| 6.10.10.9, 6.10.10.10, 7.1.1.1, 7.1.1.2, 7.1.1.3, 7.1.1.4, 7.1.1.5, 7.1.1.6, 7.1.1.7, 7.1.1.8, 7.1.1.9, 7.1.1.10, 7.1.2.1, |
| 7.1.2.2, 7.1.2.3, 7.1.2.4, 7.1.2.5, 7.1.2.6, 7.1.2.7, 7.1.2.8, 7.1.2.9, 7.1.2.10, 7.1.3.1, 7.1.3.2, 7.1.3.3, 7.1.3.4, |
| 7.1.3.5, 7.1.3.6, 7.1.3.7, 7.1.3.8, 7.1.3.9, 7.1.3.10, 7.1.4.1, 7.1.4.2, 7.1.4.3, 7.1.4.4, 7.1.4.5, 7.1.4.6, 7.1.4.7, |
| 7.1.4.8, 7.1.4.9, 7.1.4.10, 7.1.5.1, 7.1.5.2, 7.1.5.3, 7.1.5.4, 7.1.5.5, 7.1.5.6, 7.1.5.7, 7.1.5.8, 7.1.5.9, 7.1.5.10, |
| 7.1.6.1, 7.1.6.2, 7.1.6.3, 7.1.6.4, 7.1.6.5, 7.1.6.6, 7.1.6.7, 7.1.6.8, 7.1.6.9, 7.1.6.10, 7.1.7.1, 7.1.7.2, 7.1.7.3, |
| 7.1.7.4, 7.1.7.5, 7.1.7.6, 7.1.7.7, 7.1.7.8, 7.1.7.9, 7.1.7.10, 7.1.8.1, 7.1.8.2, 7.1.8.3, 7.1.8.4, 7.1.8.5, 7.1.8.6, |
| 7.1.8.7, 7.1.8.8, 7.1.8.9, 7.1.8.10, 7.1.9.1, 7.1.9.2, 7.1.9.3, 7.1.9.4, 7.1.9.5, 7.1.9.6, 7.1.9.7, 7.1.9.8, 7.1.9.9, |
| 7.1.9.10, 7.1.10.1, 7.1.10.2, 7.1.10.3, 7.1.10.4, 7.1.10.5, 7.1.10.6, 7.1.10.7, 7.1.10.8, 7.1.10.9, 7.1.10.10, 7.2.1.1, |
| 7.2.1.2, 7.2.1.3, 7.2.1.4, 7.2.1.5, 7.2.1.6, 7.2.1.7, 7.2.1.8, 7.2.1.9, 7.2.1.10, 7.2.2.1, 7.2.2.2, 7.2.2.3, 7.2.2.4, |
| 7.2.2.5, 7.2.2.6, 7.2.2.7, 7.2.2.8, 7.2.2.9, 7.2.2.10, 7.2.3.1, 7.2.3.2, 7.2.3.3, 7.2.3.4, 7.2.3.5, 7.2.3.6, 7.2.3.7, |
| 7.2.3.8, 7.2.3.9, 7.2.3.10, 7.2.4.1, 7.2.4.2, 7.2.4.3, 7.2.4.4, 7.2.4.5, 7.2.4.6, 7.2.4.7, 7.2.4.8, 7.2.4.9, 7.2.4.10, |
| 7.2.5.1, 7.2.5.2, 7.2.5.3, 7.2.5.4, 7.2.5.5, 7.2.5.6, 7.2.5.7, 7.2.5.8, 7.2.5.9, 7.2.5.10, 7.2.6.1, 7.2.6.2, 7.2.6.3, |
| 7.2.6.4, 7.2.6.5, 7.2.6.6, 7.2.6.7, 7.2.6.8, 7.2.6.9, 7.2.6.10, 7.2.7.1, 7.2.7.2, 7.2.7.3, 7.2.7.4, 7.2.7.5, 7.2.7.6, |
| 7.2.7.7, 7.2.7.8, 7.2.7.9, 7.2.7.10, 7.2.8.1, 7.2.8.2, 7.2.8.3, 7.2.8.4, 7.2.8.5, 7.2.8.6, 7.2.8.7, 7.2.8.8, 7.2.8.9, |
| 7.2.8.10, 7.2.9.1, 7.2.9.2, 7.2.9.3, 7.2.9.4, 7.2.9.5, 7.2.9.6, 7.2.9.7, 7.2.9.8, 7.2.9.9, 7.2.9.10, 7.2.10.1, 7.2.10.2, |
| 7.2.10.3, 7.2.10.4, 7.2.10.5, 7.2.10.6, 7.2.10.7, 7.2.10.8, 7.2.10.9, 7.2.10.10, 7.3.1.1, 7.3.1.2, 7.3.1.3, 7.3.1.4, |
| 7.3.1.5, 7.3.1.6, 7.3.1.7, 7.3.1.8, 7.3.1.9, 7.3.1.10, 7.3.2.1, 7.3.2.2, 7.3.2.3, 7.3.2.4, 7.3.2.5, 7.3.2.6, 7.3.2.7, |
| 7.3.2.8, 7.3.2.9, 7.3.2.10, 7.3.3.1, 7.3.3.2, 7.3.3.3, 7.3.3.4, 7.3.3.5, 7.3.3.6, 7.3.3.7, 7.3.3.8, 7.3.3.9, 7.3.3.10, |
| 7.3.4.1, 7.3.4.2, 7.3.4.3, 7.3.4.4, 7.3.4.5, 7.3.4.6, 7.3.4.7, 7.3.4.8, 7.3.4.9, 7.3.4.10, 7.3.5.1, 7.3.5.2, 7.3.5.3, |
| 7.3.5.4, 7.3.5.5, 7.3.5.6, 7.3.5.7, 7.3.5.8, 7.3.5.9, 7.3.5.10, 7.3.6.1, 7.3.6.2, 7.3.6.3, 7.3.6.4, 7.3.6.5, 7.3.6.6, |
| 7.3.6.7, 7.3.6.8, 7.3.6.9, 7.3.6.10, 7.3.7.1, 7.3.7.2, 7.3.7.3, 7.3.7.4, 7.3.7.5, 7.3.7.6, 7.3.7.7, 7.3.7.8, 7.3.7.9, |
| 7.3.7.10, 7.3.8.1, 7.3.8.2, 7.3.8.3, 7.3.8.4, 7.3.8.5, 7.3.8.6, 7.3.8.7, 7.3.8.8, 7.3.8.9, 7.3.8.10, 7.3.9.1, 7.3.9.2, |
| 7.3.9.3, 7.3.9.4, 7.3.9.5, 7.3.9.6, 7.3.9.7, 7.3.9.8, 7.3.9.9, 7.3.9.10, 7.3.10.1, 7.3.10.2, 7.3.10.3, 7.3.10.4, 7.3.10.5, |
| 7.3.10.6, 7.3.10.7, 7.3.10.8, 7.3.10.9, 7.3.10.10, 7.4.1.1, 7.4.1.2, 7.4.1.3, 7.4.1.4, 7.4.1.5, 7.4.1.6, 7.4.1.7, 7.4.1.8, |
| 7.4.1.9, 7.4.1.10, 7.4.2.1, 7.4.2.2, 7.4.2.3, 7.4.2.4, 7.4.2.5, 7.4.2.6, 7.4.2.7, 7.4.2.8, 7.4.2.9, 7.4.2.10, 7.4.3.1, |
| 7.4.3.2, 7.4.3.3, 7.4.3.4, 7.4.3.5, 7.4.3.6, 7.4.3.7, 7.4.3.8, 7.4.3.9, 7.4.3.10, 7.4.4.1, 7.4.4.2, 7.4.4.3, 7.4.4.4, |
| 7.4.4.5, 7.4.4.6, 7.4.4.7, 7.4.4.8, 7.4.4.9, 7.4.4.10, 7.4.5.1, 7.4.5.2, 7.4.5.3, 7.4.5.4, 7.4.5.5, 7.4.5.6, 7.4.5.7, |
| 7.4.5.8, 7.4.5.9, 7.4.5.10, 7.4.6.1, 7.4.6.2, 7.4.6.3, 7.4.6.4, 7.4.6.5, 7.4.6.6, 7.4.6.7, 7.4.6.8, 7.4.6.9, 7.4.6.10, |
| 7.4.7.1, 7.4.7.2, 7.4.7.3, 7.4.7.4, 7.4.7.5, 7.4.7.6, 7.4.7.7, 7.4.7.8, 7.4.7.9, 7.4.7.10, 7.4.8.1, 7.4.8.2, 7.4.8.3, |
| 7.4.8.4, 7.4.8.5, 7.4.8.6, 7.4.8.7, 7.4.8.8, 7.4.8.9, 7.4.8.10, 7.4.9.1, 7.4.9.2, 7.4.9.3, 7.4.9.4, 7.4.9.5, 7.4.9.6, |
| 7.4.9.7, 7.4.9.8, 7.4.9.9, 7.4.9.10, 7.4.10.1, 7.4.10.2, 7.4.10.3, 7.4.10.4, 7.4.10.5, 7.4.10.6, 7.4.10.7, 7.4.10.8, |
| 7.4.10.9, 7.4.10.10, 7.5.1.1, 7.5.1.2, 7.5.1.3, 7.5.1.4, 7.5.1.5, 7.5.1.6, 7.5.1.7, 7.5.1.8, 7.5.1.9, 7.5.1.10, 7.5.2.1, |
| 7.5.2.2, 7.5.2.3, 7.5.2.4, 7.5.2.5, 7.5.2.6, 7.5.2.7, 7.5.2.8, 7.5.2.9, 7.5.2.10, 7.5.3.1, 7.5.3.2, 7.5.3.3, 7.5.3.4, |
| 7.5.3.5, 7.5.3.6, 7.5.3.7, 7.5.3.8, 7.5.3.9, 7.5.3.10, 7.5.4.1, 7.5.4.2, 7.5.4.3, 7.5.4.4, 7.5.4.5, 7.5.4.6, 7.5.4.7, |
| 7.5.4.8, 7.5.4.9, 7.5.4.10, 7.5.5.1, 7.5.5.2, 7.5.5.3, 7.5.5.4, 7.5.5.5, 7.5.5.6, 7.5.5.7, 7.5.5.8, 7.5.5.9, 7.5.5.10, |
| 7.5.6.1, 7.5.6.2, 7.5.6.3, 7.5.6.4, 7.5.6.5, 7.5.6.6, 7.5.6.7, 7.5.6.8, 7.5.6.9, 7.5.6.10, 7.5.7.1, 7.5.7.2, 7.5.7.3, |
| 7.5.7.4, 7.5.7.5, 7.5.7.6, 7.5.7.7, 7.5.7.8, 7.5.7.9, 7.5.7.10, 7.5.8.1, 7.5.8.2, 7.5.8.3, 7.5.8.4, 7.5.8.5, 7.5.8.6, |
| 7.5.8.7, 7.5.8.8, 7.5.8.9, 7.5.8.10, 7.5.9.1, 7.5.9.2, 7.5.9.3, 7.5.9.4, 7.5.9.5, 7.5.9.6, 7.5.9.7, 7.5.9.8, 7.5.9.9, |
| 7.5.9.10, 7.5.10.1, 7.5.10.2, 7.5.10.3, 7.5.10.4, 7.5.10.5, 7.5.10.6, 7.5.10.7, 7.5.10.8, 7.5.10.9, 7.5.10.10, 7.6.1.1, |
| 7.6.1.2, 7.6.1.3, 7.6.1.4, 7.6.1.5, 7.6.1.6, 7.6.1.7, 7.6.1.8, 7.6.1.9, 7.6.1.10, 7.6.2.1, 7.6.2.2, 7.6.2.3, 7.6.2.4, |
| 7.6.2.5, 7.6.2.6, 7.6.2.7, 7.6.2.8, 7.6.2.9, 7.6.2.10, 7.6.3.1, 7.6.3.2, 7.6.3.3, 7.6.3.4, 7.6.3.5, 7.6.3.6, 7.6.3.7, |
| 7.6.3.8, 7.6.3.9, 7.6.3.10, 7.6.4.1, 7.6.4.2, 7.6.4.3, 7.6.4.4, 7.6.4.5, 7.6.4.6, 7.6.4.7, 7.6.4.8, 7.6.4.9, 7.6.4.10, |
| 7.6.5.1, 7.6.5.2, 7.6.5.3, 7.6.5.4, 7.6.5.5, 7.6.5.6, 7.6.5.7, 7.6.5.8, 7.6.5.9, 7.6.5.10, 7.6.6.1, 7.6.6.2, 7.6.6.3, |
| 7.6.6.4, 7.6.6.5, 7.6.6.6, 7.6.6.7, 7.6.6.8, 7.6.6.9, 7.6.6.10, 7.6.7.1, 7.6.7.2, 7.6.7.3, 7.6.7.4, 7.6.7.5, 7.6.7.6, |
| 7.6.7.7, 7.6.7.8, 7.6.7.9, 7.6.7.10, 7.6.8.1, 7.6.8.2, 7.6.8.3, 7.6.8.4, 7.6.8.5, 7.6.8.6, 7.6.8.7, 7.6.8.8, 7.6.8.9, |
| 7.6.8.10, 7.6.9.1, 7.6.9.2, 7.6.9.3, 7.6.9.4, 7.6.9.5, 7.6.9.6, 7.6.9.7, 7.6.9.8, 7.6.9.9, 7.6.9.10, 7.6.10.1, 7.6.10.2, |
| 7.6.10.3, 7.6.10.4, 7.6.10.5, 7.6.10.6, 7.6.10.7, 7.6.10.8, 7.6.10.9, 7.6.10.10, 7.7.1.1, 7.7.1.2, 7.7.1.3, 7.7.1.4, |
| 7.7.1.5, 7.7.1.6, 7.7.1.7, 7.7.1.8, 7.7.1.9, 7.7.1.10, 7.7.2.1, 7.7.2.2, 7.7.2.3, 7.7.2.4, 7.7.2.5, 7.7.2.6, 7.7.2.7, |
| 7.7.2.8, 7.7.2.9, 7.7.2.10, 7.7.3.1, 7.7.3.2, 7.7.3.3, 7.7.3.4, 7.7.3.5, 7.7.3.6, 7.7.3.7, 7.7.3.8, 7.7.3.9, 7.7.3.10, |
| 7.7.4.1, 7.7.4.2, 7.7.4.3, 7.7.4.4, 7.7.4.5, 7.7.4.6, 7.7.4.7, 7.7.4.8, 7.7.4.9, 7.7.4.10, 7.7.5.1, 7.7.5.2, 7.7.5.3, |
| 7.7.5.4, 7.7.5.5, 7.7.5.6, 7.7.5.7, 7.7.5.8, 7.7.5.9, 7.7.5.10, 7.7.6.1, 7.7.6.2, 7.7.6.3, 7.7.6.4, 7.7.6.5, 7.7.6.6, |
| 7.7.6.7, 7.7.6.8, 7.7.6.9, 7.7.6.10, 7.7.7.1, 7.7.7.2, 7.7.7.3, 7.7.7.4, 7.7.7.5, 7.7.7.6, 7.7.7.7, 7.7.7.8, 7.7.7.9, |
| 7.7.7.10, 7.7.8.1, 7.7.8.2, 7.7.8.3, 7.7.8.4, 7.7.8.5, 7.7.8.6, 7.7.8.7, 7.7.8.8, 7.7.8.9, 7.7.8.10, 7.7.9.1, 7.7.9.2, |
| 7.7.9.3, 7.7.9.4, 7.7.9.5, 7.7.9.6, 7.7.9.7, 7.7.9.8, 7.7.9.9, 7.7.9.10, 7.7.10.1, 7.7.10.2, 7.7.10.3, 7.7.10.4, 7.7.10.5, |
| 7.7.10.6, 7.7.10.7, 7.7.10.8, 7.7.10.9, 7.7.10.10, 7.8.1.1, 7.8.1.2, 7.8.1.3, 7.8.1.4, 7.8.1.5, 7.8.1.6, 7.8.1.7, 7.8.1.8, |
| 7.8.1.9, 7.8.1.10, 7.8.2.1, 7.8.2.2, 7.8.2.3, 7.8.2.4, 7.8.2.5, 7.8.2.6, 7.8.2.7, 7.8.2.8, 7.8.2.9, 7.8.2.10, 7.8.3.1, |
| 7.8.3.2, 7.8.3.3, 7.8.3.4, 7.8.3.5, 7.8.3.6, 7.8.3.7, 7.8.3.8, 7.8.3.9, 7.8.3.10, 7.8.4.1, 7.8.4.2, 7.8.4.3, 7.8.4.4, |
| 7.8.4.5, 7.8.4.6, 7.8.4.7, 7.8.4.8, 7.8.4.9, 7.8.4.10, 7.8.5.1, 7.8.5.2, 7.8.5.3, 7.8.5.4, 7.8.5.5, 7.8.5.6, 7.8.5.7, |
| 7.8.5.8, 7.8.5.9, 7.8.5.10, 7.8.6.1, 7.8.6.2, 7.8.6.3, 7.8.6.4, 7.8.6.5, 7.8.6.6, 7.8.6.7, 7.8.6.8, 7.8.6.9, 7.8.6.10, |
| 7.8.7.1, 7.8.7.2, 7.8.7.3, 7.8.7.4, 7.8.7.5, 7.8.7.6, 7.8.7.7, 7.8.7.8, 7.8.7.9, 7.8.7.10, 7.8.8.1, 7.8.8.2, 7.8.8.3, |
| 7.8.8.4, 7.8.8.5, 7.8.8.6, 7.8.8.7, 7.8.8.8, 7.8.8.9, 7.8.8.10, 7.8.9.1, 7.8.9.2, 7.8.9.3, 7.8.9.4, 7.8.9.5, 7.8.9.6, |
| 7.8.9.7, 7.8.9.8, 7.8.9.9, 7.8.9.10, 7.8.10.1, 7.8.10.2, 7.8.10.3, 7.8.10.4, 7.8.10.5, 7.8.10.6, 7.8.10.7, 7.8.10.8, |
| 7.8.10.9, 7.8.10.10, 7.9.1.1, 7.9.1.2, 7.9.1.3, 7.9.1.4, 7.9.1.5, 7.9.1.6, 7.9.1.7, 7.9.1.8, 7.9.1.9, 7.9.1.10, 7.9.2.1, |
| 7.9.2.2, 7.9.2.3, 7.9.2.4, 7.9.2.5, 7.9.2.6, 7.9.2.7, 7.9.2.8, 7.9.2.9, 7.9.2.10, 7.9.3.1, 7.9.3.2, 7.9.3.3, 7.9.3.4, |
| 7.9.3.5, 7.9.3.6, 7.9.3.7, 7.9.3.8, 7.9.3.9, 7.9.3.10, 7.9.4.1, 7.9.4.2, 7.9.4.3, 7.9.4.4, 7.9.4.5, 7.9.4.6, 7.9.4.7, |
| 7.9.4.8, 7.9.4.9, 7.9.4.10, 7.9.5.1, 7.9.5.2, 7.9.5.3, 7.9.5.4, 7.9.5.5, 7.9.5.6, 7.9.5.7, 7.9.5.8, 7.9.5.9, 7.9.5.10, |
| 7.9.6.1, 7.9.6.2, 7.9.6.3, 7.9.6.4, 7.9.6.5, 7.9.6.6, 7.9.6.7, 7.9.6.8, 7.9.6.9, 7.9.6.10, 7.9.7.1, 7.9.7.2, 7.9.7.3, |
| 7.9.7.4, 7.9.7.5, 7.9.7.6, 7.9.7.7, 7.9.7.8, 7.9.7.9, 7.9.7.10, 7.9.8.1, 7.9.8.2, 7.9.8.3, 7.9.8.4, 7.9.8.5, 7.9.8.6, |
| 7.9.8.7, 7.9.8.8, 7.9.8.9, 7.9.8.10, 7.9.9.1, 7.9.9.2, 7.9.9.3, 7.9.9.4, 7.9.9.5, 7.9.9.6, 7.9.9.7, 7.9.9.8, 7.9.9.9, |
| 7.9.9.10, 7.9.10.1, 7.9.10.2, 7.9.10.3, 7.9.10.4, 7.9.10.5, 7.9.10.6, 7.9.10.7, 7.9.10.8, 7.9.10.9, 7.9.10.10, |
| 7.10.1.1, 7.10.1.2, 7.10.1.3, 7.10.1.4, 7.10.1.5, 7.10.1.6, 7.10.1.7, 7.10.1.8, 7.10.1.9, 7.10.1.10, 7.10.2.1, |
| 7.10.2.2, 7.10.2.3, 7.10.2.4, 7.10.2.5, 7.10.2.6, 7.10.2.7, 7.10.2.8, 7.10.2.9, 7.10.2.10, 7.10.3.1, 7.10.3.2, |
| 7.10.3.3, 7.10.3.4, 7.10.3.5, 7.10.3.6, 7.10.3.7, 7.10.3.8, 7.10.3.9, 7.10.3.10, 7.10.4.1, 7.10.4.2, 7.10.4.3, |
| 7.10.4.4, 7.10.4.5, 7.10.4.6, 7.10.4.7, 7.10.4.8, 7.10.4.9, 7.10.4.10, 7.10.5.1, 7.10.5.2, 7.10.5.3, 7.10.5.4, |
| 7.10.5.5, 7.10.5.6, 7.10.5.7, 7.10.5.8, 7.10.5.9, 7.10.5.10, 7.10.6.1, 7.10.6.2, 7.10.6.3, 7.10.6.4, 7.10.6.5, |
| 7.10.6.6, 7.10.6.7, 7.10.6.8, 7.10.6.9, 7.10.6.10, 7.10.7.1, 7.10.7.2, 7.10.7.3, 7.10.7.4, 7.10.7.5, 7.10.7.6, |
| 7.10.7.7, 7.10.7.8, 7.10.7.9, 7.10.7.10, 7.10.8.1, 7.10.8.2, 7.10.8.3, 7.10.8.4, 7.10.8.5, 7.10.8.6, 7.10.8.7, |
| 7.10.8.8, 7.10.8.9, 7.10.8.10, 7.10.9.1, 7.10.9.2, 7.10.9.3, 7.10.9.4, 7.10.9.5, 7.10.9.6, 7.10.9.7, 7.10.9.8, |
| 7.10.9.9, 7.10.9.10, 7.10.10.1, 7.10.10.2, 7.10.10.3, 7.10.10.4, 7.10.10.5, 7.10.10.6, 7.10.10.7, 7.10.10.8, |
| 7.10.10.9, 7.10.10.10, 8.1.1.1, 8.1.1.2, 8.1.1.3, 8.1.1.4, 8.1.1.5, 8.1.1.6, 8.1.1.7, 8.1.1.8, 8.1.1.9, 8.1.1.10, 8.1.2.1, |
| 8.1.2.2, 8.1.2.3, 8.1.2.4, 8.1.2.5, 8.1.2.6, 8.1.2.7, 8.1.2.8, 8.1.2.9, 8.1.2.10, 8.1.3.1, 8.1.3.2, 8.1.3.3, 8.1.3.4, |
| 8.1.3.5, 8.1.3.6, 8.1.3.7, 8.1.3.8, 8.1.3.9, 8.1.3.10, 8.1.4.1, 8.1.4.2, 8.1.4.3, 8.1.4.4, 8.1.4.5, 8.1.4.6, 8.1.4.7, |
| 8.1.4.8, 8.1.4.9, 8.1.4.10, 8.1.5.1, 8.1.5.2, 8.1.5.3, 8.1.5.4, 8.1.5.5, 8.1.5.6, 8.1.5.7, 8.1.5.8, 8.1.5.9, 8.1.5.10, |
| 8.1.6.1, 8.1.6.2, 8.1.6.3, 8.1.6.4, 8.1.6.5, 8.1.6.6, 8.1.6.7, 8.1.6.8, 8.1.6.9, 8.1.6.10, 8.1.7.1, 8.1.7.2, 8.1.7.3, |
| 8.1.7.4, 8.1.7.5, 8.1.7.6, 8.1.7.7, 8.1.7.8, 8.1.7.9, 8.1.7.10, 8.1.8.1, 8.1.8.2, 8.1.8.3, 8.1.8.4, 8.1.8.5, 8.1.8.6, |
| 8.1.8.7, 8.1.8.8, 8.1.8.9, 8.1.8.10, 8.1.9.1, 8.1.9.2, 8.1.9.3, 8.1.9.4, 8.1.9.5, 8.1.9.6, 8.1.9.7, 8.1.9.8, 8.1.9.9, |
| 8.1.9.10, 8.1.10.1, 8.1.10.2, 8.1.10.3, 8.1.10.4, 8.1.10.5, 8.1.10.6, 8.1.10.7, 8.1.10.8, 8.1.10.9, 8.1.10.10, 8.2.1.1, |
| 8.2.1.2, 8.2.1.3, 8.2.1.4, 8.2.1.5, 8.2.1.6, 8.2.1.7, 8.2.1.8, 8.2.1.9, 8.2.1.10, 8.2.2.1, 8.2.2.2, 8.2.2.3, 8.2.2.4, |
| 8.2.2.5, 8.2.2.6, 8.2.2.7, 8.2.2.8, 8.2.2.9, 8.2.2.10, 8.2.3.1, 8.2.3.2, 8.2.3.3, 8.2.3.4, 8.2.3.5, 8.2.3.6, 8.2.3.7, |
| 8.2.3.8, 8.2.3.9, 8.2.3.10, 8.2.4.1, 8.2.4.2, 8.2.4.3, 8.2.4.4, 8.2.4.5, 8.2.4.6, 8.2.4.7, 8.2.4.8, 8.2.4.9, 8.2.4.10, |
| 8.2.5.1, 8.2.5.2, 8.2.5.3, 8.2.5.4, 8.2.5.5, 8.2.5.6, 8.2.5.7, 8.2.5.8, 8.2.5.9, 8.2.5.10, 8.2.6.1, 8.2.6.2, 8.2.6.3, |
| 8.2.6.4, 8.2.6.5, 8.2.6.6, 8.2.6.7, 8.2.6.8, 8.2.6.9, 8.2.6.10, 8.2.7.1, 8.2.7.2, 8.2.7.3, 8.2.7.4, 8.2.7.5, 8.2.7.6, |
| 8.2.7.7, 8.2.7.8, 8.2.7.9, 8.2.7.10, 8.2.8.1, 8.2.8.2, 8.2.8.3, 8.2.8.4, 8.2.8.5, 8.2.8.6, 8.2.8.7, 8.2.8.8, 8.2.8.9, |
| 8.2.8.10, 8.2.9.1, 8.2.9.2, 8.2.9.3, 8.2.9.4, 8.2.9.5, 8.2.9.6, 8.2.9.7, 8.2.9.8, 8.2.9.9, 8.2.9.10, 8.2.10.1, 8.2.10.2, |
| 8.2.10.3, 8.2.10.4, 8.2.10.5, 8.2.10.6, 8.2.10.7, 8.2.10.8, 8.2.10.9, 8.2.10.10, 8.3.1.1, 8.3.1.2, 8.3.1.3, 8.3.1.4, |
| 8.3.1.5, 8.3.1.6, 8.3.1.7, 8.3.1.8, 8.3.1.9, 8.3.1.10, 8.3.2.1, 8.3.2.2, 8.3.2.3, 8.3.2.4, 8.3.2.5, 8.3.2.6, 8.3.2.7, |
| 8.3.2.8, 8.3.2.9, 8.3.2.10, 8.3.3.1, 8.3.3.2, 8.3.3.3, 8.3.3.4, 8.3.3.5, 8.3.3.6, 8.3.3.7, 8.3.3.8, 8.3.3.9, 8.3.3.10, |
| 8.3.4.1, 8.3.4.2, 8.3.4.3, 8.3.4.4, 8.3.4.5, 8.3.4.6, 8.3.4.7, 8.3.4.8, 8.3.4.9, 8.3.4.10, 8.3.5.1, 8.3.5.2, 8.3.5.3, |
| 8.3.5.4, 8.3.5.5, 8.3.5.6, 8.3.5.7, 8.3.5.8, 8.3.5.9, 8.3.5.10, 8.3.6.1, 8.3.6.2, 8.3.6.3, 8.3.6.4, 8.3.6.5, 8.3.6.6, |
| 8.3.6.7, 8.3.6.8, 8.3.6.9, 8.3.6.10, 8.3.7.1, 8.3.7.2, 8.3.7.3, 8.3.7.4, 8.3.7.5, 8.3.7.6, 8.3.7.7, 8.3.7.8, 8.3.7.9, |
| 8.3.7.10, 8.3.8.1, 8.3.8.2, 8.3.8.3, 8.3.8.4, 8.3.8.5, 8.3.8.6, 8.3.8.7, 8.3.8.8, 8.3.8.9, 8.3.8.10, 8.3.9.1, 8.3.9.2, |
| 8.3.9.3, 8.3.9.4, 8.3.9.5, 8.3.9.6, 8.3.9.7, 8.3.9.8, 8.3.9.9, 8.3.9.10, 8.3.10.1, 8.3.10.2, 8.3.10.3, 8.3.10.4, 8.3.10.5, |
| 8.3.10.6, 8.3.10.7, 8.3.10.8, 8.3.10.9, 8.3.10.10, 8.4.1.1, 8.4.1.2, 8.4.1.3, 8.4.1.4, 8.4.1.5, 8.4.1.6, 8.4.1.7, 8.4.1.8, |
| 8.4.1.9, 8.4.1.10, 8.4.2.1, 8.4.2.2, 8.4.2.3, 8.4.2.4, 8.4.2.5, 8.4.2.6, 8.4.2.7, 8.4.2.8, 8.4.2.9, 8.4.2.10, 8.4.3.1, |
| 8.4.3.2, 8.4.3.3, 8.4.3.4, 8.4.3.5, 8.4.3.6, 8.4.3.7, 8.4.3.8, 8.4.3.9, 8.4.3.10, 8.4.4.1, 8.4.4.2, 8.4.4.3, 8.4.4.4, |
| 8.4.4.5, 8.4.4.6, 8.4.4.7, 8.4.4.8, 8.4.4.9, 8.4.4.10, 8.4.5.1, 8.4.5.2, 8.4.5.3, 8.4.5.4, 8.4.5.5, 8.4.5.6, 8.4.5.7, |
| 8.4.5.8, 8.4.5.9, 8.4.5.10, 8.4.6.1, 8.4.6.2, 8.4.6.3, 8.4.6.4, 8.4.6.5, 8.4.6.6, 8.4.6.7, 8.4.6.8, 8.4.6.9, 8.4.6.10, |
| 8.4.7.1, 8.4.7.2, 8.4.7.3, 8.4.7.4, 8.4.7.5, 8.4.7.6, 8.4.7.7, 8.4.7.8, 8.4.7.9, 8.4.7.10, 8.4.8.1, 8.4.8.2, 8.4.8.3, |
| 8.4.8.4, 8.4.8.5, 8.4.8.6, 8.4.8.7, 8.4.8.8, 8.4.8.9, 8.4.8.10, 8.4.9.1, 8.4.9.2, 8.4.9.3, 8.4.9.4, 8.4.9.5, 8.4.9.6, |
| 8.4.9.7, 8.4.9.8, 8.4.9.9, 8.4.9.10, 8.4.10.1, 8.4.10.2, 8.4.10.3, 8.4.10.4, 8.4.10.5, 8.4.10.6, 8.4.10.7, 8.4.10.8, |
| 8.4.10.9, 8.4.10.10, 8.5.1.1, 8.5.1.2, 8.5.1.3, 8.5.1.4, 8.5.1.5, 8.5.1.6, 8.5.1.7, 8.5.1.8, 8.5.1.9, 8.5.1.10, 8.5.2.1, |
| 8.5.2.2, 8.5.2.3, 8.5.2.4, 8.5.2.5, 8.5.2.6, 8.5.2.7, 8.5.2.8, 8.5.2.9, 8.5.2.10, 8.5.3.1, 8.5.3.2, 8.5.3.3, 8.5.3.4, |
| 8.5.3.5, 8.5.3.6, 8.5.3.7, 8.5.3.8, 8.5.3.9, 8.5.3.10, 8.5.4.1, 8.5.4.2, 8.5.4.3, 8.5.4.4, 8.5.4.5, 8.5.4.6, 8.5.4.7, |
| 8.5.4.8, 8.5.4.9, 8.5.4.10, 8.5.5.1, 8.5.5.2, 8.5.5.3, 8.5.5.4, 8.5.5.5, 8.5.5.6, 8.5.5.7, 8.5.5.8, 8.5.5.9, 8.5.5.10, |
| 8.5.6.1, 8.5.6.2, 8.5.6.3, 8.5.6.4, 8.5.6.5, 8.5.6.6, 8.5.6.7, 8.5.6.8, 8.5.6.9, 8.5.6.10, 8.5.7.1, 8.5.7.2, 8.5.7.3, |
| 8.5.7.4, 8.5.7.5, 8.5.7.6, 8.5.7.7, 8.5.7.8, 8.5.7.9, 8.5.7.10, 8.5.8.1, 8.5.8.2, 8.5.8.3, 8.5.8.4, 8.5.8.5, 8.5.8.6, |
| 8.5.8.7, 8.5.8.8, 8.5.8.9, 8.5.8.10, 8.5.9.1, 8.5.9.2, 8.5.9.3, 8.5.9.4, 8.5.9.5, 8.5.9.6, 8.5.9.7, 8.5.9.8, 8.5.9.9, |
| 8.5.9.10, 8.5.10.1, 8.5.10.2, 8.5.10.3, 8.5.10.4, 8.5.10.5, 8.5.10.6, 8.5.10.7, 8.5.10.8, 8.5.10.9, 8.5.10.10, 8.6.1.1, |
| 8.6.1.2, 8.6.1.3, 8.6.1.4, 8.6.1.5, 8.6.1.6, 8.6.1.7, 8.6.1.8, 8.6.1.9, 8.6.1.10, 8.6.2.1, 8.6.2.2, 8.6.2.3, 8.6.2.4, |
| 8.6.2.5, 8.6.2.6, 8.6.2.7, 8.6.2.8, 8.6.2.9, 8.6.2.10, 8.6.3.1, 8.6.3.2, 8.6.3.3, 8.6.3.4, 8.6.3.5, 8.6.3.6, 8.6.3.7, |
| 8.6.3.8, 8.6.3.9, 8.6.3.10, 8.6.4.1, 8.6.4.2, 8.6.4.3, 8.6.4.4, 8.6.4.5, 8.6.4.6, 8.6.4.7, 8.6.4.8, 8.6.4.9, 8.6.4.10, |
| 8.6.5.1, 8.6.5.2, 8.6.5.3, 8.6.5.4, 8.6.5.5, 8.6.5.6, 8.6.5.7, 8.6.5.8, 8.6.5.9, 8.6.5.10, 8.6.6.1, 8.6.6.2, 8.6.6.3, |
| 8.6.6.4, 8.6.6.5, 8.6.6.6, 8.6.6.7, 8.6.6.8, 8.6.6.9, 8.6.6.10, 8.6.7.1, 8.6.7.2, 8.6.7.3, 8.6.7.4, 8.6.7.5, 8.6.7.6, |
| 8.6.7.7, 8.6.7.8, 8.6.7.9, 8.6.7.10, 8.6.8.1, 8.6.8.2, 8.6.8.3, 8.6.8.4, 8.6.8.5, 8.6.8.6, 8.6.8.7, 8.6.8.8, 8.6.8.9, |
| 8.6.8.10, 8.6.9.1, 8.6.9.2, 8.6.9.3, 8.6.9.4, 8.6.9.5, 8.6.9.6, 8.6.9.7, 8.6.9.8, 8.6.9.9, 8.6.9.10, 8.6.10.1, 8.6.10.2, |
| 8.6.10.3, 8.6.10.4, 8.6.10.5, 8.6.10.6, 8.6.10.7, 8.6.10.8, 8.6.10.9, 8.6.10.10, 8.7.1.1, 8.7.1.2, 8.7.1.3, 8.7.1.4, |
| 8.7.1.5, 8.7.1.6, 8.7.1.7, 8.7.1.8, 8.7.1.9, 8.7.1.10, 8.7.2.1, 8.7.2.2, 8.7.2.3, 8.7.2.4, 8.7.2.5, 8.7.2.6, 8.7.2.7, |
| 8.7.2.8, 8.7.2.9, 8.7.2.10, 8.7.3.1, 8.7.3.2, 8.7.3.3, 8.7.3.4, 8.7.3.5, 8.7.3.6, 8.7.3.7, 8.7.3.8, 8.7.3.9, 8.7.3.10, |
| 8.7.4.1, 8.7.4.2, 8.7.4.3, 8.7.4.4, 8.7.4.5, 8.7.4.6, 8.7.4.7, 8.7.4.8, 8.7.4.9, 8.7.4.10, 8.7.5.1, 8.7.5.2, 8.7.5.3, |
| 8.7.5.4, 8.7.5.5, 8.7.5.6, 8.7.5.7, 8.7.5.8, 8.7.5.9, 8.7.5.10, 8.7.6.1, 8.7.6.2, 8.7.6.3, 8.7.6.4, 8.7.6.5, 8.7.6.6, |
| 8.7.6.7, 8.7.6.8, 8.7.6.9, 8.7.6.10, 8.7.7.1, 8.7.7.2, 8.7.7.3, 8.7.7.4, 8.7.7.5, 8.7.7.6, 8.7.7.7, 8.7.7.8, 8.7.7.9, |
| 8.7.7.10, 8.7.8.1, 8.7.8.2, 8.7.8.3, 8.7.8.4, 8.7.8.5, 8.7.8.6, 8.7.8.7, 8.7.8.8, 8.7.8.9, 8.7.8.10, 8.7.9.1, 8.7.9.2, |
| 8.7.9.3, 8.7.9.4, 8.7.9.5, 8.7.9.6, 8.7.9.7, 8.7.9.8, 8.7.9.9, 8.7.9.10, 8.7.10.1, 8.7.10.2, 8.7.10.3, 8.7.10.4, 8.7.10.5, |
| 8.7.10.6, 8.7.10.7, 8.7.10.8, 8.7.10.9, 8.7.10.10, 8.8.1.1, 8.8.1.2, 8.8.1.3, 8.8.1.4, 8.8.1.5, 8.8.1.6, 8.8.1.7, 8.8,1.8, |
| 8.8.1.9, 8.8.1.10, 8.8.2.1, 8.8.2.2, 8.8.2.3, 8.8.2.4, 8.8.2.5, 8.8.2.6, 8.8.2.7, 8.8.2.8, 8.8.2.9, 8.8.2.10, 8.8.3.1, |
| 8.8.3.2, 8.8.3.3, 8.8.3.4, 8.8.3.5, 8.8.3.6, 8.8.3.7, 8.8.3.8, 8.8.3.9, 8.8.3.10, 8.8.4.1, 8.8.4.2, 8.8.4.3, 8.8.4.4, |
| 8.8.4.5, 8.8.4.6, 8.8.4.7, 8.8.4.8, 8.8.4.9, 8.8.4.10, 8.8.5.1, 8.8.5.2, 8.8.5.3, 8.8.5.4, 8.8.5.5, 8.8.5.6, 8.8.5.7, |
| 8.8.5.8, 8.8.5.9, 8.8.5.10, 8.8.6.1, 8.8.6.2, 8.8.6.3, 8.8.6.4, 8.8.6.5, 8.8.6.6, 8.8.6.7, 8.8.6.8, 8.8.6.9, 8.8.6.10, |
| 8.8.7.1, 8.8.7.2, 8.8.7.3, 8.8.7.4, 8.8.7.5, 8.8.7.6, 8.8.7.7, 8.8.7.8, 8.8.7.9, 8.8.7.10, 8.8.8.1, 8.8.8.2, 8.8.8.3, |
| 8.8.8.4, 8.8.8.5, 8.8.8.6, 8.8.8.7, 8.8.8.8, 8.8.8.9, 8.8.8.10, 8.8.9.1, 8.8.9.2, 8.8.9.3, 8.8.9.4, 8.8.9.5, 8.8.9.6, |
| 8.8.9.7, 8.8.9.8, 8.8.9.9, 8.8.9.10, 8.8.10.1, 8.8.10.2, 8.8.10.3, 8.8.10.4, 8.8.10.5, 8.8.10.6, 8.8.10.7, 8.8.10.8, |
| 8.8.10.9, 8.8.10.10, 8.9.1.1, 8.9.1.2, 8.9.1.3, 8.9.1.4, 8.9.1.5, 8.9.1.6, 8.9.1.7, 8.9.1.8, 8.9.1.9, 8.9.1.10, 8.9.2.1, |
| 8.9.2.2, 8.9.2.3, 8.9.2.4, 8.9.2.5, 8.9.2.6, 8.9.2.7, 8.9.2.8, 8.9.2.9, 8.9.2.10, 8.9.3.1, 8.9.3.2, 8.9.3.3, 8.9.3.4, |
| 8.9.3.5, 8.9.3.6, 8.9.3.7, 8.9.3.8, 8.9.3.9, 8.9.3.10, 8.9.4.1, 8.9.4.2, 8.9.4.3, 8.9.4.4, 8.9.4.5, 8.9.4.6, 8.9.4.7, |
| 8.9.4.8, 8.9.4.9, 8.9.4.10, 8.9.5.1, 8.9.5.2, 8.9.5.3, 8.9.5.4, 8.9.5.5, 8.9.5.6, 8.9.5.7, 8.9.5.8, 8.9.5.9, 8.9.5.10, |
| 8.9.6.1, 8.9.6.2, 8.9.6.3, 8.9.6.4, 8.9.6.5, 8.9.6.6, 8.9.6.7, 8.9.6.8, 8.9.6.9, 8.9.6.10, 8.9.7.1, 8.9.7.2, 8.9.7.3, |
| 8.9.7.4, 8.9.7.5, 8.9.7.6, 8.9.7.7, 8.9.7.8, 8.9.7.9, 8.9.7.10, 8.9.8.1, 8.9.8.2, 8.9.8.3, 8.9.8.4, 8.9.8.5, 8.9.8.6, |
| 8.9.8.7, 8.9.8.8, 8.9.8.9, 8.9.8.10, 8.9.9.1, 8.9.9.2, 8.9.9.3, 8.9.9.4, 8.9.9.5, 8.9.9.6, 8.9.9.7, 8.9.9.8, 8.9.9.9, |
| 8.9.9.10, 8.9.10.1, 8.9.10.2, 8.9.10.3, 8.9.10.4, 8.9.10.5, 8.9.10.6, 8.9.10.7, 8.9.10.8, 8.9.10.9, 8.9.10.10, |
| 8.10.1.1, 8.10.1.2, 8.10.1.3, 8.10.1.4, 8.10.1.5, 8.10.1.6, 8.10.1.7, 8.10.1.8, 8.10.1.9, 8.10.1.10, 8.10.2.1, |
| 8.10.2.2, 8.10.2.3, 8.10.2.4, 8.10.2.5, 8.10.2.6, 8.10.2.7, 8.10.2.8, 8.10.2.9, 8.10.2.10, 8.10.3.1, 8.10.3.2, |
| 8.10.3.3, 8.10.3.4, 8.10.3.5, 8.10.3.6, 8.10.3.7, 8.10.3.8, 8.10.3.9, 8.10.3.10, 8.10.4.1, 8.10.4.2, 8.10.4.3, |
| 8.10.4.4, 8.10.4.5, 8.10.4.6, 8.10.4.7, 8.10.4.8, 8.10.4.9, 8.10.4.10, 8.10.5.1, 8.10.5.2, 8.10.5.3, 8.10.5.4, |
| 8.10.5.5, 8.10.5.6, 8.10.5.7, 8.10.5.8, 8.10.5.9, 8.10.5.10, 8.10.6.1, 8.10.6.2, 8.10.6.3, 8.10.6.4, 8.10.6.5, |
| 8.10.6.6, 8.10.6.7, 8.10.6.8, 8.10.6.9, 8.10.6.10, 8.10.7.1, 8.10.7.2, 8.10.7.3, 8.10.7.4, 8.10.7.5, 8.10.7.6, |
| 8.10.7.7, 8.10.7.8, 8.10.7.9, 8.10.7.10, 8.10.8.1, 8.10.8.2, 8.10.8.3, 8.10.8.4, 8.10.8.5, 8.10.8.6, 8.10.8.7, |
| 8.10.8.8, 8.10.8.9, 8.10.8.10, 8.10.9.1, 8.10.9.2, 8.10.9.3, 8.10.9.4, 8.10.9.5, 8.10.9.6, 8.10.9.7, 8.10.9.8, |
| 8.10.9.9, 8.10.9.10, 8.10.10.1, 8.10.10.2, 8.10.10.3, 8.10.10.4, 8.10.10.5, 8.10.10.6, 8.10.10.7, 8.10.10.8, |
| 8.10.10.9, 8.10.10.10, 9.1.1.1, 9.1.1.2, 9.1.1.3, 9.1.1.4, 9.1.1.5, 9.1.1.6, 9.1.1.7, 9.1.1.8, 9.1.1.9, 9.1.1.10, 9.1.2.1, |
| 9.1.2.2, 9.1.2.3, 9.1.2.4, 9.1.2.5, 9.1.2.6, 9.1.2.7, 9.1.2.8, 9.1.2.9, 9.1.2.10, 9.1.3.1, 9.1.3.2, 9.1.3.3, 9.1.3.4, |
| 9.1.3.5, 9.1.3.6, 9.1.3.7, 9.1.3.8, 9.1.3.9, 9.1.3.10, 9.1.4.1, 9.1.4.2, 9.1.4.3, 9.1.4.4, 9.1.4.5, 9.1.4.6, 9.1.4.7, |
| 9.1.4.8, 9.1.4.9, 9.1.4.10, 9.1.5.1, 9.1.5.2, 9.1.5.3, 9.1.5.4, 9.1.5.5, 9.1.5.6, 9.1.5.7, 9.1.5.8, 9.1.5.9, 9.1.5.10, |
| 9.1.6.1, 9.1.6.2, 9.1.6.3, 9.1.6.4, 9.1.6.5, 9.1.6.6, 9.1.6.7, 9.1.6.8, 9.1.6.9, 9.1.6.10, 9.1.7.1, 9.1.7.2, 9.1.7.3, |
| 9.1.7.4, 9.1.7.5, 9.1.7.6, 9.1.7.7, 9.1.7.8, 9.1.7.9, 9.1.7.10, 9.1.8.1, 9.1.8.2, 9.1.8.3, 9.1.8.4, 9.1.8.5, 9.1.8.6, |
| 9.1.8.7, 9.1.8.8, 9.1.8.9, 9.1.8.10, 9.1.9.1, 9.1.9.2, 9.1.9.3, 9.1.9.4, 9.1.9.5, 9.1.9.6, 9.1.9.7, 9.1.9.8, 9.1.9.9, |
| 9.1.9.10, 9.1.10.1, 9.1.10.2, 9.1.10.3, 9.1.10.4, 9.1.10.5, 9.1.10.6, 9.1.10.7, 9.1.10.8, 9.1.10.9, 9.1.10.10, 9.2.1.1, |
| 9.2.1.2, 9.2.1.3, 9.2.1.4, 9.2.1.5, 9.2.1.6, 9.2.1.7, 9.2.1.8, 9.2.1.9, 9.2.1.10, 9.2.2.1, 9.2.2.2, 9.2.2.3, 9.2.2.4, |
| 9.2.2.5, 9.2.2.6, 9.2.2.7, 9.2.2.8, 9.2.2.9, 9.2.2.10, 9.2.3.1, 9.2.3.2, 9.2.3.3, 9.2.3.4, 9.2.3.5, 9.2.3.6, 9.2.3.7, |
| 9.2.3.8, 9.2.3.9, 9.2.3.10, 9.2.4.1, 9.2.4.2, 9.2.4.3, 9.2.4.4, 9.2.4.5, 9.2.4.6, 9.2.4.7, 9.2.4.8, 9.2.4.9, 9.2.4.10, |
| 9.2.5.1, 9.2.5.2, 9.2.5.3, 9.2.5.4, 9.2.5.5, 9.2.5.6, 9.2.5.7, 9.2.5.8, 9.2.5.9, 9.2.5.10, 9.2.6.1, 9.2.6.2, 9.2.6.3, |
| 9.2.6.4, 9.2.6.5, 9.2.6.6, 9.2.6.7, 9.2.6.8, 9.2.6.9, 9.2.6.10, 9.2.7.1, 9.2.7.2, 9.2.7.3, 9.2.7.4, 9.2.7.5, 9.2.7.6, |
| 9.2.7.7, 9.2.7.8, 9.2.7.9, 9.2.7.10, 9.2.8.1, 9.2.8.2, 9.2.8.3, 9.2.8.4, 9.2.8.5, 9.2.8.6, 9.2.8.7, 9.2.8.8, 9.2.8.9, |
| 9.2.8.10, 9.2.9.1, 9.2.9.2, 9.2.9.3, 9.2.9.4, 9.2.9.5, 9.2.9.6, 9.2.9.7, 9.2.9.8, 9.2.9.9, 9.2.9.10, 9.2.10.1,9.2.10.2, |
| 9.2.10.3, 9.2.10.4, 9.2.10.5, 9.2.10.6, 9.2.10.7, 9.2.10.8, 9.2.10.9, 9.2.10.10, 9.3.1.1, 9.3.1.2, 9.3.1.3, 9.3.1.4, |
| 9.3.1.5, 9.3.1.6, 9.3.1.7, 9.3.1.8, 9.3.1.9, 9.3.1.10, 9.3.2.1, 9.3.2.2, 9.3.2.3, 9.3.2.4, 9.3.2.5, 9.3.2.6, 9.3.2.7, |
| 9.3.2.8, 9.3.2.9, 9.3.2.10, 9.3.3.1, 9.3.3.2, 9.3.3.3, 9.3.3.4, 9.3.3.5, 9.3.3.6, 9.3.3.7, 9.3.3.8, 9.3.3.9, 9.3.3.10, |
| 9.3.4.1, 9.3.4.2, 9.3.4.3, 9.3.4.4, 9.3.4.5, 9.3.4.6, 9.3.4.7, 9.3.4.8, 9.3.4.9, 9.3.4.10, 9.3.5.1, 9.3.5.2, 9.3.5.3, |
| 9.3.5.4, 9.3.5.5, 9.3.5.6, 9.3.5.7, 9.3.5.8, 9.3.5.9, 9.3.5.10, 9.3.6.1, 9.3.6.2, 9.3.6.3, 9.3.6.4, 9.3.6.5, 9.3.6.6, |
| 9.3.6.7, 9.3.6.8, 9.3.6.9, 9.3.6.10, 9.3.7.1, 9.3.7.2, 9.3.7.3, 9.3.7.4, 9.3.7.5, 9.3.7.6, 9.3.7.7, 9.3.7.8, 9.3.7.9, |
| 9.3.7.10, 9.3.8.1, 9.3.8.2, 9.3.8.3, 9.3.8.4, 9.3.8.5, 9.3.8.6, 9.3.8.7, 9.3.8.8, 9.3.8.9, 9.3.8.10, 9.3.9.1, 9.3.9.2, |
| 9.3.9.3, 9.3.9.4, 9.3.9.5, 9.3.9.6, 9.3.9.7, 9.3.9.8, 9.3.9.9, 9.3.9.10, 9.3.10.1, 9.3.10.2, 9.3.10.3, 9.3.10.4, 9.3.10.5, |
| 9.3.10.6, 9.3.10.7, 9.3.10.8, 9.3.10.9, 9.3.10.10, 9.4.1.1, 9.4.1.2, 9.4.1.3, 9.4.1.4, 9.4.1.5, 9.4.1.6, 9.4.1.7, 9.4.1.8, |
| 9.4.1.9, 9.4.1.10, 9.4.2.1, 9.4.2.2, 9.4.2.3, 9.4.2.4, 9.4.2.5, 9.4.2.6, 9.4.2.7, 9.4.2.8, 9.4.2.9, 9.4.2.10, 9.4.3.1, |
| 9.4.3.2, 9.4.3.3, 9.4.3.4, 9.4.3.5, 9.4.3.6, 9.4.3.7, 9.4.3.8, 9.4.3.9, 9.4.3.10, 9.4.4.1, 9.4.4.2, 9.4.4.3, 9.4.4.4, |
| 9.4.4.5, 9.4.4.6, 9.4.4.7, 9.4.4.8, 9.4.4.9, 9.4.4.10, 9.4.5.1, 9.4.5.2, 9.4.5.3, 9.4.5.4, 9.4.5.5, 9.4.5.6, 9.4.5.7, |
| 9.4.5.8, 9.4.5.9, 9.4.5.10, 9.4.6.1, 9.4.6.2, 9.4.6.3, 9.4.6.4, 9.4.6.5, 9.4.6.6, 9.4.6.7, 9.4.6.8, 9.4.6.9, 9.4.6.10, |
| 9.4.7.1, 9.4.7.2, 9.4.7.3, 9.4.7.4, 9.4.7.5, 9.4.7.6, 9.4.7.7, 9.4.7.8, 9.4.7.9, 9.4.7.10, 9.4.8.1, 9.4.8.2, 9.4.8.3, |
| 9.4.8.4, 9.4.8.5, 9.4.8.6, 9.4.8.7, 9.4.8.8, 9.4.8.9, 9.4.8.10, 9.4.9.1, 9.4.9.2, 9.4.9.3, 9.4.9.4, 9.4.9.5, 9.4.9.6, |
| 9.4.9.7, 9.4.9.8, 9.4.9.9, 9.4.9.10, 9.4.10.1, 9.4.10.2, 9.4.10.3, 9.4.10.4, 9.4.10.5, 9.4.10.6, 9.4.10.7, 9.4.10.8, |
| 9.4.10.9, 9.4.10.10, 9.5.1.1, 9.5.1.2, 9.5.1.3, 9.5.1.4, 9.5.1.5, 9.5.1.6, 9.5.1.7, 9.5.1.8, 9.5.1.9, 9.5.1.10, 9.5.2.1, |
| 9.5.2.2, 9.5.2.3, 9.5.2.4, 9.5.2.5, 9.5.2.6, 9.5.2.7, 9.5.2.8, 9.5.2.9, 9.5.2.10, 9.5.3.1, 9.5.3.2, 9.5.3.3, 9.5.3.4, |
| 9.5.3.5, 9.5.3.6, 9.5.3.7, 9.5.3.8, 9.5.3.9, 9.5.3.10, 9.5.4.1, 9.5.4.2, 9.5.4.3, 9.5.4.4, 9.5.4.5, 9.5.4.6, 9.5.4.7, |
| 9.5.4.8, 9.5.4.9, 9.5.4.10, 9.5.5.1, 9.5.5.2, 9.5.5.3, 9.5.5.4, 9.5.5.5, 9.5.5.6, 9.5.5.7, 9.5.5.8, 9.5.5.9, 9.5.5.10, |
| 9.5.6.1, 9.5.6.2, 9.5.6.3, 9.5.6.4, 9.5.6.5, 9.5.6.6, 9.5.6.7, 9.5.6.8, 9.5.6.9, 9.5.6.10, 9.5.7.1, 9.5.7.2, 9.5.7.3, |
| 9.5.7.4, 9.5.7.5, 9.5.7.6, 9.5.7.7, 9.5.7.8, 9.5.7.9, 9.5.7.10, 9.5.8.1, 9.5.8.2, 9.5.8.3, 9.5.8.4, 9.5.8.5, 9.5.8.6, |
| 9.5.8.7, 9.5.8.8, 9.5.8.9, 9.5.8.10, 9.5.9.1, 9.5.9.2, 9.5.9.3, 9.5.9.4, 9.5.9.5, 9.5.9.6, 9.5.9.7, 9.5.9.8, 9.5.9.9, |
| 9.5.9.10, 9.5.10.1, 9.5.10.2, 9.5.10.3, 9.5.10.4, 9.5.10.5, 9.5.10.6, 9.5.10.7, 9.5.10.8, 9.5.10.9, 9.5.10.10, 9.6.1.1, |
| 9.6.1.2, 9.6.1.3, 9.6.1.4, 9.6.1.5, 9.6.1.6, 9.6.1.7, 9.6.1.8, 9.6.1.9, 9.6.1.10, 9.6.2.1, 9.6.2.2, 9.6.2.3, 9.6.2.4, |
| 9.6.2.5, 9.6.2.6, 9.6.2.7, 9.6.2.8, 9.6.2.9, 9.6.2.10, 9.6.3.1, 9.6.3.2, 9.6.3.3, 9.6.3.4, 9.6.3.5, 9.6.3.6, 9.6.3.7, |
| 9.6.3.8, 9.6.3.9, 9.6.3.10, 9.6.4.1, 9.6.4.2, 9.6.4.3, 9.6.4.4, 9.6.4.5, 9.6.4.6, 9.6.4.7, 9.6.4.8, 9.6.4.9, 9.6.4.10, |
| 9.6.5.1, 9.6.5.2, 9.6.5.3, 9.6.5.4, 9.6.5.5, 9.6.5.6, 9.6.5.7, 9.6.5.8, 9.6.5.9, 9.6.5.10, 9.6.6.1, 9.6.6.2, 9.6.6.3, |
| 9.6.6.4, 9.6.6.5, 9.6.6.6, 9.6.6.7, 9.6.6.8, 9.6.6.9, 9.6.6.10, 9.6.7.1, 9.6.7.2, 9.6.7.3, 9.6.7.4, 9.6.7.5, 9.6.7.6, |
| 9.6.7.7, 9.6.7.8, 9.6.7.9, 9.6.7.10, 9.6.8.1, 9.6.8.2, 9.6.8.3, 9.6.8.4, 9.6.8.5, 9.6.8.6, 9.6.8.7, 9.6.8.8, 9.6.8.9, |
| 9.6.8.10, 9.6.9.1, 9.6.9.2, 9.6.9.3, 9.6.9.4, 9.6.9.5, 9.6.9.6, 9.6.9.7, 9.6.9.8, 9.6.9.9, 9.6.9.10, 9.6.10.1, 9.6.10.2, |
| 9.6.10.3, 9.6.10.4, 9.6.10.5, 9.6.10.6, 9.6.10.7, 9.6.10.8, 9.6.10.9, 9.6.10.10, 9.7.1.1, 9.7.1.2, 9.7.1.3, 9.7.1.4, |
| 9.7.1.5, 9.7.1.6, 9.7.1.7, 9.7.1.8, 9.7.1.9, 9.7.1.10, 9.7.2.1, 9.7.2.2, 9.7.2.3, 9.7.2.4, 9.7.2.5, 9.7.2.6, 9.7.2.7, |
| 9.7.2.8, 9.7.2.9, 9.7.2.10, 9.7.3.1, 9.7.3.2, 9.7.3.3, 9.7.3.4, 9.7.3.5, 9.7.3.6, 9.7.3.7, 9.7.3.8, 9.7.3.9, 9.7.3.10, |
| 9.7.4.1, 9.7.4.2, 9.7.4.3, 9.7.4.4, 9.7.4.5, 9.7.4.6, 9.7.4.7, 9.7.4.8, 9.7.4.9, 9.7.4.10, 9.7.5.1, 9.7.5.2, 9.7.5.3, |
| 9.7.5.4, 9.7.5.5, 9.7.5.6, 9.7.5.7, 9.7.5.8, 9.7.5.9, 9.7.5.10, 9.7.6.1, 9.7.6.2, 9.7.6.3, 9.7.6.4, 9.7.6.5, 9.7.6.6, |
| 9.7.6.7, 9.7.6.8, 9.7.6.9, 9.7.6.10, 9.7.7.1, 9.7.7.2, 9.7.7.3, 9.7.7.4, 9.7.7.5, 9.7.7.6, 9.7.7.7, 9.7.7.8, 9.7.7.9, |
| 9.7.7.10, 9.7.8.1, 9.7.8.2, 9.7.8.3, 9.7.8.4, 9.7.8.5, 9.7.8.6, 9.7.8.7, 9.7.8.8, 9.7.8.9, 9.7.8.10, 9.7.9.1, 9.7.9.2, |
| 9.7.9.3, 9.7.9.4, 9.7.9.5, 9.7.9.6, 9.7.9.7, 9.7.9.8, 9.7.9.9, 9.7.9.10, 9.7.10.1, 9.7.10.2, 9.7.10.3, 9.7.10.4, 9.7.10.5, |
| 9.7.10.6, 9.7.10.7, 9.7.10.8, 9.7.10.9, 9.7.10.10, 9.8.1.1, 9.8.1.2, 9.8.1.3, 9.8.1.4, 9.8.1.5, 9.8.1.6, 9.8.1.7, 9.8.1.8, |
| 9.8.1.9, 9.8.1.10, 9.8.2.1, 9.8.2.2, 9.8.2.3, 9.8.2.4, 9.8.2.5, 9.8.2.6, 9.8.2.7, 9.8.2.8, 9.8.2.9, 9.8.2.10, 9.8.3.1, |
| 9.8.3.2, 9.8.3.3, 9.8.3.4, 9.8.3.5, 9.8.3.6, 9.8.3.7, 9.8.3.8, 9.8.3.9, 9.8.3.10, 9.8.4.1, 9.8.4.2, 9.8.4.3, 9.8.4.4, |
| 9.8.4.5, 9.8.4.6, 9.8.4.7, 9.8.4.8, 9.8.4.9, 9.8.4.10, 9.8.5.1, 9.8.5.2, 9.8.5.3, 9.8.5.4, 9.8.5.5, 9.8.5.6, 9.8.5.7, |
| 9.8.5.8, 9.8.5.9, 9.8.5.10, 9.8.6.1, 9.8.6.2, 9.8.6.3, 9.8.6.4, 9.8.6.5, 9.8.6.6, 9.8.6.7, 9.8.6.8, 9.8.6.9, 9.8.6.10, |
| 9.8.7.1, 9.8.7.2, 9.8.7.3, 9.8.7.4, 9.8.7.5, 9.8.7.6, 9.8.7.7, 9.8.7.8, 9.8.7.9, 9.8.7.10, 9.8.8.1, 9.8.8.2, 9.8.8.3, |
| 9.8.8.4, 9.8.8.5, 9.8.8.6, 9.8.8.7, 9.8.8.8, 9.8.8.9, 9.8.8.10, 9.8.9.1, 9.8.9.2, 9.8.9.3, 9.8.9.4, 9.8.9.5, 9.8.9.6, |
| 9.8.9.7, 9.8.9.8, 9.8.9.9, 9.8.9.10, 9.8.10.1, 9.8.10.2, 9.8.10.3, 9.8.10.4, 9.8.10.5, 9.8.10.6, 9.8.10.7, 9.8.10.8, |
| 9.8.10.9, 9.8.10.10, 9.9.1.1, 9.9.1.2, 9.9.1.3, 9.9.1.4, 9.9.1.5, 9.9.1.6, 9.9.1.7, 9.9.1.8, 9.9.1.9, 9.9.1.10, 9.9.2.1, |
| 9.9.2.2, 9.9.2.3, 9.9.2.4, 9.9.2.5, 9.9.2.6, 9.9.2.7, 9.9.2.8, 9.9.2.9, 9.9.2.10, 9.9.3.1, 9.9.3.2, 9.9.3.3, 9.9.3.4, |
| 9.9.3.5, 9.9.3.6, 9.9.3.7, 9.9.3.8, 9.9.3.9, 9.9.3.10, 9.9.4.1, 9.9.4.2, 9.9.4.3, 9.9.4.4, 9.9.4.5, 9.9.4.6, 9.9.4.7, |
| 9.9.4.8, 9.9.4.9, 9.9.4.10, 9.9.5.1, 9.9.5.2, 9.9.5.3, 9.9.5.4, 9.9.5.5, 9.9.5.6, 9.9.5.7, 9.9.5.8, 9.9.5.9, 9.9.5.10, |
| 9.9.6.1, 9.9.6.2, 9.9.6.3, 9.9.6.4, 9.9.6.5, 9.9.6.6, 9.9.6.7, 9.9.6.8, 9.9.6.9, 9.9.6.10, 9.9.7.1, 9.9.7.2, 9.9.7.3, |
| 9.9.7.4, 9.9.7.5, 9.9.7.6, 9.9.7.7, 9.9.7.8, 9.9.7.9, 9.9.7.10, 9.9.8.1, 9.9.8.2, 9.9.8.3, 9.9.8.4, 9.9.8.5, 9.9.8.6, |
| 9.9.8.7, 9.9.8.8, 9.9.8.9, 9.9.8.10, 9.9.9.1, 9.9.9.2, 9.9.9.3, 9.9.9.4, 9.9.9.5, 9.9.9.6, 9.9.9.7, 9.9.9.8, 9.9.9.9, |
| 9.9.9.10, 9.9.10.1, 9.9.10.2, 9.9.10.3, 9.9.10.4, 9.9.10.5, 9.9.10.6, 9.9.10.7, 9.9.10.8, 9.9.10.9, 9.9.10.10, |
| 9.10.1.1, 9.10.1.2, 9.10.1.3, 9.10.1.4, 9.10.1.5, 9.10.1.6, 9.10.1.7, 9.10.1.8, 9.10.1.9, 9.10.1.10, 9.10.2.1, |
| 9.10.2.2, 9.10.2.3, 9.10.2.4, 9.10.2.5, 9.10.2.6, 9.10.2.7, 9.10.2.8, 9.10.2.9, 9.10.2.10, 9.10.3.1, 9.10.3.2, |
| 9.10.3.3, 9.10.3.4, 9.10.3.5, 9.10.3.6, 9.10.3.7, 9.10.3.8, 9.10.3.9, 9.10.3.10, 9.10.4.1, 9.10.4.2, 9.10.4.3, |
| 9.10.4.4, 9.10.4.5, 9.10.4.6, 9.10.4.7, 9.10.4.8, 9.10.4.9, 9.10.4.10, 9.10.5.1, 9.10.5.2, 9.10.5.3, 9.10.5.4, |
| 9.10.5.5, 9.10.5.6, 9.10.5.7, 9.10.5.8, 9.10.5.9, 9.10.5.10, 9.10.6.1, 9.10.6.2, 9.10.6.3, 9.10.6.4, 9.10.6.5, |
| 9.10.6.6, 9.10.6.7, 9.10.6.8, 9.10.6.9, 9.10.6.10, 9.10.7.1, 9.10.7.2, 9.10.7.3, 9.10.7.4, 9.10.7.5, 9.10.7.6, |
| 9.10.7.7, 9.10.7.8, 9.10.7.9, 9.10.7.10, 9.10.8.1, 9.10.8.2, 9.10.8.3, 9.10.8.4, 9.10.8.5, 9.10.8.6, 9.10.8.7, |
| 9.10.8.8, 9.10.8.9, 9.10.8.10, 9.10.9.1, 9.10.9.2, 9.10.9.3, 9.10.9.4, 9.10.9.5, 9.10.9.6, 9.10.9.7, 9.10.9.8, |
| 9.10.9.9, 9.10.9.10, 9.10.10.1, 9.10.10.2, 9.10.10.3, 9.10.10.4, 9.10.10.5, 9.10.10.6, 9.10.10.7, 9.10.10.8, |
| 9.10.10.9, 9.10.10.10, 10.1.1.1, 10.1.1.2, 10.1.1.3, 10.1.1.4, 10.1.1.5, 10.1.1.6, 10.1.1.7, 10.1.1.8, 10.1.1.9, |
| 10.1.1.10, 10.1.2.1, 10.1.2.2, 10.1.2.3, 10.1.2.4, 10.1.2.5, 10.1.2.6, 10.1.2.7, 10.1.2.8, 10.1.2.9, 10.1.2.10, |
| 10.1.3.1, 10.1.3.2, 10.1.3.3, 10.1.3.4, 10.1.3.5, 10.1.3.6, 10.1.3.7, 10.1.3.8, 10.1.3.9, 10.1.3.10, 10.1.4.1, |
| 10.1.4.2, 10.1.4.3, 10.1.4.4, 10.1.4.5, 10.1.4.6, 10.1.4.7, 10.1.4.8, 10.1.4.9, 10.1.4.10, 10.1.5.1, 10.1.5.2, |
| 10.1.5.3, 10.1.5.4, 10.1.5.5, 10.1.5.6, 10.1.5.7, 10.1.5.8, 10.1.5.9, 10.1.5.10, 10.1.6.1, 10.1.6.2, 10.1.6.3, |
| 10.1.6.4, 10.1.6.5, 10.1.6.6, 10.1.6.7, 10.1.6.8, 10.1.6.9, 10.1.6.10, 10.1.7.1, 10.1.7.2, 10.1.7.3, 10.1.7.4, |
| 10.1.7.5, 10.1.7.6, 10.1.7.7, 10.1.7.8, 10.1.7.9, 10.1.7.10, 10.1.8.1, 10.1.8.2, 10.1.8.3, 10.1.8.4, 10.1.8.5, |
| 10.1.8.6, 10.1.8.7, 10.1.8.8, 10.1.8.9, 10.1.8.10, 10.1.9.1, 10.1.9.2, 10.1.9.3, 10.1.9.4, 10.1.9.5, 10.1.9.6, |
| 10.1.9.7, 10.1.9.8, 10.1.9.9, 10.1.9.10, 10.1.10.1, 10.1.10.2, 10.1.10.3, 10.1.10.4, 10.1.10.5, 10.1.10.6, 10.1.10.7, |
| 10.1.10.8, 10.1.10.9, 10.1.10.10, 10.2.1.1, 10.2.1.2, 10.2.1.3, 10.2.1.4, 10.2.1.5, 10.2.1.6, 10.2.1.7, 10.2.1.8, |
| 10.2.1.9, 10.2.1.10, 10.2.2.1, 10.2.2.2, 10.2.2.3, 10.2.2.4, 10.2.2.5, 10.2.2.6, 10.2.2.7, 10.2.2.8, 10.2.2.9, |
| 10.2.2.10, 10.2.3.1, 10.2.3.2, 10.2.3.3, 10.2.3.4, 10.2.3.5, 10.2.3.6, 10.2.3.7, 10.2.3.8, 10.2.3.9, 10.2.3.10, |
| 10.2.4.1, 10.2.4.2, 10.2.4.3, 10.2.4.4, 10.2.4.5, 10.2.4.6, 10.2.4.7, 10.2.4.8, 10.2.4.9, 10.2.4.10, 10.2.5.1, |
| 10.2.5.2, 10.2.5.3, 10.2.5.4, 10.2.5.5, 10.2.5.6, 10.2.5.7, 10.2.5.8, 10.2.5.9, 10.2.5.10, 10.2.6.1, 10.2.6.2, |
| 10.2.6.3, 10.2.6.4, 10.2.6.5, 10.2.6.6, 10.2.6.7, 10.2.6.8, 10.2.6.9, 10.2.6.10, 10.2.7.1, 10.2.7.2, 10.2.7.3, |
| 10.2.7.4, 10.2.7.5, 10.2.7.6, 10.2.7.7, 10.2.7.8, 10.2.7.9, 10.2.7.10, 10.2.8.1, 10.2.8.2, 10.2.8.3, 10.2.8.4, |
| 10.2.8.5, 10.2.8.6, 10.2.8.7, 10.2.8.8, 10.2.8.9, 10.2.8.10, 10.2.9.1, 10.2.9.2, 10.2.9.3, 10.2.9.4, 10.2.9.5, |
| 10.2.9.6, 10.2.9.7, 10.2.9.8, 10.2.9.9, 10.2.9.10, 10.2.10.1, 10.2.10.2, 10.2.10.3, 10.2.10.4, 10.2.10.5, 10.2.10.6, |
| 10.2.10.7, 10.2.10.8, 10.2.10.9, 10.2.10.10, 10.3.1.1, 10.3.1.2, 10.3.1.3, 10.3.1.4, 10.3.1.5, 10.3.1.6, 10.3.1.7, |
| 10.3.1.8, 10.3.1.9, 10.3.1.10, 10.3.2.1, 10.3.2.2, 10.3.2.3, 10.3.2.4, 10.3.2.5, 10.3.2.6, 10.3.2.7, 10.3.2.8, |
| 10.3.2.9, 10.3.2.10, 10.3.3.1, 10.3.3.2, 10.3.3.3, 10.3.3.4, 10.3.3.5, 10.3.3.6, 10.3.3.7, 10.3.3.8, 10.3.3.9, |
| 10.3.3.10, 10.3.4.1, 10.3.4.2, 10.3.4.3, 10.3.4.4, 10.3.4.5, 10.3.4.6, 10.3.4.7, 10.3.4.8, 10.3.4.9, 10.3.4.10, |
| 10.3.5.1, 10.3.5.2, 10.3.5.3, 10.3.5.4, 10.3.5.5, 10.3.5.6, 10.3.5.7, 10.3.5.8, 10.3.5.9, 10.3.5.10, 10.3.6.1, |
| 10.3.6.2, 10.3.6.3, 10.3.6.4, 10.3.6.5, 10.3.6.6, 10.3.6.7, 10.3.6.8, 10.3.6.9, 10.3.6.10, 10.3.7.1, 10.3.7.2, |
| 10.3.7.3, 10.3.7.4, 10.3.7.5, 10.3.7.6, 10.3.7.7, 10.3.7.8, 10.3.7.9, 10.3.7.10, 10.3.8.1, 10.3.8.2, 10.3.8.3, |
| 10.3.8.4, 10.3.8.5, 10.3.8.6, 10.3.8.7, 10.3.8.8, 10.3.8.9, 10.3.8.10, 10.3.9.1, 10.3.9.2, 10.3.9.3, 10.3.9.4, |
| 10.3.9.5, 10.3.9.6, 10.3.9.7, 10.3.9.8, 10.3.9.9, 10.3.9.10, 10.3.10.1, 10.3.10.2, 10.3.10.3, 10.3.10.4, 10.3.10.5, |
| 10.3.10.6, 10.3.10.7, 10.3.10.8, 10.3.10.9, 10.3.10.10, 10.4.1.1, 10.4.1.2, 10.4.1.3, 10.4.1.4, 10.4.1.5, 10.4.1.6, |
| 10.4.1.7, 10.4.1.8, 10.4.1.9, 10.4.1.10, 10.4.2.1, 10.4.2.2, 10.4.2.3, 10.4.2.4, 10.4.2.5, 10.4.2.6, 10.4.2.7, |
| 10.4.2.8, 10.4.2.9, 10.4.2.10, 10.4.3.1, 10.4.3.2, 10.4.3.3, 10.4.3.4, 10.4.3.5, 10.4.3.6, 10.4.3.7, 10.4.3.8, |
| 10.4.3.9, 10.4.3.10, 10.4.4.1, 10.4.4.2, 10.4.4.3, 10.4.4.4, 10.4.4.5, 10.4.4.6, 10.4.4.7, 10.4.4.8, 10.4.4.9, |
| 10.4.4.10, 10.4.5.1, 10.4.5.2, 10.4.5.3, 10.4.5.4, 10.4.5.5, 10.4.5.6, 10.4.5.7, 10.4.5.8, 10.4.5.9, 10.4.5.10, |
| 10.4.6.1, 10.4.6.2, 10.4.6.3, 10.4.6.4, 10.4.6.5, 10.4.6.6, 10.4.6.7, 10.4.6.8, 10.4.6.9, 10.4.6.10, 10.4.7.1, |
| 10.4.7.2, 10.4.7.3, 10.4.7.4, 10.4.7.5, 10.4.7.6, 10.4.7.7, 10.4.7.8, 10.4.7.9, 10.4.7.10, 10.4.8.1, 10.4.8.2, |
| 10.4.8.3, 10.4.8.4, 10.4.8.5, 10.4.8.6, 10.4.8.7, 10.4.8.8, 10.4.8.9, 10.4.8.10, 10.4.9.1, 10.4.9.2, 10.4.9.3, |
| 10.4.9.4, 10.4.9.5, 10.4.9.6, 10.4.9.7, 10.4.9.8, 10.4.9.9, 10.4.9.10, 10.4.10.1, 10.4.10.2, 10.4.10.3, 10.4.10.4, |
| 10.4.10.5, 10.4.10.6, 10.4.10.7, 10.4.10.8, 10.4.10.9, 10.4.10.10, 10.5.1.1, 10.5.1.2, 10.5.1.3, 10.5.1.4, 10.5.1.5, |
| 10.5.1.6, 10.5.1.7, 10.5.1.8, 10.5.1.9, 10.5.1.10, 10.5.2.1, 10.5.2.2, 10.5.2.3, 10.5.2.4, 10.5.2.5, 10.5.2.6, |
| 10.5.2.7, 10.5.2.8, 10.5.2.9, 10.5.2.10, 10.5.3.1, 10.5.3.2, 10.5.3.3, 10.5.3.4, 10.5.3.5, 10.5.3.6, 10.5.3.7, |
| 10.5.3.8, 10.5.3.9, 10.5.3.10, 10.5.4.1, 10.5.4.2, 10.5.4.3, 10.5.4.4, 10.5.4.5, 10.5.4.6, 10.5.4.7, 10.5.4.8, |
| 10.5.4.9, 10.5.4.10, 10.5.5.1, 10.5.5.2, 10.5.5.3, 10.5.5.4, 10.5.5.5, 10.5.5.6, 10.5.5.7, 10.5.5.8, 10.5.5.9, |
| 10.5.5.10, 10.5.6.1, 10.5.6.2, 10.5.6.3, 10.5.6.4, 10.5.6.5, 10.5.6.6, 10.5.6.7, 10.5.6.8, 10.5.6.9, 10.5.6.10, |
| 10.5.7.1, 10.5.7.2, 10.5.7.3, 10.5.7.4, 10.5.7.5, 10.5.7.6, 10.5.7.7, 10.5.7.8, 10.5.7.9, 10.5.7.10, 10.5.8.1, |
| 10.5.8.2, 10.5.8.3, 10.5.8.4, 10.5.8.5, 10.5.8.6, 10.5.8.7, 10.5.8.8, 10.5.8.9, 10.5.8.10, 10.5.9.1, 10.5.9.2, |
| 10.5.9.3, 10.5.9.4, 10.5.9.5, 10.5.9.6, 10.5.9.7, 10.5.9.8, 10.5.9.9, 10.5.9.10, 10.5.10.1, 10.5.10.2, 10.5.10.3, |
| 10.5.10.4, 10.5.10.5, 10.5.10.6, 10.5.10.7, 10.5.10.8, 10.5.10.9, 10.5.10.10, 10.6.1.1, 10.6.1.2, 10.6.1.3, 10.6.1.4, |
| 10.6.1.5, 10.6.1.6, 10.6.1.7, 10.6.1.8, 10.6.1.9, 10.6.1.10, 10.6.2.1, 10.6.2.2, 10.6.2.3, 10.6.2.4, 10.6.2.5, |
| 10.6.2.6, 10.6.2.7, 10.6.2.8, 10.6.2.9, 10.6.2.10, 10.6.3.1, 10.6.3.2, 10.6.3.3, 10.6.3.4, 10.6.3.5, 10.6.3.6, |
| 10.6.3.7, 10.6.3.8, 10.6.3.9, 10.6.3.10, 10.6.4.1, 10.6.4.2, 10.6.4.3, 10.6.4.4, 10.6.4.5, 10.6.4.6, 10.6.4.7, |
| 10.6.4.8, 10.6.4.9, 10.6.4,10, 10.6.5,1, 10.6.5.2, 10.6.5.3, 10.6.5.4, 10.6.5.5, 10.6.5.6, 10.6.5.7, 10.6.5.8, |
| 10.6.5.9, 10.6.5,10, 10.6.6,1, 10.6.6.2, 10.6.6.3, 10.6.6.4, 10.6.6.5, 10.6.6.6, 10.6.6.7, 10.6.6.8, 10.6.6.9, |
| 10.6.6,10, 10.6.7,1, 10.6.7.2, 10.6.7.3, 10.6.7.4, 10.6.7.5, 10.6.7.6, 10.6.7.7, 10.6.7.8, 10.6.7.9, 10.6.7,10, |
| 10.6.8,1, 10.6.8.2, 10.6.8.3, 10.6.8.4, 10.6.8.5, 10.6.8.6, 10.6.8.7, 10.6.8.8, 10.6.8.9, 10.6.8,10, 10.6.9,1, |
| 10.6.9.2, 10.6.9.3, 10.6.9.4, 10.6.9.5, 10.6.9.6, 10.6.9.7, 10.6.9.8, 10.6.9.9, 10.6.9,10, 10.6,10,1, 10.6,10.2, |
| 10.6,10.3, 10.6,10.4, 10.6,10.5, 10.6,10.6, 10.6.10.7, 10.6.10.8, 10.6.10.9, 10.6.10.10, 10.7.1.1, 10.7.1.2, |
| 10.7.1.3, 10.7.1.4, 10.7.1.5, 10.7.1.6, 10.7.1.7, 10.7.1.8, 10.7.1.9, 10.7.1.10, 10.7.2.1, 10.7.2.2, 10.7.2.3, |
| 10.7.2.4, 10.7.2.5, 10.7.2.6, 10.7.2.7, 10.7.2.8, 10.7.2.9, 10.7.2.10, 10.7.3.1, 10.7.3.2, 10.7.3.3, 10.7.3.4, |
| 10.7.3.5, 10.7.3.6, 10.7.3.7, 10.7.3.8, 10.7.3.9, 10.7.3.10, 10.7.4.1, 10.7.4.2, 10.7.4.3, 10.7.4.4, 10.7.4.5, |
| 10.7.4.6, 10.7.4.7, 10.7.4.8, 10.7.4.9, 10.7.4.10, 10.7.5.1, 10.7.5.2, 10.7.5.3, 10.7.5.4, 10.7.5.5, 10.7.5.6, |
| 10.7.5.7, 10.7.5.8, 10.7.5.9, 10.7.5.10, 10.7.6.1, 10.7.6.2, 10.7.6.3, 10.7.6.4, 10.7.6.5, 10.7.6.6, 10.7.6.7, |
| 10.7.6.8, 10.7.6.9, 10.7.6.10, 10.7.7.1, 10.7.7.2, 10.7.7.3, 10.7.7.4, 10.7.7.5, 10.7.7.6, 10.7.7.7, 10.7.7.8, |
| 10.7.7.9, 10.7.7.10, 10.7.8.1, 10.7.8.2, 10.7.8.3, 10.7.8.4, 10.7.8.5, 10.7.8.6, 10.7.8.7, 10.7.8.8, 10.7.8.9, |
| 10.7.8.10, 10.7.9.1, 10.7.9.2, 10.7.9.3, 10.7.9.4, 10.7.9.5, 10.7.9.6, 10.7.9.7, 10.7.9.8, 10.7.9.9, 10.7.9.10, |
| 10.7.10.1, 10.7.10.2, 10.7.10.3, 10.7.10.4, 10.7.10.5, 10.7.10.6, 10.7.10.7, 10.7.10.8, 10.7.10.9, 10.7.10.10, |
| 10.8.1.1, 10.8.1.2, 10.8.1.3, 10.8.1.4, 10.8.1.5, 10.8.1.6, 10.8.1.7, 10.8.1.8, 10.8.1.9, 10.8.1.10, 10.8.2.1, |
| 10.8.2.2, 10.8.2.3, 10.8.2.4, 10.8.2.5, 10.8.2.6, 10.8.2.7, 10.8.2.8, 10.8.2.9, 10.8.2.10, 10.8.3.1, 10.8.3.2, |
| 10.8.3.3, 10.8.3.4, 10.8.3.5, 10.8.3.6, 10.8.3.7, 10.8.3.8, 10.8.3.9, 10.8.3.10, 10.8.4.1, 10.8.4.2, 10.8.4.3, |
| 10.8.4.4, 10.8.4.5, 10.8.4.6, 10.8.4.7, 10.8.4.8, 10.8.4.9, 10.8.4.10, 10.8.5.1, 10.8.5.2, 10.8.5.3, 10.8.5.4, |
| 10.8.5.5, 10.8.5.6, 10.8.5.7, 10.8.5.8, 10.8.5.9, 10.8.5.10, 10.8.6.1, 10.8.6.2, 10.8.6.3, 10.8.6.4, 10.8.6.5, |
| 10.8.6.6, 10.8.6.7, 10.8.6.8, 10.8.6.9, 10.8.6.10, 10.8.7.1, 10.8.7.2, 10.8.7.3, 10.8.7.4, 10.8.7.5, 10.8.7.6, |
| 10.8.7.7, 10.8.7.8, 10.8.7.9, 10.8.7.10, 10.8.8.1, 10.8.8.2, 10.8.8.3, 10.8.8.4, 10.8.8.5, 10.8.8.6, 10.8.8.7, |
| 10.8.8.8, 10.8.8.9, 10.8.8.10, 10.8.9.1, 10.8.9.2, 10.8.9.3, 10.8.9.4, 10.8.9.5, 10.8.9.6, 10.8.9.7, 10.8.9.8, |
| 10.8.9.9, 10.8.9.10, 10.8.10.1, 10.8.10.2, 10.8.10.3, 10.8.10.4, 10.8.10.5, 10.8.10.6, 10.8.10.7, 10.8.10.8, |
| 10.8.10.9, 10.8.10.10, 10.9.1.1, 10.9.1.2, 10.9.1.3, 10.9.1.4, 10.9.1.5, 10.9.1.6, 10.9.1.7, 10.9.1.8, 10.9.1.9, |
| 10.9.1.10, 10.9.2.1, 10.9.2.2, 10.9.2.3, 10.9.2.4, 10.9.2.5, 10.9.2.6, 10.9.2.7, 10.9.2.8, 10.9.2.9, 10.9.2.10, |
| 10.9.3.1, 10.9.3.2, 10.9.3.3, 10.9.3.4, 10.9.3.5, 10.9.3.6, 10.9.3.7, 10.9.3.8, 10.9.3.9, 10.9.3.10, 10.9.4.1, |
| 10.9.4.2, 10.9.4.3, 10.9.4.4, 10.9.4.5, 10.9.4.6, 10.9.4.7, 10.9.4.8, 10.9.4.9, 10.9.4.10, 10.9.5.1, 10.9.5.2, |
| 10.9.5.3, 10.9.5.4, 10.9.5.5, 10.9.5.6, 10.9.5.7, 10.9.5.8, 10.9.5.9, 10.9.5.10, 10.9.6.1, 10.9.6.2, 10.9.6.3, |
| 10.9.6.4, 10.9.6.5, 10.9.6.6, 10.9.6.7, 10.9.6.8, 10.9.6.9, 10.9.6.10, 10.9.7.1, 10.9.7.2, 10.9.7.3, 10.9.7.4, |
| 10.9.7.5, 10.9.7.6, 10.9.7.7, 10.9.7.8, 10.9.7.9, 10.9.7.10, 10.9.8.1, 10.9.8.2, 10.9.8.3, 10.9.8.4, 10.9.8.5, |
| 10.9.8.6, 10.9.8.7, 10.9.8.8, 10.9.8.9, 10.9.8.10, 10.9.9.1, 10.9.9.2, 10.9.9.3, 10.9.9.4,10.9.9.5, 10.9.9.6, |
| 10.9.9.7, 10.9.9.8, 10.9.9.9, 10.9.9.10, 10.9.10.1, 10.9.10.2, 10.9.10.3, 10.9.10.4, 10.9.10.5, 10.9.10.6, 10.9.10.7, |
| 10.9.10.8, 10.9.10.9, 10.9.10.10, 10.10.1.1, 10.10.1.2, 10.10.1.3, 10.10.1.4, 10.10.1.5, 10.10.1.6, 10.10.1.7, |
| 10.10.1.8, 10.10.1.9, 10.10.1.10, 10.10.2.1, 10.10.2.2, 10.10.2.3, 10.10.2.4, 10.10.2.5, 10.10.2.6, 10.10.2.7, |
| 10.10.2.8, 10.10.2.9, 10.10.2.10, 10.10.3.1, 10.10.3.2, 10.10.3.3, 10.10.3.4, 10.10.3.5, 10.10.3.6, 10.10.3.7, |
| 10.10.3.8, 10.10.3.9, 10.10.3.10, 10.10.4.1, 10.10.4.2, 10.10.4.3, 10.10.4.4, 10.10.4.5, 10.10.4.6, 10.10.4.7, |
| 10.10.4.8, 10.10.4.9, 10.10.4.10, 10.10.5.1, 10.10.5.2, 10.10.5.3, 10.10.5.4, 10.10.5.5, 10.10.5.6, 10.10.5.7, |
| 10.10.5.8, 10.10.5.9, 10.10.5.10, 10.10.6.1, 10.10.6.2, 10.10.6.3, 10.10.6.4, 10.10.6.5, 10.10.6.6, 10.10.6.7, |
| 10.10.6.8, 10.10.6.9, 10.10.6.10, 10.10.7.1, 10.10.7.2, 10.10.7.3, 10.10.7.4, 10.10.7.5, 10.10.7.6, 10.10.7.7, |
| 10.10.7.8, 10.10.7.9, 10.10.7.10, 10.10.8.1, 10.10.8.2, 10.10.8.3, 10.10.8.4, 10.10.8.5, 10.10.8.6, 10.10.8.7, |
| 10.10.8.8, 10.10.8.9, 10.10.8.10, 10.10.9.1, 10.10.9.2, 10.10.9.3, 10.10.9.4, 10.10.9.5, 10.10.9.6, 10.10.9.7, |
| 10.10.9.8,10.10.9.9,10.10.9.10, 10.10.10.1, 10.10.10.2, 10.10.10.3, 10.10.10.4, 10.10.10.5, 10.10.10.6, |
| 10.10.10.7, 10.10.10.8, 10.10.10.9, 10.10.10.10 |

Additional exemplary formula B compound groups include the following compound groups disclosed below. Unless otherwise specified, the configurations of all hydrogen atoms and R groups for the following compound groups are as defined for the group 1 compounds of formula B above.

**Group 2**. This group comprises compounds named in Table B having R¹, R², R³ and R⁴ substituents defined in Table A wherein the R¹, R², R³ and R⁴ substituents are bonded to the steroid nucleus described for group 1 compounds, except that a double bond at the 5-6 position is present. Thus, group 2 compound 1.3.1.1 has the structure

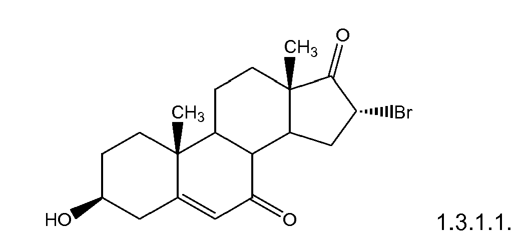

**Group 3**. This group comprises compounds named in Table B having R¹, R², R³ and R⁴ substituents defined in Table A wherein the R¹, R², R³ and R⁴ substituents are bonded to the steroid nucleus as described for group 1 compounds, except that double bonds at the 1-2- and 5-6 positions are present. Thus, group 3 compound 2.2.5.1 has the structure

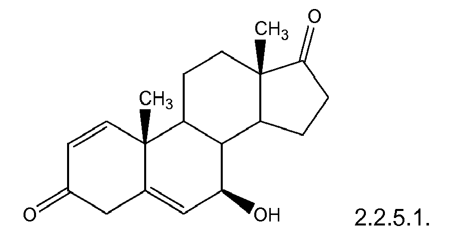

**Group 4**. This group comprises compounds named in Table B having R¹, R², R³ and R⁴ substituents defined in Table A wherein the R¹, R², R³ and R⁴ substituents are bonded to the steroid nucleus described for group 1 compounds, except that a double bond at the 1-2 position is present. Thus, group 4 compound 5.2.7.8 has the structure

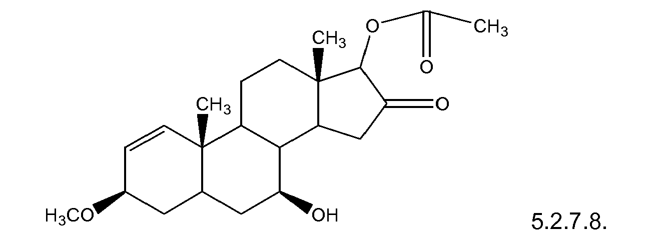

**Group 5**. This group comprises compounds named in Table B having R¹, R², R³ and R⁴ substituents defined in Table A wherein the R¹, R², R³ and R⁴ substituents are bonded to the steroid nucleus described for group 1 compounds, except that a double bond at the 4-5 position is present. Thus, the group 5 compound named 3.5.2.9 has the structure

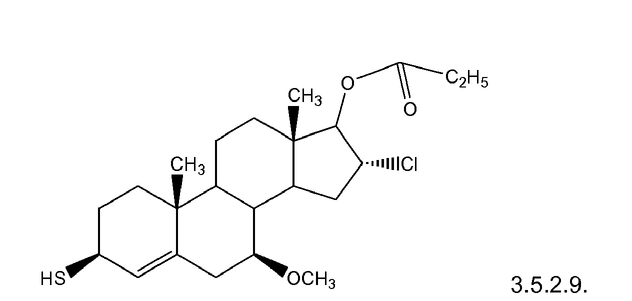

**Group 6.** This group comprises compounds named in Table B having R¹, R², R³ and R⁴ substituents defined in Table A wherein the R¹, R², R³ and R⁴ substituents are bonded to the steroid nucleus described for group 1 compounds, except that double bonds at both the 1-2 and 4-5 positions are present. Thus, the group 6 compound named 10.2.7.8 has the structure

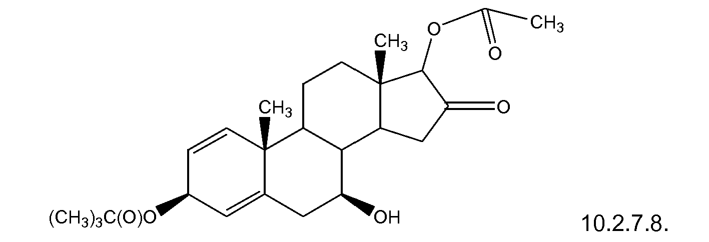

**Group 7**. Group 7 comprises the 6 compound groups described above, except that R⁵ is hydrogen instead of methyl, i.e., it comprises 6 subgroups, 7-1, 7-2, 7-3, 7-4, 7-5 and 7-6. Thus, subgroup 7-1 has the same steroid nucleus as group 1 above, i.e., no double bond is present, but R⁵ is -H. Subgroup 7-2 comprises the same steroid nucleus as group 2 above, i.e., a double bond is present at the 5-6-position, but R⁵ is -H. Compound subgroups 7-3 through 7-6 are assigned a steroid nucleus in the same manner. Thus, the subgroup 7-1 through subgroup 7-6 compounds named 1.2.1.9 have the structures

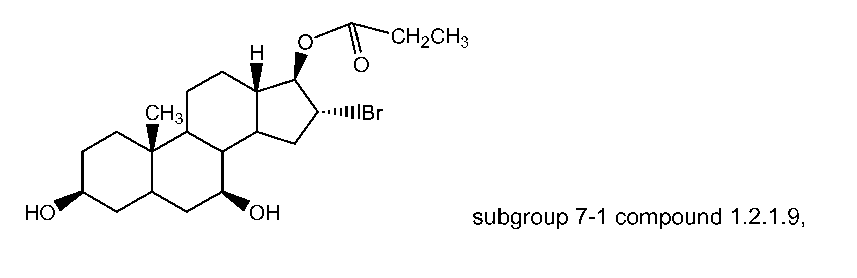

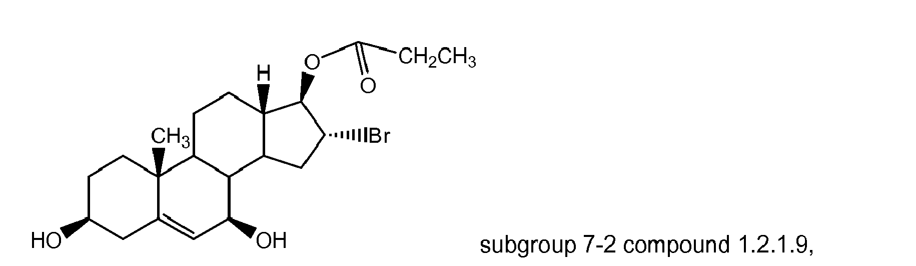

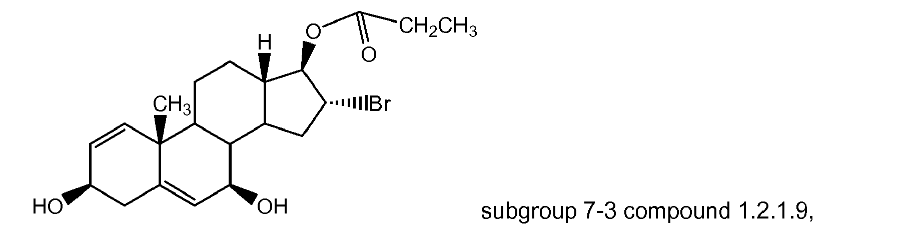

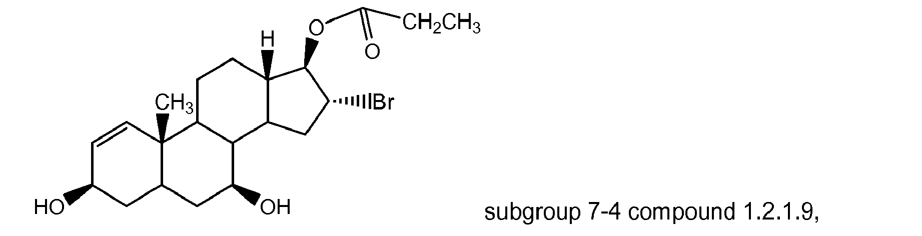

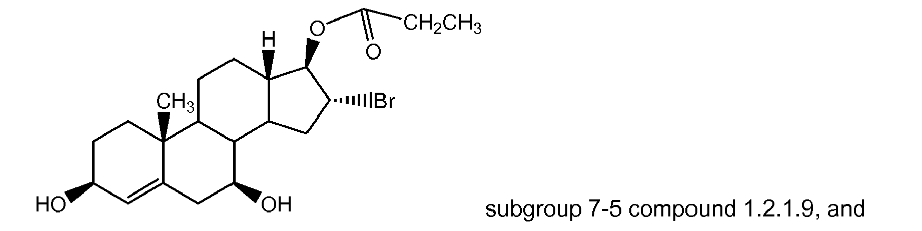

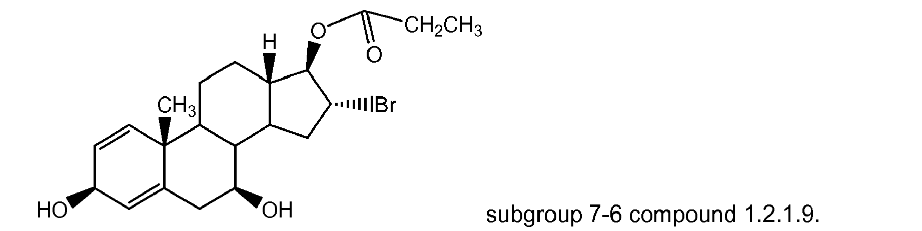

**Group 8.** Group 8 comprises 6 subgroups of compounds, i.e., each compound named in groups 1-6, except that R⁵ of formula B is -CH₂OH instead of methyl. The subgroups 8-1 through subgroup 8-6 compounds have structures that are named in the same manner as group 1-6 compounds, except that -CH₂OH instead of methyl is present at R⁵. These groups are named in essentially the same manner as subgroups 7-1 through 7-6. Thus, subgroup 8-1 and subgroup 8-2 compounds named 1.2.1.9 have the structures

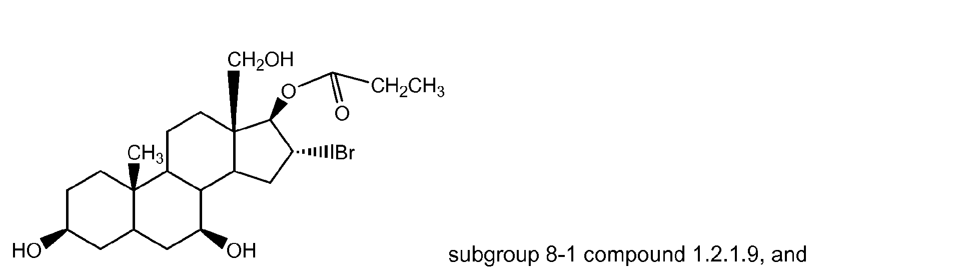

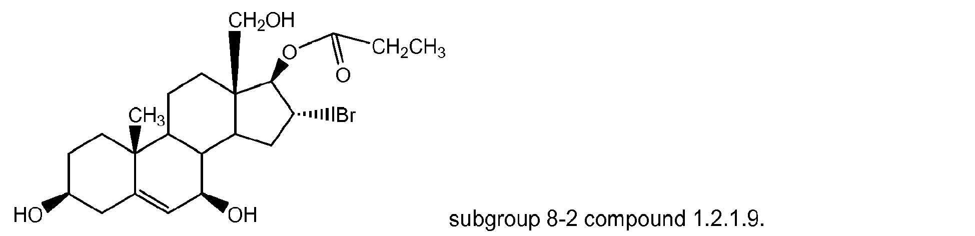

**Group 9**. Group 9 comprises each compound named in compound groups 1-8, except that R⁶ of formula B is hydrogen instead of methyl. Thus group 9 comprises subgroups 9-1 through 9-8-6, i.e., 9-1, 9-2, 9-3, 9-4, 9-5, 9-6, 9-7-1, 9-7-2, 9-7-3, 9-7-4, 9-7-5, 9-7-6, 9-8-1, 9-8-2, 9-8-3, 9-8-4, 9-8-5 and 9-8-6. Subgroups 9-1 through 9-8-6 compounds have structures that are named in essentially the same manner as subgroup 7-1 through 7-6 compounds, except that -H instead of methyl is present at R⁶. Thus, subgroup 9-1 and subgroup 9-2 compounds named 1.2.1.9 have the structures

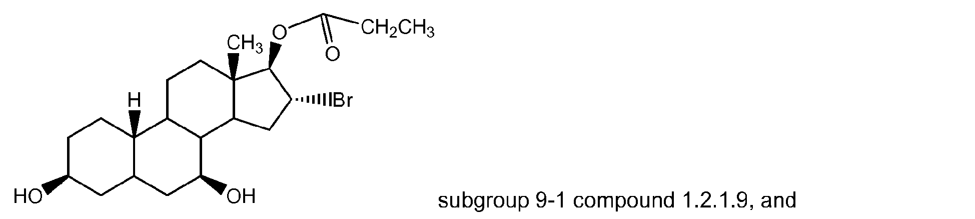

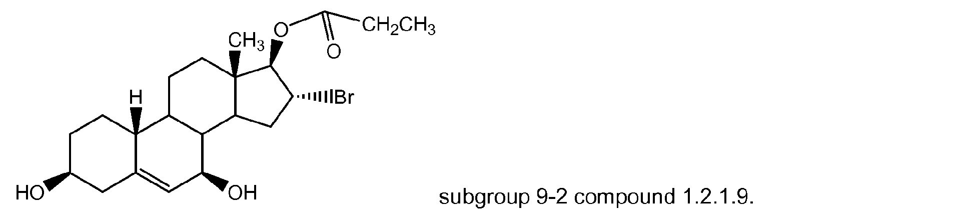

Subgroup 9-7-1 compound 1.2.1.9 has the same structure as group 9-1 compound 1.2.1.9, except that R⁵ is hydrogen in the β configuration, instead of a methyl group in the β configuration. Similarly, the group 9-8-1 compound 1.2.1.9 has the same structure as group 9-1 compound 1.2.1.9, except that R⁵ is hydroxymethyl (-CH₂OH) in the β configuration, instead of a methyl group in the β configuration. Group 9-7-2 compound 1.2.1.9 has the same structure as the group 9-7-1 compound, except that a double bond is present at the 5-6 position.

Thus, subgroups 9-1 through 9-6 have hydrogen at R⁶, but each has a different double bond structure, e.g., no double bond in subgroup 9-1 and double bonds at 1-2 and 4-5 in subgroup 9-6. Subgroups 9-7-1 through 9-7-6 also comprises six subgroups, but they have hydrogen at R⁵ and R⁶, but each has a different double bond structure for each of the six subgroups, e.g., no double bond in subgroup 9-7-1 and double bonds at positions 1-2 and 4-5 in subgroup 9-7-6. Subgroups 9-8-1 through 9-8-6 all have hydrogen at R⁶ and - CH₂OH at R⁵, but each has a different double bond structure in each, e.g., no double bond in subgroup 9-8-1 and double bonds at positions 1-2 and 4-5 in group 9-8-6.

**Groups 10**. Group 10 comprises each compound named in groups 1 through 8, but where R⁶ of formula B is -CH₂OH instead of methyl. The subgroups 10-1 through group 10-6 compounds have structures that are named in essentially the same manner as compounds in group 9, except that -CH₂OH instead of methyl is present at R⁶. Thus, subgroup 10-1 and subgroup 10-2 compounds named 1.2.1.9 have the structures

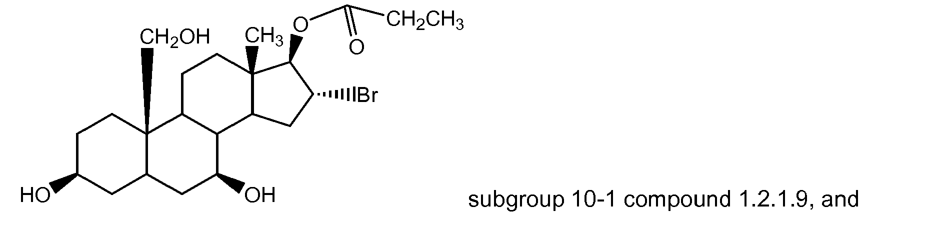

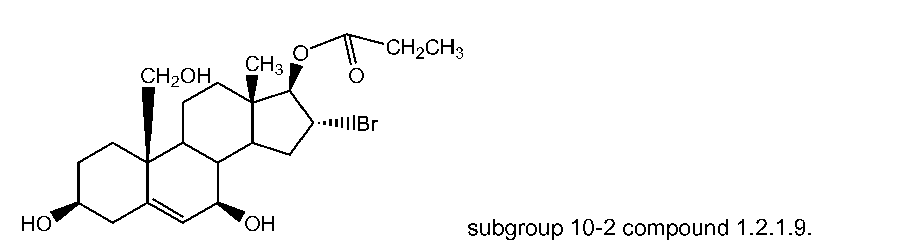

Subgroup 10-7-1 compound 1.2.1.9 has the same structure as subgroup 10-1 compound 1.2.1.9, except that R⁵ is hydrogen in the β configuration, instead of a methyl group in the β configuration. Similarly, the subgroup 10-8-1 compound 1.2.1.9 has the same structure as group 10-1 compound 1.2.1.9, except that R⁵ is hydroxymethyl (-CH₂OH) in the β configuration, instead of a methyl group in the β configuration. Subgroup 10-7-2 compound 1.2.1.9 has the same structure as the subgroup 10-7-1 compound, except that a double bond is present at the 5-6 position.

Thus, subgroups 10-1 through 10-8-6 comprise 18 separate groups, each of which has -CH₂OH at R⁶. Subgroups 10-1 through 10-6 comprise different six subgroups where each has a different double bond structure, e.g., no double bond in subgroup 10-1 and double bonds at 1-2 and 4-5 in subgroup 10-6. Subgroups 10-7-1 through 10-7-6 all have - CH₂OH at R⁶ and hydrogen at R⁵, but each has a different double bond structure for each of the six groups, e.g., no double bond in subgroup 10-7-1 and double bonds at positions 1-2 and 4-5 in subgroup 10-7-6. Similarly, subgroups 10-8-1 through 10-8-6 all six have -CH₂OH at R⁶ and at R⁵, but each has a different double bond structure in each of the six subgroups, e.g., no double bond in subgroup 10-8-1 and double bonds at positions 1-2 and 4-5 in subgroup 10-8-6. The 18 groups are 10-1, 10-2, 10-3, 10-4, 10-5, 10-6, 10-7-1, 10-7-2, 10-7-3, 10-7-4, 10-7-5, 10-7-6, 10-8-1, 10-8-2, 10-8-3, 10-8-4, 10-8-5 and 10-8-6.

**Group 11.** Group 11 comprises each compound named in compound groups 1-10, but where R¹ moieties (or substituents) 1-10 listed in Table A are replaced with the following moieties:
1 -O-C(O)-CH₂CH₂CH₂CH₃ (-O-C(O)-CH₂CH₂CH₂CH₃ replaces -OH, which is R¹ moiety 1 in Table A)
2 -O-C(O)-CH₂CH₂CH₂CH₂CH₂CH₃
3 -O-C(O)-CH₂CH₂OCH₂CH₃
4 -O-C(O)-CH₂CH₂OCH₂CH₂OCH₂CH₃
5 -O-C(O)-CH₂CH₂CH₂CH₂OCH₂CH₃
6 -O-C(O)-CH₂CH₂OCH₂CH₂CH₂CH₃
7 -O-C₆H₄Cl
8 -O-C₆H₃F₂
9 -O-C₆H₄-O(CH₂)₂-O-CH₂CH₃
10 -O-C₆H₄-C(O)O(CH₂)₀₋₉CH₃

The subgroup 11-1 through subgroup 11-6 compounds have structures that are named in essentially the same manner as described for the groups above, except that moieties 1-10 of table A are replaced by the moieties 1-10 at R¹. Thus subgroup 11-1 and 11-2 compounds named 1.2.1.9 have the structures

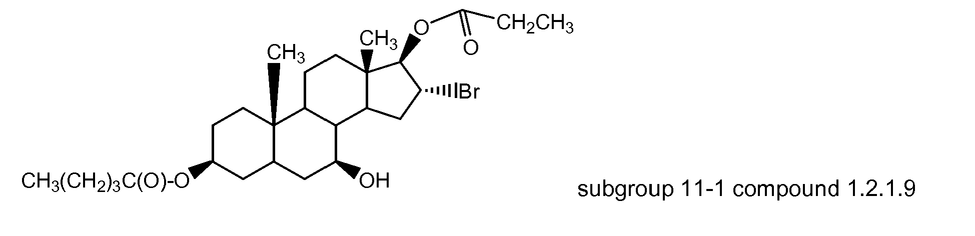

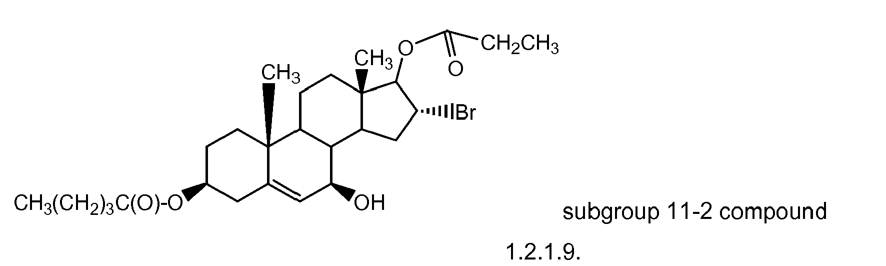

Subgroup 11-7-1 and 11-7-2 compounds named 1.2.1.9 have the structures

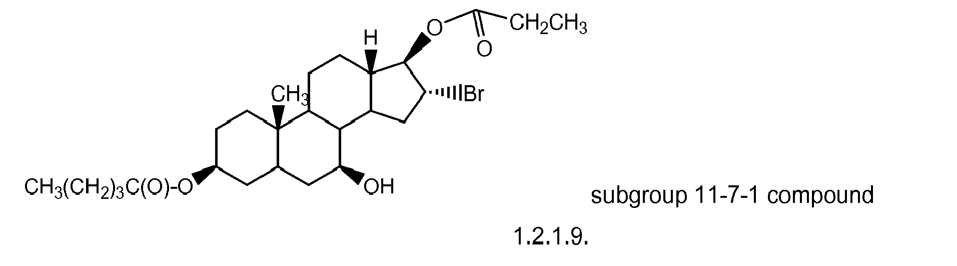

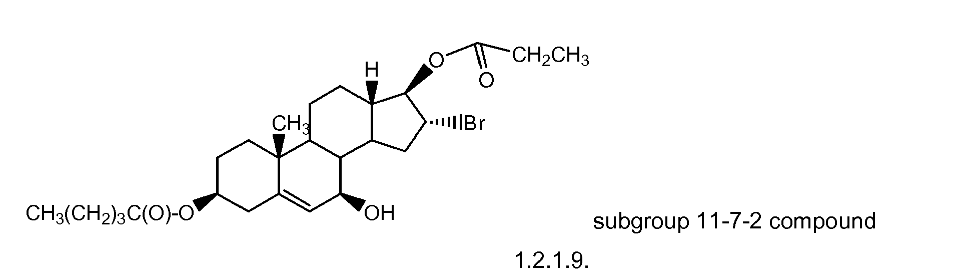

Subgroup 11-8-1 and 11-8-2 compounds named 1.2.1.9 have the structures

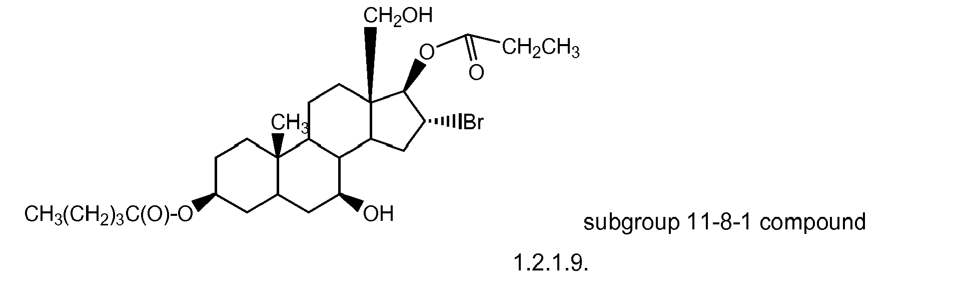

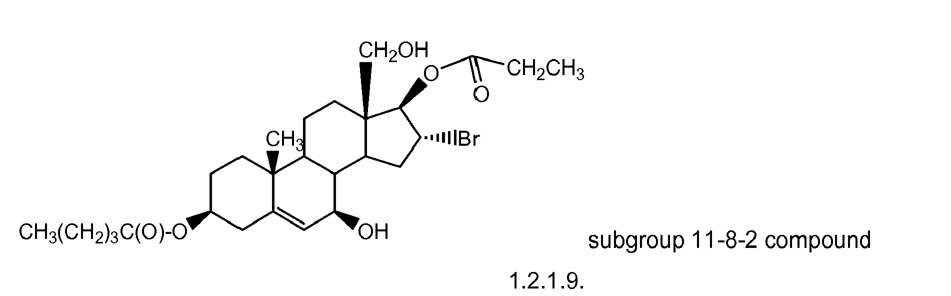

Group 11 comprises 54 separate subgroups, subgroups 11-1 through 11-10-8-6, where each of which has the R¹ moieties shown in this group and the remaining moieties as shown in the other groups described above. Subgroups 11-1 through 11-6 each have a different double bond structure, e.g., no double bond in subgroup 11-1 and double bonds at 1-2 and 4-5 in subgroup 11-6. Subgroups 11-7-1 through 11-7-6 all have -CH₂OH at R⁶ and hydrogen at R⁵, but each has a different double bond structure, e.g., no double bond in subgroup 11-7-1 and double bonds at positions 1-2 and 4-5 in subgroup 11-7-6. Subgroups 11-8-1 through 11-8-6 comprise all have -CH₂OH at R⁶ and at R⁵, but each has a different double bond structure in each of the six groups, e.g., no double bond in group 11-8-1 and double bonds at positions 1-2 and 4-5 in group 11-8-6. The compounds in the remaining groups are named in essentially the same manner.

The 54 groups are 11-1, 11-2, 11-3, 11-4, 11-5, 11-6, 11-7-1, 11-7-2, 11-7-3, 11-7-4, 11-7-5, 11-7-6, 11-8-1, 11-8-2, 11-8-3, 11-8-4, 11-8-5, 11-8-6, 11-9-1, 11-9-2, 11-9-3, 11-9-4, 11-9-5, 11-9-6, 11-10-1, 11-10-2, 11-10-3, 11-10-4, 11-10-5, 11-10-6, 11-9-7-1, 11-9-7-2, 11-9-7-3, 11-9-7-4, 11-9-7-5, 11-9-7-6, 11-10-7-1, 11-10-7-2, 11-10-7-3, 11-10-7-4, 11-10-7-5, 11-10-7-6, 11-9-8-1, 11-9-8-2, 11-9-8-3, 11-9-8-4, 11-9-8-5, 11-9-8-6, 11-10-8-1, 11-10-8-2, 11-10-8-3, 11-10-8-4, 11-10-8-5 and 11-10-8-6.

**Group 12.** Group 12 comprises each compound named in groups 1 through 10, but where R¹ moieties 1-10 listed in Table A are replaced with the following moieties:
1 -O-P(O)(O)-OCH₂CH(CH₃)CH₃ (-O-P(O)(O)-OCH₂CH(CH₃)CH₃ replaces -OH, which is R¹ moiety 1 in Table A)
2 -O-P(O)(O)-OCH₂CH₂CH₂CH₂CH₃
3 -O-P(O)(O)-OCH₂CH₂CH₂CH₂CH₂CH₃
4 -O-P(O)(O)-OCH₂CH₂CH(CH₂CH₂)CH₃
5 -O-CH₂CH₂CH₂CH₂CH₂CH₃
6 -O-C1-C6 alkyl(OH)₀₋₂
7 -C1-C6 alkyl(OH)₀₋₂
8 -C(O)-C1-C6 alkyl(OH)₀₋₂
9 -O-monosaccharide
10 -O-disaccharide

Group 12 comprises 54 separate subgroups, subgroups 12-1 through 12-10-8-6, where each of which has the R¹ moieties shown in this group and the remaining moieties as shown in the other groups described above. The subgroups are defined essentially as described for group 11 above. The 54 subgroups are 12-1, 12-2, 12-3, 12-4, 12-5, 12-6, 12-7-1, 12-7-2, 12-7-3, 12-7-4, 12-7-5, 12-7-6, 12-8-1, 12-8-2, 12-8-3, 12-8-4, 12-8-5, 12-8-6, 12-9-1, 12-9-2, 12-9-3, 12-9-4, 12-9-5, 12-9-6, 12-10-1, 12-10-2, 12-10-3, 12-10-4, 12-10-5, 12-10-6, 12-9-7-1, 12-9-7-2, 12-9-7-3, 12-9-7-4, 12-9-7-5, 12-9-7-6, 12-10-7-1, 12-10-7-2, 12-10-7-3, 12-10-7-4, 12-10-7-5, 12-10-7-6, 12-9-8-1, 12-9-8-2, 12-9-8-3, 12-9-8-4, 12-9-8-5, 12-9-8-6, 12-10-8-1, 12-10-8-2, 12-10-8-3, 12-10-8-4, 12-10-8-5 and 12-10-8-6.

**Group 13**. Group 13 comprises each compound named in groups 1 through 10, but where R¹ moieties 1-10 listed in Table A are replaced with the following moieties:
1 -O-(CH₂)₄CH₃ (-O-(CH₂)₄CH₃ replaces -OH, which is R¹ moiety 1 in Table A)
2 -O-oligosaccharide
3 -O-polyethylene glycol (e.g., PEG20, PEG100, PEG200 or PEG400)
4 -O-C(O)-NH₀₋₂(C1-C6 alkyl)₀₋₂
5 -C(O)-NH₀₋₂(C1-C6 alkyl)₀₋₂
6 -O-C(O)-NH(CH₂)₂-₄-O-C1-C4 alkyl(OH)₀₋₂
7 -O-C(O)-CH₃
8 -O-C(O)-C2-C5 alkyl(OH)₀₋₂
9 -O-C(O)- CH₂CH₂CH₂CH₃
10 -O-C(O)-CH(NH₂)-R⁴² (R⁴² is -H, C2-C6 alkyl or an amino acid side chain)

**Group 13** comprises 54 separate subgroups, subgroups 13-1 through 13-10-8-6, where each of which has the R¹ moieties shown in this group and the remaining moieties as shown in the other groups described above. The subgroups are defined essentially as described for group 11 above. The 54 subgroups are 13-1, 13-2, 13-3, 13-4, 13-5, 13-6, 13-7-1, 13-7-2, 13-7-3, 13-7-4, 13-7-5, 13-7-6, 13-8-1, 13-8-2, 13-8-3, 13-8-4, 13-8-5, 13-8-6, 13-9-1, 13-9-2, 13-9-3, 13-9-4, 13-9-5, 13-9-6, 13-10-1, 13-10-2, 13-10-3, 13-10-4, 13-10-5, 13-10-6, 13-9-7-1, 13-9-7-2, 13-9-7-3, 13-9-7-4, 13-9-7-5, 13-9-7-6, 13-10-7-1, 13-10-7-2, 13-10-7-3, 13-10-7-4, 13-10-7-5, 13-10-7-6, 13-9-8-1, 13-9-8-2, 13-9-8-3, 13-9-8-4, 13-9-8-5, 13-9-8-6, 13-10-8-1, 13-10-8-2, 13-10-8-3, 13-10-8-4, 13-10-8-5 and 13-10-8-6.

Group 14. Group 14 comprises each compound named in groups 1 through 10, but where R¹ moieties 1-10 listed in Table A are replaced with the following moieties:
1 -C(O)-CH₃
2 -O-CH₂C₆H₅
3 -C(S)-CH₃
4 -O-CO-C6 alkyl-heterocycle
5 -CO-C6 alkyl-heterocycle
6 -O-CH₂C₆H₄F
7 -O-CH₂C₆H₃(OCH₃)₂
8 -C(O)-C2-C4 alkyl-O-C1-C3 alkyl
9 -C(O)-CO-C4 alkyl-NH-(C1-C3 alkyl)₀₋₁-H
10 -O-CH₂C₆H₄OCH₂CH₃

Group 14 comprises 54 separate subgroups, subgroups 14-1 through 14-10-8-6, where each of which has the R¹ moieties shown in this group and the remaining moieties as shown in the other groups described above. These subgroups are defined essentially as described for group 11 above. The 54 subgroups are 14-1, 14-2, 14-3, 14-4, 14-5, 14-6, 14-7-1, 14-7-2, 14-7-3, 14-7-4, 14-7-5, 14-7-6, 14-8-1, 14-8-2, 14-8-3, 14-8-4, 14-8-5, 14-8-6, 14-9-1, 14-9-2, 14-9-3, 14-9-4, 14-9-5, 14-9-6, 14-10-1, 14-10-2, 14-10-3, 14-10-4, 14-10-5, 14-10-6, 14-9-7-1, 14-9-7-2, 14-9-7-3, 14-9-7-4, 14-9-7-5, 14-9-7-6, 14-10-7-1, 14-10-7-2, 14-10-7-3, 14-10-7-4, 14-10-7-5, 14-10-7-6, 14-9-8-1, 14-9-8-2, 14-9-8-3, 14-9-8-4, 14-9-8-5, 14-9-8-6, 14-10-8-1, 14-10-8-2, 14-10-8-3, 14-10-8-4, 14-10-8-5 and 14-10-8-6.

**Group 15.** Group 15 comprises each compound named in groups 1 through 10, but where R¹ moieties 1-10 listed in Table A are replaced with the following groups:
1 -O-C(O)-CH₂CH₂NH₂ (-O-C(O)-CH₂CH₂NH₂ replaces -OH, which is R¹ moiety 1 in Table A)
2 -O-C(O)-C1-C6 alkyl-NH₂
3 -C(O)-C1-C6 alkyl-NH₂
4 -O-C(O)-C1-C6 alkyl-(OH)₀₋₂
5 -C(O)-C1-C6 alkyl-(OH)₀₋₂
6 -O-C(O)-C1-C6 alkyl-(SH)₀₋₂
7 -C(O)-C1-C6 alkyl-(SH)₀₋₂
8 -O-C(O)-CH₂CH₂CH₂SH
9 -S-C(O)-C1-C6 alkyl-(OH)₀₋₂
10 -C(S)-C1-C6 alkyl-(OH)₀₋₂

Group 15 comprises 54 separate subgroups, subgroups 15-1 through 15-10-8-6, where each of which has the R¹ moieties shown in this group and the remaining moieties as shown in the other groups described above. These subgroups are defined essentially as described for group 11 above. The 54 subgroups are 15-1, 15-2, 15-3, 15-4, 15-5, 15-6, 15-7-1, 15-7-2, 15-7-3, 15-7-4, 15-7-5, 15-7-6, 15-8-1, 15-8-2, 15-8-3, 15-8-4, 15-8-5, 15-8-6, 15-9-1, 15-9-2, 15-9-3, 15-9-4, 15-9-5, 15-9-6, 15-10-1, 15-10-2, 15-10-3, 15-10-4, 15-10-5, 15-10-6, 15-9-7-1, 15-9-7-2, 15-9-7-3, 15-9-7-4, 15-9-7-5, 15-9-7-6, 15-10-7-1, 15-10-7-2, 15-10-7-3, 15-10-7-4, 15-10-7-5, 15-10-7-6, 15-9-8-1, 15-9-8-2, 15-9-8-3, 15-9-8-4, 15-9-8-5, 15-9-8-6, 15-10-8-1, 15-10-8-2, 15-10-8-3, 15-10-8-4, 15-10-8-5 and 15-10-8-6.

**Group 16**. Groups 16 comprises each compound named in groups 1 through 10, but where R¹ moieties 1-10 listed in Table A are replaced with the following groups:
1 -O-C(O)-A4-NH₂, where A4-NH₂ is a 4 carbon alkyl group substituted with -NH₂ (-O-C(O)-A4-NH₂ replaces -OH, which is R¹ moiety 1 in Table A)
2 -O-C(O)-A6-NH₂, where A6-NH₂ is a 6 carbon alkyl group substituted with -NH₂
3 -O-C(O)-A8-NH₂, where A8-NH₂ is a 8 carbon alkyl group substituted with -NH₂
4 -O-C(O)-A4-OH, where A4-OH is a 4 carbon alkyl group substituted with -OH or -O-
5 -O-C(O)-A6-OH, where A6-OH is a 6 carbon alkyl group substituted with -OH or -O-
6 -O-C(O)-A8-OH, where A8-OH is a 8 carbon alkyl group substituted with -OH or -O-
7 -F
8 -Cl
9 -Br
10 -I

Group 16 comprises 54 separate subgroups, subgroups 16-1 through 16-10-8-6, where each of which has the R¹ moieties shown in this group and the remaining moieties as shown in the other groups described above. These groups are defined essentially as described for group 11 above. The 54 subgroups are 16-1, 16-2, 16-3, 16-4, 16-5, 16-6, 16-7-1, 16-7-2, 16-7-3, 16-7-4, 16-7-5, 16-7-6, 16-8-1, 16-8-2, 16-8-3, 16-8-4, 16-8-5, 16-8-6, 16-9-1, 16-9-2, 16-9-3, 16-9-4, 16-9-5, 16-9-6, 16-10-1, 16-10-2, 16-10-3, 16-10-4, 16-10-5, 16-10-6, 16-9-7-1, 16-9-7-2, 16-9-7-3, 16-9-7-4, 16-9-7-5, 16-9-7-6, 16-10-7-1, 16-10-7-2, 16-10-7-3, 16-10-7-4, 16-10-7-5, 16-10-7-6, 16-9-8-1, 16-9-8-2, 16-9-8-3, 16-9-8-4, 16-9-8-5, 16-9-8-6, 16-10-8-1, 16-10-8-2, 16-10-8-3, 16-10-8-4, 16-10-8-5 and 16-10-8-6.

**Group 17**. Group 17 comprises each compound named in compound groups 1 through 10, but where R¹ moieties 1-10 listed in Table A are replaced with the following groups:
1 -O-S(O)(O)-O-C1-C8 optionally substituted alkyl
2 -O-P(O)(OH)-O-C1-C8 optionally substituted alkyl
3 -O-P(S)(OH)-O-C1-C8 optionally substituted alkyl
4 -O-P(O)(OH)-S-C1-C8 optionally substituted alkyl
5 -O-S(O)(O)-OR⁴⁴ (R⁴⁴ is H, NH₄⁺, Na⁺, K⁺, HN⁺(CH₃)₃, N⁺(CH₃)₄, HN⁺(C₂H₅)₃ C1-C8 alkyl (e.g., -CH₃, -C₂H₅ or -C₃H₇), or pyridinium⁺)
6 -O-P(O)(OH)-OR⁴⁴
7 -O-P(O)(OH)-SR⁴⁴
8 -O-S(O)(O)-O-2',3'-dipalmitoyl-1'-glyceryl
9 -O-(3β-O-1β)-D-glucuronic acid-R⁴⁴
10 -O-(3β-O-1β)-tri-O-acetyl-D-glucuronic acid-R⁴⁴

Group 17 comprises 54 separate subgroups, subgroups 17-1 through 17-10-8-6, where each of which has the R¹ moieties shown in this group and the remaining moieties as shown in the other groups described above. These subgroups are defined essentially as described for group 11 above. The 54 subgroups are 17-1, 17-2, 17-3, 17-4, 17-5, 17-6, 17-7-1, 17-7-2, 17-7-3, 17-7-4, 17-7-5, 17-7-6, 17-8-1, 17-8-2, 17-8-3, 17-8-4, 17-8-5, 17-8-6, 17-9-1, 17-9-2, 17-9-3, 17-9-4, 17-9-5, 17-9-6, 17-10-1, 17-10-2, 17-10-3, 17-10-4, 17-10-5, 17-10-6, 17-9-7-1, 17-9-7-2, 17-9-7-3, 17-9-7-4, 17-9-7-5, 17-9-7-6, 17-10-7-1, 17-10-7-2, 17-10-7-3, 17-10-7-4, 17-10-7-5, 17-10-7-6, 17-9-8-1, 17-9-8-2, 17-9-8-3, 17-9-8-4, 17-9-8-5, 17-9-8-6, 17-10-8-1, 17-10-8-2, 17-10-8-3, 17-10-8-4, 17-10-8-5 and 17-10-8-6.

**Group 18**. Group 18 comprises each compound named in groups 1 through 17, but where R⁴ moieties 1-10 listed in Table A are replaced with the following moieties:
1 -O-C(O)CH₂NH₂
2 -O-C(O)C(CH₃)H-NH₂
3 -O-C(O)C(CH₂C₆H₅)H-NH₂
4 -O-C(O)-O-NHC(CH₃)H-CO₂H
5 -O-C(O)-O-NHCH₂-CO₂H
6 -O-C(O)-O-NH(CH₂C₆H₅)H-CO₂H
7 -O-C(O)-CF₃
8 -O-C(O)-CH₂CF₃
9 -O-C(O)-(CH₂)₃CF₃
10 -O-C(O)-(CH₂)₅CH₃

Group 18 comprises 432 separate subgroups, 18-1 through 18-17-10-8-6, where each of which has the R⁴ moieties shown in this group and the remaining moieties as shown in the other groups described above. These groups are defined essentially as described for the groups described above. The groups are 18-1 through 18-6, 18-7-1 through 18-7-6, 18-8-1 through 18-8-6, 18-9-1 through 18-9-6, 18-10-1 through 18-10-6, 18-9-7-1 through 18-9-7-6, 18-9-8-1 through 18-9-8-6, 18-10-7-1 through 18-10-7-6, 18-10-8-1 through 18-10-8-6, 18-11-1 through 18-11-6, 18-11-7-1 through 18-11-7-6, 18-11-8-1 through 18-11-8-6, 18-11-9-1 through 18-11-9-6, 18-11-10-1 through 18-11-10-6, 18-11-9-7-1 through 18-11-9-7-6, 18-11-9-8-1 through 18-11-9-8-6, 18-11-10-7-1 through 18-11-10-7-6, 18-11-10-8-1 through 18-11-10-8-6, 18-12-1 through 18-12-6, 18-12-7-1 through 18-12-7-6, 18-12-8-1 through 18-12-8-6, 18-12-9-1 through 18-12-9-6, 18-12-10-1 through 18-12-10-6, 18-12-9-7-1 through 18-12-9-7-6, 18-12-9-8-1 through 18-12-9-8-6, 18-12-10-7-1 through 18-12-10-7-6, 18-12-10-8-1 through 18-12-10-8-6, 18-13-1 through 18-13-6, 18-13-7-1 through 18-13-7-6, 18-13-8-1 through 18-13-8-6, 18-13-9-1 through 18-13-9-6, 18-13-10-1 through 18-13-10-6, 18-13-9-7-1 through 18-13-9-7-6, 18-13-9-8-1 through 18-13-9-8-6, 18-13-10-7-1 through 18-13-10-7-6, 18-13-10-8-1 through 18-13-10-8-6, 18-14-1 through 18-14-6, 18-14-7-1 through 18-14-7-6, 18-14-8-1 through 18-14-8-6, 18-14-9-1 through 18-14-9-6, 18-14-10-1 through 18-14-10-6, 18-14-9-7-1 through 18-14-9-7-6, 18-14-9-8-1 through 18-14-9-8-6, 18-14-10-7-1 through 18-14-10-7-6, 18-14-10-8-1 through 18-14-10-8-6, 18-15-1 through 18-15-6, 18-15-7-1 through 18-15-7-6, 18-15-8-1 through 18-15-8-6, 18-15-9-1 through 18-15-9-6, 18-15-10-1 through 18-15-10-6, 18-15-9-7-1 through 18-15-9-7-6, 18-15-9-8-1 through 18-15-9-8-6, 18-15-10-7-1 through 18-15-10-7-6, 18-15-10-8-1 through 18-15-10-8-6, 18-16-1 through 18-16-6, 18-16-7-1 through 18-16-7-6, 18-16-8-1 through 18-16-8-6, 18-16-9-1 through 18-16-9-6, 18-16-10-1 through 18-16-10-6, 18-16-9-7-1 through 18-16-9-7-6, 18-16-9-8-1 through 18-16-9-8-6, 18-16-10-7-1 through 18-16-10-7-6, 18-16-10-8-1 through 18-16-10-8-6, 18-17-1 through 18-17-6, 18-17-7-1 through 18-17-7-6, 18-17-8-1 through 18-17-8-6, 18-17-9-1 through 18-17-9-6, 18-17-10-1 through 18-17-10-6, 18-17-9-7-1 through 18-17-9-7-6, 18-17-9-8-1 through 18-17-9-8-6, 18-17-10-7-1 through 18-17-10-7-6 and 18-17-10-8-1 through 18-17-10-8-6.

**Group 19.** Group 19 comprises each compound named in compound groups 1 through 17, but where R⁴ moieties 1-10 listed in Table A are replaced with the following moieties:
1 -O-C(O)-O-CH₃
2 -O-C(O)-O-CH₂CH₃
3 -O-C(O)-O-C₃H₇
4 -O-C(O)-O-C₄H₉
5 -O-C(O)-O-C₆H₁₃
6 -O-C(O)-O-C₆H₅
7 -O-C(O)-O-C₆H₄OH
8 -O-C(O)-O-C₆H₄OCH₃
9 -O-C(O)-O-C₆H₄OCH₂CH₃
10 -O-C(O)-O-C₆H₄F

Group 19 comprises 432 separate subgroups, 19-1 through 19-17-10-8-6, where each of which has the R⁴ moieties shown in this group and the remaining moieties as shown in the other groups described above. These subgroups are defined essentially as described for group 18 above. The subgroups are 19-1 through 19-6, 19-7-1 through 19-7-6, 19-8-1 through 19-8-6, 19-9-1 through 19-9-6 and so on essentially as described for group 18 compounds.

**Group 20.** Group 20 comprises each compound named in groups 1 through 17, but where R⁴ moieties 1-10 listed in Table A are replaced with the following moieties:
1 -O-S(O)(O)-OR⁴⁴ (R⁴⁴ is H, NH₄⁺, Na⁺, K⁺, HN⁺(CH₃)₃, N⁺(CH₃)₄, HN⁺(C₂H₅)₃ C1-C8 optionally substituted alkyl (e.g., -CH₃, -C₂H₅ or -C₃H₇), or pyridinium⁺)
2 -O-P(O)(OH)-SR⁴⁴
3 -C(O)-C1-C8 optionally substituted alkyl
4 -CH(OH)-C1-C8 optionally substituted alkyl
5 -C≡CH
6 -C≡C-(CH₂)₁₋₄-H
7 -C(O)-CH₂-OH
8 -C(S)-CH₂-OH
9 -O-S(O)(O)-O-2',3'-dipalmitoyl-1'-glyceryl
10 -O-(3-O-1β)-tri-O-acetyl-D-glucuronic acid-R⁴⁴

Group 20 comprises 432 separate subgroups, 20-1 through 20-17-10-8-6 comprise 432 separate groups, each of which has the R⁴ moieties defined for this group and the remaining moieties as shown in the other groups described above. These subgroups are defined essentially as described for group 18 above. The subgroups are 20-1 through 20-6, 20-7-1 through 20-7-6, 20-8-1 through 20-8-6, 20-9-1 through 20-9-6 and so on essentially as described for group 18 compounds.

**Group 21.** Group 21 comprises each compound named in compound groups 1 through 17, but where R⁴ moieties 1-10 listed in Table A are replaced with the following moieties:
1 -O-C(S)-O-C1-C4 alkyl
2 -S-C(S)-O-C1-C4 alkyl
3 -SH
4 =S
5 -O-C1-C6 optionally substituted alkyl
6 -O-C1-C6-optionally substituted alkyl-optionally substituted aryl
7 -S-C1-C6 optionally substituted alkyl
8 -O-C(O)-CH(NH₂)-R⁴² (R⁴² is -H, C2-C6 alkyl or an amino acid side chain)
9 -C0-C4 alkyl-heterocycle
10 -O-polyethylene glycol (e.g., PEG20, PEG100, PEG200 or PEG400)

Group 21 comprises 432 separate subgroups, 21-1 through 21-17-10-8-6 comprise 432 separate groups, each of which has the R⁴ moieties defined for this group and the remaining moieties as shown in the other groups described above. These subgroups are defined essentially as described for group 18 above. The subgroups are 21-1 through 21-6, 21-7-1 through 21-7-6, 21-8-1 through 21-8-6, 21-9-1 through 21-9-6 and so on essentially as described for group 18 compound.

**Group 22.** Group 22 comprises each compound named in compound groups 1 through 21, but where R² moieties 1-10 listed in Table A are replaced with the following moieties:
1 -O-C(S)-O-C1-C8 alkyl-(OH)₀₋₂
2 -O-C(O)-O-C1-C8 alkyl-(OH)₀₋₂
3 -C(O)-C1-C6 alkyl-O-C1-C2 alkyl
4 -C(O)-C1-C6 alkyl-(S)₀₋₁-C1-C2 alkyl-(OH)₀₋₁
5 -C(O)-C1-C6 alkyl-NH₀₋₂(C1-C4 alkyl)₀₋₂
6 -O-C(O)-CO-C4 alkyl-heterocycle
7 -C(O)-O-C1-C4 alkyl-C₆H₃₋₅-(OH)₀₋₂
8 -O-C(O)-O-C1-C4 alkyl-C₆H₃₋₅-(OH)₀₋₂
9 -O-C(O)-C1-C4 alkyl-C₆H₃₋₅-(O-C1-C4 alkyl)₀₋₂
10 -O-C(O)-C1-C4 alkyl-C₆H₃₋₅-(halogen)₀₋₂

Group 22 comprises subgroups 22-1 through 22-21-17-10-8-6, which name compounds or genera of compounds essentially as described for the other compound groups above. The 1728 subgroups in group 22 are 22-1 through 22-6, 22-7-1 through 22-7-6, 22-8-1 through 22-8-6, 22-9-1 through 22-9-6 and so on essentially as described for the groups above.

**Group 23.** Group 23 comprises each compound named in compound groups 1 through 21, but where R² moieties 1-10 listed in Table A are replaced with the following moieties:
1 -O-C₀₋₄ alkyl-heterocycle
2 -O-C(O)-C₀₋₄ alkyl-heterocycle
3 -SH
4 =S
5 -C2-C6 alkyl-(OH)₁₋₂
6 -O-CHR²⁴-C(O)-R²⁵
7 -O-CHR²⁴-C(O)-N(R²⁵)₂
8 -O-CHR²⁴-C(O)-NHR²⁵
9 -O-CHR²⁴-C(O)-NH₂
10 -O-CHR²⁴-C(O)-OC₆H₅

Group 23 comprises subgroups 24-1 through 24-23-21-17-10-8-6, which name compounds or genera of compounds essentially as described for the other compound groups above. The subgroups in group 24 are 24-1 through 24-6, 24-7-1 through 24-7-6, 24-8-1 through 24-8-6, 24-9-1 through 24-9-6 and so on essentially as described for the groups above.

**Group 24.** Group 24 comprises each compound named in compound groups 1 through 23 where R³ moieties 1-10 listed in Table A are replaced with the following moieties:
1 -O-C(S)-O-C1-C8 alkyl-(OH)₀₋₂
2 -O-C(O)-O-C1-C8 alkyl-(OH)₀₋₂
3 -C(O)-C1-C6 alkyl-O-C1-C2 alkyl
4 -C(O)-C1-C6 alkyl-(S)₀₋₁-C1-C2 alkyl-(OH)₀₋₁
5 -C(O)-C1-C6 alkyl-NH₀₋₂(C1-C4 alkyl)₀₋₂
6 -O-C(O)-CO-C4 alkyl-heterocycle
7 -C(O)-O-C1-C4 alkyl-C₆H₃₋₅-(OH)₀₋₂
8 -O-C(O)-O-C1-C4 alkyl-C₆H₃₋₅-(OH)₀₋₂
9 -O-C(O)-C1-C4 alkyl-C₆H₃₋₅-(O-C1-C4 alkyl)₀₋₂
10 -O-C(O)-C1-C4 alkyl-C₆H₃₋₅-(halogen)₀₋₂

Group 24 comprises subgroups 23-1 through 23-21-17-10-8-6, which name compounds or genera of compounds essentially as described for the other compound groups above. The 1728 subgroups in group 23 are 23-1 through 23-6, 23-7-1 through 23-7-6, 23-8-1 through 23-8-6, 23-9-1 through 23-9-6 and so on essentially as described for the groups above.

**Group 25.** Group 25 comprises each compound named in compound groups 1 through 23, but where R³ moieties 1-10 listed in Table A are replaced with the following moieties:
1 -O-C₀₋₄ alkyl-heterocycle
2 -O-C(O)-C₀₋₄ alkyl-heterocycle
3 -SH
4 =S
5 -C2-C6 alkyl-(OH)₁₋₂
6 -O-CHR²⁴-C(O)-R²⁵
7 -O-CHR²⁴-C(O)-N(R²⁵)₂
8 -O-CHR²⁴-C(O)-NHR²⁵
9 -O-CHR²⁴-C(O)-NH₂
10 -O-CHR²⁴-C(O)-OC₆H₅

Group 25 comprises subgroups 25-1 through 25-23-21-17-10-8-6, which name compounds or genera of compounds essentially as described for the other compound groups above. The subgroups in group 25 are 25-1 through 25-6, 25-7-1 through 25-7-6, 25-8-1 through 25-8-6, 25-9-1 through 25-9-6 and so on essentially as described for the groups above.

**Group 26.** Group 26 comprises each compound or genus named in compound groups 1 through 25, but wherein R¹ is not divalent, i.e., it is not bonded to the carbon atom at the 3 position by a double bond (e.g., R¹ is not =O) and it is in the α-configuration, instead of the β-configuration as shown in formula B.

Group 26 comprises subgroups 26-1 through 26-25-23-21-17-10-8-6, which name compounds or genera of compounds essentially as described for the other compound groups above. The subgroups in group 26 are 26-1 through 26-6, 26-7-1 through 26-7-6, 26-8-1 through 26-8-6, 26-9-1 through 26-9-6 and so on essentially as described for the groups above.

**Group 27.** Group 27 comprises each compound or genus named in compound groups 1 through 26, but wherein R² is not divalent, i.e., it is not bonded to the carbon atom at the 3 position by a double bond (e.g., R² is not =O) and it is in the α-configuration, instead of the β-configuration as shown in formula B.

Group 27 comprises subgroups 27-1 through 27-26-25-23-21-17-10-8-6, which name compounds or genera of compounds essentially as described for the other compound groups above. The subgroups in group 27 are 27-1 through 27-6, 27-7-1 through 27-7-6, 27-8-1 through 27-8-6, 27-9-1 through 27-9-6 and so on essentially as described for the groups above.

**Group 28.** Group 28 comprises each compound or genus named in compound groups 1 through 27, but wherein R³ is not divalent, i.e., it is not bonded to the carbon atom at the 3 position by a double bond (e.g., R³ is not =O) and it is in the β-configuration, instead of the α-configuration as shown in formula B.

Group 28 comprises subgroups 28-1 through 28-27-26-25-23-21-17-10-8-6, which name compounds or genera of compounds essentially as described for the other compound groups above. The subgroups in group 28 are 28-1 through 28-6, 28-7-1 through 28-7-6, 28-8-1 through 28-8-6, 28-9-1 through 28-9-6 and so on essentially as described for the groups above.

**Group 29.** Group 29 comprises each compound or genus named in compound groups 1 through 28, but wherein R⁴ is not divalent, i.e., it is not bonded to the carbon atom at the 3 position by a double bond (e.g., R⁴ is not =O) and it is in the α-configuration, instead of the β-configuration as shown in formula B.

Group 29 comprises subgroups 29-1 through 29-28-27-26-25-23-21-17-10-8-6, which name compounds or genera of compounds essentially as described for the other compound groups above. The subgroups in group 29 are 29-1 through 29-6, 29-7-1 through 29-7-6, 29-8-1 through 29-8-6, 29-9-1 through 29-9-6 and so on essentially as described for the groups above.

**Group 30.** Group 30 comprises each compound or genus named in compound groups 1 through 29, but wherein R⁵ is in the α-configuration, instead of the β-configuration as shown in formula B.

Group 30 comprises subgroups 30-1 through 30-29-28-27-26-25-23-21-17-10-8-6, which name compounds or genera of compounds essentially as described for the other compound groups above. The subgroups in group 30 are 30-1 through 30-6, 30-7-1 through 30-7-6, 30-8-1 through 30-8-6, 30-9-1 through 30-9-6 and so on essentially as described for the groups above.

**Group 31.** Group 31 comprises each compound or genus named in compound groups 1 through 30, but wherein R⁵ is in the α-configuration, instead of the β-configuration as shown in formula B.

Group 31 comprises subgroups 31-1 through 31-30-29-28-27-26-25-23-21-17-10-8-6, which name compounds or genera of compounds essentially as described for the other compound groups above. The subgroups in group 31 are 31-1 through 31-6, 31-7-1 through 31-7-6, 31-8-1 through 31-8-6, 31-9-1 through 31-9-6 and so on essentially as described for the groups above.

**Group 32.** Group 32 comprises each compound or genus named in compound groups 1 through 31, but wherein the hydrogen atom at the 5 position is in the β-configuration, instead of the α-configuration as shown in formula B.

Group 32 comprises subgroups 32-1 through 32-31-30-29-28-27-26-25-23-21-17-10-8-6, which name compounds or genera of compounds essentially as described for the other compound groups above. The subgroups in group 32 are 32-1 through 32-6, 32-7-1 through 32-7-6, 32-8-1 through 32-8-6, 32-9-1 through 32-9-6 and so on essentially as described for the groups above.

**Group 33.** Group 33 comprises each compound or genus named in compound groups 1 through 32, but wherein the hydrogen atom at the 8 position is in the α-configuration, instead of the β-configuration as shown in formula B.

Group 33 comprises subgroups 33-1 through 33-32-31-30-29-28-27-26-25-23-21-17-10-8-6, which name compounds or genera of compounds essentially as described for the other compound groups above. The subgroups in group 33 are 33-1 through 33-6, 33-7-1 through 33-7-6, 33-8-1 through 33-8-6, 33-9-1 through 33-9-6 and so on essentially as described for the groups above.

**Group 34.** Group 34 comprises each compound or genus named in compound groups 1 through 33, but wherein the hydrogen atom at the 9 position is in the β-configuration, instead of the α-configuration as shown in formula B.

Group 34 comprises subgroups 34-1 through 34-33-32-31-30-29-28-27-26-25-23-21-17-10-8-6, which name compounds or genera of compounds essentially as described for the other compound groups above. The subgroups in group 34 are 34-1 through 34-6, 34-7-1 through 34-7-6, 34-8-1 through 34-8-6, 34-9-1 through 34-9-6 and so on essentially as described for the groups above.

**Group 35.** Group 35 comprises each compound or genus named in compound groups 1 through 34, but wherein the hydrogen atom at the 14 position is in the β-configuration, instead of the α-configuration as shown in formula B.

Group 35 comprises subgroups 35-1 through 35-34-33-32-31-30-29-28-27-26-25-23-21-17-10-8-6, which name compounds or genera of compounds essentially as described for the other compound groups above. The subgroups in group 35 are 35-1 through 35-6, 35-7-1 through 35-7-6, 35-8-1 through 35-8-6, 35-9-1 through 35-9-6 and so on essentially as described for the groups above.

**Group 36.** Group 36 comprises each compound or genus named in compound groups 1 through 35, but wherein R⁴ in formula B is not divalent, and a second monovalent R⁴ is present at the 17 position, and the second R⁴ is a moiety other than hydrogen. As used here, monovalent R⁴ means that the second R⁴ moiety is bonded to the carbon atom at the 17 position by a single bond.

The second R⁴ optionally comprises -OH, -OR^{PR}, -SH, -SR^{PR}, -NH₂, -NHR^{PR}, a halogen, optionally substituted alkyl, optionally substituted alkenyl, optionally substituted alkynyl, optionally substituted aryl, optionally substituted alkylaryl, an optionally substituted heterocycle, an ester, an ether, a thioester, a thionoester, a thioether, an optionally substituted monosaccharide, an optionally substituted oligosaccharide, a carbonate, a carbamate, an amide or an amino acid. Any of these moieties, may comprise any R⁴ structure disclosed herein.

Exemplary second R⁴ moieties include -C≡C-(CH₂)ₙH (e.g., -C≡CH and -C≡C-CH₃), -C=C-(CH₂)ₙH, -(CH₂)ₙH (e.g., -CH₃, -C₂H₅, -C₃H₇), -(CH₂)ₙC₆H₅, wherein n is 0, 1, 2, 3, 4, 5, 6, 7 or 8 and any of these exemplary second R⁴ moieties optionally comprise 1, 2, 3, 4 or more independently selected -O-, -OH, =O, -S-, -SH, =S, -NH-, -NH2, -COOH, -COOR^{PR}, -F, -Cl, -Br, -I, -SCN, -CN, -NO₂, =NHO, -CH₃, -CF₃, -C₂H₅ or -C₆H₅ moieties that replace (or substitute) one or more hydrogen or carbon atoms, wherein such moieties may be adjacent to one another, e.g., they can comprise -C(O)-NH- or -NH-C(O)-NH-. Typically moieties that replace a hydrogen or carbon atom will not replace a divalent or trivalent carbon atom, e.g., in -CH=CH- or in -C≡C- and specific embodiments include one or more substitutions at carbons that are separated from a -CH=CH- or -C≡C- moiety by one, two, three or more - CH₂- moieties. In some embodiments, one or two hydrogen atoms that are bonded to the distal carbon atom is substituted by one or two -OH, =O -SH, =S, -NH2, -COOH, -COOR^{PR}, -F, -Cl, -Br, -I, -SCN, -CN, -NO₂ or =NHO moieties.

Group 36 comprises subgroups 36-1 through 36-35-34-33-32-31-30-29-28-27-26-25-23-21-17-10-8-6, which name compounds or genera of compounds essentially as described for the other compound groups above. The subgroups in group 36 are 36-1 through 36-6, 36-7-1 through 36-7-6, 36-8-1 through 36-8-6, 36-9-1 through 36-9-6 and so on essentially as described for the groups above.

Group 37. Group 37 comprises each compound or genus named in compound groups 1 through 36, but wherein R⁷ in formula B is not -CH₂- or a heteroatom, i.e., R¹⁰ is bonded to R⁷ in formula B and it is not a hydrogen atom.

The R¹⁰ optionally comprises -OH, =O, -OR^{PR}, -SH, =S, -SR^{PR}, -NH₂, -NHR^{PR}, a halogen, optionally substituted alkyl, optionally substituted alkenyl, optionally substituted alkynyl, optionally substituted aryl, optionally substituted alkylaryl, an optionally substituted heterocycle, an ester, an ether, a thioester, a thionoester, a thioether, an optionally substituted monosaccharide, an optionally substituted oligosaccharide, a carbonate, a carbamate, an amide or an amino acid. Any of these moieties, may comprise any R¹⁰ structure disclosed herein.

Exemplary second R⁴ moieties include -C≡C-(CH₂)ₙH (e.g., -C≡CH and -C≡C-CH₃), -C=C-(CH₂)ₙH, -(CH₂)ₙH (e.g., -CH₃, -C₂H₅, -C₃H₇), -(CH₂)ₙC₆H₅, wherein n is 0, 1, 2, 3, 4, 5, 6, 7 or 8 and any of these exemplary second R⁴ moieties optionally comprise 1, 2, 3, 4 or more independently selected -O-, -OH, =O, -S-, -SH, =S, -NH-, -NH2, -COOH, -COOR^{PR}, -F, -Cl, -Br, -I, -SCN, -CN, -NO₂, =NHO, -CH₃, -CF₃, -C₂H₅ or -C₆H₅ moieties that replace (or substitute) one or more hydrogen or carbon atoms, wherein such moieties may be adjacent to one another, e.g., they can comprise -C(O)-NH- or -NH-C(O)-NH-. In some embodiments, the moieties that replace a hydrogen or carbon atom will not replace a divalent or trivalent carbon atom, or a hydrogen that is bonded to such a carbon atom, e.g., in -CH=CH- or in - C≡C- and specific embodiments include one or more substitutions at carbons that are separated from a -CH=CH- or -C≡C- moiety by one, two, three or more -CH₂- moieties. In some embodiments, one or two hydrogen atoms that are bonded to the distal carbon atom is substituted by one, two or three -OH, =O -SH, =S, -NH₂, -COOH, -COOR^{PR}, -F, -Cl, -Br, -I, - SCN, -CN, -NO₂ or =NHO moieties.

Group 37 comprises subgroups 37-1 through 37-36-35-34-33-32-31-30-29-28-27-26-25-23-21-17-10-8-6, which name compounds or genera of compounds essentially as described for the other compound groups above. The subgroups in group 37 are 37-1 through 37-6, 37-7-1 through 37-7-6, 37-8-1 through 37-8-6, 37-9-1 through 37-9-6 and so on essentially as described for the groups above.

**Group 38.** Group 38 comprises each compound or genus named in compound groups 1 through 37, but wherein R⁸ in formula B is not -CH₂- or a heteroatom, i.e., R¹⁰ is bonded to R⁸ in formula B and it is not a hydrogen atom.

This R¹⁰ optionally comprises -OH, =O, -OR^{PR}, -SH, =S, -SR^{PR}, -NH₂, -NHR^{PR}, a halogen, optionally substituted alkyl, optionally substituted alkenyl, optionally substituted alkynyl, optionally substituted aryl, optionally substituted alkylaryl, an optionally substituted heterocycle, an ester, an ether, a thioester, a thionoester, a thioether, an optionally substituted monosaccharide, an optionally substituted oligosaccharide, a carbonate, a carbamate, an amide or an amino acid. Any of these moieties, may comprise any R¹⁰ structure disclosed herein.

Other exemplary R¹⁰ moieties include -C≡C-(CH₂)ₙH (e.g., -C≡CH and -C≡C-CH₃), -C=C-(CH₂)ₙH, -(CH₂)ₙH (e.g., -CH₃, -C₂H₅, -C₃H₇), -(CH₂)ₙC₆H₅, wherein n is 0, 1, 2, 3, 4, 5, 6, 7 or 8 and any of these exemplary second R⁴ moieties optionally comprise 1, 2, 3, 4 or more independently selected -O-, -OH, =O, -S-, -SH, =S, -NH-, -NH2, -COOH, -COOR^{PR}, -F, -Cl, -Br, -I, -SCN, -CN, -NO₂, =NHO, -CH₃, -CF₃, -C₂H₅ or -C₆H₅ moieties that replace (or substitute) one or more hydrogen or carbon atoms, wherein such moieties may be adjacent to one another, e.g., they can comprise -C(O)-NH- or -NH-C(O)-NH-. In some embodiments, moieties that replace a hydrogen or carbon atom will not replace a divalent or trivalent carbon atom, or a hydrogen that is bonded to such a carbon atom, e.g., in -CH=CH- or in - C≡C- and specific embodiments include one or more substitutions at carbons that are separated from a -CH=CH- or -C≡C- moiety by one, two, three or more -CH₂- moieties. In some embodiments, one or two hydrogen atoms that are bonded to the distal carbon atom is substituted by one, two or three -OH, =O -SH, =S, -NH₂, -COOH, -COOR^{PR}, -F, -Cl, -Br, -I, - SCN, -CN, -NO₂ or =NHO moieties.

Group 38 comprises subgroups 38-1 through 38-37-36-35-34-33-32-31-30-29-28-27-26-25-23-21-17-10-8-6, which name compounds or genera of compounds essentially as described for the other compound groups above. The subgroups in group 38 are 38-1 through 38-6, 38-7-1 through 38-7-6, 38-8-1 through 38-8-6, 38-9-1 through 38-9-6 and so on essentially as described for the groups above.

**Group 39.** Group 39 comprises each compound or genus named in compound groups 1 through 38, but wherein R⁹ in formula B is not -CH₂- or a heteroatom, i.e., R¹⁰ is bonded to R⁹ in formula B and it is not a hydrogen atom and wherein when a double bond is present at the 1-2 position, this R¹⁰ is not bonded to R⁹ by a double bond. Thus, the carbon atom at the 2 position is not pentavalent or charged.

This R¹⁰ optionally comprises -OH, =O, -OR^{PR}, -SH, =S, -SR^{PR}, -NH₂, -NHR^{PR}, a halogen, optionally substituted alkyl, optionally substituted alkenyl, optionally substituted alkynyl, optionally substituted aryl, optionally substituted alkylaryl, an optionally substituted heterocycle, an ester, an ether, a thioester, a thionoester, a thioether, an optionally substituted monosaccharide, an optionally substituted oligosaccharide, a carbonate, a carbamate, an amide or an amino acid. Any of these moieties, may comprise any R¹⁰ structure disclosed herein.

Other exemplary R¹⁰ moieties include -C≡C-(CH₂)ₙH (e.g., -C≡CH and -C≡C-CH₃), -C=C-(CH₂)ₙH, -(CH₂)ₙH (e.g., -CH₃, -C₂H₅, -C₃H₇), -(CH₂)ₙC₆H₅, wherein n is 0, 1, 2, 3, 4, 5, 6, 7 or 8 and any of these exemplary second R⁴ moieties optionally comprise 1, 2, 3, 4 or more independently selected -O-, -OH, =O, -S-, -SH, =S, -NH-, -NH2, -COOH, -COOR^{PR}, -F, -Cl, -Br, -I, -SCN, -CN, -NO₂, =NHO, -CH₃, -CF₃, -C₂H₅ or -C₆H₅ moieties that replace (or substitute) one or more hydrogen or carbon atoms, wherein such moieties may be adjacent to one another, e.g., they can comprise -C(O)-NH- or -NH-C(O)-NH-. In some embodiments the moieties that replace a hydrogen or carbon atom will not replace a divalent or trivalent carbon atom, or a hydrogen that is bonded to such a carbon atom, e.g., in -CH=CH- or in - C≡C- and specific embodiments include one or more substitutions at carbons that are separated from a -CH=CH- or -C≡C- moiety by one, two, three or more -CH₂- moieties. In some embodiments, one or two hydrogen atoms that are bonded to the distal carbon atom is substituted by one, two or three -OH, =O -SH, =S, -NH₂, -COOH, -COOR^{PR}, -F, -Cl, -Br, -I, - SCN, -CN, -NO₂ or =NHO moieties.

Group 39 comprises subgroups 39-1 through 39-38-37-36-35-34-33-32-31-30-29-28-27-26-25-23-21-17-10-8-6, which name compounds or genera of compounds essentially as described for the other compound groups above. The subgroups in group 39 are 39-1 through 39-6, 39-7-1 through 39-7-6, 39-8-1 through 39-8-6, 39-9-1 through 39-9-6 and so on essentially as described for the groups above.

**Group 40**. Group 40 comprises each compound or genus named in compound groups 1 through 39, wherein R⁷ in formula B is -O-, instead of a -CH₂- or -CHR¹⁰- moiety, where R¹⁰ is not hydrogen. Group 40 comprises subgroups 40-1 through 40-39-38-37-36-35-34-33-32-31-30-29-28-27-26-25-23-21-17-10-8-6, which name compounds or genera of compounds essentially as described for the other compound groups. The subgroups in group 40 are 40-1 through 40-6, 40-7-1 through 40-7-6, 40-8-1 through 40-8-6, 40-9-1 through 40-9-6 and so on essentially as described for the groups above. The subgroup 40-1, 40-2 40-8-1, 40-8-2, 40-11-1 and 40-11-2 compounds named 1.2.5.9 have the structures

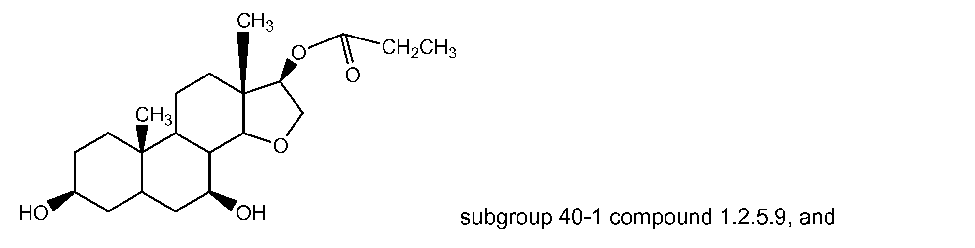

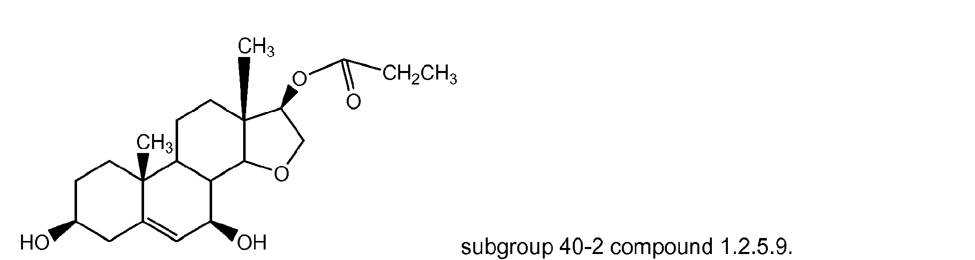

Subgroup 40-8-1 and 40-8-2 compounds named 1.2.5.9 have the structures

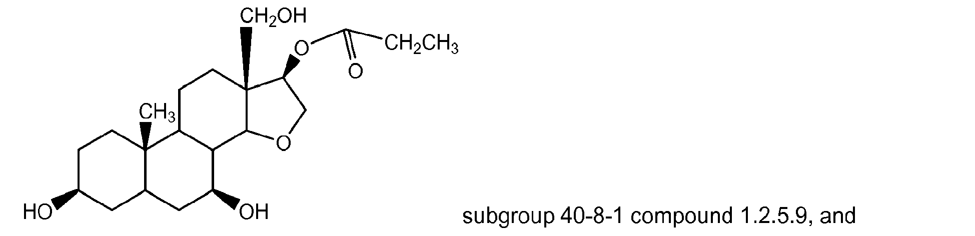

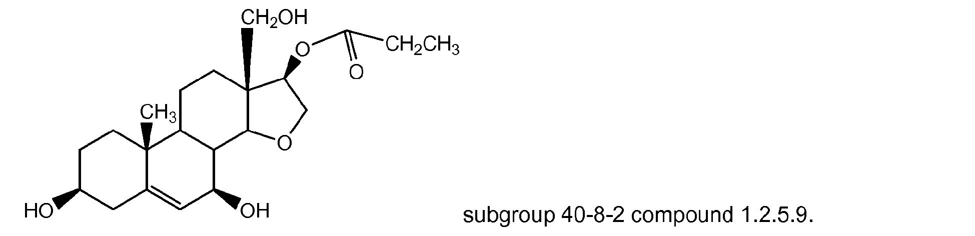

The subgroup 40-11-1 and 40-11-2 compounds named 1.2.5.9 have the structures

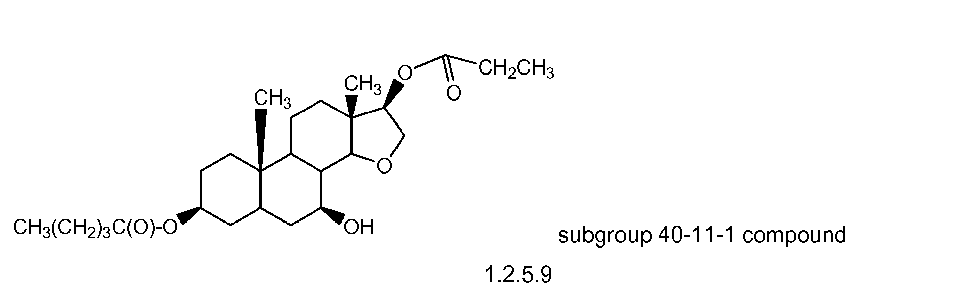

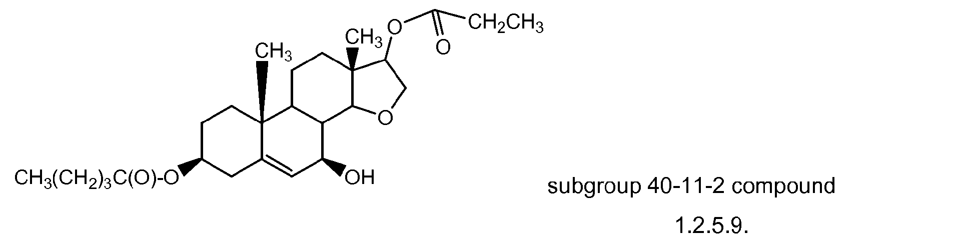

**Group 41.** Group 41 comprises each compound or genus named in compound groups 1 through 39, wherein R⁸ in formula B is -O-, instead of a -CH₂- or -CHR¹⁰- moiety, where R¹⁰ is not hydrogen. Group 41 comprises subgroups 41-1 through 41-39-38-37-36-35-34-33-32-31-30-29-28-27-26-25-23-21-17-10-8-6, which name compounds or genera of compounds essentially as described for the other compound groups. The subgroups in group 41 are 41-1 through 41-6, 41-7-1 through 41-7-6, 41-8-1 through 41-8-6, 41-9-1 through 41-9-6 and so on essentially as described for the groups above. Group 41 compounds are named in essentially the same manner as described for group 40 and other compound groups. Thus, for example, subgroup 41-1, 41-2, 41-8-1, 41-8-2, 41-11-1 and 41-11-2 compounds named 1.2.5.9 have the structures shown for these compounds in group 40, except that an oxygen atom is present at the 11 position and no oxygen is present at the 15 position.

**Group 42**. Group 42 comprises each compound or genus named in compound groups 1 through 39, wherein R⁸ in formula B is -O-, instead of a -CH₂- or -CHR¹⁰- moiety, where R¹⁰ is not hydrogen. Group 42 comprises subgroups 42-1 through 42-39-38-37-36-35-34-33-32-31-30-29-28-27-26-25-23-21-17-10-8-6, which name compounds or genera of compounds essentially as described for the other compound groups. The subgroups in group 42 are 42-1 through 42-6, 42-7-1 through 42-7-6, 42-8-1 through 42-8-6, 42-9-1 through 42-9-6 and so on essentially as described for the groups above. Group 42 compounds are named in essentially the same manner as described for group 40 and other compound groups. Thus, for example, subgroup 42-1, 42-2, 42-8-1, 42-8-2, 42-11-1 and 42-11-2 compounds named 1.2.5.9 have the structures shown for these compounds in group 40, except that an oxygen atom is present at the 2 position and no oxygen is present at the 15 position.

This group does not include species or genera of compounds wherein a double bond is present at the 1-2 position, since this would make the oxygen atom charged. Therefore, there is, e.g., no group 42-3, 42-4, 42-6, 42-7-3, 42-7-4 or 42-7-6, since the 3, 4 and 6 groups and their variants all have a double bond at the 1-2 position.

**Group 43.** Group 43 comprises each compound or genus named in compound groups 1 through 39, wherein R⁷ in formula B is -NH-, instead of a -CH₂- or -CHR¹⁰- moiety, where R¹⁰ is not hydrogen. Group 43 comprises subgroups 43-1 through 43-39-38-37-36-35-34-33-32-31-30-29-28-27-26-25-23-21-17-10-8-6, which name compounds or genera of compounds essentially as described for the other compound groups. The subgroups in group 43 are 43-1 through 43-6, 43-7-1 through 43-7-6, 43-8-1 through 43-8-6, 43-9-1 through 43-9-6 and so on essentially as described for the groups above. The subgroup 43-1, 43-2 43-8-1, 43-8-2, 43-11-1 and 43-11-2 compounds named 1.2.5.9 have the structures shown for these compounds in group 40, except that -NH- is present at the 15 position instead of oxygen.

**Group 44.** Group 44 comprises each compound or genus named in compound groups 1 through 39, wherein R⁸ in formula B is -NH-, instead of a -CH₂- or -CHR¹⁰- moiety, where R¹⁰ is not hydrogen. Group 44 comprises subgroups 44-1 through 44-39-38-37-36-35-34-33-32-31-30-29-28-27-26-25-23-21-17-10-8-6, which name compounds or genera of compounds essentially as described for the other compound groups. The subgroups in group 44 are 44-1 through 44-6, 44-7-1 through 44-7-6, 44-8-1 through 44-8-6, 44-9-1 through 44-9-6 and so on essentially as described for the groups above. Group 44 compounds are named in essentially the same manner as described for group 40 and other compound groups. Thus, for example, subgroup 44-1, 44-2, 44-8-1, 44-8-2, 44-11-1 and 44-11-2 compounds named 1.2.5.9 have the structures shown for these compounds in group 40, except that -NH- is present at the 11 position and no oxygen is present at the 15 position.

**Group 45.** Group 45 comprises each compound or genus named in compound groups 1 through 39, wherein R⁹ in formula B is -NH- or -N=, instead of a -CH₂- or -CHR¹⁰-moiety, where R¹⁰ is not hydrogen. Group 45 comprises subgroups 45-1 through 45-39-38-37-36-35-34-33-32-31-30-29-28-27-26-25-23-21-17-10-8-6, which name compounds or genera of compounds essentially as described for the other compound groups. The subgroups in group 45 are 45-1 through 45-6, 45-7-1 through 45-7-6, 45-8-1 through 45-8-6, 45-9-1 through 45-9-6 and so on essentially as described for the groups above. Group 45 compounds are named in essentially the same manner as described for group 40 and other compound groups. Thus, for example, subgroup 45-1, 45-2, 45-8-1, 45-8-2, 45-11-1 and 45-11-2 compounds named 1.2.5.9 have the structures shown for these compounds in group 40, except that -NH- is present at the 2 position and no oxygen is present at the 15 position.

**Group 46.** Group 46 comprises each compound or genus named in compound groups 1 through 39, wherein R⁷ in formula B is -S-, instead of a -CH₂- or -CHR¹⁰- moiety, where R¹⁰ is not hydrogen. Group 46 comprises subgroups 46-1 through 46-39-38-37-36-35-34-33-32-31-30-29-28-27-26-25-23-21-17-10-8-6, which name compounds or genera of compounds essentially as described for the other compound groups. The subgroups in group 46 are 46-1 through 46-6, 46-7-1 through 46-7-6, 46-8-1 through 46-8-6, 46-9-1 through 46-9-6 and so on essentially as described for the groups above. The subgroup 46-1, 46-2 46-8-1, 46-8-2, 46-11-1 and 46-11-2 compounds named 1.2.5.9 have the structures shown for these compounds in group 40, except that -S- is present at the 15 position instead of oxygen.

**Group 47.** Group 47 comprises each compound or genus named in compound groups 1 through 39, wherein R⁸ in formula B is -S-, instead of a -CH₂- or -CHR¹⁰- moiety, where R¹⁰ is not hydrogen. Group 47 comprises subgroups 47-1 through 47-39-38-37-36-35-34-33-32-31-30-29-28-27-26-25-23-21-17-10-8-6, which name compounds or genera of compounds essentially as described for the other compound groups. The subgroups in group 47 are 47-1 through 47-6, 47-7-1 through 47-7-6, 47-8-1 through 47-8-6, 47-9-1 through 47-9-6 and so on essentially as described for the groups above. Group 47 compounds are named in essentially the same manner as described for group 40 and other compound groups. Thus, for example, subgroup 47-1, 47-2, 47-8-1, 47-8-2, 47-11-1 and 47-11-2 compounds named 1.2.5.9 have the structures shown for these compounds in group 40, except that -S- is present at the 11 position and no oxygen is present at the 15 position.

**Group 48.** Group 48 comprises each compound or genus named in compound groups 1 through 39, wherein R⁹ in formula B is -S-, instead of a -CH₂- or -CHR¹⁰- moiety, where R¹⁰ is not hydrogen. Group 48 comprises subgroups 48-1 through 48-39-38-37-36-35-34-33-32-31-30-29-28-27-26-25-23-21-17-10-8-6, which name compounds or genera of compounds essentially as described for the other compound groups. The subgroups in group 48 are 48-1 through 48-6, 48-7-1 through 48-7-6, 48-8-1 through 48-8-6, 48-9-1 through 48-9-6 and so on essentially as described for the groups above. Group 48 compounds are named in essentially the same manner as described for group 40 and other compound groups. Thus, for example, subgroup 48-1, 48-2, 48-8-1, 48-8-2, 48-11-1 and 48-11-2 compounds named 1.2.5.9 have the structures shown for these compounds in group 40, except that -S- is present at the 2 position and no oxygen is present at the 15 position.

This group does not include species or genera of compounds wherein a double bond is present at the 1-2 position. Therefore, there is, e.g., no group 48-3, 48-4, 48-6, 48-7-3, 48-7-4 or 48-7-6, since the 3, 4 and 6 groups and their variants all have a double bond at the 1-2 position.

**Group 49.** Group 49 comprises each compound or genus named in compound groups 1 through 39, but wherein two of R⁷, R⁸ and R⁹ in formula B independently are -O-, - NH-, =NH- or -S-, instead of -CH₂- or -CHR¹⁰-, where R¹⁰ is not hydrogen. This group includes 27 combinations of two heteroatoms (O, N or S) that are at any two of R⁷, R⁸ and R⁹. These are (49c1, i.e., combination number 1) O2-O11 (i.e., oxygen at the 2 and 11 positions), (49c2) 02-015, (49c3) 011-015, (49c4) O2-N11 (i.e., oxygen at the 2-position and nitrogen at the 11 position), (49c5) O2-N15, (49c6) O11-N15, (49c7) O2-S11 (i.e., oxygen at the 2-position and sulfur at the 11 position), (49c8) O2-S15, (49c9) O11-S15, (49c10) N2-N11, (49c11) N2-N15, (49c12) N11-N15, (49c13) N2-O11, (49c14) N2-O15, (49c15) N11-015, (49c16) N2-S11, (49c17) N2-S15, (49c18) N11-S15, (49c19) S2-S11, (49c20) S2-S 15, (49c21) S11-S15, (49c22) S2-O11, (49c23) S2-O15, (49c24) S11-O15, (49c25) S2-N11, (49c26) S2-N15 and (49c27) S11-N15.

Group 49 comprises subgroups 49c1-1 through 49c27-39-38-37-36-35-34-33-32-31-30-29-28-27-26-25-23-21-17-10-8-6, which name compounds or genera of compounds essentially as described for the other compound groups. The subgroups in group 49 are 49c1-1 through 49c1-6, 49c1-7-1 through 49c1-7-6, 49c1-8-1 through 49c1-8-6, 49c1-9-1 through 49c1-9-6 and so on essentially as described for the groups above. Group 49 compounds are named in essentially the same manner as described for group 40 and other compound groups. Thus, for example, subgroup 49c1-1, 49c1-2, 49c1-8-1, 49c1-8-2, 49c1-11-1 and 49c1-11-2 compounds named 1.2.5.9 have the structures shown for these compounds in group 40, except that -O- is present at the 2 and 11 positions and no oxygen is present at the 15 position. Similarly, subgroup 49c10-1, 49c10-2, 49c10-8-1, 49c10-8-2, 49c10-11-1 and 49c10-11-2 compounds named 1.2.5.9 have the structures shown for these compounds in group 40, except that -NH- or =N- is present at the 2 and 11 positions and no oxygen is present at the 15 position. This group does not include species or genera of compounds wherein a double bond and either -O- or -S- is present at the 2-position.

**Group 50.** Group 50 comprises each compound or genus named in compound groups 1 through 39, but wherein all three of R⁷, R⁸ and R⁹ in formula B independently are - O-, -NH-, =NH- or -S-, instead of -CH₂- or -CHR¹⁰-, where R¹⁰ is not hydrogen. This group includes all combinations of 3 heteroatoms (O, N or S) that are at R⁷, R⁸ and R⁹. The combinations are defined essentially as described for the combinations in group 49. They are (50c1) 02-O11-O15, (50c2) O2-O11-N15, (50c3) O2-N11-O15, (50c4) O2-N11-N15, (50c5) O2-O11-S15, (50c6) O2-S11-O15, (50c7) O2-S11-S15, (50c8) N2-N11-N15, (50c9) N2-N11-O15, (50c10) N2-O11-N15, (50c11) N2-O11-O15, (50c12) N2-N11-S15, (50c13) N2-S11-N15, (50c14) N2-S11-S15, (50c15) S2-S11-S15, (50c16) S2-S11-O15, (50c17) S2-O11-S15, (50c18) S2-S11-O15, (50c19) S2-S11-N15, (50c20) S2-N11-S15, (50c21) S2-N11-N15, (50c22) S2-N11-S15, (50c23) O2-S11-N15, (50c24) N2-O11-S15, (50c25) N2-S11-O15, (50c26) S2-011-N15 and (50c27) S2-N11-015.

Group 50 comprises subgroups 50c1-1 through 50c27-39-38-37-36-35-34-33-32-31-30-29-28-27-26-25-23-21-17-10-8-6, which name compounds or genera of compounds essentially as described for the other compound groups. The subgroups in group 50 are 50c1-1 through 50c1-6, 50c1-7-1 through 50c1-7-6, 50c1-8-1 through 50c1-8-6, 50c1-9-1 through 50c1-9-6 and so on essentially as described for the groups above. Group 50 compounds are named in essentially the same manner as described for group 40 and other compound groups. Thus, for example, subgroup 50c1-1, 50c1-2, 50c1-8-1, 50c1-8-2, 50c1-11-1 and 50c1-11-2 compounds named 1.2.5.9 have the structures shown for these compounds in group 40, except that -O- is also present at the 2 and 11 positions. Similarly, subgroup 50c10-1, 50c10-2, 50c10-8-1, 50c10-8-2, 50c10-11-1 and 50c10-11-2 compounds named 1.2.5.9 have the structures shown for these compounds in group 40, except that - NH- or =N- is present at the 2 and 15 positions and oxygen is present at the 11 position. This group does not include species or genera of compounds wherein a double bond and either -O- or -S- is present at the 2-position.

**Group 51.** Group 51 comprises each compound or genus named in compound groups 1 through 50, but wherein R⁷ comprises a -X-CHR¹⁰- moiety, where X is -O-, -NR^{PR}-or -S-. This group includes all R⁷ moieties, i.e., (51 a1) -O-CHR¹⁰-, (51 a2) -NR^{PR}-CHR¹⁰-, (51 a3) -S-CHR¹⁰-, (51 a4) -CHR¹⁰-O-, (51 a5) -CHR¹⁰-NR^{PR}- and (51 a6) -CHR¹⁰-S-. Group 51 comprises subgroups 51a1-1 through 51a6-50c27-49c27-48-47-46-45-44-43-42-41-40-39-38-37-36-35-34-33-32-31-30-29-28-27-39-38-37-36-35-34-33-32-31-30-29-28-27-26-25-23-21-17-10-8-6, which name compounds or genera of compounds essentially as described for the other compound groups. The subgroups in group 51 are 51a1-1 through 51a1-6, 51a1-7-1 through 51a1-7-6, 51a1-8-1 through 51a1-8-6, 5a1-9-1 through 51a1-9-6 and so on essentially as described for the groups above.

In some embodiments, the R¹⁰ included in R⁷ is hydrogen. In others, the R¹⁰ included in R⁷ is optionally comprises -OH, =O, -OR^{PR}, -SH, =S, -SR^{PR}, -NH₂, -NHR^{PR}, a halogen, optionally substituted alkyl, optionally substituted alkenyl, optionally substituted alkynyl, optionally substituted aryl, optionally substituted alkylaryl, an optionally substituted heterocycle, an ester, an ether, a thioester, a thionoester, a thioether, an optionally substituted monosaccharide, an optionally substituted oligosaccharide, a carbonate, a carbamate, an amide or an amino acid. The R¹⁰ may comprise any R¹⁰ structure disclosed herein.

Other exemplary R¹⁰ moieties include -C≡C-(CH₂)ₙH (e.g., -C≡CH and -C≡C-CH₃), -C=C-(CH₂)ₙH, -(CH₂)ₙH (e.g., -CH₃, -C₂H₅, -C₃H₇), -(CH₂)ₙC₆H₅, wherein n is 0, 1, 2, 3, 4, 5, 6, 7 or 8 and any of these exemplary R¹⁰ moieties optionally comprise 1, 2, 3, 4 or more independently selected -O-, -OH, =O, -S-, -SH, =S, -NH-, -NH₂, -COOH, -COOR^{PR}, -F, -Cl, - Br, -I, -SCN, -CN, -NO₂, =NHO, -CH₃, -CF₃, -C₂H₅ or -C₆H₅ moieties that replace (or substitute) one or more hydrogen or carbon atoms, wherein such moieties may be adjacent to one another, e.g., they can comprise -C(O)-NH- or -NH-C(O)-NH-. In some embodiments the moieties that replace a hydrogen or carbon atom will not replace a divalent or trivalent carbon atom, or a hydrogen that is bonded to such a carbon atom, e.g., in -CH=CH- or in - C≡C- and specific embodiments include one or more substitutions at carbons that are separated from a -CH=CH- or -C≡C- moiety by one, two, three or more -CH₂- moieties. In some embodiments, one or two hydrogen atoms that are bonded to the distal carbon atom is substituted by one, two or three -OH, =O -SH, =S, -NH₂, -COOH, -COOR^{PR}, -F, -Cl, -Br, -I, - SCN, -CN, -NO₂ or =NHO moieties.

**Group 52.** Group 52 comprises each compound or genus named in compound groups 1 through 49, but wherein R⁷ is absent and the ring in formula B that contains R⁷ comprises a cyclobutyl moiety with R³ and one or two R⁴ bonded to it. Group 52 comprises subgroups 52-1 through 52-51a6-50c27-49c27-48-47-46-45-44-43-42-41-40-39-38-37-36-35-34-33-32-31-30-29-28-27-39-38-37-36-35-34-33-32-31-30-29-28-27-26-25-23-21-17-10-8-6, which name compounds or genera of compounds essentially as described for the other compound groups. The subgroups in group 52 are 52-1 through 52-6, 52-7-1 through 52-7-6, 52-8-1 through 52-8-6, 52-9-1 through 52-9-6 and so on essentially as described for the groups above.

**Group 53.** Group 53 comprises each compound or genus named in compound groups 1 through 52, but wherein R⁸ is absent and the ring in formula B that contains R⁸ comprises a 5 membered ring moiety. Group 53 comprises subgroups 53-1 through 53-52-51 a6-50c27-49c27-48-47-46-45-44-43-42-41-40-39-38-37-36-35-34-33-32-31-30-29-28-27-39-38-37-36-35-34-33-32-31-30-29-28-27-26-25-23-21-17-10-8-6, which name compounds or genera of compounds essentially as described for the other compound groups. The subgroups in group 53 are 53-1 through 53-6, 53-7-1 through 53-7-6, 53-8-1 through 53-8-6, 53-9-1 through 53-9-6 and so on essentially as described for the groups above.

The subgroups here do not include compounds or genera where two ring heteroatoms are present as described in group 49 and where both R⁷ and R⁸ are absent ("group 53-52-49-.... "), since such groups are mutually incompatible. This holds for all of the compound groups described herein, i.e., whenever the structures that a first group or subgroup specifies is incompatible with the structure that a second group or subgroup specifies, then the structure that the first group or subgroup specifies is not included. However, all other possible compounds and genera are included in such compound groups.

**Group 54.** Group 54 comprises each compound or genus named in compound groups 1 through 53, but wherein R⁹ is absent and the ring in formula B that contains R⁹ comprises a 5 membered ring moiety. Group 54 comprises subgroups 54-1 through 54-53-52-51 a6-50c27-49c27-48-47-46-45-44-43-42-41-40-39-38-37-36-35-34-33-32-31-30-29-28-27-39-38-37-36-35-34-33-32-31-30-29-28-27-26-25-23-21-17-10-8-6, which name compounds or genera of compounds essentially as described for the other compound groups. The subgroups in group 54 are 54-1 through 54-6, 54-7-1 through 54-7-6, 54-8-1 through 54-8-6, 54-9-1 through 54-9-6 and so on essentially as described for the groups above. The subgroups here do not include, e.g., compounds or genera where two or three ring heteroatoms are present as described in group 49 or 50 and where two or three of R⁷, R⁸ and R⁹ are absent (e.g., "group 54-52-49-.... ").

The individual compounds and genera named in groups 1-54 above may also be named using any suitable formal or informal chemical nomenclature. Thus, as will be apparent, individual compounds in these groups incude 16α-bromoepiandrosterone, 16α-hydroxyepiandrosterone, 3α,16α-dihydroxy-5α-androstane-17-one, 3α,16α,17β-trihydroxy-5α-androstane, 3α,16α,17α-trihydroxy-5α-androstane, 3β,17β-dihydroxyandrost-5-ene or 3β,7β,17β-trihydroxyandrost-5-ene, 7-oxodehydroepiandrosterone, 16α-fluoroandrost-5-ene-17-one, 7α-hydroxy-16α-fluoroandrost-5-ene-17-one, 7β-hydroxy-16α-fluoroandrost-5-ene-17-one, 17α-hydroxy-16α-fluoroandrost-5-ene, 17β-hydroxy-16α-fluoroandrost-5-ene and the like.

Any of the species of compounds or genera of compounds that are disclosed herein, e.g., as named in compound groups 1 through 54-53-52-51a6-50c27-49c27-48-47-46-45-44-43-42-41-40-39-38-37-36-35-34-33-32-31-30-29-28-27-39-38-37-36-35-34-33-32-31-30-29-28-27-26-25-23-21-17-10-8-6 or elsewhere in this disclosure, are suitable for use in the methods as described herein or in the cited references.

Additional embodiments of the formula 1 compounds include any compound or genus of compounds that are disclosed herein, e.g., any of the compounds or genera of compounds in groups 1 through 54 wherein one or both of R⁵ or R⁶ independently comprises -CH₂SH, -CHO, -CH₂NRPR, -CH₂NH₂, -C₂H₅, -C₂H₄OH, -C₂H₄SH, -C₂H₄NH₂, -CH₂CHO, - CH₂CH₂NR^{PR}, -CH₂CH₂OH, -CH₂CH₂SH, -CH₂CH₂C₆H₅, -CH₂C₆H₅ or -C₆H₅ wherein any phenyl (C₆H₅) moiety in the foregoing groups is optionally substituted at the phenyl ring with 1, 2, 3, 4 or 5 moieties independently selected from those described for esters herein and including C1-6 alkyl (optionally substituted with 1 or 2 independently selected -OH, -SH, -O-, -S- or -NH-) C1-6 alkoxy, -F, -Cl, -Br, -I, -CN, -NO₂, -OH, -SH, -COOR^{PR}, -NHR^{PR} and -C(O)-C1-6 alkyl.

Exemplary formula 1 compounds will comprise compounds where the steroid has the structure

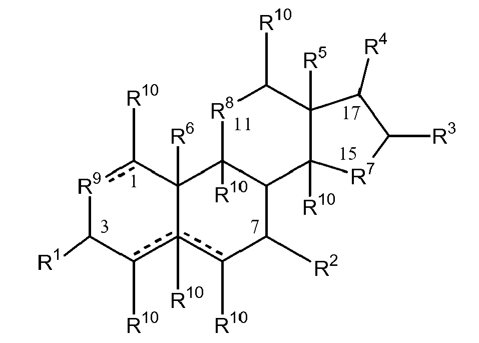

and (1) one of the atoms or groups described immediately above at the 1, 2, 3, 4, 5, 6, 7, 8, 9, 11, 12, 15, 16 or 17 positions, with independently selected groups at the remaining variable group positions, e.g., -H, -OH, =O, -SH, -CHO, -CH₂-, -O-, -S-, -NH-, halogen, optionally substituted alkyl, acyl, ester or any other moiety described herein, (2) two of these groups, which are the same or are independently selected and are at, e.g., the 2,3, 2,7, 2,11, 2,15, 2,16, 2,17, 3,7, 3,11, 3,15, 3,16, 3,17, 7,11, 7,15, 7,16, 7,17, 11,15, 11,16, 11,17 or 16,17 positions, with independently selected groups at the remaining variable group positions, e.g., -H, -OH, =O, -SH, -CHO, -CH₂-, -O-, -S-, -NH-, halogen, optionally substituted alkyl, acyl, ester or any other moiety described herein, (3) three of these groups, which are the same or are independently selected and are at, e.g., the 2,3,7, 2,3,11, 2,3,15, 2,3,16, 2,3,17, 3,7,11, 3,7,15, 3,7,16, 3,7,17, 7,11,15, 7,11,16, 7,11,17, 11,15,16, 11,15,17, or 15,16,17 positions, with independently selected groups at the remaining variable group positions, e.g., -H, -OH, =O, -SH, -CHO, -CH₂-, -O-, -S-, -NH-, halogen, optionally substituted alkyl, acyl, ester or any other moiety described herein, (4) four of these groups, which are the same or are independently selected and are at, e.g., the 2,3,7,11, 2,3,7,15, 2,3,7,16, 2,3,7,17, 3,7,11,15, 3,7,11,16, 3,7,11,17, 7,11,15,16, 7,11,15,17 or 11,15,16,17 positions, with independently selected groups at the remaining variable group positions, e.g., -H, -OH, =O, -SH, -CHO, -CH₂-, -O-, -S-, -NH-, halogen, optionally substituted alkyl, acyl, ester or any other moiety described herein or (5) any of the foregoing compounds in (1) through (4) wherein 1, 2 or 3 R¹⁰ at the 1, 4, 5, 6, 9, 12 and 14 positions are -OH, -SH, halogen, an ester, a thioester, a thionoester, optionally substituted alkyl (e.g., C1-C8), optionally substituted alkoxy (e.g., C1-C8), optionally substituted alkenyl (e.g., C2-C8), or an optionally substituted heterocycle or any other moiety described herein and the remaining R¹⁰ are -H.

When a substituent is an oligonucleotide or a polymer usually only a one of these is bonded to the formula 1 compound. Typically, when R¹-R² and R⁴-R⁶ comprise one or more of these substituents (or others described herein), the substituent is present in the β-configuration, while R³ typically comprises a substituent in the β-configuration. In some embodiments, R² is in the α-configuration.

In some embodiments, one or more of the variable groups that are bonded to the formula 1 compounds, e.g., R¹-R⁶, R¹⁰, R¹⁵, R¹⁷ and R¹⁸, independently comprise a nucleoside, a nucleotide, an oligonucleotide or an analog of any of these moieties. Typically such moieties are linked to the steroid nucleus through a terminal hydroxyl, thiol, acyl moiety or amine at the 5', 3' or 2' positions, when a hydroxyl, thiol, acyl moiety or amine is present at that position. For oligonucleotides and oligonucleotide analogs, the linkage to the steroid occasionally is through a sugar hydroxyl at an internal 2' position.

Individual formula 1 compounds, e.g., those named in any of the compound groups 1 through 54 are suitable for use as standards for various analytical methods, e.g., for use in HPLC, MS, NMR, IR or other analytical methods. Thus, to aid in the determination of, e.g., the structure of a metabolite of a formula 1 compound or a structurally related compound, another structurally related formula 1 compound could be used. Metabolism of formula 1 compounds will include one or more of hydroxylation or conjugation, usually to a - OH moiety, with a moiety such as sulfate, phosphate or a monosaccharide such as glucuronic acid at, e.g., the 2, 3, 7, 11, 15, 16 or 17 positions. In these embodiments, the appropriate use of a formula 1 compound of known structure as a standard can aid in or verify the identification of metabolites that are projected to have closely related structures. Information regarding the identification can be useful or sometimes is necessary for, e.g., obtaining regulatory approval to market a therapeutic agent such as a formula 1 compound or understanding the potential biological role that a formula 1 compound or its metabolite can play in one of the applications disclosed herein or in a cited reference. To facilitate such uses, the formula 1 compound may be labeled as appropriate, e.g., using a formula 1 compound with, e.g., a ¹³C atom at 1, 2 or more of the 1, 2, 3, 4, 6, 7, 11, 12, 15, 16, 17, 18 or 19 positions in the steroid.

Typical containers for storage of compositions and formulations that comprise a formula 1 compound will limit the amount of water that reaches the materials contained therein. Typically, formulations are packaged in hermetically or induction sealed containers. The containers are usually induction sealed. Water permeation characteristics of containers have been described, e.g., Containers--Permeation, chapter, USP 23 <671 >, United States Pharmacopeial Convention, Inc., 12601 Twinbrook Parkway, Rockville, MD 20852, pp.: 1787 et seq. (1995).

Dosing protocols or methods. In treating any of the conditions or symptoms disclosed herein, one can continuously (daily) or intermittently administer the formula 1 compound(s) to a subject suffering from or susceptible to the condition or symptom. In treating a condition such as an infection, a hyperproliferation condition, an inflammation condition or another condition disclosed herein with a formula 1 compound intermittent dosing can avoid or ameliorate some of the undesired aspects normally associated with discontinuous dosing.

In any of the continuous (daily) or intermittent dosing protocols described herein, or in treating any of the diseases, conditions or symptoms described herein, the formula 1 compound(s) can be administered by one or more suitable routes, e.g., oral, buccal, sublingual, intramuscular (i.m.), subcutaneous (s.c.), intravenous (i.v.), intradermal, another parenteral route or by an aerosol. The daily dose in such methods will typically comprise about 0.05 mg/kg/day to about 200 mg/kg/day, or about 0.1 to about 100 mg/kg/day, including about 0.2 mg/kg/day, 0.5 mg/kg/day, about 1 mg/kg/day, about 2 mg/kg/day, about 4 mg/kg/day, about 6 mg/kg/day, about 10 mg/kg/day, about 20 mg/kg/day, about 40 mg/kg/day or about 100 mg/kg/day. Higher dosages, e.g., about 250 mg/kg/day, about 300 mg/kg/day or about 350 mg/kg/day can also be utilized, e.g., in some veterinary applications or for human short term dosing, e.g., for about 1-7 days, optionally followed by lower dosages. One can administer the formula 1 compound(s) orally using about 4 to about 60 mg/kg/day, usually about 6-30 mg/kg/day. In some embodiments, the intermittent dosing methods exclude dosing protocols that are commonly used to deliver contraceptive steroids to, e.g., human females, such as daily dosing for 21 days, followed by no dosing for 7 days. In some embodiments, the non-aqueous formulations described herein that comprise a formula 1 compound(s) are administered i.m. or s.c., while aqueous formulations that contain formula 1 compound(s) is administered by i.v., i.m., s.c. or other parenteral routes.

Dosages of the formula 1 compound, routes of administration and the use of combination therapies with other standard therapeutic agents or treatments could be applied essentially as described above for any of the diseases or conditions that are disclosed herein. Thus, the formula 1 compounds may be administered prophylactically or therapeutically in chronic conditions or they may be administered at the time of or relatively soon after an acute event such as the onset of surgery, a migraine or the occurrence of trauma, e.g., accidental central nervous system injury or a cerebral stroke or infarction. For acute events, the formula1 compounds may thus be administered concurrently, e.g., within about 15 minutes or about 30 minutes of the onset or occurrence of the acute event, or at a later time, e.g., at about 1, 2, 3, 4, 5, 6, 7, 8, 9, 10, 11, 12, 13, 14, 15, 16, 18, 20, 22, 24, 26, 28, 30, 36, 42, 48, 54, 60, 72, 84, 96, 108 or 120 hours after the onset or occurrence of the acute event. The formula 1 compounds may thus be administered at about 6-120 hours, or about 8-48 hours, about 10-24 hours or about 12-16 hours after an acute event starts or occurs.

Alternatively, the formula1 compounds may thus be administered before, e.g., within about 15 minutes or about 30 minutes before the onset or occurrence of a planned or anticipated acute event, or at an earlier time, e.g., at about 1, 2, 3, 4, 5, 6, 7, 8, 9, 10, 11, 12, 13, 14, 15, 16, 18, 20, 22, 24, 26, 28, 30, 36, 42, 48, 54, 60, 72, 84, 96, 108 or 120 hours before the onset or occurrence of the acute event. The formula 1 compounds may thus be administered at about 6-120 hours, or about 8-48 hours, about 10-24 hours or about 12-16 hours before the planned or anticipated acute event starts or occurs.

Synthesis methods.

Exemplary synthesis methods are shown below.

Scheme 1. For the structures shown in scheme 1, R⁵-R⁹ are as defined for formula 1 compounds. Thus, when R⁵ and R⁶ are both -CH₃ in the β-configuration, R⁷, R⁸ and R⁹ are all -CH₂-, H at the 9 and 14 positions are in the α-configuration, acetate at the 3-position is in the β-configuration, and H at the 8 position is in the β-configuration, the first compound in scheme 1 is DHEA acetate. The acetate groups at the 3, 7, 16, 17 or other positions in this scheme and in other schemes disclosed herein may independently be other ester moieties as described herein, e.g., C₂₋₅₀ esters including -C(O)-(CH₂)₀₋₄-(CF₂)₀₋₄-CF₃, including -C(O)-CF₃, -C(O)-C₂₋₂₉ optionally substituted alkyl, -C(O)-CH₂-C₂₋₂₈ optionally substituted alkenyl, -C(O)-CH₂-C₂₋₂₈ optionally substituted alkynyl, -C(O)-(CH₂)₀₋₆-optionally substituted phenyl, or -C(O)-(CH₂)₀₋₆-optionally substituted heterocycle or other organic moieties as disclosed herein or in the cited references.

Typical substituents for these organic moieties are as described herein, including one, two, three or more independently selected -O-, =O, optionally protected hydroxyl, -S-, optionally protected thiol, -NH-, optionally protected -NH₂, optionally protected -C(O)OH, -C(O)-NH-, -C(O)-NH₂, -NH₂-C(O)-H, -NH₂-C(O)-C₀₋₄H₁₋₉, -NH₂-C(O)-O-C₀₋₄H₁₋₉, -CN, -NO₂, -N₃ or halogen. Reactive groups are protected as needed, e.g., =O would usually be protected in the LiCR reaction that is used to generate compound 1 in scheme 1 below.

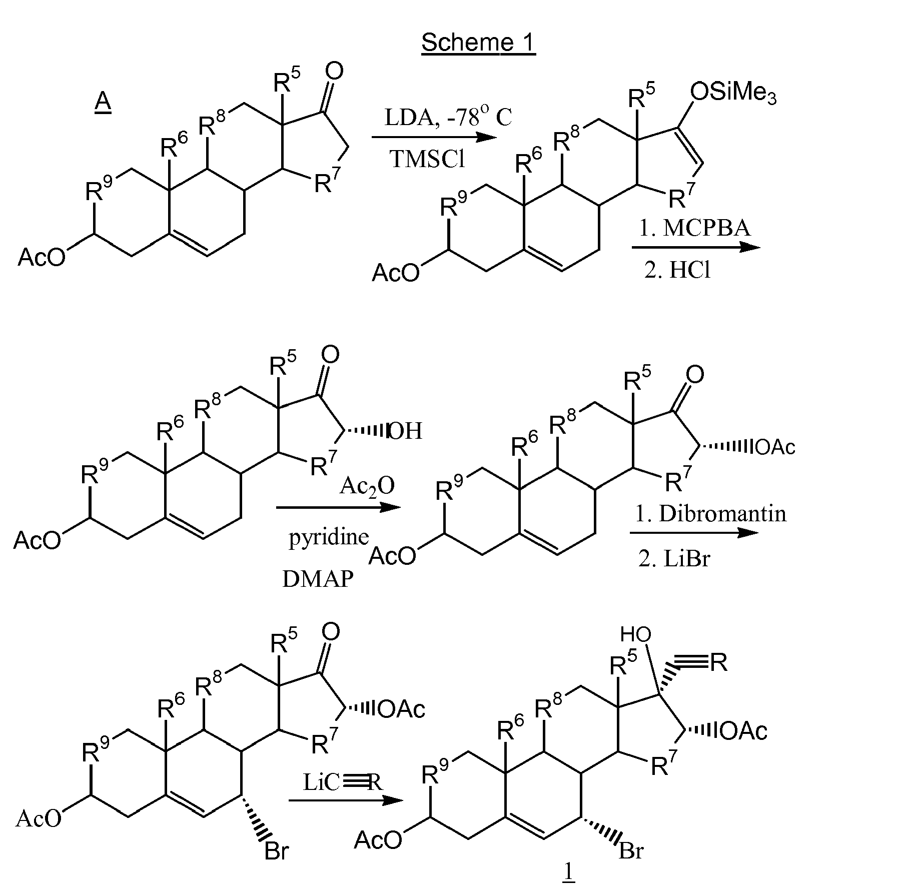

### Abbreviations:

LDA = lithium diisopropyl amide; MCPBA = m-chloroperbenzoic acid; TMSCI = trimethychlorosilane; DMAP = 4-dimethylaminopyridine; Dibromantin = 1,3-dibromo-4,4-dimethylhydantoin.
R = CR^{A}; R^{A} = -H or a C1-C50 organic moiety as described herein, e.g., -H, -C₁₋₂₀ optionally substituted alkyl, -C₁₋₂₀ optionally substituted alkenyl, -C₁₋₂₀ optionally substituted alkynyl, -(CH₂)₀₋₆-optionally substituted phenyl or -(CH₂)₀₋₆-optionally substituted heterocycle.

Scheme 2. Compounds of formula 2 are prepared from structure A compounds shown in scheme 1 using the last two steps of Scheme 1: (1a) dibromantin, (1b) LiBr, (2) Li-C≡R, where R is CR^{A} and R^{A} is as defined above, e.g., -H, -CH₃, -CH₂N₃, -CH₂NH₂, -CH₂-O-organic moiety, -CH₂-S-organic moiety, -C₁₋₁₂ optionally substituted alkyl. When R⁷, R⁸ and R⁹ are all -CH₂-, H at the 9 and 14 positions are in the α-configuration and H at the 8 position is in the β-configuration the first compound in scheme 1 is DHEA acetate. Typical substituents for the R^{A} alkyl moiety includes one, two or more independently selected -O-, optionally protected =O, optionally protected hydroxyl, -S-, optionally protected thiol, -NH-, optionally protected -NH₂, optionally protected -C(O)OH, -C(O)-NH-, -C(O)-NH₂, -NH₂-C(O)-H, -NH₂-C(O)-C₀₋₄H₁₋₉, -NH₂-C(O)-O-C₀₋₄H₁₋₉, -CN, -NO₂, -N₃ or halogen.

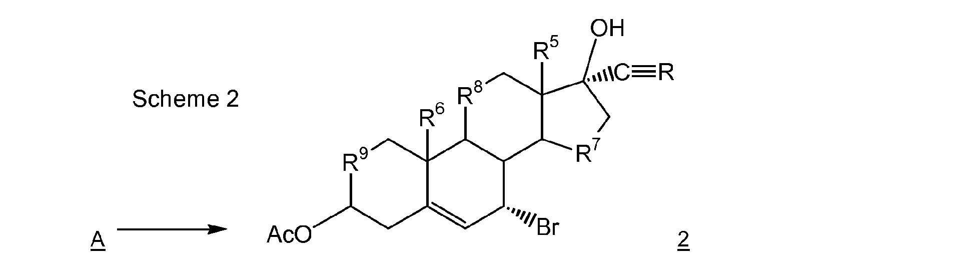

Scheme 3. The allylic bromination at C-7 is done as in Scheme 1. R and R^{A} are as defined in Schemes 1 and 2.

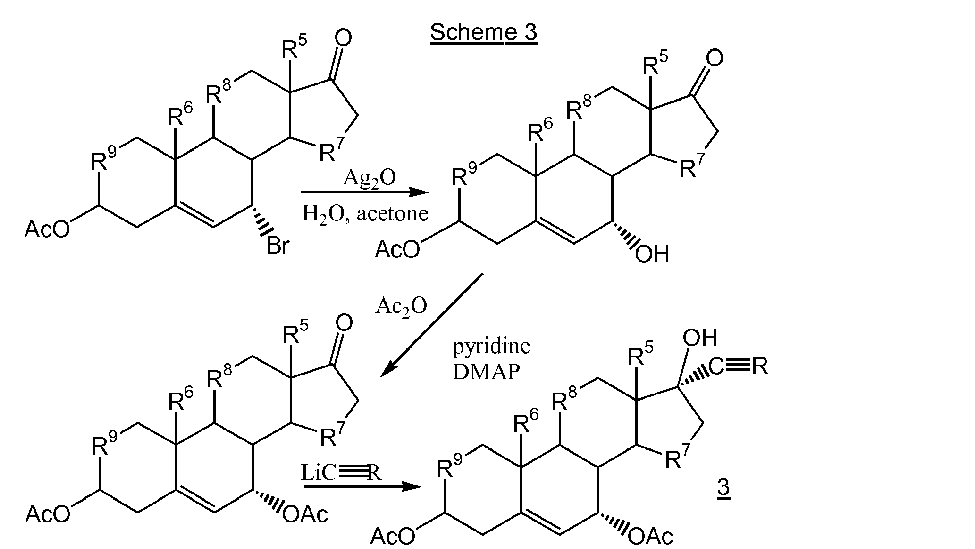

Scheme 4. The addition of lithium reagent (lithium acetylide when R is -CH) to the 17-position >C=O in the presence of the bromide at C-16 results in epoxide formation or in a pinacol rearrangement (not shown). Alternatively, compounds without the C-7 acetate of structure 3 can be dehydrated by mild acid catalysis to form compounds of formula 4 by treatment of the alkene with Br₂, H₂O. R and R^{A} are as defined in Schemes 1 and 2.

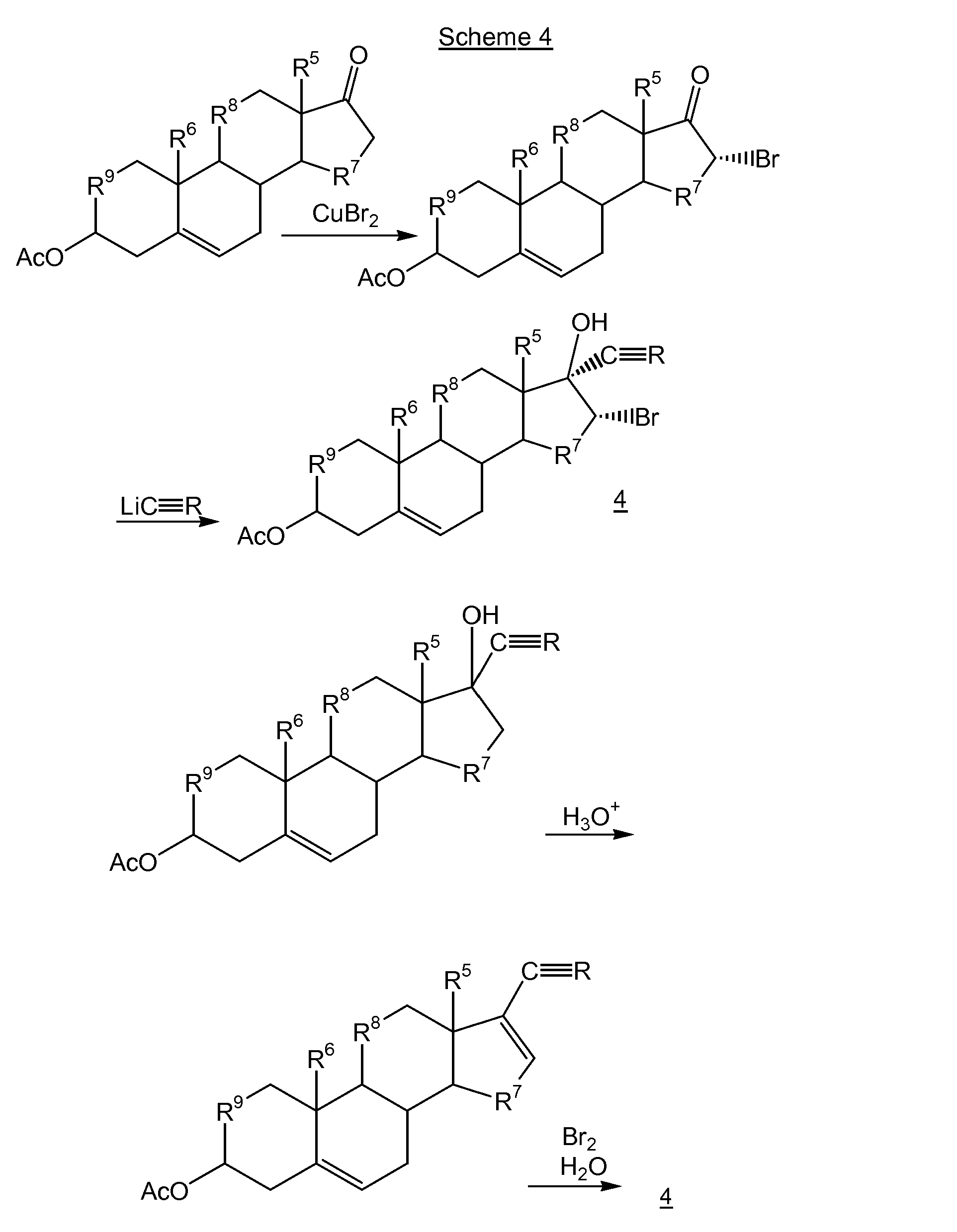

Scheme 5. Sodium borohydride gives a mixture of epimers at C-7, which may be separated by standard methods, e.g., HPLC, TLC or column chromatography. To obtain the pure 7α-OH compound, allylic bromination followed by hydrolysis is accomplished as described in Schemes 1 and 3.

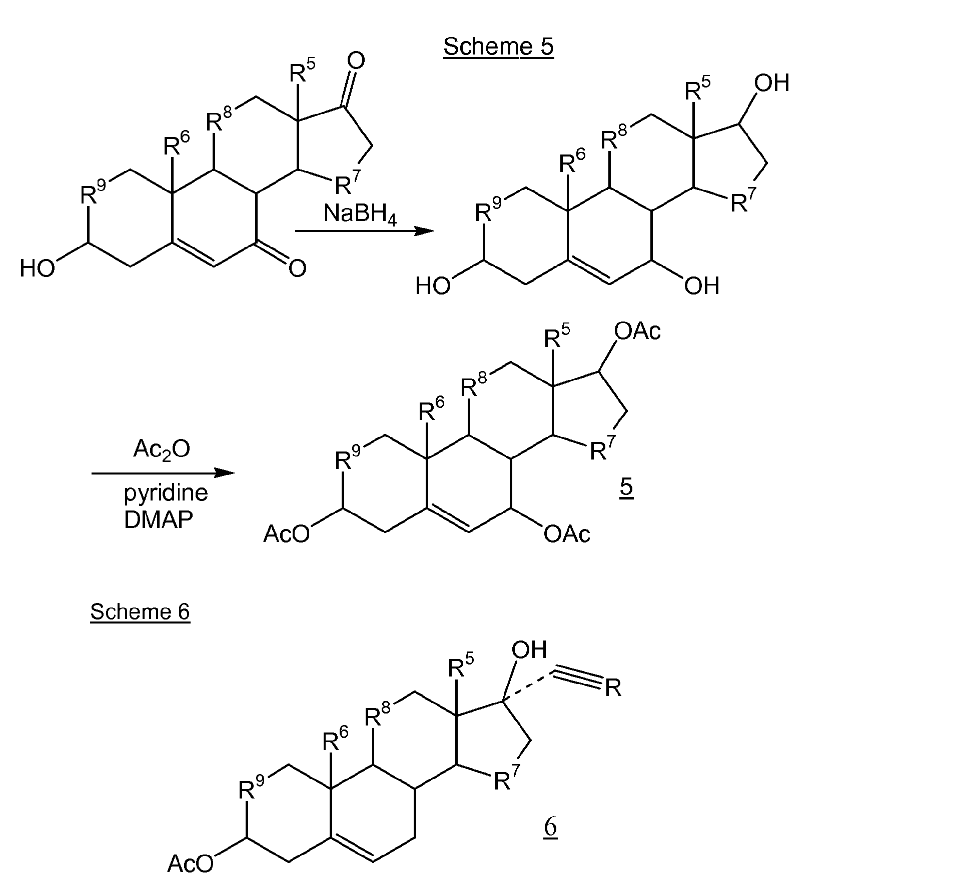

Scheme 6. Formula 6 compounds are prepared by treatment of the acetate A with lithium acetylide as in Schemes 1, 2, 3 or 4. R and R^{A} are as defined in Schemes 1 and 2.

Scheme 7. Formula 7 compounds are prepared from the 3-acetate A with reagents described in Schemes 1 and 4. R and R^{A} are as defined in Schemes 1 and 2.

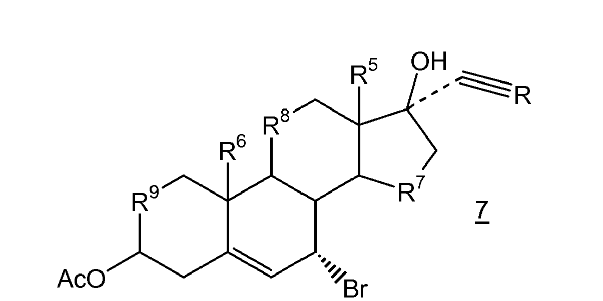

Scheme 8. Formula 8 compounds are prepared from the formula A compounds by sodium borohydride reduction at C-17 followed by acetylation.

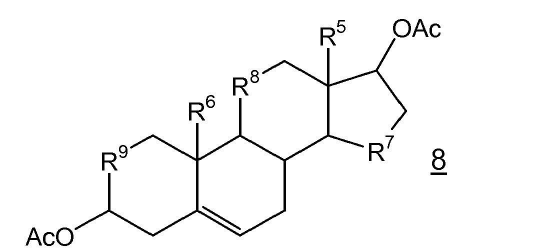

Scheme 9. The starting material is made using reactions described in Schemes 1 and 3. Rearrangement of the C-17 carbonyl to the C-16 position followed by reduction with NaBH₄ selectively gives the C-16 β hydroxy function.

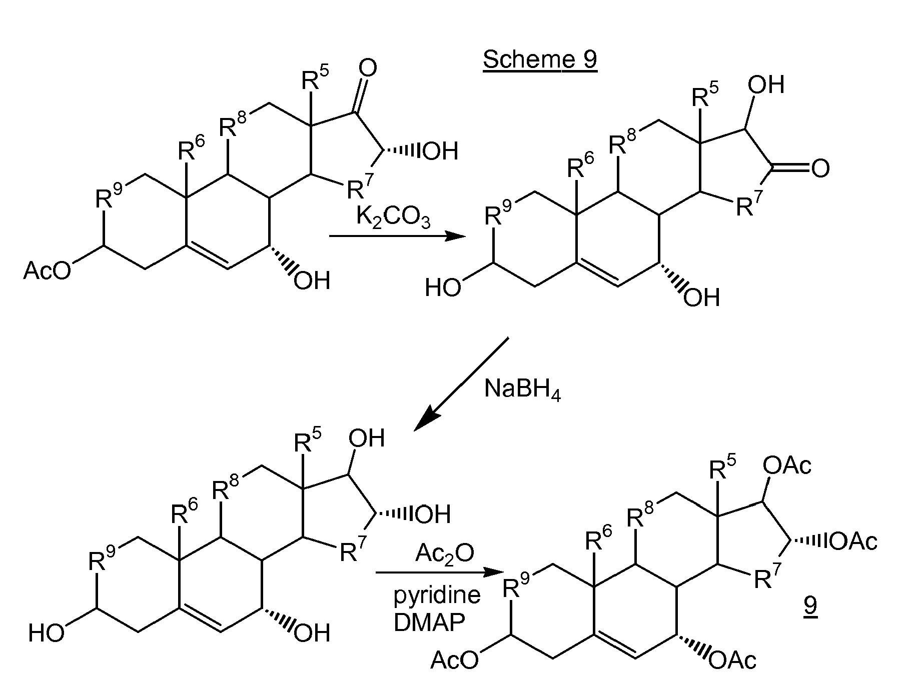

Scheme 10. Reduction and acetylation at C-3 and hydrolysis and oxidation at C-17 will allow formula 10a and 10b compounds to undergo functionalization as shown in Schemes 1-9 at C-3, C-16 and C-17. The 7-oxo acetate can be substituted for the formula A compound 3-acetate and functionalization at C-3, C-16 and C-17 is achieved similarly for 7-oxo compounds using the reactions shown in schemes 1-9.

Treatment of 10a with LDA, followed by alkylation of the enolate allows introduction of side chains such as R¹⁰ which may be, e.g., C1-C20 alkyl (methyl, ethyl), C1-C20 alkenyl (CH₂=CH-(CH₂)₀₋₆-), benzyl, -(CH₂)₁₋₄-O-(CH₂)₀₋₄-CH₃.

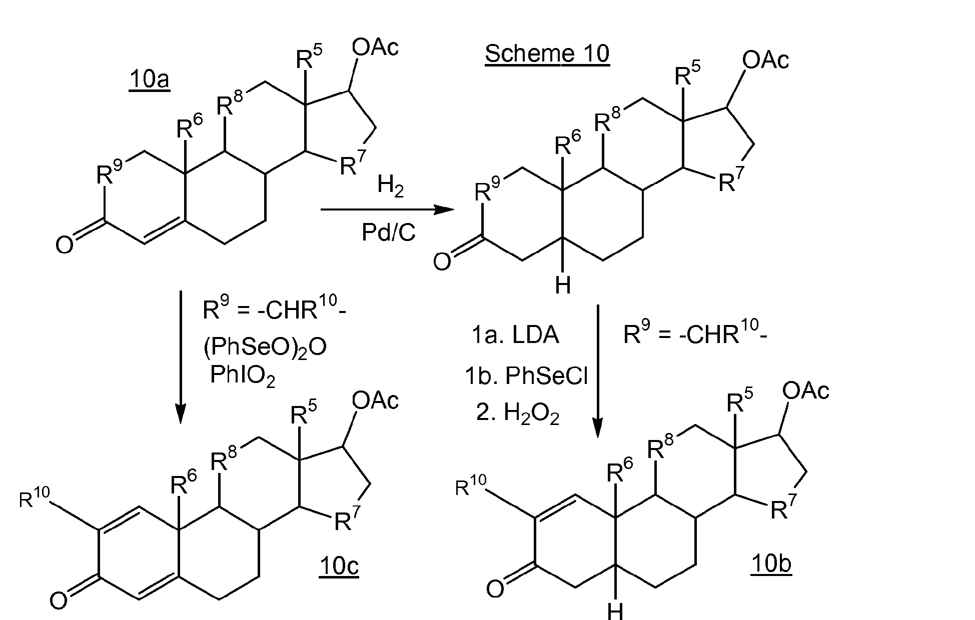

Schemes 1-9 show the introduction of the hydroxyl function at the positions shown. Methods to convert hydroxyl to other functional groups are accomplished essentially as described, e.g., in the references cited herein. For example, esters, of formula 1 - 10c compounds, such as -O-C(O)-R^{B} where R^{B} is a C₁₋₅₀ organic moiety, are prepared from the steroid alcohol by treatment with the appropriate acid anhydride or acid chloride (R^{B}-C(O)-Cl) to form any desired ester. Ethers, such as -O-R^{B}, are prepared from alcohols by formation of the alkaline metal alkoxide (Na⁺ or K⁺) followed by treatment with a primary or secondary iodide (R^{B}-I).

Oxidation of hydroxyl groups that are linked to the steroid nucleus is used to obtain ketones and related functionalities. For example, conversion of alcohols to ketones can be achieved using a variety of oxidizing agents such as CrO₃ in AcOH, or pyridinium cholorchromate, pyridinium dichromate or oxalyl chloride with triethylamine (Swern oxidation).

Substitution of hydroxyl groups is used to generate a number of functionalities. For example, thiols, -SH, are prepared from alcohols by conversion of the alcohol with inversion to the bromide using PBr₃. Treatment of the bromide with thiourea followed by NaOH gives the thiol. Thioethers, R^{B}-S-, are prepared from thiols by treatment with NaOH and the required halide, e.g., alkyl halide. Alternatively, alcohol derivatives like tosylates or mesylates can be displaced by thiolate anions, R^{B}-S-, to yield the thioether. Thioesters, R-C(O)-S-, are prepared by treating the tosylate (mesylate) of the alcohol with the sodium salt of the thioacid.

Substitution of hydroxyl groups can be used to generate both esters, R^{B}O-C(O)-, and amides, NHR^{B}-C(O)-, linked to the steroid at carbon atoms. For amides and amines, R^{B} is -H, a protecting group or a C₁₋₅₀ organic moiety. These are synthesized from the steroid bromide with inversion by displacement with NaCN. The cyanide group can be hydrolyzed to the amide or the acid. The acid is esterified or treated by standard peptide coupling reactions with an acid-protected amino acid in the presence of a suitable carboxyl activating agents such as dicyclohexylcarbodiimide (DCC) to form steroid -C(O)-NH-CHY-C(O)-OR, where Y is the side chain of an amino acid or a C1-C10 organic moiety and R is a protecting group (or hydrogen when deprotected).

Amines and derivatives of amines, e.g., R^{B}NH-, R^{B}-C(O)NH-, R^{B}OC(O)-NH- or R^{B}O-C(O)-CHR^{B}-NH- linked to steroid carbon atoms, are typically prepared by standard methods. For example, amines (NH₂-steroid) are generally prepared using the Hoffmann rearrangement (Br₂, NaOH) from the amide (NH₂-C(O)-steroid) or the Curtius rearrangement (NaN₃) from the acid chloride of the steroid. The R^{B} substituent can subsequently be introduced by alkylation. Steroid alcohols can be used as starting materials under standard Mitsunobu conditions (PPh₃, DEAD) to yield N-Boc sulfonamides using N-(t-butoxycarbonyl)-p-toluenesulfonamide. One can selectively remove either protecting group. Treatment with trifluoroacetic acid affords the sulfonamide (R^{B}-S(O)(O)-NH-steroid). Alternatively, sodium napthalenide deprotects to give the N-Boc compound. Amines (NH₂-steroid) can be converted to amides (R^{B}-C(O)-NH-steroid) using acyl chlorides (R^{B}-C(O)-Cl). Treatment with ethyl chloroformate gives the N-carbamate (R^{B}O-C(O)-NH-steroid). The amine (NH₂-steroid) can be alkylated with an α-bromoester to yield the amino acid substituted steroid (R^{B}-O-C(O)-CHY-NH-steroid).

Where reactions such as substitutions give a product mixture, the desired intermediate is optionally separated from other products or at least partially enriched (e.g., enriched at least about 10-fold, usually at least about 50-1 00-fold) from other products before subsequent reactions are conducted. Substitution at steroid carbon atoms will generally proceed with greatest efficiency at the 3-position, which is relatively sterically unhindered and C-17 is generally somewhat less accessible than the C-3 position. The relative reactivities of the C-3, C-7, C-17 and C-16 positions allows one to use their reactivities to control the sequential introduction of different functional groups into the same steroid molecule. Also, groups, such as hydroxyl at more reactive positions, C-3 or C-17, may be sequentially protected or deprotected to allow introduction of functional groups at other positions, such as C-7 or C-16.

Several methods to introduce a functional group into the 7-position are suitable. For example, formula 1 compounds that have a double bond at the 5-6 position and are unsubstituted at the 7-position are optionally protected, e.g., hydroxyl groups are protected with acetate, and a ketone is introduced into the 7-position by oxidation with chromic acid essentially as described (U.S. patent 2170124). The carbonyl (=O) at 7 is reduced to a hydroxyl using mild conditions, e.g., Al(Oi-Pr)₃, to avoid reducing the 5-6 double bond.

Selective hydrogenation of a double bond at the 16-17 position without reduction of a double bond at 5-6 is accomplished using H₂ and Pd. In general, ketones (=O) can be protected using a glycol, e.g., reaction with ethylene glycol in p-toluenesulfonic acid and benzene, before subsequent oxidation or reduction reactions are conducted.

Various groups that may comprise the formula 1 compounds described herein, e.g., hydroxyl groups or ketones bonded to the steroid nucleus, or substituted alkyl groups, substituted heterocycles, amino acids and peptides, can contain one or more reactive moieties such as hydroxyl, carboxyl, amino or thiol. Intermediates used to make formula 1 compounds may be protected as is apparent in the art. Noncyclic and cyclic protecting groups and corresponding cleavage reactions are described in "Protective Groups in Organic Chemistry", Theodora W. Greene (John Wiley & Sons, Inc., New York, 1991, ISBN 0-471-62301-6) (hereafter "Greene") and will not be detailed here. In the context of the present invention, these protecting groups are groups that can be removed from a formula 1 compound without irreversibly changing the covalent bond structure or oxidation/reduction state of the remainder of the molecule. For example, the protecting group, -R^{PR}, that is bonded to an -O- or -NH- group can be removed to form -OH or -NH₂, respectively, without affecting other covalent bonds in the molecule. At times, when desired, more than one protecting group can be removed at a time, or they can be removed sequentially. In formula 1 compounds containing more than one protecting group, the protecting groups are the same or different.

Protecting groups are removed by known procedures, although it will be understood that the protected intermediates fall within the scope of this invention. The removal of the protecting group may be arduous or straight-forward, depending upon the economics and nature of the conversions involved. In general, one will use a protecting group with exocyclic amines or with carboxyl groups during synthesis of a formula 1 compound. For most therapeutic applications amine groups should be deprotected. Protecting groups commonly are employed to protect against covalent modification of a sensitive group in reactions such as alkylation or acylation. Ordinarily, protecting groups are removed by, e.g. hydrolysis, elimination or aminolysis. Thus, simple functional considerations will suffice to guide the selection of a reversible or an irreversible protecting group at a given locus on the formula 1 compounds. Suitable protecting groups and criteria for their selection are described in T.W. Greene and P.G.M. Wuts, Eds. "Protective Groups in Organic Synthesis" 2nd edition, Wiley Press, at pps. 10-142, 143-174, 175-223, 224-276, 277-308, 309-405 and 406-454.

Determination of whether a group is a protecting group is made in the conventional manner, e.g., as described by Kocienski, Philip J.; "Protecting Groups" (Georg Thieme Verlag Stuttgart, New York, 1994) (hereafter "Kocienski"), Section 1.1, page 2, and Greene Chapter 1, pages 1-9. In particular, a group is a protecting group if when, based on mole ratio, 90% of that protecting group has been removed by a deprotection reaction, no more than 50%, typically 25%, more typically 10%, of the deprotected product molecules have undergone changes to their covalent bond structure or oxidation/reduction state other than those occasioned by the removal of the protecting group. When multiple protecting groups of the same type are present in the molecule, the mole ratios are determined when all of the groups of that type are removed. When multiple protecting groups of different types are present in the molecule, each type of protecting group is treated (and the mole ratios are determined) independently or together with others depending on whether the deprotection reaction conditions pertinent to one type are also pertinent to the other types present. In one embodiment, a group is a protecting group if when, based on mole ratio determined by conventional techniques, 90% of that protecting group has been removed by a conventional deprotection reaction, no more than 50%, typically 25%, more typically 10%, of the deprotected product molecules have undergone irreversible changes to their covalent bond structure or oxidation/reduction state other than those occasioned by the removal of the protecting group. Irreversible changes require chemical reactions (beyond those resulting from aqueous hydrolysis, acid/base neutralization or conventional separation, isolation or purification) to restore the covalent bond structure or oxidation/reduction state of the deprotected formula 1 compound.

Formulations and compositions for preparing formulations. Invention embodiments include formulations described here and elsewhere in this disclosure. While it is possible for the formula 1 compound(s) to be administered alone it is usual to present them as formulations. The formulations, both for veterinary and for human use, comprise at least one formula 1 compound, together with one or more excipients and optionally one or more additional therapeutic ingredients.

This aspect of the invention includes compositions comprising one or more pharmaceutically acceptable excipients or carriers. The compositions are used to prepare formulations suitable for human or animal use. Suitable administration routes for formulations include oral, rectal, nasal, topical (including buccal and sublingual), vaginal, rectal and parenteral (including subcutaneous, intramuscular, intravenous, intradermal, intrathecal, intraocular and epidural). In general, aqueous and non-aqueous liquid or cream formulations are delivered by a parenteral, oral or topical route. In other embodiments, such as the invention intermittent dosing methods, the formula 1 compound(s) may be present as an aqueous or a non-aqueous liquid formulation or a solid formulation suitable for administration by any of the routes disclosed herein, e.g., oral, topical, buccal, sublingual, parenteral, inhaled aerosol or a depot such as a subcutaneous depot or an intraperitoneal or intramuscular depot. It will be appreciated that the preferred route may vary with, for example, the subject's pathological condition or weight or the subject's response to therapy with a formula 1 compound or other therapy that is used or that is appropriate to the circumstances.

The formulations include those suitable for the foregoing administration routes. The formulations may conveniently be presented in unit dosage form and may be prepared by any of the methods known in the art of pharmacy. Techniques, excipients and formulations generally are found in, e.g., Remington's Pharmaceutical Sciences, Mack Publishing Co., Easton, PA 1985, 17th edition, Nema et al., PDA J. Pharm. Sci. Tech. 1997 51:166-171, G. Cole, et al., editors, Pharmaceutical Coating Technology, 1995, Taylor & Francis, ISBN 0 136628915, H.A. Lieberman, et al., editors, Pharmaceutical Dosage Forms, 1992 2nd revised edition, volumes 1 and 2, Marcel Dekker, ISBN 0824793870, J.T. Carstensen. Pharmaceutical Preformulation, 1998, pages 1-306, Technomic Publishing Co. ISBN 1566766907. Exemplary excipients for formulations include emulsifying wax, propyl gallate, citric acid, lactic acid, polysorbate 80, sodium chloride, isopropyl palmitate, glycerin, white petrolatum and other excipients disclosed herein.

Methods to make invention formulations include the step of bringing into association or contacting a formula 1 compound(s) with one or more excipient, such as one described herein or in the cited references. In general the formulations are prepared by uniformly and intimately bringing into association the formula 1 compound(s) with liquid excipients or finely divided solid excipients or both, and then, if appropriate, shaping the product.

Formulations suitable for oral administration are prepared as discrete units such as capsules, cachets or tablets each containing a predetermined amount of the formula 1 compound(s); as a powder or granules; as solution or a suspension in an aqueous liquid or a non-aqueous liquid; or as an oil-in-water liquid emulsion or a water-in-oil liquid emulsion. The formula 1 compound(s) may also be presented as a bolus, electuary or paste.

A tablet is made by compression or molding, optionally with one or more accessory ingredients. Compressed tablets may be prepared by compressing in a suitable machine the formula 1 compound(s) in a free-flowing form such as a powder or granules, optionally mixed with a binder, lubricant, inert diluent, preservative, surface active or dispersing agent. Molded tablets may be made by molding in a suitable machine a mixture of the powdered or granulated formula 1 compound and one or more excipients, which are optionally moistened, with an inert liquid diluent or excipient. The tablets may optionally be coated or scored and optionally are formulated so as to provide slow or controlled release of the formula 1 compound(s) therefrom. An exemplary tablet or caplet (a capsule shaped tablet) formulation suitable for buccal or sublingual delivery of a formula 1 compound to a subject's tissues comprises about 20, 25, 40, 50 or 60 mg of a formula 1 compound such as 16α-bromo-3β-hydroxy-5α-androstane-17-one (16α-bromoepiandrosterone or "BrEA") or BrEA hemihydrate comprising per 25 mg of the formula 1 compound about 6.2 mg povidone, about 0.62 mg magnesium stearate, about 45 mg mannitol and about 48 mg of compressible sucrose. BrEA hemihydrate has the formula (16α-bromo-3β-hydroxy-5α-androstane-17-one)₂ • H₂O and is described in WO 00/56757.

Formulations suitable for topical administration to the eye include eye drops wherein the formula 1 compound(s) is dissolved or suspended in a suitable excipient(s), including an aqueous solvent for a formula 1 compound(s) that comprise at least about 0.5, one, two or more charges at pH values near neutrality, e.g., about pH 6-8. The formula 1 compound(s) is typically present in such formulations in a concentration of about 0.5-20% w/w, about 1-10% w/w or about 2-5% w/w.

Formulations suitable for topical administration to oral mucosa include lozenges or tablets comprising the formula 1 compound(s) in a flavored basis or a monosaccharide or disaccharide such as sucrose, lactose or glucose and acacia or tragacanth; pastilles comprising the formula 1 compound(s) in an inert basis such as gelatin and glycerin, or sucrose and acacia; and mouthwashes comprising the formula 1 compound(s) in a suitable liquid excipient(s). In some embodiments, the lozenges or tablets optionally comprise the property of rapid dissolution or disintegration, e.g., disintegration within about 15 seconds to about 2 minutes, while in others, the lozenges or tablets comprise the property of slower dissolution or disintegration, e.g., disintegration within about 2 minutes to about 10 minutes or more.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the formula 1 compound(s) such excipients as are known in the art to be appropriate.

Formulations suitable for parenteral administration include aqueous and non-aqueous sterile injection solutions which may contain anti-oxidants, buffers, bacteriostats, salts (e.g., NaCl, potassium or sodium carbonate or bicarbonate or potassium or sodium phosphates) and solutes which render the formulation isotonic with the blood of the intended subject; and aqueous and non-aqueous sterile suspensions which may include suspending agents or thickening agents. In general, the formula 1 compound that is present in liquid compositions or formulations is completely dissolved in aqueous or non-aqueous excipients. However, in some embodiments, e.g., transient compositions or some formulations, the formula 1 compound is partially dissolved while the remaining portion is present as a solid, which can be a suspension or a colloid.

Exemplary formulations suitable for parenteral delivery of formula 1 compounds to subjects such as humans or animals typically comprise one, two, three or more excipients. Exemplary embodiments include (1) any two, three or four of propylene glycol, PEG200, PEG300, ethanol and benzyl benzoate and (2) any two, three or four of propylene glycol, PEG100, PEG200, PEG300, PEG400 and benzyl benzoate.

Exemplary compositions and formulations generally comprise about 0.01-10% of a formula 1 compound, usually about 1-5%, and about 0.01-3% water, typically about 0.05-3%, usually about 0.1-1%. The formulations include unit or multi-unit dosages suitable for parenteral administration once or twice per day or once per 2-3 days. Unit dosages comprise about 3-1000 mg of formula 1 compound per unit dose, typically about 5-500 mg, usually about 10-200 mg. For treating retroviruses such as HIV in humans, a unit dose usually comprises about 10-250 mg of BrEA hemihydrate, usually about 100-200 mg, in a volume of about 1-6 mL, usually about 2-4 mL.

Formulations, or compositions disclosed herein for use to make formulations suitable for administration by the routes disclosed herein optionally comprise an average particle size in the range of about 0.01 to about 500 microns, about 0.1 to about 100 microns or about 0.5 to about 75 microns. Average particle sizes include a range between 0.01 and 500 microns in 0.05 micron or in 0.1 micron or other increments, e.g., an average particle size of about 0.05, 0.1, 0.5, 1, 1.5, 2.0, 2.5, 3.0, 3.5, 4.0, 4.5, 5.0, 5.5, 6, 7, 8, 9, 10, 15, 20, 25, 30, 35, 40, 50, 60, 75, 85, 100, 120, etc. microns). When formula 1 compounds or compositions that comprise a formula 1 compound are used as intermediates to make a formulation, they may comprise one, two, three or more of these average particle sizes, or size ranges.

In preparing any of the compositions or formulations that are disclosed herein and that comprise a formula 1 compound (and optionally one or more excipients), one may optionally mill, sieve or otherwise granulate the compound or composition to obtain a desired particle size, e.g., as described above.

Milling may occur before or after the formula 1 compound is contacted with one or more excipients. For example, one may mill a formula 1 compound such as 16α-bromoepiandrosterone hemihydrate, to obtain an average particle size (or diameter) of about 0.05-50 µM or about 0.5-10 µM (e.g., about 0.04, 0.1, 0.5, 1, 1.5, 2, 2.5, 5, 10, 15, 20, 40, 60, 80, 100 or 120 µM average particle size or diameter) before contacting the milled formula 1 compound with a liquid or solid excipient. In some cases the formula 1 compound is milled or sieved to obtain an average particle size of about 5 µm or about 10 µm before it is contacted with a solid or liquid excipient(s) to obtain a solution or suspension or a powder suitable for making a tablet, capsule or other dosage form as described herein or in the cited references.

As used herein, reference to an average particle size or an average particle diameter means that the material, e.g., a formula 1 compound(s), an excipient(s) or a composition that comprises both, is ground, milled, sieved or otherwise treated so as to comprise the specified average size. It is to be understood that some particles may be larger or smaller, but the composition or the formula 1 compound(s) will comprise a significant proportion of the material with the specified size or within an acceptable range of the specified size. Micronization methods include milling by ball mills, pin mills, jet mills (e.g., fluid energy jet mills) and grinding, sieving and precipitation of a compound(s) from a solution, see, e.g., U.S. patents 4919341, 5202129, 5271944, 5424077 and 5455049. Average particle size is determined by, e.g., transmission electron microscopy, scanning electron microscopy, light microscopy, X-ray diffractometry and light scattering methods or Coulter counter analysis.

Thus, the formula 1 compounds may comprise a powder that consists of one, two or more of these average particle sizes and the powder may be contacted with a solid excipient(s), suitably mixed and optionally compressed or formed into a desired shape. Alternatively, such a formula 1 compound(s) is contacted with a liquid excipient(s) to prepare a liquid formulation or a liquid composition that is incorporated into a solid formulation. Suitable micronized formulations thus include aqueous or oily solutions or suspensions of the formula 1 compound(s).

Formulations suitable for aerosol administration typically will comprise a fine powder, e.g., having an average particle size of about 0.1 to about 20 microns or any one, two or more of the average particle sizes within this range that are described above. The powder is typically delivered by rapid inhalation through the nasal passage or by inhalation through the mouth so as to reach the bronchioles or alveolar sacs of the lungs.

Formulations suitable for aerosol, dry powder or tablet administration may be prepared according to conventional methods and may be delivered with other therapeutic agents such as compounds heretofore used in the treatment or prophylaxis of viral or other infections as described herein. Such formulations may be administered, e.g., orally, parenterally (e.g., intravenous, intramuscular, subcutaneous, intradermal, intrathecal), topically, sublingually or by a buccal or sublingual route.

Micronized formula 1 compound is useful, e.g., to facilitate mixing, dissolution or uniform suspension of the formula 1 compound in one or more liquid or solid excipients, e.g., a PEG such as PEG 300 or PEG 400, or propylene glycol or benzyl benzoate, a complexing agent, such as a cyclodextrin (e.g., an α-, β- or γ-cyclodextrin such as hydroxypropyl-β-cyclodextrin). Micronized formula 1 compound is also useful to facilitate uniformly distributing drug substance when the micronized compound is contacted with one or more solid excipients (e.g., a filler, a binder, a disintegrant, complexing agent (e.g., a cyclodextrin such as hydroxypropyl-β-cyclodextrin), a preservative, a buffer or a lubricant).

The formulations are presented in unit-dose or multi-dose containers, for example sealed ampules and vials, and may be stored in a freeze-dried (lyophilized) condition requiring only the addition of the sterile liquid excipient, for example water for injection, immediately prior to use. Extemporaneous injection solutions and suspensions are prepared from sterile powders, granules and tablets as described above. Unit dosage formulations are those containing a daily dose or unit daily sub-dose, as recited herein, or an appropriate fraction thereof, of the formula 1 compound(s).

It should be understood that in addition to the ingredients particularly mentioned above the formulations of this invention may include other agents or excipients conventional in the art having regard to the type of formulation in question, for example those suitable for oral administration may include flavoring agents.

Invention formulations include controlled release pharmaceutical formulations containing a formula 1 compound(s) ("controlled release formulations", "slow release formulations" or the like) in which the release of the formula 1 compound(s) is controlled or regulated to allow less frequency dosing or to improve the pharmacokinetic or toxicity profile of a given formula 1 compound(s). Polymers and other materials that are suitable to prepare controlled release formulations that comprise a formula 1 compound have been described, e.g., U.S. patents 4652443, 4800085, 4808416, 5013727, 5188840.

An effective dose of formula 1 compound(s) depends in part on one or more of the nature of the condition being treated, toxicity, whether the formula 1 compound(s) is being used prophylactically (generally lower doses) or against an active infection or condition, the method of delivery, and the formulation. For use in humans, the effective dosage will typically be determined by the clinician using conventional dose escalation studies. In other subjects, the dose is obtained by similar dose escalation analyses. For humans, an effective dose can be expected to be from about 0.05 to about 50 mg/kg body weight per day, e.g., about 2 mg/kg/day, about 4 mg/kg/day, about 6 mg/kg/day, or about 8 mg/kg/day. For example, for topical delivery the daily candidate dose for an adult human of approximately 70 kg body weight will range from about 1 mg to about 500 mg, generally between about 5 mg and about 40 mg, and may take the form of single or multiple doses or administration sites. For non-human subjects, e.g., mammals such as rodents or primates, the effective daily dosage may comprise about 0.05 mg/kg/day to about 300 mg/kg/day, including about 1 mg/kg/day to about 100 mg/kg/day. As used herein, an "effective dosage" or an "effective amount" of a formula 1 compound(s) is one that is sufficient to result in, e.g., a detectable change in a symptom or an immune parameter such as one described herein. An effective dosage (or daily dosage) may be administered to a subject over a period of time, e.g., at least about 1-14 days before a symptom change or an immune parameter detectably changes. Effective dosages may include any of the dosages as described herein.

Embodiments include formulations that comprise a liposome or lipid complex that comprises a formula 1 compound(s). Such formulations are prepared according to known methods, e.g., U.S. patents 4427649, 5043165, 5714163, 5744158, 5783211, 5795589, 5795987, 5798348, 5811118, 5820848, 5834016 and 5882678. The liposomes optionally contain an additional therapeutic agent(s), e.g., amphotericin B, cis-platin, adriamycin, a protease inhibitor, a nucleoside or a nucleotide analog, such as one of those mentioned herein. Formulations that comprise liposomes can be delivered to a subject by any standard route, e.g., oral, aerosol or parenteral (e.g., s.c., i.v. or i.m.).

Liposome formulations can be used to enhance delivery of the formula 1 compound(s) to certain cell types such as tumor cells (see e.g., U.S. patent 5714163) or to cells of the reticuloendothelial system ("RES"). The RES includes macrophages, mononuclear phagocytic cells, Kupfer cells, cells lining the sinusoids of the spleen, lymph nodes, and bone marrow, and the fibroblastic reticular cells of hematopoietic tissues. In general, RES cells are phagocytic and they are targets for targeted delivery of a formula 1 compound(s) *in vitro* or *in vivo* using liposomes, or other compositions or formulations. Thus, one can deliver formula 1 compound to a neoplasm that is derived from reticuloendothelial tissue (reticuloendothelioma). The liposomes may optionally comprise a peptide from an infectious agent such as a malaria parasite, a virus or a tumor associated antigen. The peptides may facilitate the generation of a MHC class II and B cell response.

Invention embodiments include the product made by a process of combining, mixing or otherwise contacting a formula 1 compound and one, two, three or more excipients. Such products are produced by routine methods of contacting the ingredients. Such products optionally contain a diluent, a disintegrant, a lubricant, a binder, or other excipients described herein or in references cited herein.

Immune modulation. As noted elsewhere, the formula 1 compounds, or the biologically active substances produced from these compounds by hydrolysis or metabolism *in vivo*, have a number of clinical and non-clinical applications. The compounds are generally useful to correct immune dysregulation, e.g., imbalanced immune responses to disease conditions, pathogens or the like, suppression of an innate or acquired immune response(s) and inflammation conditions in vertebrate or mammalian subjects, e.g., as disclosed herein. Thus, while the compounds will generally enhance a deficient immune response in a given clinical condition, they will generally reduce the same immune response when it is too active in a different clinical condition. For example, they can enhance insufficient or suboptimal Th1 immune responses, reduce excess or undesirable Th2 immune responses, reduce excess or undesirable Th1 immune responses or enhance insufficient or suboptimal Th2 immune responses or they can reduce excess or undesirable inflammation or one or more of its symptoms. The compounds will generally also modulate dysregulated Tc1 and Tc2 immune responses (associated with CD8⁺ T cells) in a similar manner, e.g., excessive Tc1 or Tc2 responses will be detectably decreased and deficient or suboptimal Tc1 or Tc2 responses will generally be detectably enhanced.

In modulating one or more activities of Th1, Th2, Tc1 or Tc2 cells or their function(s), the formula 1 compounds will typically detectably modulate one, two, three or more factors, e.g., immune cell subsets or populations, cytokines, interleukins, surface antigens such as a CD molecule(s) and/or their receptors that affect the development, migration, numbers or biological function(s) of such cells. When a Th1 or Tc1 cell or population is affected, the formula 1 compounds will typically increase or decrease the synthesis or level of one, two or more of an associated effector factor, e.g., IFNγ, IL-2, IL-12, IL-18, T-bet, PPARα and PPARγ or a cell surface molecule, e.g., as disclosed herein or in the cited references, that is associated with or needed for normal, optimal or enhanced Th1 or Tc1 cells or cell function. Such molecules are generally associated with development or enhancement of Th1 or Tc1 cells or their biological function(s). When a Th2 or Tc2 cell or population is affected, the formula 1 compounds will typically increase or decrease the synthesis or level of one, two or more of an associated effector factor, e.g., IL-4, IL-5, IL-6, IL-8, IL-10, IL-13, GATA-3, COX-2 or a cell surface molecule, e.g., as disclosed herein or in the cited references, that is associated with or needed for normal, optimal or enhanced Th2 or Tc2 cells or cell function(s). Such molecules are generally associated with development or enhancement of Th2 or Tc2 cells or their biological function(s).

Similarly, when a subject has or is subject to developing an unwanted or excessive inflammation, the formula 1 compounds will generally detectably modulate one or more relevant effector factors for inflammation, e.g., a decrease of one, two, three or more of IL-1α, IL-1β, TNFα, MIP-1α, γIFN, IL-6, IL-8, IL-10 and COX-2, or an increase of one or more suppressor factors or antagonists of inflammation. Such modulation can comprise increases or decreases of at least about 2%, 5%, 10%, 15%, 20%, 25%, 30%, 40%, 50%, 60%, 70%, 75%, 80%, 85%, 90%, 95%, 98%, 100%, 200%, 300%, 500%, 1000%, 5000% or within a range between any two of these values, e.g., between about 5-95%, about 10-90%, about 5-60% or about 40-95%. In general, such changes leads to a detectable amelioration of an inflammation-associated disease, condition, symptom or to the detectable slowing of the progression thereof or to a detectably reduced incidence or severity of or susceptibility to developing an unwanted inflammatory response.

In conditions where an unwanted or excessive Th1, Tc1, Th2 or Tc2 response is associated with or causes a disease(s), disease(s) progression, disease(s) state maintenance, condition(s) or symptom(s), the formula 1 compounds will generally decrease the level or one or more biological activity of one, two or more of their respective associated effector molecules. In conditions where a deficient or suboptimal Th1, Tc1, Th2 or Tc2 response is associated with or causes a disease(s), disease(s) progression, disease(s) state maintenance, condition(s) or symptom(s), the formula 1 compounds will generally increase the level or one or more biological activity of one, two, three or more of their respective associated effector molecules. Such changes in the level or biological activity(ies) the associated effector molecules is generally detectable using standard methods and is typically an increase (when a response is insufficient) or a decrease (when a response is in excess) of at least about 2%, 5%, 10%, 15%, 20%, 25%, 30%, 40%, 50%, 60%, 70%, 75%, 80%, 85%, 90%, 95%, 98% or within a range between any two of these values, e.g., between about 5-95%, about 10-90%, about 5-60% or about 40-95%. In general, such changes leads to a detectable amelioration of a disease, condition, symptom or to the detectable slowing of the progression thereof or to a detectably reduced incidence or severity of or susceptibility to developing a disease(s) or the occurrence of a symptom(s) for a at least a portion of subjects that are treated with a formula 1 compound, e.g., at least about 5%, 10%, 20%, 40%, 60% or 80% of treated subjects. The formula 1 compounds may facilitate the clinical cure of a disease(s), prolong remission of a disease(s) or eliminate or ameliorate a clinically detectable symptom(s).

The formula 1 compound will generally also affect the function of other immune cell subsets in a similar manner. Thus, when an insufficient macrophage, dendritic cell or neutrophil response is associated with the establishment, maintenance or progression of a disease, symptom or a condition, the formula 1 compounds will generally enhance of the level or a biological activity(ies) of one or more effector molecule associated with or needed for an optimal or more normal response or immune function that is mediated by the macrophages, dendritic cells or neutrophils. Similarly, when the subject suffers from a excessive or pathological activity associated with macrophages, dendritic cells or neutrophils, which is associated with the establishment, maintenance or progression of a disease, symptom or a condition, the formula 1 compounds will generally detectably reduce the level or a biological activity(ies) of one or more effector molecule associated with or needed for an optimal or more normal response or immune function that is mediated by the macrophages, dendritic cells or neutrophils. Such effector molecules are as described herein or in the cited references.

As used herein, reference to Th1 or Th2 immune responses means such responses as observed in mammals generally and not as observed in the murine system, from which the Th1 and Th2 terminology originated. Thus, in humans, Th1 cells are CD4⁺ T lymphocytes and they usually preferentially display chemokine receptors CXCR3 and CCR5, while Th2 cells are CD4⁺ T lymphocytes and usually preferentially express the CCR4, CCR8 and/or CXCR4 chemokine receptor molecule(s) and generally a smaller amount of CCR3, see, e.g., U. Syrbe et al., Springer Semin. Immunopathol. 1999 21:263-285, S. Sebastiani et al., J. Immunol. 2001 166:996-1002. Tc1 and Tc2 immune responses are mediated by CD8⁺ lymphocytes and means to identify these cells and their associated lymphokines, cell specific antigens and biological activities have been described, see, e.g., M.B. Faries et al., Blood 2001 98:2489-2497, W.L. Chan et al., J. Immunol. 2001 167:1238-1244, C. Prezzi et al., Eur. J. Immunol. 2001 31:894-906, H. Ochi et al., J. Neuroimmunol. 2001 119:297-305, D.H. Fowler and R.E. Gress, Leukemia and Lymphoma 2000 38:221-234.

The formula 1 compounds are useful in reestablishing normal immune function in various immune dysregulation or immune suppression conditions. For example, they are useful to treat, slow progression of or to ameliorate one or more symptoms associated with one or more of an autoimmune condition(s), a inflammation condition(s), an infection(s), a cancer(s), a precancer(s), a chemotherapy(ies), radiation therapy, a burn(s), a trauma(s), a surgery(ies), a pulmonary condition, a cardiovascular disease(s) and a neurological or neurodegenerative disease(s). Without being limited to any theory, the formula 1 compounds are believed to act through several mechanisms, including by directly or indirectly modulating steroid receptor activity or by affecting or modulating other biological targets such as transcription factors, steroid binding proteins or enzymes in at least some of the diseases, conditions or symptoms disclosed herein.

The formula 1 compounds are useful to modulate delayed-type hypersensitivity ("DTH") responses and anergic conditions in subjects having to subject to developing abnormal DHT responses or anergy. Means to measure such responses and conditions are known and can be used to characterize the effects of the formula 1 compounds on these responses and conditions. See, e.g., A.E. Brown, et al., J. Med. Assoc. Thailand 83:633-639 2000, R.A. Smith et al., J. Adolesc. Health 27:384-390 2000, N.M. Ampel, Med. Mycology 37:245-250 1999. The compounds will generally detectably enhance or restore DTH in immune suppression conditions. They will also generally detectably reduce or eliminate anergy in subjects having significantly reduced or no immune response to, e.g., specific antigens or pathogens.

Clinical indications that have an association with or have a symptom(s) that is consistent or associated with an excessive or unwanted Th2 immune response include, e.g., fatigue, pain, fever or an increased incidence of infection, schizophrenia, acute myelitis, tumor progression, progressive systemic sclerosis, Omenn's syndrome, atopic disease, atopy, allergen hypersensitivity, atopic asthma, atopic dermatitis, burns, trauma (e.g., bone fracture, hemorrhage, surgery), immune responses to xenotransplantation, chronic periodontitis, SLE (systemic lupus erythematosus), discoid lupus erythematosus, osteoporosis, myasthenia gravis, Graves disease, mite-associated ulcerative dermatitis, rheumatoid arthritis and osteoarthritis. Excessive Th2 immune responses are also associated with an unwanted symptom or pathology, e.g., fatigue, pain, fever or an increased incidence of infection, that is associated with aging, allergy and inflammation conditions such as allergic bronchopulmonary aspergillosis in cystic fibrosis patients, allergic respiratory disease, allergic rhinitis, atopic dermatitis, subepithelial fibrosis in airway hyperresponsiveness, chronic sinusitis, perennial allergic rhinitis, fibrosing alveolitis (lung fibrosis). This common underlying immune component is at least part of the pathology or symptoms of all of these conditions. This allows a formula 1 compound to be effectively used to prevent or treat the condition or to treat or ameliorate one or more symptoms that are associated with these conditions. Thus, in some embodiments, an unwanted or excessive Th2 response is present and amelioration of one or more symptoms associated with this condition is accomplished by administering an effective amount of a formula 1 compound according to the methods described herein, e.g., formula 1 compound is administered using a formulation and a route of administration essentially as described herein on an intermittent or a daily basis.

Typically, unwanted Th2 immune responses are associated with, or caused by, increased expression of one or more cytokines or interleukins such as one, two, three or more of cortisol, IL-4, IL-5, IL-6, IL-10 and IL-13. Administration of a formula 1 compound will generally reduce the expression of one or more of the Th2-associated cytokines or interleukins. At the same time, the compounds generally enhance the expression of one or more cytokines or interleukins associated with Th1 immune responses. Because of their capacity to modulate or to balance Th1 and Th2 immune responses, the compounds are useful for a variety of clinical conditions, e.g., infection, immunosuppression or cancer, where an enhanced Th1 immune response is desired. Effects of the formula 1 compounds in treating, preventing or slowing the progression of the clinical conditions described herein can include one or more of (1) enhancing the Th1 character of a subject's immune response or immune status, (2) increasing the intensity of a Th1 or a Th2 immune response or both and (3) decreasing inflammation or a symptom thereof.

Exemplary conditions where an immune imbalance or an excessive Th1 immune response is involved include autoimmune diseases such as multiple sclerosis, Crohn's disease (regional enteritis), ulcerative colitis, inflammatory bowel disease, rheumatoid arthritis, reactive arthritis, acute allograft rejection, sarcoidosis, type 1 diabetes mellitus, *Helicobacter pylori* associated peptic ulcer, graft versus host disease and Hashimotos' thyroiditis. Because these conditions are associated with a similar type immune dysfunction, a formula 1 compound can be effectively used to prevent or treat these conditions or to treat or ameliorate one or more symptoms associated therewith. Thus, in some embodiments, an unwanted or excessive Th1 response is present and amelioration of one or more symptoms associated with this condition is accomplished by administering an effective amount of a formula 1 compound according to the methods described herein, e.g., formula 1 compound is administered using a formulation and a route of administration essentially as described herein on an intermittent or a daily basis. In other embodiments, an deficient Th1 response is enhanced, which is optionally observed as a detectable increase in one or more of IFNγ, IL-2, IL-12 or IL-18 in Th1 cells or in accessory cells such as a dendritic cell or macrophage. In all of the conditions where an insufficient or excess Th1, Th2, Tc1 or Tc2 response is present, amelioration of one or more symptoms associated with the condition is accomplished by administering an effective amount of a formula 1 compound according to the methods described herein.

Expression of one or more homing or other receptors or receptor subunits such as CD62L, CLA-1, LFA1, CD44, ICAM, VCAM or ECAM may also be detectably affected after administration of the formula 1 compounds to a subject. The numbers of cells that express these molecules, or the relative amounts per cell of, e.g., CD44 or CD62L, may be increased where a desired immune response is desired, e.g., migration of T cells to mucosal tissues or exposure of naïve T cells to antigen in lymph nodes. Alternatively, numbers of cells that express these molecules, or the relative amounts per cell of, e.g., CLA-1, may be decreased where inhibition of an undesired immune response, such as an inflammatory response is desired. The subject's response to such enhanced expression includes migration of cells such as movement of naïve T cells to peripheral lymph nodes in response to modulation of CD62L or other homing receptor expression. Thus, the formula 1 compounds can also facilitate migration of various immune cell types, e.g., dendritic cells, NK cells, LAK cells, macrophages or lymphocytes, from one location to another within a subject. For example, the compounds can enhance dendritic cell or lymphocyte migration from areas such as the skin tissues to the gut associated lymphoid tissue ("GALT"), lymph nodes or spleen. Such migration may facilitate the function of those cell types by increasing their transit to tissues where their effector functions, e.g., antigen presentation by dendritic cells, normally occur. The migration period is often relatively transient (e.g., observable over about 1-7 days) or occasionally longer (e.g., occurring for about 8-40 days), depending on the dosing regimen and other factors. This migration can be observed by standard methods, e.g., by cell staining, by PCR analyses or by determining the presence of a given cell type in circulation or determining a decrease in the number circulating cells. A decrease would generally reflect sequestration of an immune cell population(s) in a tissue(s) where the immune cell normally exercises its effector functions.

Thus, in some embodiments, the migration of one or more immune cell subsets such as CD11C⁺ cells from tissue such as skin or lung through the blood to immune tissue such as lymph nodes or GALT is seen as a transient increase in the level of circulating CD11C⁺ cells in response to exposure of the subject's tissues to a suitable amount of a formula 1 compound. Thus, the level of CD11C⁺ cells in the blood will generally detectably increase, e.g., a statistically significant increase, plateau and then decrease as migration of the cells to immune tissue subsides. In these embodiments, the proportion of the cells of the affected immune cell subset is typically relatively low in most physiological immune states, e.g., normal or abnormal immune conditions, compared to the total white blood cell population in circulation. In other embodiments, the migration of one or more immune cell subsets such as CD123⁺ cells from the circulation to immune tissue such as lymph nodes or GALT results in a decrease. In these embodiments, the decrease in the numbers of circulating immune cells reflects the migration of the immune cells from the blood to immune tissue such as lymph nodes or GALT. Such a decrease may be transient and followed by recovery of the affected immune cell subset(s) over about 2 to 24 weeks. In conducting these embodiments, administration of the formula 1 compound to the subject is accomplished using the formulations or the methods as described herein.

Thus, an aspect of the invention is a method to enhance the migration of one or more immune cell types in a subject from one location (e.g., bone marrow, circulating blood or a tissue such as the skin, liver, central nervous system or lung) to another (e.g., to the blood or to a lymphoid tissue such as a lymph node, spleen or a mucosal tissue such as GALT) by administration to a subject as described herein of an effective amount of a formula 1 compound essentially as described by any of the methods disclosed herein. A related aspect is the monitoring, e.g., by suitable blood counts or tissue biopsy, of the subject's response to determine the timing and extent of such immune cell migration.

Other CD molecules that are modulated by the presence of the formula 1 compounds in a subject include cytokine receptor molecules such as one or more of CD115, CDW116, CD117, CD118, CDW119, CD120a, CD120b, CD121a, CD121b, CD122, CD123, CD124, CD125 CD126, CDW127, CDW128 or CDW130. Often, the numbers of receptor molecules per cell will be modulated. For example, receptors for cytokines that mediate or facilitate Th1 immune responses or innate immune responses (e.g., one or more of IL-1α, IL-1β, IL-2, IL-4, IL-12, γIFN or α-interferon) will typically increase in or on cells that mediate Th1 or innate immune responses. Modulation of these molecules may be by direct interactions with a receptor(s) in the cell that expresses the cytokine receptor or indirectly by modulation of cytokine synthesis in the affected cells or in other cells, typically immune cells that may interact with the cells whose receptor synthesis is being modulated. Thus, autocrine or paracrine mechanisms may underlie some of the effects associated with administration of a formula 1 compound(s) such as altered cytokine profiles in immune cells or altered immune cell populations. Endocrine cytokine mechanisms may also contribute to desired immune responses.

Treatment of a subject with a formula 1 compound can result in a change of at least about 20-80% or about 25-50% above or below (e.g., at least 30% or at least 40% above or below) the control or basal level of affected immune cell subsets. For example, increases of more than about 30% in the total numbers of activated CD8⁺ T cells, e.g., CD8⁺, CD69⁺, CD25⁺ T cells, CD8⁺, CD69⁺, CD25- T cells or CD8⁺, CD69⁻, CD25⁺ T cells, can occur by 7 days after a single dose of a formula 1 compound to a subject. Such increases may be greater than 50%, 60% or 100% in the total numbers of activated CD8⁺ T cells or subsets of activated CD8⁺ T cells in individual subjects. Typically such increases are about in the total numbers of activated CD8⁺ T cells or subsets of activated CD8⁺ T cells averages about 30-40%, with individual subjects experiencing increases over 100% in the numbers of activated CD8⁺ T cells per unit blood volume compared to the basal level.

Administration of the formula 1 compounds can affect other immune cell subsets. For example, the concentration of circulating CD4⁺, CD69⁺, CD25- (Th1 helper cells) and CD8⁺, CD16⁺, CD38⁺ LAK cells or CD8⁻, CD16⁺, CD38⁺ LAK cells typically increases during or after the course of dosing a subject with a formula 1 compound. Also, CD8⁻, CD16⁺, CD38⁺ and CD8⁺, CD16⁺, CD38⁺ (ADCC effector cells) and low side scatter Lin⁻, DR⁺, CD123⁺ (dendritic precursors) or low side scatter Lin⁻, DR⁺, CD11c⁺ (dendritic cells or precursors) may show modest to significant increases.

The formula 1 compounds can thus be used to shift the nature of a subject's immune response to result in a more balanced immune. Alternatively, the compounds can selectively suppress inappropriate or unwanted immune responses. Enhanced Th1 responses appears to be at least partly due to one or more of (i) a reduction in biological restraints, e.g., high levels of IL-4 or IL-10, on Th1 functions by preexisting primed Th1 effector cells, (ii) enhanced differentiation of Th0 cells to Th1 cells or enhanced responses mediated by Th1 cells, (iii) enhanced function of accessory cell function, e.g., antigen presentation by dendritic cells, dendritic precursor or progenitor cells or by macrophages or their precursors or progenitors, (iv) enhanced proliferation and differentiation of Th1 precursor or progenitor cells, (v) enhanced IL-12 expression in dendritic cells or their precursors, which results in enhanced differentiation of Th1 cells from Th0 precursors, (vi) enhanced expression or activity of factors associated with Th1 functions, e.g., IL-2, gamma interferon (γIFN or IFNγ), IL-18 or lymphotoxin.

An aspect of the invention methods is an alteration in the expression of IL-4 or IL-10 that occurs after administration of a formula 1 compound to a subject. A consistent observation is that extracellular IL-4 or IL-10 levels rapidly decrease to levels that are undetectable by ELISA. Intracellular IL-10 levels are reduced to levels that are near or below the limits of detection by flow cytometry. The administration of a formula 1 compound to a subject thus provides a means to inhibit either or both of these interleukins. Such inhibition may be associated with enhancement of Th1 immune responses relative to Th2 or Th0 responses, e.g., in subjects where Th1 responses are suppressed (e.g., from viral, bacterial or parasite infection (HIV, HCV, etc) or chemotherapy) or are otherwise suboptimal. In many subjects, levels of either IL-4 or IL-10, usually IL-10, before dosing with a formula 1 compound is low or undetectable. In these subjects, dosing with the formula 1 compound results in a rapid drop in the interleukin that is detectable, usually IL-4.

Clinical conditions are described in more detail below where the formula 1 compounds are useful for treating, preventing, slowing the progression of, or ameliorating one or more symptoms associated with the described conditions. In any these conditions, any formula 1 compound disclosed herein can be used according to one or more of the dosing methods that are disclosed herein. For these conditions, dosages for the formula 1 compounds, formulations and routes of administration are as described herein. Additional information regarding these and other clinical conditions or symptoms that can be treated, prevented or ameliorated with the formula 1 compounds are found at e.g., The Merck Manual, 17th edition, M.H. Beers and R. Berkow editors, 1999, Merck Research Laboratories, Whitehouse Station, NJ, ISBN 0911910-10-7, or in other references cited herein.

Responses to treatment of a subject having a condition disclosed herein with a formula 1 compound is optionally monitored by observing changes in one or more immune or other appropriate clinical parameters, e.g., as described herein or in D.S. Jacobs et al., editors, Laboratory Test Handbook, 4th edition, pages 11-686, Lexi-Comp Inc., Hudson, Ohio, ISBN 0-916589-36-6, or in any of the references cited herein, or by monitoring the progression or severity of the underlying condition according to known methods, e.g., J.B. Peter, editor, Use and Interpretation of Laboratory Tests in Infectious Disease, 5th Edition, pages 1-309, 1998, Specialty Laboratories, Santa Monica, California, ISBN 1-889342-13-0.

Other uses for the formula 1 compound(s) include administering the compound(s) to a subject who suffers from a pathological condition(s). The treatment may treat or ameliorate the source of the condition(s) and/or symptoms associated with the pathological condition(s) such as infection with a pathogen(s) (viruses, bacteria, fungi), a malignancy, unwanted immune response, i.e., an immune response that causes pathology and/or symptoms, e.g., autoimmune conditions or allergy or conditions such as hypoproliferation conditions, e.g., normal or impaired tissue growth, or wound healing or burn healing, or in immunosuppression conditions, e.g., conditions characterized by an absence of a desired response and/or an inadequate degree of a desired response.

Cancer and hyperproliferation conditions. Many cancers, precancers, malignancies or hyperproliferation conditions are associated with an unwanted Th2 immune response, a deficient Th1 response or unwanted inflammation. An insufficient Th1 immune response may play a role in the capacity of malignant or premalignant cells to escape immune surveillance. The formula 1 compounds, including those in the compound groups and embodiments disclosed herein, may thus be used to treat, prevent or slow the progression of one or more cancers, precancers or cell hyperproliferation conditions or they may be used to ameliorate one or more symptoms thereof. In these conditions, the formula 1 compounds are useful to enhance the subject's Th1 responses or to reestablish a more normal Th1-Th2 balance in the subject's immune responses. The formula 1 compounds may function at least in part by decreasing inflammation or inflammation associated markers such as IL-6 and/or by enhancing hemopoiesis in many of these conditions.

These conditions include cancers or precancers comprising carcinomas, sarcomas, adenomas, blastoma, disseminated tumors and solid tumors such as one associated with or arising from prostate, lung, breast, ovary, skin, stomach, intestine, pancreas, neck, larynx, esophagus, throat, tongue, lip, oral cavity, oral mucosa, salivary gland, testes, liver, parotid, biliary tract, colon, rectum, cervix, uterus, vagina, pelvis, endometrium, kidney, bladder, central nervous system, glial cell, astrocyte, squamous cell, blood, bone marrow, muscle or thyroid cells or tissue. The formula 1 compounds are thus useful to treat, prevent, slow the progression of, or ameliorate one or more symptoms of a precancer, cancer or related hyperproliferation condition such as myelodysplastic syndrome, actinic keratoses, endometriosis, Barrett's esophagus, leiomyoma, fibromyoma, benign or precancerous intestinal or bowel polyps or benign prostatic hyperplasia. The compounds can also be used to treat, prevent, slow the progression of, slow the replication or growth of, or to ameliorate one or more symptoms of a primary tumor, a metastasis, an advanced malignancy, a blood born malignancy, a leukemia or a lymphoma.

The formula 1 compounds can be used to treat paraneoplastic syndromes or conditions such as ones associated with lung or breast cancers that secrete calcitonin or that enhance osteoclast activity. Such conditions include hypercalcemia, Cushing's syndrome, acromegaly and non-islet cell tumor hypoglycemia. The compounds are used to decrease osteoclast activity or other symptoms associated with such conditions.

Hyperproliferation conditions that can be treated include melanoma, Kaposi's sarcoma, leiomyosarcoma, non-small cell lung cancer, small cell lung cancer, bronchogenic carcinoma, renal cell cancer or carcinoma, glioma, glioblastoma, pancreatic or gastric adenocarcinoma, gastrointestinal adenocarcinoma, human papillomavirus associated cervical intraepithelial neoplasia, cervical carcinoma, hepatoma, hepatocellular carcinoma, hepatocellular adenoma, cutaneous T-cell lymphoma (mycosis fungoides, Sezary syndrome), colorectal cancer, chronic lymphocytic leukemia, chronic myelogenous leukemia, ALL or follicular lymphoma, multiple myeloma, carcinomas with p53 mutations, colon cancer, cardiac tumors, adrenal tumors, pancreatic cancer, retinoblastoma, a small cell lung cancer, a non-small cell lung cancer, intestinal cancer, testicular cancer, stomach cancer, neuroblastoma, neuroma, myxoma, myoma, endothelioma, osteoblastoma, osteoclastoma, osteosarcoma, chondrosarcoma, adenoma, breast cancer, prostate cancer, Kaposi's sarcoma, ovarian cancer, squamous cell carcinoma of the gastrointestinal tract. Treating a subject with a formula 1 compound can ameliorate one or more side effects of chemotherapy or cancer symptoms such as alopecia, pain, fever, malaise, chronic fatigue and cachexia or weight loss. Other cancers, precancers or their symptoms that can be treated, prevented or ameliorated are described in, e.g., Holland. Frei Cancer Medicine e.5, 5th edition, R.C. Bast et al., editors, 2000, ISBN 1-55009-113-1, pages 168-2453, B.C. Becker Inc. Hamilton, Ontario, Canada or The Merck Manual, 17th edition, M.H. Beers and R. Berkow editors, 1999, Merck Research Laboratories, Whitehouse Station, NJ, ISBN 0911910-10-7.

In some of these embodimants, the subject's hyperproliferation or malignant condition may be associated with or caused by one or more pathogens. Such conditions include hepatocellular carcinoma associated with HCV or HBV, Kaposi's sarcoma associated with HIV-1 or HIV-2, T cell leukemia associated with HTLV I, Burkitt's lymphoma associated with Epstein-Barr virus or papillomas or carcinoma associated with papilloma viruses (e.g., human HPV 6, HPV 11, HPV 16, HPV 18, HPV 31, HPV 45) or gastric adenocarcinoma, gastric MALT lymphoma or gastric inflammation associated with *Helicobacter pylori,* lactobacillus, enterobacter, staphylococcus or propionibacteria infection.

In some of these embodiments, the formula 1 compounds may be used to treat, prevent or slow the progression of or ameliorate one or more conditions in a subject having or subject to developing a hyperproliferation condition where angiogenesis contributes to the pathology. Abnormal or unwanted angiogenesis or neovascularization contributes to the development or progression of solid tumor growth and metastases, as well as to arthritis, some types of eye diseases such as diabetic retinopathy, retinopathy of prematurity, macular degeneration, corneal graft rejection, neovascular glaucoma, rubeosis, retinoblastoma, uvietis and pterygia or abnormal blood vessel growth of the eye, and psoriasis. See, e.g., Moses et al., Biotech. 9:630-634 1991, Folkman et al., N. Engl. J. Med., 333:1757-1763 1995, and Auerbach et al., J. Microvasc. Res. 29:401-411 1985.

Dosages of the formula 1 compound, routes of administration and the use of combination therapies with other standard therapeutic agents or treatments could be applied essentially as described above for cancer or hyperproliferation conditions or other conditions as disclosed herein. This, in some embodiments, the use of the formula 1 compound is optionally combined with one or more additional therapies for a cancer or precancer(s), e.g., one or more of surgery and treatment with an antiandrogen or an antiestrogen as described herein or in the cited references, an antineoplastic agent such as an alkylating agent, a nitrogen mustard, a nitrosourea, an antimetabolite or cytotoxic agent, or an analgesic such as propoxyphene napsylate, acetaminophen or codeine. Exemplary anticancer and adjunct agents include methotrexate, thioguanine, mercaptopurine, adriamycin, chlorambucil, cyclophosphamide, cisplatin, procarbazine, hydroxyurea, allopurinol, erythropoietin, G-CSF, bicalutamide, anastrozole, fludarabine phosphate and doxorubicin. Such therapies would be used essentially according to standard protocols and they would precede, be essentially concurrent with and/or follow treatment with a formula 1 compound. In some embodiments, such additional therapies will be administered at the same time that a formula 1 compound is being used or within about 1 day to about 16 weeks before or after at least one round of treatment with the formula 1 compound is completed. Other exemplary therapeutic agents and their use have been described in detail, see, e.g., Physicians Desk Reference 54th edition, 2000, pages 303-3250, ISBN 1-56363-330-2, Medical Economics Co., Inc., Montvale, NJ. One or more of these exemplary agents can be used in combination with a formula 1 compound to ameliorate, slow the establishment or progression of, prevent or treat any of the appropriate cancers, precancers or related conditions described herein, or any of their symptoms.

In treating cancers or hyperproliferation conditions, the formula 1 compounds may detectably modulate, e.g., decrease or increase, the expression or level or activity of one or more biomolecules associated with the prevention, establishment, maintenance or progression of the cancer or hyperproliferation condition. Such biomolecules include one or more of carcinoembryonic antigen, prostate specific antigen, her2/neu, Bcl-XL, bcl-2, p53, IL-1α, IL-1β, IL-6, or TNFα, GATA-3, COX-2, NFκB, IkB, an IkB kinase, e.g., IkB kinase-α, IkB kinase-β or IkB kinase-γ, NFAT, calcineurin, calmodulin, a ras protein such as H-ras or K-ras, cyclin D, cyclin E, xanthine oxidase, or their isoforms, homologs or mutant forms, which may have either reduced or enhanced biological activity(ies), and which may be detectably decreased. Biomolecules or their activity(ies) that can be detectably increased include IL-2, IFNγ, IL-12, T-bet, O6-methylguanine-DNA-methyltransferase, calcineurin, calmodulin, a superoxide dismutase (e.g., Mn, Zn or Cu), a tumor suppressor protein such as the retinoblastoma protein (Rb) or CDKN2A (p16), BRCA1, BRCA2, MeCP2, MBD2, PTEN, NBR1, NBR2 or the isoforms, homologs or mutant forms, which may have either attenuated or enhanced biological activity(ies), of any of these molecules. One or more of these biomolecules may be modulated in any the cancers or conditions described herein.

The formula 1 compounds can modulate the synthesis or a biological activity of one or more other gene products such as transcription factors, enzymes or steroid or other receptors that are associated with the establishment, progression or maintenance of a cancer or precancer or associated symptom. The compounds can inhibit AIB-1 coactivator or HER2/neu synthesis or activity in breast cancer cells or breast cancer conditions. They can enhance the synthesis or an activity of an estrogen receptor such as ERα, ERβ1 or ERβ2 or progesterone receptor in breast cancer or colon cancer cells or conditions. These effects can include modulation of the expression or one or more biological activities of proteins or enzymes that contribute to disease establishment or progression. Thus, the compounds can decrease IL-4, IL-6 or IL-13 expression by stromal cells or immune cells that are in proximity to or adjacent to solid or diffuse tumor cells in a subject such as a human or another mammal. In the cancers or precancers described herein, the compounds can thus directly or indirectly modulate (e.g., decrease) the activity or expression of relevant enzymes such as STAT-6, neutral endopeptidase, a hydroxysteroid dehydrogenase, such as a 17β-hydroxysteroid dehydrogenase or a 3β-hydroxysteroid dehydrogenase.

In an exemplary embodiment, human patients suffering from melanoma or melanoma precursor lesions are treated with a topical cream formulation containing 2-20% BrEA (w/w). The cream is applied to primary nevi (dysplastic nevi or common acquired nevi), primary cutaneous melanomas, secondary cutaneous melanomas and the skin surrounding the nevi or melanomas. The areas to be treated are washed with soap or swabbed with an alcohol (e.g., ethanol or isopropanol) prior to administering the cream, when this is practical. About 0.1-0.4 g of cream, depending on the size of the treated area, is applied once or twice per day per treated region or lesion for about 10-20 days. The cream is left undisturbed at the administration site for about 15-30 minutes before the patient resumes normal activity. Progression of the nevi and melanomas is retarded in the majority of patients and significant regression is observed for some lesions. Following initial treatment, the formulation is administered every other day for at least 1 to 4 months using the same dosing described for the initial round of treatment. For these patients, standard therapy to treat precursor lesion or melanoma, e.g., dimethyl triazeno imidazole carboxamide or nitrosoureas (e.g., BCNU, CCNU), is optionally started or continued according to the recommendations of the patient's doctor and with the patient's informed approval. In cases where a tumor or precursor lesion is surgically removed and the site has sufficiently healed, the patient optionally continues using the topical formulation at the site and the adjacent surrounding area every other day for at least 1 to 4 months. In some of these embodiments, a formula 1 compound(s) is administered daily continuously as an oral composition or formulation, e.g., for a formula 1 compound(s) that is a new compound *per se.* The compound are optionally also administered systemically using, e.g., a formulation essentially as described in the examples below or elsewhere herein to deliver about 0.1-25 mg/kg/day every other day for about 1 week to about to 4 months, e.g., in the case of malignant melanoma.

Cardiovascular applications. The formula 1 compounds, including those in the compound groups and embodiments disclosed herein, may be used to treat, prevent or slow the progression of one or more of congenital heart defects, cardiovascular diseases, disorders, abnormalities and/or conditions, or to ameliorate one or more symptoms thereof in a subject. These include peripheral artery disease, arterio-arterial fistula, arteriovenous fistula, cerebral arteriovenous malformations, aortic coarctation, cor triatum, coronary vessel anomalies, patent ductus arteriosus, Ebstein's anomaly, hypoplastic left heart syndrome, levocardia, transposition of great vessels, double outlet right ventricle, tricuspid atresia, persistent truncus arteriosus, and heart septal defects, such as aortopulmonary septal defect, endocardial cushion defects, Lutembacher's Syndrome, ventricular heart septal defects, cardiac tamponade, endocarditis (including bacterial), heart aneurysm, cardiac arrest, congestive heart failure, congestive cardiomyopathy, paroxysmal dyspnea, cardiac edema, post-infarction heart rupture, ventricular septal rupture, heart valve diseases, myocardial diseases, pericardial effusion, pericarditis (including constrictive and tuberculous), pneumopericardium, postpericardiotomy syndrome, pulmonary heart disease, rheumatic heart disease, ventricular dysfunction, hyperemia, cardiovascular pregnancy complications, cardiovascular syphilis, cardiovascular tuberculosis, arrhythmias such as sinus arrhythmia, atrial fibrillation, atrial flutter, bradycardia, extrasystole, Adams-Stokes Syndrome, bundle-branch block, sinoatrial block, long QT syndrome, parasystole, sick sinus syndrome, ventricular fibrillations, tachycardias such as paroxysmal tachycardia, supraventricular tachycardia, accelerated idioventricular rhythm, atrioventricular nodal reentry tachycardia, ectopic atrial tachycardia, ectopic junctional tachycardia, sinoatrial nodal reentry tachycardia, sinus tachycardia, Torsades de Pointes, and ventricular tachycardia and heart valve diseases such as aortic valve insufficiency, aortic valve stenosis, hear murmurs, aortic valve prolapse, mitral valve prolapse, tricuspid valve prolapse, mitral valve insufficiency, mitral valve stenosis, pulmonary atresia, pulmonary valve insufficiency, pulmonary valve stenosis, tricuspid atresia, tricuspid valve insufficiency, and tricuspid valve stenosis.

The formula 1 compounds can be used to treat, prevent or ameliorate one or more symptoms of myocardial diseases or pathological myocardial or vascular conditions such as alcoholic cardiomyopathy, congestive cardiomyopathy, hypertrophic cardiomyopathy, aortic subvalvular stenosis, pulmonary subvalvular stenosis, restrictive cardiomyopathy, Chagas cardiomyopathy, endocardial fibroelastosis, myocardial fibrosis, endomyocardial fibrosis, Kearns Syndrome, myocardial reperfusion injury, myocarditis, cardiovascular or vascular diseases such as dissecting aneurysms, false aneurysms, infected aneurysms, ruptured aneurysms, aortic aneurysms, cerebral aneurysms, coronary aneurysms, heart aneurysms, and iliac aneurysms, angiodysplasia, angiomatosis, bacillary angiomatosis, Sturge-Weber Syndrome, angioneurotic edema, aortic diseases, Takayasu's Arteritis, aortitis, Leriche's Syndrome, arterial occlusive diseases, arteritis, enarteritis, polyarteritis nodosa, cerebrovascular diseases, disorders, and/or conditions, diabetic angiopathies, diabetic retinopathy, thrombosis, erythromelalgia, hemorrhoids, hepatic veno-occlusive disease, hypertension, hypotension, idiopathic pulmonary fibrosis, peripheral vascular diseases, phlebitis, pulmonary veno-occlusive disease, Raynaud's disease, CREST syndrome, retinal vein occlusion, Scimitar syndrome, superior vena cava syndrome, telangiectasia, atacia telangiectasia, hereditary hemorrhagic telangiectasia, varicocele, varicose veins, varicose ulcer, vasculitis, venous insufficiency and arterial occlusive diseases such as arteriosclerosis, intermittent claudication, carotid stenosis, fibromuscular dysplasias, mesenteric vascular occlusion, Moyamoya disease retinal artery occlusion, thromboangiitis obliterans or atherosclerosis, any of which may be at an early stage or at a more advanced or late stage.

The formula 1 compounds can also be used to treat, prevent or ameliorate one or more symptoms of cerebrovascular diseases, thrombosis, and/or conditions such as carotid artery diseases, cerebral amyloid angiopathy, cerebral aneurysm, cerebral anoxia, cerebral arteriosclerosis, cerebral arteriovenous malformation, cerebral artery diseases, cerebral embolism and thrombosis, carotid artery thrombosis, sinus thrombosis, Wallenberg's syndrome, cerebral hemorrhage, epidural hematoma, subdural hematoma, subarachnoid hemorrhage, cerebral infarction, cerebral ischemia (including transient), subclavian steal syndrome, periventricular leukomalacia, vascular headache, cluster headache, migraine, vertebrobasilar insufficiency, air embolisms, embolisms such as cholesterol embolisms, fat embolisms, pulmonary embolisms or amniotic fluid embolism, thromoboembolisms, thrombosis such as coronary thrombosis, hepatic vein thrombosis, retinal vein occlusion, carotid artery thrombosis, sinus thrombosis, Wallenberg's syndrome, and thrombophlebitis.

The formula 1 compounds can also be used to treat, prevent or ameliorate one or more symptoms of vascular ischemia or myocardial ischemias, vasculitis and coronary diseases, including angina pectoris, coronary aneurysm, coronary arteriosclerosis, coronary thrombosis, coronary vasospasm, myocardial infarction and myocardial stunning, cerebral ischemia, ischemic colitis, compartment syndromes, anterior compartment syndrome, myocardial ischemia, reperfusion injuries, peripheral limb ischemia, aortitis, arteritis, Behcet's Syndrome, mucocutaneous lymph node syndrome, thromboangiitis obliterans, hypersensitivity vasculitis, Schoenlein-Henoch purpura, allergic cutaneous vasculitis, and Wegener's granulomatosis.

Exemplary symptoms that the use of the formula 1 compounds can ameliorate include one or more of pain such as arm, jaw or chest pain, edema or swelling, high blood pressure, shortness of breath or dyspnea, e.g., on exertion or while prone, fatigue or malaise and low cardiac injection fraction. In treating a cardiovascular condition in a subject or in improving one or more symptoms thereof, the formula 1 compounds may accomplish one or more of increasing cardiac ejection volume or fraction, decreasing levels of IL-6, decreasing levels of C reactive protein, fibrinogen, cardiac creatinine kinase, increasing fatty acid metabolism or utlization by cardiac tissue, increasing carnityl palmitoyl fatty acid transferase or other cardiac metabolic enzymes, activating potassium dependent calcium channels, vasodilating or enhancing oxygen delivery to ischemic tissues or decreasing levels of scarring or plaque formation that occurs, e.g., after vascular damage. Symptoms associated with a cardiovascular condition such as ischemia that can be ameliorated also include acidosis, expression of one or more immediate early genes in, e.g., glial cells, vascular smooth muscle cells or endothelial cells, neuronal membrane depolarization and increased neuronal extracellular calcium and glutamate concentration. Other biological effects associated with treatment using a formula 1 compound may also be monitored, e.g., and increase or decrease of a cell surface antigen, a cytokine or an interleukin as disclosed herein.

Useful biological effects of the formula 1 compounds in cardiovascular indications such as myocardial ischemias also include preventing or reducing heart or vascular cell death and subsequent fibrosis. These effects are associated with a decreased oxidative capacity of heart cells or myocytes, which is associated with a decreased capacity of the cells to metabolize fatty acids efficiently. The compounds enhance fatty acid metabolism and ameliorate the deleterious effects of a limited oxidative capacity.

The formula 1 compounds also can limit inflammation or cell injury that is associated with ischemia or oxygen reperfusion after ischemia. Ischemia, which is a detrimental decrease in oxygenated blood delivery to affected cells or tissues, may arise from a cardiovascular condition or event such as an infarction, or from thermal injury or burns. Ischemia may also arise from accidental or surgical trauma. Reperfusion after cells have become hypoxic for a sufficient period of time can lead to tissue or cell injury that varies from slight to lethal. The compounds can reduce cell or tissue injury or death associated with ischemia and reperfusion, by, e.g., reducing inflammation or the level of a molecule associated with inflammation. Thus, levels of a proinflammatory cytokine or molecule such as leukotriene B4, platelet activating factor or levels of extracellular P-selectin may result from administration of a formula 1 compound to a subject who may experience reperfusion injury. Thus, the compounds can reduce injury or death of, e.g., neuron, cardiac, vascular endothelium, myocardial, pulmonary, hepatic or renal cells or tissues. Without wishing to be bound by any theory, the compounds may act in part by reducing one or more of neutrophil activation, platelet activation, platelet aggregation, endothelial cell activation and neutrophil adherence or adhesion to endothelial cells in these conditions.

The use of any formula 1 compound or species in any genus of formula 1 compounds disclosed herein to treat, prevent or ameliorate any of these cardiovascular conditions or symptoms will generally use one or more of the routes of administration, dosages and dosing protocols as disclosed herein.

Applications in autoimmunity, allergy, inflammation and related conditions. As mentioned above, the formula 1 compounds, including those in the compound groups and embodiments disclosed herein, may be used to treat, prevent or slow the progression of one or more autoimmune allergic or inflammatory diseases, disorders, or conditions, or to ameliorate one or more symptoms thereof in a subject. These diseases and conditions include Addison's Disease, autoimmune hemolytic anemia, antiphospholipid syndrome, acute or chronic rheumatoid arthritis and other synovial disorders, an osteoarthritis including post-traumatic osteoarthritis and hypertrophic pulmonary osteoarthropathy, psoriatic arthritis, polyarthritis, epichondylitis, type I diabetes, type II diabetes, rheumatic carditis, bursitis, ankylosing spondylitis, multiple sclerosis, a dermatitis such as contact dermatitis, atopic dermatitis, exfoliative dermatitis or seborrheic dermatitis, mycosis fungoides, allergic encephalomyelitis, autoimmune glomerulonephritis, Goodpasture's Syndrome, Graves' Disease, Hashimoto's Thyroiditis, multiple sclerosis, myasthenia gravis, neuritis, bullous pemphigoid, pemphigus, polyendocrinopathies, purpura, Reiter's Disease, autoimmune thyroiditis, systemic lupus erythematosus, scleroderma, fibromyalgia, chronic fatigue syndrome, autoimmune pulmonary inflammation, Guillain-Barre Syndrome, type 1 or insulin dependent diabetes mellitus, autoimmune inflammatory eye disease, hepatitis C virus associated autoimmunity, postinfectious autoimmunity associated with, e.g., virus or bacterial infection such as a parvovirus such as human parvovirus B19 or with rubella virus, autoimmune skin and muscle conditions such as pemphigus vulgaris, pemphigus foliaceus, systemic dermatomyositis or polymyositis or another inflammatory myopathy, myocarditis, asthma such as allergic asthma, allergic encephalomyelitis, allergic rhinitis, a vasculitis condition such as polyarteritis nodosa, giant cell arteritis or systemic necrotizing vasculitis, chronic and an acute or chronic inflammation condition such as chronic prostatitis, granulomatous prostatitis and malacoplakia, ischemia-reperfusion injury, endotoxin exposure, complement- mediated hyperacute rejection, nephritis, cytokine or chemokine induced lung injury, cachexia, sarcoidosis, inflammatory bowel disease, regional enteritis, ulcerative colitis, Crohn's disease, inflammatory bowel disease or inflammation associated with an infection, e.g., septic shock, sepsis, or systemic inflammatory response syndrome. Any of these diseases or conditions or their symptoms may be acute, chronic, mild, moderate, severe, stable or progressing before, during or after the time administration of the formula 1 compound to a subject such as a human, is initiated. In general, a detectable improvement is observed in the subject within a period of about 3 days to about 12 months after initiation of a dosing protocol, e.g., the severity of the disease or condition will detectably decrease, the rate of progression will detectably slow or the severity of a symptom(s) will detectably decrease.

As used herein, acute inflammation conditions are characterized as an inflammation that typically has a fairly rapid onset, quickly becomes moderate or severe and usually lasts for only a few days or for a few weeks. Chronic inflammation conditions as used herein are characterized as an inflammation that may begin with a relatively rapid onset or in a slow, or even unnoticed manner, tends to persist for at least several weeks, e.g., about 3-6 weeks, months, or years and may have a vague or indefinite termination. Chronic inflammation may result when the injuring agent (or products resulting from its presence) persists in the lesion, and the subject's tissues respond in a manner (or to a degree) that is not sufficient to overcome completely the continuing effects of the injuring agent. Other exemplary conditions are described in, e.g., Textbook ofAutoimmune Diseases, R.G. Lahita, editor, Lippincott Williams & Wikins, Philadelphia, PA, 2000, ISBN 0-7817-1505-9, pages 175-851 and Rheumatology, 2nd edition, J.H. Klippel et al., editors, 1998, ISBN 0-7234-2405-5, volume 1, sections 1-5 and volume 2, sections 6-8, Mosby International, London, UK.

A formula 1 compound can be used to inhibit or ameliorate one or more inappropriate immune responses or their symptoms in autoimmunity, inflammation, allergy or related conditions. The effects of the formula 1 compounds include detectably ameliorating one or more of (1) the proliferation, differentiation or chemotaxis of T cells, (2) reducing unwanted cytotoxic T cell responses, (3) reducing unwanted autoantibody or other antibody synthesis, e.g., an unwanted IgA, IgE, IgG or IgM, in allergy, asthma or another autoimmune or inflammation condition, (4) inhibiting the development, proliferation or unwanted activity of autoreactive T or B cells, (5) altering the expression of one or more cytokines, interleukins or cell surface antigens, e.g., a cytokine, interleukin or cell surface antigen described herein (decreasing IL-8 in an autoimmune condition, decreasing the level of acute phase proteins such as C reactive protein or fibrinogen in inflammation conditions, (6) decreasing eosinophilia in allergy conditions, (7) detectably decreasing the level or activity of one or more of ICAM-1, IL-1α, IL-1β, TNFα or IL-6 in, e.g., inflammation conditions or in autoimmune conditions such as an arthritis or a myocarditis condition such as osteoarthritis, rheumatoid arthritis, toxic myocarditis, indurative myocarditis or idiopathic myocarditis, (8) decreasing the level or biological activity of one or more of anti-islet antibody, TNF, IFN-γ, IL-1, an arthritis symptom(s), nephritis, skin rash, photosensitivity, headache frequency or pain, migraine frequency or pain, abdominal pain, nausea or anti-DNA antibodies in , e.g., insulin dependent diabetes mellitus or an autoimmune or inflammation condition such as systemic lupus erythematosus, rheumatoid arthritis or Crohn's disease, (9) reducing induction of arachidonic acid metabolism or reducing eicosanoid metabolites such as thromboxanes or prostaglandins in, e.g., inflammation, asthma or allergy, (10) reducing IL-4, IL-8 or IL-10 synthesis, levels or activity in, e.g., allergy or inflammation such as idiopathic pulmonary fibrosis or allergic asthma or (11) reducing or interfering with neutrophil chemotaxis by, e.g., reducing thioredoxin release from affected cells in conditions such as cancer, infections, inflammation or autoimmunity.

Exemplary symptoms that the use of the formula 1 compounds can ameliorate in these autoimmune, inflammatory and allergy conditions include one or more of pain such as shoulder, hip, joint, abdominal or spine pain, joint stiffness or gelling, bursitis, tendonitis, edema or swelling, fatigue or malaise, headache, dyspnea, skin rash, fever, night sweats, anorexia, weight loss, skin or intestine ulceration, muscle weakness, pericarditis, coronary occlusion, neuropathy and diarrhea. In treating one of these conditions in a subject or in improving one or more symptoms thereof, the formula 1 compounds may accomplish one or more of decreasing levels of one or more of IL-1, IL-4, IL-6 or TNFα, decreasing levels of C reactive protein, fibrinogen or creatinine kinase. Other biological effects associated with treatment using a formula 1 compound may also be monitored or observed, e.g., an increase or decrease of a cell surface antigen, a cytokine or an interleukin as disclosed herein.

In treating inflammation or any condition described herein where inflammation contributes to the condition, the formula 1 compounds may detectably modulate, e.g., decrease or increase, the expression or level or activity of one or more biomolecules associated with the prevention, establishment, maintenance or progression of the inflammation condition. Such biomolecules include one or more of carcinoembryonic antigen, prostate specific antigen, her2/neu, Bcl-XL, bcl-2, p53, IL-1α, IL-1β, IL-6, or TNFα, GATA-3, COX-2, NFκB, IkB, an IkB kinase, e.g., IkB kinase-α, IkB kinase-β or IkB kinase-γ, NFAT, a ras protein such as H-ras or K-ras, cyclin D, cyclin E xanthine oxidase, or their isoforms, homologs or mutant forms, which may have either reduced or enhanced biological activity(ies), and which may be detectably decreased. Biomolecules that can be detectably increased include IL-2, IFNγ, IL-12, T-bet, O6-methylguanine-DNA-methyltransferase, calcineurin, calmodulin, a superoxide dismutase (e.g., Mn, Zn or Cu), a tumor suppressor protein such as the retinoblastoma protein (Rb) or CDKN2A (p16), BRCA1, BRCA2, MeCP2, MBD2, PTEN, NBR1, NBR2 or the isoforms, homologs or mutant forms, which may have either attenuated or enhanced biological activity(ies), of any of these molecules. One or more of these biomolecules may be modulated in any inflammation condition described herein.

The use of any formula 1 compound or species in any genus of formula 1 compounds disclosed herein to treat, prevent or ameliorate any of these autoimmune, inflammatory or allergy conditions or symptoms will generally use one or more of the routes of administration, dosages and dosing protocols as disclosed herein. Thus, in exemplary embodiments, about 0.5 to about 100 mg/kg or about 1 mg/kg to about 15 mg/kg, of the formula 1 compound will be administered per day by, e.g., an oral, buccal, sublingual, topical or parenteral route. Such administration can be, e.g., daily for about 5 to about 60 days in acute conditions or it can be intermittent for about 3 months to about 2 years or more for chronic conditions. Alternatively, intermittent dosing can be used essentially as described herein for acute autoimmune, inflammatory and allergy conditions.

In related embodiments, the use of the formula 1 compound is optionally combined with one or more additional therapies for an autoimmune, inflammatory or allergy disorder(s), e.g., one or more of surgery and treatment with a corticosteroid or glucocorticoid such as hydrocortisone, hydrocortisone acetate, prednisone, prednisolone, prednisolone acetate, methylprednisolone, dexamethasone, dexamethasone acetate or triamcinolone acetonide, leflunomide, an antibody, e.g., a human or humanized monoclonal antibody, that decreases the activity or level of C5 complement, TNFα or TNFα receptor, an antirheumatic drug such as methorexate, D-penicillamine, sodium aurothiomalate, sulfasalazine or hydroxychloroquine, immunosuppressive agents such as 6-thioguanylic acid, chlorambucil, cyclophosphamide or cyclosporin, a non-steroidal antiinflammatory agent such as celecoxib, ibuprofin, piroxicam or naproxin, an antihistamine such as loratidine or promethazine hydrochloride or an analgesic such as propoxyphene napsylate, acetaminophen or codeine. Such therapies would be used essentially according to standard protocols and such they would precede, be concurrent with or follow treatment with a formula 1 compound. In some embodiments, such additional therapies will be administered at the same time that a formula 1 compound is being used or within about 1 day to about 16 weeks before or after at least one round of treatment with the formula 1 compound is completed. Other exemplary therapeutic agents and their use have been described in detail, see, e.g., Physicians Desk Reference 54th edition, 2000, pages 303-3267, ISBN 1-56363-330-2, Medical Economics Co., Inc., Montvale, NJ. One or more of these exemplary agents can be used in combination with a formula 1 compound to ameliorate, prevent or treat any of the appropriate autoimmune, inflammatory or allergy conditions or disorders described herein or any of their symptoms.

Regeneration and wound healing. The formula 1 compounds can be used to facilitate cell differentiation or proliferation where regeneration of tissues is desired. The regeneration of tissues could be used to repair, replace, protect or limit the effects of tissue damaged by congenital defects, trauma (wounds, burns, incisions, or ulcers), age, disease (e.g. osteoporosis, osteoarthritis, periodontal disease, liver failure), surgery, including cosmetic plastic surgery, fibrosis, reperfusion injury, or systemic cytokine damage. Tissues for which regeneration may be enhanced include organs (e.g., pancreas, liver, intestine, kidney, skin, endothelium, oral mucosa, gut mucosa), muscle (e.g., smooth, skeletal or cardiac), vasculature (including vascular and lymphatics), nervous tissue, hematopoietic tissue, and skeletal tissue (e.g., bone, cartilage, tendon, and ligament). These effects may be accompanied by decreased scarring or an increased rate of healing.

The formula 1 compounds are thus useful to enhance healing or tissue repair in a subject having a bone fracture(s), e.g., a simple or compound skull, spine, hip, arm or leg bone fracture. Similarly, nerve or brain tissue treatment using a formula 1 compound allows treating, slowing the progression of, ameliorating or preventing diseases such as central and peripheral nervous system diseases, neuropathies, or mechanical and traumatic diseases, disorders, and/or conditions (e.g., spinal cord disorders, head trauma, cerebrovascular disease, and stoke). The compounds are useful to treat diseases associated with peripheral nerve injuries, peripheral neuropathy (e.g., resulting from chemotherapy or other medical therapies), localized neuropathies, and central nervous system diseases such as Alzheimer's disease, Parkinson's disease, Huntington's disease and amyotrophic lateral sclerosis. The subjects undergoing treatment in these conditions may be elderly, e.g., a human at least 55, 60, 65 or 70 years of age. Where the condition is acute, e.g., a bone fracture or a burn, the treatment may comprise administration of a formula 1 compound to the subject on a daily or intermittent basis for about 3 days to about 12 months, e.g., administration for about 2-12 weeks beginning after the subject sustains an injury.

Dosages of the formula 1 compound, routes of administration and the use of combination therapies with other standard therapeutic agents or treatments could be applied essentially as described herein, e.g., for cardiovascular conditions or other conditions as disclosed herein.

Neurological conditions. Nervous system diseases, disorders, or conditions, which can be ameliorated, treated or prevented with any of the formula 1 compounds disclosed herein include, but are not limited to, nervous system trauma or injury, and diseases or conditions which result in either a disconnection of axons, a diminution of neuron function or degeneration of neurons, demyelination or pain.

Nervous system lesions which may be treated, prevented, or ameliorated in a subject include but are not limited to, the following lesions of either the central (including spinal cord, brain) or peripheral nervous systems: (1) ischemic lesions, in which a lack of oxygen in a portion of the nervous system results in neuronal injury or death, including cerebral infarction or ischemia, or spinal cord infarction or ischemia, (2) traumatic lesions, including lesions caused by physical injury or associated with surgery, for example, lesions which sever a portion of the nervous system, or compression injuries, (3) malignant lesions, in which a portion of the nervous system is destroyed or injured by malignant tissue which is either a nervous system associated malignancy or a malignancy derived from non-nervous system tissue, (4) infectious lesions, in which a portion of the nervous system is destroyed or injured as a result of infection, for example, by an abscess or associated with infection by human immunodeficiency virus, herpes zoster, or herpes simplex virus or with Lyme disease, tuberculosis, syphilis, (5) degenerative lesions, in which a portion of the nervous system is destroyed or injured as a result of a degenerative process including but not limited to degeneration associated with Parkinson's disease, Alzheimer's disease, Huntington's chorea, or amyotrophic lateral sclerosis (ALS), (6) lesions associated with nutritional diseases, disorders, and/or conditions, in which a portion of the nervous system is destroyed or injured by a nutritional disorder or disorder of metabolism including but not limited to, vitamin B 12 deficiency, folic acid deficiency, Wernicke disease, tobacco-alcohol amblyopia, Marchiafava-Bignami disease (primary degeneration of the corpus callosum), and alcoholic cerebellar degeneration, (7) neurological lesions associated with systemic diseases including, but not limited to, diabetes (diabetic neuropathy, Bell's palsy), systemic lupus erythematosus, carcinoma, or sarcoidosis, (8) lesions caused by toxic substances including alcohol, lead, or particular neurotoxins, (9) demyelinated lesions in which a portion of the nervous system is destroyed or injured by a demyelinating disease including, but not limited to, multiple sclerosis, human immunodeficiency virus-associated myelopathy, progressive multifocal leukoencephalopathy, and central pontine myelinolysis or a myelopathy, e.g., diabetic meylopathy or a transverse myelopathy, (10) neurological conditions such as insomnia, epilepsy, schizophrenia, depression, addiction to a drug, substance such as tobacco, nicotine, caffeine, alcohol, a barbiturate, a tranquilizer, a narcotic such as hydromorphone HCl, propoxyphene napsylate, meperidine HCl, codeine, cocaine, morphine, heroin or methadone and (11) cognitive dysfunction conditions or diseases such as one or more of impaired long-term or short-term memory, impaired concentration impaired attention or impaired learning, where the cognitive dysfunction condition or disease is optionally associated with chemotherapy, radiation therapy or exposure, aging, trauma, e.g., CNS trauma, or neurodegeneration.

The formula 1 compounds are useful to ameliorate, treat or prevent the onset, severity or length of other neurological diseases or conditions such as headache or a migraine condition or symptom such as classic migraine, cluster headache, abdominal migraine, common migraine, hemiplegic migraine, ocular migraine, fulminating migraine, complicated migraine or a symptom of any of these such as head pain, vertigo, nausea, vomiting or potophobia.

In some embodiments, the formula 1 compound is used to protect neural cells from the damaging effects of cerebral hypoxia, cerebral ischemia or neural cell injury associated with cerebral infarction, heart attack, stroke or elevated levels of glucocorticoids such as cortisol. The compounds are also useful for treating or preventing a nervous system disorder may be selected, e.g., by assaying their biological activity in promoting the survival or differentiation of neurons. For example, and not by way of limitation, compositions of the invention which elicit any of the following effects are useful: (1) increased survival time of neurons in culture, (2) increased sprouting of neurons in culture or *in vivo*, (3) increased production of a neuron-associated molecule in culture or *in vivo*, *e*.*g*., dopamine or choline acetyltransferase or acetylcholinesterase with respect to motor neurons or (4) decreased symptoms of neuron dysfunction *in vivo*. Such effects may be measured by any method known in the art. Increased survival of neurons may be measured using known methods, such as, for example, the method set forth in Arakawa et al. (J. Neurosci. 10:3507-3515 1990); increased sprouting of neurons may be detected by methods known in the art, such as the methods set forth in Pestronk et al. (Exp. Neurol. 70:65-82 1980) or Brown et al. (Ann. Rev. Neurosci. 4:17-42 1981). Increased production of neuron-associated molecules may be measured by, e.g., bioassay, enzymatic assay, antibody binding or Northern blot assay, using techniques known in the art and depending on the molecule to be measured. Motor neuron dysfunction may be measured by assessing the physical manifestation of motor neuron disorder, e.g., weakness, motor neuron conduction velocity, or functional disability. In other embodiments, motor neuron diseases, disorders, and/or conditions that may be treated, prevented, and/or ameliorated include diseases, disorders, and/or conditions such as infarction, infection, exposure to toxin, trauma, surgical damage, degenerative disease or malignancy that may affect motor neurons as well as other components of the nervous system, as well as diseases, disorders, and/or conditions that selectively affect neurons such as amyotrophic lateral sclerosis, and including, but not limited to, progressive spinal muscular atrophy, progressive bulbar palsy, primary lateral sclerosis, infantile and juvenile muscular atrophy, poliomyelitis and the post polio syndrome, and hereditary motorsensory neuropathy.

In some conditions such as mood changes, depression anxiety, memory loss or motor function impairment, the formula 1 compounds can modulate one or more biological activities of a transcription factor or a steroid receptor such as ERα in tissue such as the hypothalamus or amygdala or ERβ in tissue such as the hippocampus, thalamus or entorhinal cortex.

For any of these diseases, conditions or their associated symptoms, the presence of the disease, condition or symptom may be determined by suitable objective or subjective means, e.g., assays to detect tissue damage, levels of diagnostic markers, or an etiological agent, patient questionaires or behavior performance tests, measurement of a diagnostic marker(s), e.g., an enzyme, hormone, cytokine or drug substance in blood or tissue, electroencephalography, imaging methods such as X-ray, MRI scan or CAT scan, observation and diagnosis of clinical features or symptoms or biopsy of affected tissue or cells, e.g., aspiration biopsy, needle biopsy, incision biopsy or punch biopsy of tissue or cells. Neurological conditions, diseases and symptoms, which the formula 1 compounds can be used to treat or ameliorate and methods to diagnose and characterize such conditions or diseases have been described. See, e.g., Ph. Demaerel, A.L. Baert et al., eds. Recent Advances in Diagnostic Neuroradiology (Medical Radiology: Diagnostic Imaging) 2001 Springer Verlag, ISBN: 3504657231, W.G. Bradley et al., Neurology in Clinical Practice: Principles of Diagnosis and Management 1995, see, e.g., vol. 1 Ch. 1-55 and vol 2. Ch. 1-66, Butterworth-Heinemann Medical, ISBN 0750694777, H.J.M. Barnett et al., eds. Stroke: Pathophysiology, Diagnosis and Management 3rd edition, 1998, see, e.g., pages 10-1450, Churchill Livingstone, ISBN 0443075514, P.J. Vinken et al., eds. Neurodystrophies and Neurolipidoses 2nd ed. 1996, see, e.g., pages 8-780, Elsevier Science, ISBN 0444812857, P.L. Peterson and J.W. Phillis eds. Novel Therapies for CNS Injuries: Rationales and Results 1995, see, e.g., pages 8-380, CRC Press, ISBN 0849376521, D. Schiffer, Brain Tumors: Pathology and Its Biological Correlates 2nd ed. 1997, see, e.g., pages 5-450, Springer Verlag, ISBN 3540616225 and E. Niedermeyer and F. Lopes Da Silva, eds. Electroencephalography: Basic Principles, Clinical Applications and Related Fields 4th ed. 1999 see, e.g., pages 13-1238, Lippincott, Williams & Wilkins, ISBN 0683302841. The use of the formula 1 compounds in these conditions is optionally combined with one or more of the therapeutic treatments that are described in these references. The formula 1 compound may be administered before, during or after another treatment is employed to treat or ameliorate a given neurological disease, condition or symptom.

Dosages of the formula 1 compound, routes of administration and the use of combination therapies with other standard therapeutic agents or treatments could be applied essentially as described above for cardiovascular conditions or as disclosed elsewhere herein. Thus, the formula 1 compounds may be administered prophylactically or therapeutically in chronic conditions or they may be administered at the time of or relatively soon after an acute event such as an epileptic seizure, onset of a migraine or occurrence of trauma, accidental head or central nervous system injury or a cereberal stroke or infarction. For acute events, the formula1 compounds may thus be administered concurrently, e.g., within about 15 minutes or about 30 minutes of the onset or occurrence of the acute event, or at a later time, e.g., at about 1, 2, 3, 4, 5, 6, 7, 8, 9, 10, 11, 12, 13, 14, 15, 16, 18, 20, 22, 24, 26, 28, 30, 36, 42, 48, 54, 60, 72, 84, 96, 108 or 120 hours after the onset or occurrence of the acute event. The formula 1 compounds may thus be administered at about 6-120 hours, or about 8-48 hours, about 10-24 hours or about 12-16 hours after an acute event starts or occurs.

Skin treatments. The affect of the formula 1 compounds on immune function permits their use to improve the function of organs or organ systems that rely on the optimal functioning of one or more immune responses. Thus, the formula 1 compounds can be administered to a subject to prevent, treat, ameliorate, slow the progression of or enhance the healing of certain skin conditions such as skin inflammation, lesions, atrophy or rash. As used here, skin includes external skin and internal skin or surfaces such as oral, intestinal and rectal mucosa. These conditions include lesions, rashes or inflammation associated with, e.g., burns, infections and the thinning or general degradation of the dermis often characterized by a decrease in collagen or elastin as well as decreased number, size and doubling potential of fibroblast cells. Such skin conditions include keratoses such as actinic keratosis, psoriasis, eczema, warts such as papillomavirus-induced warts, ulcers or lesions such as herpesvirus-induced ulcers or lesions or diabetes associated ulcers or lesions, discoid lupus erythematosus, erythema nodosum, erythema multiform, cutaneous T cell lymphoma, atopic dermatitis, inflammatory vasculitis, relapsing polychondritis, exfoliative dermatitis, sarcoidosis, burns, melanoma, rash or irritation from poison oak, poison ivy or poison Sumac, blemished or hyperpigmented skin, hyperkeratotic skin, dry skin, dandruff, acne, inflammatory dermatoses, scarring such as from a chemical or thermal burn and age-related skin changes. In these embodiments, treatment with the formula 1 compounds is optionally combined with other appropriate treatments or therapies essenitally as described herein, e.g., one or more of a corticosteroid such as hydrocortisone or cortisol, prednisone, or prednisolone, an α-hydroxybenzoic acid or an α-hydroxycarboxylic acid(s) is coadministered with a formula 1 compound to treat, prevent or ameliorate a skin condition such as atrophy or a lesion. α-Hydroxybenzoic acids and α-hydroxycarboxylic acids suitable for use in these embodiments are described in, e.g., U.S. patents 5262407, 5254343, 4246261, 4234599 and 3984566. The formula 1 compound can be used to minimize cutaneous atrophy caused by corticosteroids, a side-effect of their application to the skin.

In these embodiments that address skin conditions, dosages, routes of administration and dosing protocols for the formula 1 compounds are essentially as described herein. In some embodiments, the formula 1 compound is administered to the subject in the form of a topical cream, ointment, spray, foam or gel.

Modulation of transcription factors, receptors and gene expression. In treating any of the diseases, conditions or symptoms disclosed herein, the formula 1 compounds can modulate, i.e., detectably enhance or increase or detectably inhibit or decrease, the expression or a biological activity(ies) of one or more transcription factors or receptors. This can lead to detectable modulation of target gene activity or expression as part of the treatment or amelioration of the disease, condition or symptom. Such modulation can arise from changes in the capacity of a transcription factor or receptor to bind to or form a complex with other natural ligands such as a target DNA sequence(s), another transcription factor(s), a transcription cofactor, a receptor such as a steroid receptor or cell membrane receptor (e.g., a lipid, peptide, protein or glycoprotein receptor such as an interleukin receptor or a growth factor receptor), a receptor cofactor or an enzyme such as a polymerase, kinase, phosphatase or transferase. The effects of formula 1 compounds on these biomolecules can be exerted in immune cells or in non-immune tissue, e.g., cells or tissue adjacent to diseased tissue such as infected or malignant cells. The formula 1 compounds may directly or indirectly modulate the capacity of any of these molecules to transduce signals that are part of normal signal trandsuction processes.

In many of the clinical conditions described herein, e.g., in cancers, infections, acute inflammation, chronic inflammation or autoimmunity, the formula 1 compounds can modulate, e..g., detectably decrease or increase, a biological activity(ies), protein or molecule level or RNA level of 1, 2, 3, 4, 5, 6 or more biomolecules that are involved in establishment, maintenance or progression of a disease, condition or symptom. Such biomolecules include 1, 2, 3, 4, 5, 6 or more of AP-1, a cyclooxygenase such as cyclooxygenase-1 (COX-1) or cyclooxygenase-2 (COX-2), TNFα, TNFα receptor 1, TNFα receptor 2, TNF receptor-associated factor, TNFβ, TNFβ receptor, MIP-1α, monocyte chemoattractant-1 (MCP-1), interferon gamma (IFNγ or γIFN), IL-1α, IL-1β, IL-1α receptor, IL-1β receptor, IL-2, IL-3, IL-4, IL-4 receptor (IL-4R), IL-5, IL-6, IL-6 receptor (IL-6R), IL-8, IL-8 receptor (IL-8R), IL-10, IL-10 receptor (IL-10R), IL-12, an IL-12 receptor, (e.g., IL-12Rβ2), IL-13, IL-15, IL-17, IL-18, nuclear factor kappa B (NFκB), AP-1, c-maf, v-maf, mafB, Nrl, mafK, mafG, the maf family protein p18, reactive oxygen species, e.g., hydrogen peroxide or superoxide ion (collectively ROS), a 17β-hydroxysteroid dehydrogenase (17β-HSD) or an 11β-hydroxysteroid dehydrogenase (11β-HSD), e.g., 11β-HSD type 1, 11β-HSD type 2, 17β-HSD type 1, 17β-HSD type 2 or 17β-HSD type 5, a steroid aromatase, e.g., cytochrome P450 aromatase, steroid 5α-reductase, serum or blood cortisol cytosolic phospholipase A2 (cPLA2), calcium-independent phospholipase A2 (iPLA2), a prostaglandin, e.g., prostaglandin E2 (PGE2) or prostaglandin D2 (PGD2), a leukotriene, e.g., leukotriene B4, inducible nitric oxide synthetase (iNOS), nitric oxide (NO), GM-CSF, RANTES (regulated on activation, normal T cells expressed and secreted), eotaxin, GATA-3, CCR1, CCR3, CCR4, CCR5, CXCR4, , in, e.g., a subject's cell(s) or tissue(s) or in enzyme, tissue or cell-based assays. In these subjects, the levels of other biomolecules, their RNAs or the level of their activity can be detectably modulated include IFNα, INFα receptor, PPARα, PPARγ, PPARδ or a transcription factor such as T-bet is detectably increased. Other biomolecules or their polymorphs or homologs that the formula 1 compounds directly or indirectly modulate include one or more of, e.g., Janus kinase 1 (JAK1), Janus kinase 2 (JAK2), Janus kinase 3 (JAK3), signal transducer and activator of transcription 1 (STAT1), signal transducer and activator of transcription 2 (STAT2) and signal transducer and activator of transcription 3 (STAT3). The formula 1 compounds can modulate the other biologically active analogs of any these enzymes, chemokines, cytokines, their receptors or ligands, including their polymorphs or homologs. In some cells or tissues, one or more of these biomolecules may be detectably increased, while in other cells or tissues, the same biomolecule may be detectably decreased. Thus, the biomolecules that the formula 1 compounds can modulate, e.g., detectably increase or decrease, include the intracellular or extracellular level or biological activity of one or more enzyme, cytokine, cytokine receptor, chemokine and/or chemokine receptor.

Additional exemplary mammalian or human and other biomolecules, e.g., transcription factors or receptors, including orphan nuclear receptors, their homologs, isoforms and co-factors (e.g., co-repressors, co-activators, transcription factors, gene promoter regions or sequences) and related molecules that the formula 1 compounds can directly or indirectly from complexes with, or modulate (detectably increase or decrease) the synthesis, level or one or more biological activities of, include steroidogenic factor-1 (SF-1), steroidogenic acute regulatory protein (StAR), chicken ovalbumin upstream promoter-transcription factor (COUP-TFI) and its mammalian homologs, silencing mediator for retinoid and thyroid hormone receptor (SMRT) and its mammalian homologs, sterol regulatory element binding protein (SREBP) 1a (SREBP-1a), SREBP-1c, SREPB-2, NF-E3, FKHR-L1, COUP-TFII and its mammalian homologs, IκB, IκBα, AML-3, PEBP2αA1, Osf2, Cbfa1, RUNX2, activating transcription factor 2 (ATF2), c-Jun, c-Fos, a mitogen activated kinase (MAP) such as p38 or JNK or an isoform thereof, a mitogen activated kinase kinase (MKK) or an isoform thereof, steroid receptor coactivator-1 family (SRC-1, SRC-1/serum response factor), SRC-2, SRC-3, SET, nerve growth factor inducible protein B, STF-IT, NFAT, NFAT interacting protein 45 (NIP45), IkB, an IkB kinase, NFATp, NFAT4, an AP-1 family protein, p300, CREB, CREB-binding protein (CPB), p300/CBP, p300/CPB-associated factor, SWI/SNF and their human and other homologs, BRG-1, OCT-1/OAF, AP-1, AF-2, Ets, androgen receptor associated protein 54 (ARA54), androgen receptor associated protein 55 (ARA55), androgen receptor associated protein 70 (ARA70), androgen receptor-interacting protein 3 (ARIP3), ARIP3/PIASx α complex, PIASx α, Miz1, Miz1/PIASx β complex, PIASx β, PIAS1, PIAS3, GBP, GBP/PIAS1 complex, RAC3/ACTR complex, SRC-1α, receptor interacting protein-140 (RIP-140), transcription factor activator protein-1, activation function-2, glucocorticoid receptor-interacting protein-1 (GRIP-1), receptor interacting protein-160 (RIP-160), suppressor of gal4D lesions (SUG-1), transcription intermediary factor-1 (TIF-1), transcription intermediary factor-2 (TIF-2), SMRT, N-CoR, N-CoA-1, p/CIP, p65 (RelA), the 120KD rel-related transcription factor, heat shock proteins (HSP) such as HSP90, HSP70 and HSP72, heat shock factor-1, Vpr encoded by the human immunodeficiency virus and its isoforms and homologs thereof, testicular orphan receptor TR2, thyroid hormone α1 (TR α1), retinoid X receptor α, TR α1/RXR α heterodimer, direct repeat-4 thyroid hormone response element (DR4-TRE), an estrogen receptor (ER) such as ERα or ERβ, estrogen receptor related receptor α (ERRα), estrogen receptor related receptor β (ERRβ), estrogen receptor related receptor γ (ERRγ), steroid xenobiotic receptor (SXR), hepatocyte nuclear factor 4 (HNF-4), hepatocyte nuclear factor 3 (HNF-3), liver X receptors (LXRs), LXRα, LXRβ, estrogen receptor α (ERα), constitutive androstane receptor-β (CAR-β), RXR/CAR-β heterodimer, short heterodimer partner (SHP; NR0B2), SHP/ERα heterodimer, estrogen receptor β, SHP/ERβ heterodimer, testicular orphan receptor TR4, TR2/TR4 heterodimer, pregnane X receptor (PXR) and isoforms, cytochrome P-450 monooxygenase 3A4, including its gene promoter region and isoforms thereof, HNF-4/cytochrome P-450 monooxygenase 3A4 gene promoter region and isoforms complex, HIV-1 long terminal repeat (LTR), HIV-2 LTR, TR2/HIV-1 LTR complex, TR4/HIV-1 LTR complex, TR4/HIV-1 LTR complex, TR α1/TR4/HIV-1 LTR complex, TR2 isoforms (TR2-5, TR7, TR9, TR11), DAX-1, DAX-1/steroidogenic acute regulatory protein gene promoter region, RevErb, Rev-erbA α, Reverb β, steroid receptor coactivator amplified in breast cancer (AIB 1), p300/CREB binding protein-interacting protein (p/CIP), thyroid hormone receptor (TR, T3R), thyroid hormone response elements (T3REs), retinoblastoma protien (Rb), tumor suppressor factor p53, transcription factor E2F, mammalian acute phase response factor (APRF), constitutive androstane receptor (CAR), Xenopus xSRC-3 and mammalian (human) homologs, TAK1, TAK1/peroxisome proliferator-activated receptor α (PPARα) complex, PPARα/RXRα complex, peroxisome proliferator-activated receptor β (PPARβ), peroxisome proliferator-activated receptor γ (PPARγ), peroxisome proliferator-activated receptor δ (PPARδ), farnesoid X receptor, retina X receptor, TAK-1/RIP-140 complex, retinoic acid receptor (RAR), RARβ, TR4/RXRE complex, SF-1/steroid hydroxylase gene promoter region, SF-1/oxytocin, including its gene promoter region, bile acid receptor (FXR), nuclear receptor corepressor (NcoR), liver receptor homologous protein-1 (LRH-1; NR5A2), SF-1/ACTH receptor gene promoter region, rat Ear-2 and mammalian homologs, human TR3 orphan receptor (TR3), RLD-1, OR-1, androgen receptor, glucocorticoid receptor, estrogen receptor, progesterone receptor, mineralcorticoid receptor, aldosterone receptor, E6-associated protein (E6-AP), OR1, OR1/RXRα complex, TIF-1, CBP/P300 complex, TRIP1/SUG-1 complex, RIP-140, steroid receptor coactivator 1 (SRC1), SRC1α/P160 complex and TIF-2/GRIP-1 complex, RAR/N-CoR/RIP13 complex, RAR/SMRT/TRAC-2 complex and protein X of hepatitis B virus. The homologs, orthologs and isoforms of these transcription factors, receptors and other molecules are included among the molecules that the formula 1 compounds can modulate the synthesis or one or more biological activities of. Such factors are bioogically active or function in one or more of a number of cell types such as T cells, B cells, macrophages, dendritic cells, platelets, monocytes, neutrophils, neurons, epithelial cells, endothelial cells, cartilage cells, osteoblasts, osteoclasts, splenocytes, thymocytes and GALT associated cells. Methods to identify these molecules and their biological activities have been described, e.g., U.S. patent Nos. 6248781, 6242253, 6180681, 6174676, 6090561, 6090542, 6074850, 6063583, 6051373, 6024940, 5989810, 5958671, 5925657, 5958671, 5844082, 5837840, 5770581, 5756673, and PCT publication Nos. WO 00/24245, WO 0073453 and WO 97/39721.

In one aspect, the compounds are used to treat, prevent or to ameliorate conditions or symptoms that are associated with unwanted or expression or activity of one or more of these molecules in conditions such as, e.g., acute inflammation, chronic inflammation or their symptoms, acute allergy, chronic allergy or their symptoms, e.g., allergic rhinitis or acute or chronic asthma, psoriatic arthritis, osteoporosis, osteoarthritis, rheumatoid arthritis, neurological dysfunction or their symptoms, e.g., dementias such as Alzheimer's Disease, Parkinson's Disease, or memory loss conditions, in osteoporosis or in cancer such as breast cancer. The compounds can prevent NFκB from translocating from the cytoplasm into the nucleus and thus can increase the ratio of cytoplasmic NFκB to nuclear NFκB. The formula 1 compounds may inhibit activation of NFκB-mediated transcription while NFκB is bound to target DNA sequences in the nucleus. Alternatively, the formula 1 compounds can activate or enhance the expression of or one or more activity of a transcription factor such as T-bet in, e.g., a subject's cell(s) or tissue(s) or in enzyme or cell-based assays. In this aspect the compounds are used to treat, prevent or to ameliorate conditions or symptoms that are associated with deficient expression or activity of T-bet in conditions such as immune dysfunction in an immunosuppression condition, aging, an infection, a cancer or precancer as described herein or in the cited references.

Thus, in some embodiments, the level or a biological activity of 4, 5, 6, 7, 8, 9, 10, 11, 12, 13, 14, 15 or more of COX-2, IL-1β, TNFα, TNFα receptor 1, TNFα receptor 2, TNF receptor-associated factor, MIP-1α, MCP-1, IFNγ, IL-4, IL-4R, IL-6, IL-6R, IL-8, IL-8R, IL-10, IL-10R, NFκB, IkBα, AP-1, GATA-3, 11β-HSD1, cPLA2, iPLA2, cortisol, ROS, PGE2, PGD2, leukotriene A4, leukotriene B4, leukotriene C4, iNOS or GM-CSF are optionally measured and they are generally detectably reduced, e.g., RNA or protein levels are reduced by about 10-95% or about 20-95% or more compared to suitable untreated controls. In these embodiments, the level or a biological activity of 4, 5, 6 or more of IFNα, INFα receptor, IL-12, an IL-12 receptor, (e.g., IL-12Rβ2), PPARα, PPARγ, and T-bet are optionally measured and they are generally detectably increased. In a chronic infection condition, e.g., HIV in humans, autoimmunity, a chronic fungal or parasite infection or in a precancer or cancer condition, e.g., benign prostatic hyperplasia, the progression of the condition may be slowed over a period of 1, 2, 3, 4, 5 or more years. In these embodiments, the subject's condition becomes more manageable with a reduced incidence or severity of side effects, e.g., a detectable halt, slowing, reversal or decreased incidence of wasting, dementia, CD4 cell count decreases or viral load increases, which tend to occur over time in HIV infected humans or a halt, slowing or reversal of pathogen or precancer or cancer cell replication. The detectable halt, slowing, reversal of the condition or decreased incidence of side effects can be observed as a decrease of about 10% or more, e.g., about a 20%, 30%, 40%, 50%, 60%, 70%, 80%, 90%, 95% or more decline.

These effects are typically observed after administration of an effective amount of a formula 1 compound using, e.g., a method or dose essentially as disclosed herein. The simultaneous reduction of multiple biomolecules provides a method to modulate immune responses by modulating multiple pathways that lead to a common condition such as inflammation. This provides a method to treat or ameliorate, e.g., acute or chronic inflammation, a cancer, an infection or a symptom associated therewith, or to slow the progression of or reduce the severity of these conditions or their symptoms.

Previously described methods can be used to measure the amount, activity or cellular location of various biomolecules such as cytokines or transcription factors. See, e.g., U.S. patent 6107034, 5925657, 5658744, 4016043 and 3850752, S. Szabo et al., Cell 2000 100:655-669, Y. Nakamura et al., J. Allergy Clin. Immunol. 1999 103(2 pt. 1):215-222., R.V. Hoch et al., Int. J. Cancer 1999 84:122-128. These methods can be used to measure the effects of the formula 1 compounds on transcription factors or receptors in cells or tissues that have been exposed to the compounds.

Without wishing to be bound to any theory, the formula 1 compounds may modulate multiple biomolecules in a microenvironment sensitive manner or context. The effects of the compounds can provide a decrease in a particular molecule such as IFNγ and a decrease in inflammation associated with elevated IFNγ levels or activity without eliminating beneficial effects of the molecule. This effect arises from decreasing the level or activity of a biomolecule such as IFNγ in cells that are dysregulated, while allowing normal immune cells to produce sufficient amounts of the same molecule to perform normal immune functions. In locations where the biomolecule is needed for activity, e.g., in lymph nodes or spleen cells, sufficient amounts of the modulated molecule are present to elicit a desired response, while the level of the molecule in cells in circulation decreases. The compounds can increase IL-13, IL-15, IL-17 or IL-18 in conditions where a subject has a deficient Th1 immune response, e.g., in infection or cancer. Conversely, the compounds can decrease IL-13, IL-15 or IL-18 in conditions such as allergy or autoimmune conditions, e.g., multiple sclerosis, where an excess Th1 immune status may prevail.

The formula 1 compound will, in some cases modulate (increase or decrease) transcription of one or more genes in the cells. In other cases, the formula 1 compound will enhance lysosome movement in one or more of the subject's NK cells, phagocytes, monocytes, macrophages, neutrophils, eosinophils, dendritic cells synoviocytes, microglial cells or fibrocytes. Such effects will typically be mediated directly or indirectly through one or more transcription factors or steroid receptors that act to modulate gene transcription, e.g., cause enhanced protein kinase C (a PKC such as PKCα, PKCβ, PKCγ or PKCζ) activity in the cells used in the assay, or another effect as disclosed herein.

Other therapeutic and biological applications and activities. The formula 1 compounds are useful to treat autoimmune or metabolic conditions or disorders, or their symptoms, in subjects such as mammals or humans that relate to impaired insulin synthesis or use or that relate to abnormal or pathological lipid or cholesterol metabolism or levels. Such conditions and symptoms include Type 1 diabetes (including Immune-Mediated Diabetes Mellitus and Idiopathic Diabetes Mellitus), Type 2 diabetes (including forms with (1) predominant or profound insulin resistance, (2) predominant insulin deficiency and some insulin resistance and (3) forms intermediate between these), obesity, hyperglycemia, hyperlipidemia conditions such as hypertriglyceridemia and hypercholesterolemia. In diabetes, the compounds are useful to (1) enhance β-cell function in the islets of Langerhans (e.g., increase insulin secretion), (2) reduce the rate of islet cell damage, (3) increase insulin receptor levels or activity to increase cell sensitivity to insulin and/or (4) modulate glucocorticoid receptor activity to decrease insulin resistance in cells that are insulin resistant. The compounds are thus useful to treat, prevent, ameliorate or slow the progression of diabetes or hyperglycemia, or a related symptom or condition, in a subject such as a human or a mammal.

Beneficial effects that can the formula 1 compounds can exert on such related symptoms or conditions include improved glucose tolerance, improved glucose utilization, decreased vascular disease (e.g., decreased severity or progression of microvascular or macrovascular disease, including nephropathy, neuropathy, retinopathy, hypertension, cerebrovascular disease and coronary heart disease), decreased severity or progression of atherosclerosis, decreased severity or progression of an arteriosclerosis condition (e.g., coronary arteriosclerosis, hyperplastic arteriosclerosis, peripheral arteriosclerosis or hypertensive arteriosclerosis), decreased level or activity of inflammatory macrophages (foam cells) in atherosclerotic plaques, decreased severity or progression of diabetic osteoarthropathy, decreased severity or progression of skin lesions, decreased severity or progression of ketosis, decreased generation of autoantibodies against islet cells or decreased expression or levels of one or more of IL-1 (e.g., IL-1β), IL-6, TNF (e.g., TNFα), and IFN-γ. In these any of these diseases or conditions, the formula 1 compounds can also modulate, e.g., enhance CARβ, RXR, PPARα or PPARβ levels. As used herein, obesity includes a human with a body mass index of about 27, 28, 29, 30, 31, 32, 33, 34 or greater.

The formula 1 compounds are useful in treating insulin resistance and associated symptoms and conditions. Insulin resistance is typically observed as a diminished ability of insulin to exert its biological action across a broad range of concentrations. This leads to less than the expected biologic effect for a given level of insulin. Insulin resistant subjects or human have a diminished ability to properly metabolize glucose or fatty acids and respond poorly, if at all, to insulin therapy. Manifestations of insulin resistance include insufficient insulin activation of glucose uptake, oxidation and storage in muscle and inadequate insulin repression of lipolysis in adipose tissue and of glucose production and secretion in liver. Insulin resistance can cause or contribute to polycystic ovarian syndrome, impaired glucose tolerance, gestational diabetes, hypertension, obesity, atherosclerosis and a variety of other disorders. Insulin resistant individuals can progress to a diabetic state. The compounds can also be used in the treatment or amelioration of one or more condition associated with insulin resistance or glucose intolerance including an increase in plasma triglycerides and a decrease in high-density lipoprotein cholesterol, high blood pressure, hyperuricemia, smaller denser low-density lipoprotein particles, and higher circulating levels of plasminogen activator inhibitor-1. Such diseases and symptoms have been described, see, e.g., G.M. Reaven, J. Basic Clin. Phys. Pharm. 1998, 9: 387-406, G.M. Reaven, Physiol. Rev. 1995, 75: 473-486 and J. Flier, J. Ann. Rev. Med. 1983, 34:145-60.

The compounds can thus be used in diabetes, obesity, hyperlipidemia or hypercholesterolemia conditions to reduce body fat mass, increase muscle mass or to lower one or more of serum or blood low density lipoprotein, triglyceride, cholesterol, apolipoprotein B, free fatty acid or very low density lipoprotein compared to a subject that would otherwise be considered normal for one or more of these characteristics. These beneficial effects are typically obtained with with little or no effect on serum or blood high density lipoprotein levels. The formula 1 compounds are useful to reduce or slow the rate of myocardial tissue or myocyte damage, e.g., fibrosis, or to enhance cardiac fatty acid metabolism in conditions, such as inflammation, where fatty acid metabolism is depressed or decreased. Elevated cholesterol levels are often associated with a number of other disease states, including coronary artery disease, angina pectoris, carotid artery disease, strokes, cerebral arteriosclerosis, and xanthoma, which the formula 1 compounds can ameliorate or slow the progression or severity of. Abnormal lipid and cholseterol conditions that can be treated include exogenous hypertriglyceridemia, familial hypercholesterolemia, polygenic hypercholesterolemia, biliary cirrhosis, familial combined hyperlipidemia, dysbetalipoproteinemia, endogenous hypertriglyceridemia, mixed hypertriglyceridemia and hyperlipidemia or hypertriglycidemia secondary to alcohol consumption, diabetic lipemia, nephrosis or drug treatments, e.g., corticosteroid, estrogen, colestipol, cholestyramine or retinoid treatments. Dosages, routes of administration and dosing protocols for the formula 1 compounds are essenitally as described herein. Where the condition is chronic, the formula 1 compounds will generally be administered to a subject such as a human for a relatively long time period, e.g., for about 3 months to about 10 years or more. Dosages, routes of administration and dosing protocols for the formula 1 compounds are essenitally as described herein. Dosing of the compound can be daily or intermittent using a dosing protocol using dosages as described herein, e.g., about 0.01 to about 20 mg/kg of a formula 1 compound administered to a subject once or twice per day daily or intermittently. The use of the formula 1 compounds can be combined with other suitable treatments, e.g., diet control or HMG-CoA reductase inhibitors such as Simvastatin^{™}, Pravastatin^{™}, Mevastatin^{™} or Lovastatin^{™}.

The formula 1 compounds can modulate the biological activity of cytokines or interleukins that are associated with various immune deficiency or dysregulation conditions, which may be transient or chronic. They can thus be used to ameliorate, treat or prevent naturally occurring age-related decline in immune function in a subject or immune deficiency or dysregulation resulting from trauma, stress, burns, surgery, autoimmunity or infections as described herein. Such immune deficiency dysregulation may be associated with, e.g., an age-related increase in production of one or more of IL-4, IL-5 and IL-6 or an age-related decrease in production of one or more of IL-2, IL-3, γ-IFN, GM-CSF or antibodies. In these embodiments, the formula 1 compound is administered to the subject to detectably decrease production or levels of one or more of IL-4, IL-5 and IL-6 or to detectably increase production or levels of one or more of IL-2, IL-3, IL-5, IL-12, GM-CSF and γ-IFN. These cytokine changes facilitate normalization of the subject's immune responses. Such normalization can be observed by various means. These means include monitoring appropriate cytokine RNA or protein level(s) in the subject or by measuring biological responses such as restoration or detectable improvement of contact hypersensitivity in a subject with depressed or suboptimal contact hypersensitivity response. The formula 1 compounds can thus be used to enhance or restore a deficient or suboptimal immune response such as contact hypersensitivity response in a subject with a chronic or transient state of immune deficiency or dysregulation. In these embodiments, the formula 1 compound is administered using the dosages, routes of administration and dosing protocols for the formula 1 compounds essenitally as described herein. Treatment with the formula 1 compounds is optionally combined with other appropriate treatments or therapies essenitally as described herein, e.g., a antibacterial or antiviral agent(s) is coadministered with a formula 1 compound to treat, prevent or ameliorate an infection in an infected subject or a subject suffering from, e.g., a burn. Methods to measure changes in cytokine levels or contact hypersensitivity are known and can optionally be applied in these embodiments, see, e.g., U.S. patent 5919465, 5837269, 5827841, 5478566.

In some clinical conditions, the formula 1 compounds can inhibit activated T lymphocytes *in vivo*, and they can inhibit the expression or biological activity of one or more of TNF-α, IFN-γ, IL-6, IL-8 or insulin like growth factor-1 receptor (IGF-1 R) or IL-6 receptor. The compounds are thus useful to treat, prevent or ameliorate conditions where this is a component of pathology. Such conditions include inflammation conditions such as psoriasis, psoriatic arthritis, osteoarthritis, and rheumatoid arthritis. The compound can thus ameliorate the inflammation, e.g., by inhibiting expression of one or more of TNF-α, IFN-γ, IL-6, IL-8 or IGF-1R. Also, the compounds can inhibit unwanted T cell activity. They can thus ameliorate one or more psoriasis symptoms such as skin scaling, skin thickening, keratinocyte hyperproliferation, deficient filaggrin expression (B. Baker et al., Br. J. Dermatol.1984, 111:702), deficient strateum corneum lipid deposition or they can improve a clinical assessment such as the Psoriasis Activity and Severity Index. The formula 1 compounds can be delivered to a subject with psoriasis using topical or systemic formulations as described herein. Topical formulations include gels, lotions and creams, e.g., as described herein. Daily or intermittent administration of the compound can be used essentially as described herein. The use of the formula 1 compounds is optionally combined with one more current psoriasis treatments, e.g., topical emollients or moisturizers, tars, anthralins, systemic or topical corticosteroids, vitamin D analogs such as calcitriol, methotrexate, etretinate, acitretin, cyclosporin, FK 506, sulfasalazine, ultraviolet B radiation optionally combined with one or more of a topical corticosteroid, tar, anthralin, emollient or moisturizer or ultraviolet A plus psoralen. Such additional treatments essentially would use known dosages and routes of administration, which are applied, e.g., within a month before, during or within a month after a treatment course with a formula 1 compound.

Other desirable modulation effects of the formula 1 compounds on cells or tissues include (1) inhibition of one or more of bone resorption or calcium release or gp80, gp130, tumor necrosis factor (TNF), osteoclast differentiation factor (RANKL/ODF), RANKL/ODF receptor, IL-6 or IL-6 receptor expression or biological activity in, e.g., bone loss or osteoporosis conditions or in osteoclasts, or in cancers such as prostate cancer, metastatic breast cancer or metastatic lung cancer (e.g., with bone metastases), (2) inhibition of osteoclastogenesis or osteoclast development from progenitor cells, (3) enhancement of NFκB inhibition that is mediated by steroid receptors, e.g., enhanced inhibition of estrogen receptor-α or estrogen receptor-β mediated inhibition of NFκB in inflammation, rheumatoid arthritis or osteoporosis, (4) enhancement of osteoblastogenesis, osteoblast, bone callus or bone development, e.g., from progenitor cells in bone fractures, depressed bone healing situations (e.g., in a burn patient or in a patient being treated with a glucocorticoid), bone growth or osteoporosis or other bone loss conditions, by, e.g., modulation or enhancement of osteoblast replication or development or modulation or enhancement of the synthesis or biological activity of a transcription factor such as Cbfa1, RUNX2 or AML-3 (5) normalization of hypothalamic-pituitary-adrenal axis function in conditions where there is dysregulation such as in chronic inflammatory diseases, chronic asthma or rheumatoid arthritis (increased cortisol to ACTH ratio), (6) modulation of ligand-gated ion channels in neurons in, e.g., depression, sleep or memory disorders, (8) modulation of G-protein coupled receptors in neurons in, e.g., depression, sleep or memory disorders, (9) modulation, e.g., induction, of the synthesis or biological activity of metabolic enzymes such as a cytochrome (e.g., a CYP enzyme such as CYP1A1, CYP2B1, CYP2b10, CYP4A, CYP7A, CYP7A1, CYP7B, CYP7B1, P450 3A4, P450c17, P450scc, P450c21 or an isozyme, homolog or mutant of any of these) in cells or tissues such as liver cells, neurons, neuron precursor cells, brain, breast, testes or colon, (10) enhancement of collagen synthesis or levels in, e.g., skin in aging or skin damage from, e.g., trauma, thermal injury or solar radiation, (11) inhibition of nitric oxide production in cells or tissue, e.g., in nervous system tissue or in microglial cells in dementias such as Alzheimer's disease, (12) enhancing glucose-stimulated insulin synthesis in hyperglycemia or diabetes conditions, (13) modulation of gamma-aminobutyric acid (GABA), dopamine or N-methyl-D-aspartate (NMDA) receptor activity or levels in, e.g., brain tissue or neurons, (e.g., decreased GABA-mediated chloride currents or potentiation of neuronal response to NMDA in the hippocampus) in, e.g., conditions such as a dementia (Alzheimer's Disease), depression, anxiety, schizophrenia or memory loss due to, e.g., aging or another condition described herein, (14) modulating (e.g., enhancing) the expression or activity of a transcription factor(s), or a homolog(s) or isoform(s), such as SET, nerve growth factor inducible protein B, StF-IT, SF-1 in cells or tissues such as nerve cells, neuronal precursor cells or liver cells, (15) inhibition of eosinophil infiltration or reduction IgE levels in allergic responses or in lung or other tissue, (16) modulation, e.g., a decrease, in serum or blood of leptin levels in, e.g., obese subjects such as humans with a body mass index of about 27, 28, 29, 30, 31, 32, 33, 34 or greater, (17) increased corticotropin releasing hormone synthesis or activity in, e.g., elderly subjects such as humans at least about 60 years of age or at least about 70 years of age, (18) enhancement of memory or reduction of memory loss or disorientation in aging or dementias such as Alzheimer's Disease, (20) enhancement of the synthesis or activity of one or more enzymes responsible for thermogenesis, e.g., liver glycerol-3-phosphate dehydrogenase or malic enzyme, in subjects such as obese or diabetic humans, (21) modulation, e.g., reduction, of the synthesis or biological activity of the CXCR4 receptor or the CXCL12 chemokine in hyperproliferation conditions such as breast cancers or precancers, (22) modulation of the synthesis or biological activity of one or more of holocytochrome c, cytochrome c, second mitochondria-derived activator of caspase, Apaf-1, Bax, procaspase-9, caspase-9, procaspase-3, caspase-3, caspase-6 and caspase-7, e.g., enhanced translocation of these molecules from mitochondria to cytosol or activation of these molecules in the cytosol in cancer precancer cells, cancer cells or cells that mediate autoimmunity, (23) modulation of the synthesis or biological activity of one or more of tumor necrosis factor-α, interleukin-1β converting enzyme, IL-6, IL-8, caspase-4 and caspase-5, e.g., decreased activation of these molecules in injured cells or cells subject to injury from, e.g., ischemia or infarction (e.g., vascular, cardiac or cerebral), reperfusion of hypoxic cells or tissue or an inflammation condition such as rheumatoid arthritis, ulcerative colitis, viral hepatitis, alcoholic hepatitis, or another inflammation condition disclosed herein, (24) decrease of the synthesis, biological activity or activation of one or more of phospholipase A2, caspase-1, caspase-3 and procaspase-3 in neurodegeneration disorders or dementias such as Alzheimer's disease, Huntington's disease, or another neurological condition disclosed herein. The formula 1 compounds can thus be used where one or more of these conditions or their symptoms is present. Methods to measure the synthesis or biological activity of these molecules has been described, see, e.g., U.S. patents 6200969, 6187767, 6174901, 6110691, 6083735, 6024940, 5919465 and 5891924.

In some embodiments, the formula 1 compound is present in a formulation that comprises micronized compound, e.g., compound that is milled, sieved, ground or the like. Micronized compound may be prepared using any suitable process for micronizing, a number of which are known in the art. The micronized particles may include a percentage of particles that are less than or equal to about 0.1-20 µm in diameter. Micronized preparations may comprise formula 1 compounds having an average particle size of about 0.02, 0.05, 0.1, 0.2, 0.4, 0.5, 1, 2, 5, 10 15, 20, 30 or 50 µm. Ranges of average particle sizes include formula 1 compounds of about 0.1-0.5 µm, about 0.1-0.4 µm, about 0.5-1 µm, about 1-20 µm or about 2-50 µm. An alternative to micronizing a compound is to solubilize the compound and incorporate it into liposomes of appropriate size. The manufacture of liposomes and the insertion of active ingredients into such liposomes are known.

Numbered embodiments. Several aspects of the invention and related subject matter includes the following numbered embodiments.

In some aspects, the invention relates to non-aqueous liquid formulations that comprise a formula 1 compound. Exemplary embodiments are as follows.

1 B. A method comprising intermittently administering one or more compounds of formula 1 (or a composition comprising a formula 1 compound) to a subject or delivering to the subject's tissues a formula 1 compound(s) (or a composition comprising a formula 1 compound), e.g., any formula 1 compound named or described herein, or a metabolic precursor or a biologically active metabolite thereof.

2B. The method of embodiment 1 B wherein the subject has an infection, a hyperproliferation disorder, a hypoproliferation condition, an immunosuppression condition, an unwanted immune response or wherein the subject has recently experienced or will shortly experience trauma, surgery or a therapeutic treatment wherein the therapeutic treatment is one other than the method of embodiment 1 B.

3B. The method of embodiment 2B wherein the immunosuppression condition or the unwanted immune response is associated with a viral infection, an intracellular bacterial infection, an extracellular bacterial infection, a fungal infection, a yeast infection, an extracellular parasite infection, an intracellular parasite infection, a protozoan parasite, a multicellular parasite, an autoimmune disease, a cancer, a precancer, a chemotherapy, a radiation therapy, an immunosuppressive therapy, an anti-infective agent therapy, a wound, a burn, a neurological condition, a cardiovascular condition, the presence of an immunosuppressive molecule, gastrointestinal irritation or an inflammation condition optionally selected from or associated with irritable bowel disease, Crohn's disease or chronic diarrhea, or any combination of the foregoing.

4B. The method of embodiment 3B wherein the subject's immunosuppression condition is ameliorated or the unwanted immune response (e.g., a Th2 response) is reduced or wherein the subject's Th1 immune response is enhanced.

5B. The method of embodiment 3B wherein the subject's innate immunity, specific immunity or both is enhanced.

6B. The method of embodiment 5B wherein the subject's innate immunity is enhanced.

7B. The method of embodiment 6B wherein the subject's specific immunity is enhanced, e.g., wherein the subject's Th2 immune response is reduced or wherein the subject's Th1 immune response is enhanced.

8B. The method of embodiment 2B wherein the one or more compounds of formula 1 is or are administered according to the a dosing regimen comprising the steps, (a) administering the one or more compounds of formula 1 to the subject at least once per day for at least 2 days; (b) not administering the one or more formula 1 compounds to the subject for at least 1 day; (c) administering the one or more formula 1 compounds to the subject at least once per day for at least 2 days; and (d) optionally repeating steps (a), (b) and (c) at least once or variations of steps (a), (b) and (c) at least once.

9B. The method of embodiment 8B wherein step (c) comprises the same dosing regimen as step (a).

10B.The method of embodiment 9B wherein step (a) of the dosing regimen comprises administering the one or more formula 1 compounds once per day, twice per day, three times per day or four times per day.

11B.The method of embodiment 10B wherein step (a) of the dosing regimen comprises administering the one or more formula 1 compounds once per day or twice per day.

12B.The method of embodiment 10B wherein step (a) comprises administering the one or more formula 1 compounds for about 3 to about 24 days.

13B.The method of embodiment 12B wherein step (a) comprises administering the one or more formula 1 compounds for about 4 to about 12 days.

14B.The method of embodiment 13B wherein step (a) comprises administering the one or more formula 1 compounds for about 4 to about 8 days.

15B.The method of embodiment 14B wherein step (b) comprises not administering the one or more formula 1 compounds for about 3 to about 120 days.

16B.The method of embodiment 15B wherein step (b) comprises not administering the one or more formula 1 compounds for about 4 to about 60 days.

17B.The method of embodiment 16B wherein step (b) comprises not administering the one or more formula 1 compounds for about 5 to about 30 days.

18B.The method of embodiment 16B wherein step (b) comprises not administering the one or more formula 1 compounds for about 8 to about 60 days.19B.

The method of embodiment 15B wherein steps (a), (b), and (c) are repeated at least about 4 times.

20B.The method of embodiment 15B wherein steps (a), (b), and (c) are repeated about 5 times to about 25 times.

21 B. The method of embodiment 15B wherein steps (a), (b), and (c) and repetitions of steps (a), (b), and (c) occur over a time period of at least about 2 months.

22B.The method of embodiment 15B wherein steps (a), (b), and (c) and repetitions of steps (a), (b), and (c) occur over a time period of at least about 12 months.

23B.The method of embodiment 8B wherein step (b) comprises not administering the one or more formula 1 compounds for about 3 to about 120 days.

24B.The method of embodiment 23B wherein step (b) comprises not administering the one or more formula 1 compounds for about 4 to about 60 days.

25B.The method of embodiment 24B wherein step (b) comprises not administering the one or more formula 1 compounds for about 5 to about 30 days.

26B.The method of embodiment 23B wherein step (b) comprises not administering the one or more formula 1 compounds for about 8 to about 60 days.

27B.The method of embodiment 8B wherein step (d) comprises repeating steps (a), (b), and (c) at least once.

28B.The method of embodiment 27B wherein step (d) comprises repeating steps (a), (b), and (c) about 3 times to about 25 times.

29B.The method of embodiment 1 B wherein steps (a), (b), and (c) and repetitions of steps (a), (b), and (c) occur over a time period of at least about 2 months.

30B.The method of embodiment 29B wherein steps (a), (b), and (c) and repetitions of steps (a), (b), and (c) occur over a time period of at least about 12 months.

31B. The method of any of embodiments 8B-30B wherein the immunosuppression condition or the unwanted immune response is associated with a viral infection, an intracellular bacterial infection, an extracellular bacterial infection, a fungal infection, a yeast infection, an extracellular parasite infection, an intracellular parasite infection, a protozoan parasite, a multicellular parasite, an autoimmune disease, a cancer, a precancer, a chemotherapy, a radiation therapy, an immunosuppressive therapy, an anti-infective agent therapy, a wound, a burn, the presence of an immunosuppressive molecule, gastrointestinal irritation or an inflammation condition optionally selected from or associated with irritable bowel disease, Crohn's disease or chronic diarrhea, or any combination of the foregoing.

32B.The method of embodiment 31 B wherein the subject's immunosuppression condition is ameliorated or the unwanted immune response is reduced.

33B.The method of embodiment 32B wherein the subject's innate immunity, specific immunity or both is enhanced.

34B.The method of embodiment 33B wherein the subject's innate immunity is enhanced.

35B.The method of embodiment 34B wherein the subject's specific immunity is enhanced.

36B.The method of embodiment 8B wherein step (c) comprises the a shorter dosing regimen than step (a).

37B.The method of embodiment 36B wherein step (a) comprises administering the formula 1 compound for 7 to about 24 days.

38B.The method of embodiment 37B wherein step (c) comprises administering the formula 1 compound for 4 to about 12 days.

39B.The method of embodiment 38B wherein step (b) comprises not administering the formula 1 compound for about 3 to about 120 days.

40B.The method of embodiment 39B wherein step (b) comprises not administering the formula 1 compound for about 4 to about 60 days.

41 B. The method of embodiment 40B wherein step (b) comprises not administering the formula 1 compound for about 5 to about 30 days.

42B.The method of embodiment 36B wherein step (d) comprises repeating steps (a), (b), and (c) at least once.

43B.The method of embodiment 42B wherein step (d) comprises repeating steps (a), (b), and (c) about 3 times to about 25 times.

44B.The method of embodiment 36B wherein steps (a), (b), and (c) and repetitions of steps (a), (b), and (c) occur over a time period of at least about 2 months.

45B.The method of embodiment 44B wherein steps (a), (b), and (c) and repetitions of steps (a), (b), and (c) occur over a time period of at least about 12 months.

46B.The method of any of embodiments 36B-45B wherein the immunosuppression condition or the unwanted immune response is associated with a viral infection, an intracellular bacterial infection, an extracellular bacterial infection, a fungal infection, a yeast infection, an extracellular parasite infection, an intracellular parasite infection, a protozoan parasite, a multicellular parasite, an autoimmune disease, a cancer, a precancer, a chemotherapy, a radiation therapy, an immunosuppressive therapy, an anti-infective agent therapy, a wound, a burn, the presence of an immunosuppressive molecule, gastrointestinal irritation or an inflammation condition optionally selected from or associated with irritable bowel, Crohn's disease, chronic diarrhea, or any combination of the foregoing.

47B.The method of embodiment 46B wherein the subject's immunosuppression condition is ameliorated or the unwanted immune response is reduced.

48B.The method of embodiment 47B wherein the subject's innate immunity, specific immunity or both is enhanced.

49B.The method of embodiment 48B wherein the subject's innate immunity is enhanced.

50B.The method of embodiment 48B wherein the subject's specific immunity is enhanced.

51 B. The method of embodiment 8B wherein step (c) comprises a longer dosing period than step (a).

52B.The method of embodiment 51 B wherein step (a) comprises administering the formula 1 compound for 7 to about 24 days.

53B.The method of embodiment 52B wherein step (c) comprises administering the formula 1 compound for 4 to about 12 days.

54B.The method of embodiment 53B wherein step (b) comprises not administering the formula 1 compound for about 3 to about 120 days.

55B.The method of embodiment 54B wherein step (b) comprises not administering the formula 1 compound for about 4 to about 60 days.

56B.The method of embodiment 55B wherein step (b) comprises not administering the formula 1 compound for about 5 to about 30 days.

57B. The method of embodiment 51 B wherein step (d) comprises repeating steps (a), (b), and (c) at least once.

58B.The method of embodiment 57B wherein step (d) comprises repeating steps (a), (b), and (c) about 3 times to about 25 times.

59B.The method of embodiment 51 B wherein steps (a), (b), and (c) and repetitions of steps (a), (b), and (c) occur over a time period of at least about 2 months.

60B.The method of embodiment 59B wherein steps (a), (b), and (c) and repetitions of steps (a), (b), and (c) occur over a time period of at least about 12 months.

61B. The method of any of embodiments 51B-60B wherein the immunosuppression condition or the unwanted immune response is associated with a viral infection, an intracellular bacterial infection, an extracellular bacterial infection, a fungal infection, a yeast infection, an extracellular parasite infection, an intracellular parasite infection, a protozoan parasite, a multicellular parasite, an autoimmune disease, a cancer, a precancer, a chemotherapy, a radiation therapy, an immunosuppressive therapy, an anti-infective agent therapy, a wound, a burn, the presence of an immunosuppressive molecule, gastrointestinal irritation or an inflammation condition optionally selected from or associated with irritable bowel disease, Crohn's disease or chronic diarrhea, or any combination of the foregoing.

62B.The method of embodiment 61 B wherein the subject's immunosuppression condition is ameliorated or the unwanted immune response is reduced.

63B.The method of embodiment 62B wherein the subject's innate immunity, specific immunity or both is enhanced or wherein the subject's Th1 immune response is enhanced or the subject's Th2 immune response is decreased.

64B.The method of embodiment 8B wherein the variations of steps (a), (b) and (c) comprise conducting a first dosing regimen of steps (a), (b) and (c) once, twice or three times, followed by one or more second dosing regimens of steps (a'), (b') and (c') wherein one or more of the (a'), (b') and (c') steps in the second dosing regimen is longer than the corresponding step in the first dosing regimen.

65B.The method of embodiment 8B wherein the variations of steps (a), (b) and (c) comprise conducting a first dosing regimen of steps (a), (b) and (c) once, twice or three times, followed by one or more second dosing regimens of steps (a'), (b') and (c') wherein one or more of the (a'), (b') and (c') steps in the second dosing regimen is shorter than the corresponding step in the first dosing regimen.

66B.The method of any of embodiments 1B-67B wherein the one or more formula 1 compounds is or are administered orally, intramuscularly, intravenously, subcutaneously, topically, vaginally, rectally, intracranially, intrathecally, intradermally, as an aerosol or by a buccal route.

67B.The method of embodiment 66B wherein the one or more formula 1 compounds is or are present in a solid formulation predominantly as a solid or the one or more formula 1 compounds is or are present in a liquid formulation predominantly as a solvate, colloid or a suspension or the one or more formula 1 compounds is or are present in a gel, cream or paste.

68B.The method of any of embodiments 2B-67B wherein the subject's viral infection, intracellular bacterial infection, extracellular bacterial infection, fungal infection, yeast infection, extracellular parasite infection, intracellular parasite infection, protozoan parasite, multicellular parasite, autoimmune disease, cancer, precancer, chemotherapy, radiation therapy, immunosuppressive therapy, anti-infective agent therapy, a wound, a burn, or the presence of an immunosuppressive molecule, gastrointestinal irritation or an inflammation condition optionally selected from or associated with irritable bowel disease, Crohn's disease or chronic diarrhea, or any combination of the foregoing is (a) a DNA virus infection or an RNA virus infection (HSV, CMV, HBV, HCV, HIV, SHIV, SIV); (b) a Mycoplasma infection, a *Listeria* infection or a *Mycobacterium* infection; (c) extracellular bacteria infection; (d) fungal infection; (e) a yeast infection (*Candida, Cryptococcus*); (d) protozoa (malaria, Leishmania, Cryptosporidium, Toxoplasmosis, Pneumocystis); (e) a multicellular parasite; (f) autoimmune diseases (SLE, RA, diabetes); (g) cancers (solid cancers selected from, e.g., ovarian, breast, prostate, glioma; disseminated cancers selected from lymphoma, leukemia, colon cancer, sarcoma); (h) precancers; (i) chemotherapies (adriamycin, cisplatin, mitomycin C); (j) radiation therapies; (k) immunosuppressive therapies; (I) anti-infective agent therapies; (m) wounds (surgical or otherwise); (n) 1^{st} degree, 2^{nd} degree or 3^{rd} degree burns; (o) immunosuppressive molecules; (p) gastrointestinal irritation or an inflammation condition optionally selected from or associated with irritable bowel, Crohn's disease, chronic diarrhea; or (q) any combination of (a) through (p).

69B.The method of embodiment 68B wherein the RNA virus infection is a retroviral infection or a hepatitis virus infection.

70B.The method of embodiment 68B or 69B wherein the one or more formula 1 compounds is one formula 1 compound.

71 B. The method of embodiment 70B wherein the one or more formula 1 compounds is or are in a composition that comprises, (a) one or more nonaqueous liquid excipients, wherein the composition comprises less than about 3% v/v water; (b) a solid that comprises a pharmaceutically acceptable excipient; or (c) one or more liquid excipients, wherein the composition comprises more than about 3% v/v water.

72B. The method of embodiment 68B or 71 B wherein the formula 1 compound is 16α-bromo-3β-hydroxy-5α-androstan-17-one, 16α-bromo-3β,7β-dihydroxy-5α-androstan-17-one, 16α-bromo-3β,7β,17β-trihydroxy-5α-androstene, 16α-bromo-3β,7β-dihydroxy-5α-androstane, 16α-bromo-3β,7β-dihydroxy-5α-androstene, 16α-bromo-3β,7β,17β-trihydroxy-5α-androstane, 16β-bromo-3β,17β-dihydroxy-5α-androstane, 16β-bromo-3β,17β-dihydroxy-5α-androstene, 16β-bromo-3β,7β,17β-trihydroxy-5α-androstane, 16β-bromo-3β-hydroxy-5α-androstan-17-one, 16β-bromo-3β-hydroxy-5α-androsten-17-one, 16β-bromo-3β,7β,-dihydroxy-5α-androstan-17-one or 16β-bromo-3β,7β,-dihydroxy-5α-androsten-17-one.

73B.The method of embodiment 72B wherein the formula 1 compound is 16α-bromo-3β-hydroxy-5α-androstan-17-one.

74B.The method of embodiments 1 B-73B wherein the formula 1 compound excludes one or more of any formula 1 compounds.

75B.A method to treat involuntary weight loss, oral lesions, skin lesions, opportunistic infections, diarrhea or fatigue in an subject comprising intermittently administering one or more compounds of formula 1 to the subject (e.g., involuntary weight loss from viral infection, gastrointestinal infection, chemotherapy, anorexia, gastrointestinal irritation or an inflammation condition optionally selected from or associated with irritable bowel, Crohn's disease, chronic diarrhea).

76B.The method of embodiment 75B wherein the subject has an immunosuppression condition.

77B.The method of embodiment 76B wherein the subject is a human.

78B.The method of embodiment 77B wherein the subject is a human 1 day to 18 years old (e.g., 1 month to 6 years old).

79B.The method of any of embodiments 75B-78B wherein the subject's specific immunity remains impaired compared to a typical comparable control subject who does not have the subject's pathological condition.

80B.The method of embodiment 79B wherein the subject's CD4 cell count does not increase significantly during one or more courses of dosing (e.g., dosing for 1 week to about 2 weeks or more).

81 B. The method of clam 80B wherein the subject's CD4 cell count is about 20 to about 100 CD4⁺ cells/mm³ or about 20 to about 75 CD4⁺ cells/mm³.

82B.The method of any of embodiments 1 B-81 B wherein the subject has a pathogen(s) infection or a malignancy and the pathogen(s) or malignancy does not become resistant to the formula 1 compound over a time normally associated with the development of measurable resistance in at least about 50% of subjects who are treated with a therapeutic treatment(s) other than a formula 1 compound(s).

83B.The method of embodiment 82B wherein the pathogen infection is an HIV, SIV, SHIV or HCV infection.

84B. The method of any of embodiments 1B-83B or 85B-88B wherein the formula 1 compound is a compound named in any of compound groups 1 through 54-53-52-51a6-50c27-49c27-48-47-46-45-44-43-42-41-40-39-38-37-36-35-34-33-32-31-30-29-28-27-39-38-37-36-35-34-33-32-31-30-29-28-27-26-25-23-21-17-10-8-6, or the formula 1 compound is a species in any genus described in any of compound groups 1 through 54-53-52-51 a6-50c27-49c27-48-47-46-45-44-43-42-41-40-39-38-37-36-35-34-33-32-31-30-29-28-27-39-38-37-36-35-34-33-32-31-30-29-28-27-26-25-23-21-17-10-8-6.

85B.A method to treat, ameliorate, prevent or slow the progression of an unwanted condition or symptom associated with the presence of a natural or synthetic glucocorticosteroid ('GCS') in a subject comprising administering to the subject, or delivering to the subject's tissues, an effective amount of a formula 1 compound, whereby the condition or symptom is detectably treated, ameliorated, prevented or its progression is detectably slowed.

86B.The method of embodiment 87B wherein the unwanted condition or symptom associated with the presence of the natural or synthetic GCS is an immune suppression disease, condition or symptom.

87B.The method of embodiment 88B wherein the immune suppression disease, condition or symptom is associated with a pathogen infection, a cancer or precancer, aging, trauma (stress, burn, surgery, accidental tissue injury) or inflammation.

88B.The method of embodiment 89B wherein the chemotherapy comprises treatment of the subject with a GCS.

In other embodiments, the invention provides methods to modulate immune cells or immune responses in a subject. The following numbered embodiments describe some of these methods.

1C. A method to modulate a subject's innate immunity or to enhance a subject's Th1 immune response or to reduce a subject's Th2 immune responses comprising administering to the subject a compound(s) of formula 1, or a metabolic precursor or a biologically active metabolite thereof, including any formula 1 compound that is described or disclosed herein.

2C. The method of embodiment 1C wherein the subject's innate immunity is enhanced.

3C. The method of embodiment 1C or 2C wherein the subject suffers from an innate immunity suppression condition, a suppressed Th1 immune response or an unwanted Th2 immune response.

4C. The method of embodiment 3C wherein the innate immunity suppression condition, the suppressed Th1 immune response or the unwanted Th2 response is associated with a viral infection, an intracellular bacterial infection, an extracellular bacterial infection, a fungal infection, a yeast infection, an extracellular parasite infection, an intracellular parasite infection, a protozoan parasite, a multicellular parasite, an autoimmune disease, a cancer, a precancer, a chemotherapy, a radiation therapy, an immunosuppressive therapy, an anti-infective agent therapy, a wound, a burn, the presence of an immunosuppressive molecule or any combination of the foregoing.

5C. The method of any of embodiments 1C-3C wherein the subject's Th1 immune response is enhanced.

6C. The method of embodiment 1C wherein the subject's Th2 immune response is reduced.

7C. The method of embodiment 6C wherein the subject has a condition comprising an unwanted immune response (e.g., autoimmune disease, SLE, diabetes).

9C. The method of embodiment 6C or 7C wherein the subject is a vertebrate, a mammal, a primate or a human.

10C.The method of embodiment 9 wherein the vertebrate's, the mammal's the primate's or the human's specific immunity modulation is (i) an enhanced CTL or Th1 response to a virus infection or to a malignant cell *in vitro* or *in vivo*, (ii) enhanced antigen presentation or biological activity by dendritic cells or dendritic cell precursors, or (iii) enhanced killing of virus-infected or of malignant cells.

11C.The method of 10C wherein the vertebrate is a human, the virus infection is an HIV infection and the CTL or Th1 response comprises an enhanced response to one or more of the HIV's gag protein or to the HIV's gp120.

12C. The method of embodiment 1C, 4C, 10C or 11C wherein the subject's Th1 cells, tumor-infiltrating lymphocytes (TIL cells), NK cells, peripheral blood lymphocytes, phagocytes, monocytes, macrophage, neutrophils, eosinophils, dendritic cells or fibrocytes are activated as measured by, e.g., enhanced ³H-thymidine uptake compared to untreated controls or by an increase in the number of the cell type in circulation or demonstrable movement of the cell type from one tissue or compartment (e.g., skin) to another tissue or compartment (e.g., blood, lymph node, spleen or thymus).

13C.The method of embodiment 1C, 4C, 10C, 11C or 12C, wherein the formula 1 compound(s) modulates transcription of one or more genes in the subject's NK cells, phagocytes, monocytes, macrophages, neutrophils, eosinophils, dendritic cells or fibrocytes are detectably activated (e.g., as measured by increased protein kinase C activity or by modulation of a biological activity of a steroid receptor or an orphan nuclear hormone receptor).

14C.The method of embodiment 1C wherein the formula 1 compound(s) enhances lysosome movement in one or more of the subject's NK cells, phagocytes, monocytes, macrophages, neutrophils, eosinophils, dendritic cells or fibrocytes.

15C.The method of embodiment 1C wherein the formula 1 compound(s) enhances protein kinase C activity in one or more of the subject's NK cells, phagocytes, monocytes, macrophages, neutrophils, eosinophils, dendritic cells or fibrocytes (e.g., PKCα, PKCβ, PKCγ and PKCζ).

16C.A composition comprising a partially purified or a purified complex comprising a formula 1 compound and a steroid receptor, a serum steroid-binding protein (e.g., human serum albumin, α1-acid glycoprotein, sex hormone-binding globulin, testosterone-binding globulin, corticosteroid-binding globulin, androgen binding protein (rat)) or a binding partner (e.g., complexing agent, liposome, antibody).

17C.A product produced by the process of contacting the partially purified or the purified composition of embodiment 16C with one or more sterile containers, one or more syringes, one or more pharmaceutically acceptable excipients (e.g., excipient as defined in draft spec above and including sugars, lactose, sucrose, fillers, lubricants, binders, or any excipient named in any reference cited herein), one or more cells, one or more tissues, plasma or blood.

18C.The method of any of embodiments 1C-17C wherein the subject has an infection, a hyperproliferation disorder, a hypoproliferation condition, an immunosuppression condition, an unwanted immune response or wherein the subject has recently experienced or will shortly experience trauma, surgery or a therapeutic treatment wherein the therapeutic treatment is one other than the method of embodiment 1C.

19C.The method of embodiment 18C wherein the immunosuppression condition or the unwanted immune response is associated with a viral infection, an intracellular bacterial infection, an extracellular bacterial infection, a fungal infection, a yeast infection, an extracellular parasite infection, an intracellular parasite infection, a protozoan parasite, a multicellular parasite, an autoimmune disease, a cancer, a precancer, a chemotherapy, a radiation therapy, an immunosuppressive therapy, an anti-infective agent therapy, a wound, a burn, the presence of an immunosuppressive molecule, gastrointestinal irritation or an inflammation condition optionally selected from or associated with irritable bowel disease, Crohn's disease or chronic diarrhea, or any combination of the foregoing.

20C.The method of embodiment 19C wherein the subject's immunosuppression condition is ameliorated or the unwanted immune response is reduced.

21C.The method of embodiment 19C wherein the subject's immunosuppression condition is associated with a viral infection.

22C.The method of embodiment 21C wherein the viral infection comprises a DNA virus or an RNA virus infection.

23C.The method of embodiment 22C wherein the RNA virus infection comprises a retroviral infection or a hepatitis virus infection.

24C.The method of any of embodiments 18C-23C wherein the subject suffers from one or more of chronic diarrhea, involuntary weight loss (usually at least about 5% or more), cachexia (usually at least about 5% or more), muscle wasting, one or more oral lesions (usually at least about 1 cm²), one or more genital lesions (usually at least about 1 cm²), skin lesions (usually at least about 1 cm²) or an opportunistic infection associated with AIDS.

25C.A method (e.g., to determine a biological activity of a formula 1 compound or to modulate transcription of a gene in a cell or cell-free transcription system) comprising: (a) contacting the formula 1 compound(s) with a cell or cell population *in vitro* or *in vivo*; (b) measuring one or more of (i) a complex between a binding partner and the formula 1 compound, (ii) proliferation of the cell or cell population, (iii) differentiation of the cell or cell population (iv) an activity of a protein kinase C, (v) a level of phosphorylation of a protein kinase C substrate, (vi) transcription of one or more target genes, (vii) enhancement or inhibition of the cellular response to steroids, e.g., glucocorticoids, (viii) inhibition of steroid-induced transcription, e.g., glucocorticoids, sex steroids, (ix) inhibition of retrovirus (e.g., HIV, SIV, FIV or SHIV) LTR-driven transcription, or (x) modulation of the numbers of an immune cell population in circulation *in vivo* (e.g., circulating peripheral blood lymphocytes in a mammal such as a primate or a human); and (c) optionally comparing the result obtained in step (b) with an appropriate control.

26C.The method of embodiment 25C wherein the binding partner is a steroid receptor, a transcription factor or a steroid hormone superfamily orphan receptor.

27C.The method of embodiment 25C wherein the biological activity determined is a modulating activity of the formula 1 compound for replication or cytopathic effects associated with a retrovirus, a hepatitis virus or a protozoan parasite.

28C.The method of embodiment 25C wherein the biological activity determined is a modulating activity of the formula 1 compound for replication, cytopathic effects associated with the retrovirus, the hepatitis virus or the protozoan parasite or the biological activity determined is metabolism (assay by ³H-thymidine uptake) of a cell or cell population comprising NK cells, phagocytes, monocytes, macrophages, basophils, eosinophils, fibrocytes, transformed cells, virus-infected cells, bacteria-infected cells or parasite-infected cells.

29C.The method of embodiment 25C wherein the target gene is a virus gene, a bacterial gene, a parasite gene, a gene associated with cancer.

30C.The method of embodiment 29C wherein the virus gene is a polymerase gene, a reverse transcriptase gene, an envelope gene, a protease gene or a gene associated with viral nucleic acid replication or a viral structural gene.

31C.The method of embodiment 30C wherein the polymerase gene encodes a DNA polymerase or encodes an RNA polymerase.

32C.The method of embodiment 30C wherein the reverse transcriptase gene encodes a human, primate, avian or feline retrovirus reverse transcriptase.

33C.A method comprising administering a compound(s) of formula 1 to a human or primate who has a retroviral infection and a CD4 count of 550 or less.

34C.The method of embodiment 33C wherein the human has a CD4 count of about 20 to about 100 or about 20 to about 80.

35C.The method of embodiment 33C wherein the human has a CD4 count of about 30 to about 150.

36C.The method of embodiment 33C wherein the human has a CD4 count of about 500 or less, about 450 or less, about 400 or less, about 350 or less, about 300 or less, about 250 or less, about 200 or less, about 150 or less, about 100 or less, about 50 or less or about 25 or less or about 20 or less.

37C.The method of any of embodiments 33C-36C wherein the formula 1 compound(s) is present in a composition that comprises one or more nonaqueous liquid excipients and less than about 3% v/v water or any of the formulations as disclosed in the specification or any of the numbered embodiments above.

38C.The method of any of embodiments 33C-37C wherein the formula 1 compound(s) is administered according to an intermittent dosing protocol as disclosed in the specification or any of the numbered embodiments above.

39C.The method of any of embodiments 30C-45C wherein the human is coinfected with hepatitis C virus, hepatitis B virus, HSV-1, HSV-2, a malaria parasite, a *Pneumocystis* parasite, or a *Cryptosporidium* parasite.

40C.The method of embodiment 46C wherein level of the HCV is reduced in the human.

41C.A method comprising administering a formula 1 compound(s) to a subject, or to a nervous system cell(s) in tissue culture whereby the formula 1 compound(s) binds to a receptor associated with a cell(s) in the nervous system and (1) elicits a biological response in the cell(s) in the nervous system or in the cell(s) in tissue culture and/or (2) elicits a neuronal response that is transmitted to a distant site(s) or cell(s) where the method optionally is used to screen a formula 1 compound(s) for its biological activity, to treat a pathological condition (e.g., pathogen infection such as a virus (HIV), a malignancy or a neurological disorder, e.g., AIDS associated dementia, Alzheimer's, Parkinson's, Multiple Sclerosis) in the subject or to determine the bioavailability or metabolism of the formula 1 compound(s) to the subject or the cell(s) in the nervous system or in tissue culture, wherein metabolism is optionally determined by comparing the biological effect of a formula 1 compound(s) with a control compound, which can be a different formula 1 compound.

42C.The method of embodiment 41 wherein the receptor associated with the cell in the nervous system is a neurotransmitter receptor(s) (e.g., a γ-aminobutyric acid receptor such as type A, a NMDA receptor) and/or a steroid receptor (e.g., androgen receptor, estrogen receptor).

43C.The method of embodiment 41C or 42C wherein the cell(s) in the nervous system is a neuron(s), and astrocyte(s) and/or a glial cell(s).

44C.The method of embodiment 41C, 42C or 43C wherein the biological response in the cell(s) in the nervous system or in the cell(s) in tissue culture is increased or decreased transcription of a gene(s) (e.g., a neurotransmitter, vasopressin, a heat shock protein), increased or decreased secretion of a protein(s) (e.g., vasopressin), reduced damage from oxidative stress, enhanced nitric oxide release and/or enhanced neurite growth.

45C.The method of any of embodiments 1C-44C wherein the compound(s) of formula 1 is any one or more formula 1 compound selected from the compounds or one or more of the species of compounds within the genera named in compound groups 1 through 21-10-6.

46C.A method to (a) modulate (detectably increase or decrease) the expression of at least one immune cell antigen by an immune cell in a subject, wherein the immune cell antigen is selected from CD3, CD11c, CD14, CD16, CD19, CD25, CD36, CD38, CD56, CD62L, CD69, CD45RA, CD45RO, CD123, HLA-DR, IL-1, IL-2, IL-4, IL-6, IL-8, IL-10, IL-12, TNFα, IGF₁ and γIFN, or (b) activate CD₈⁺ T cells or CD8- T cells in a subject, wherein the activation comprises at least transiently enhanced expression of CD25 or CD69 by the T cells, or (c) increase the proportion of CD8⁺ or CD8- lymphokine activated killer cells in a subject's CD16+ cells (e.g., CD8⁺, CD16⁺, CD38⁺ or cells CD8⁻, CD16⁺, CD38⁺), or (d) increase the proportion of (i) CD8⁻, CD16⁺ natural killer cells, (ii) CD8⁺, CD16⁺ natural killer cells or (iii) CD8⁻, CD16⁺ cells that mediate antibody-dependent cell-mediated cytotoxicity, or (iv) CD8⁺, CD16⁺ cells that mediate antibody-dependent cell-mediated cytotoxicity, or (e) increase the proportion of dendritic cell precursors in a subject's circulating white blood cells (e.g., Lin⁻, HLA-DR⁺, CD123⁺ or Lin- HLA-DR⁺, CD11c⁺ cells) or (f) increase the proportion of CD45RA⁺ T cells or CD45⁺, R0⁺ T cells in a subject's circulating white blood cells, or (g) change (increase or decrease) the proportion or relative numbers of CD62L⁺ T cells in a subject's circulating white blood cells, or (h) increase the proportion of CD8⁺ or CD4⁺ T cells that express CD62L in a subject's circulating CD8⁺ or CD4⁺ T cells, or (i) decrease the proportion of CD8⁺ or CD4⁺ T cells that express CD62L in a subject's circulating CD8⁺ or CD4⁺ T cells, or (j) increase the proportion of HLA-DR⁺, CD8⁺, CD38⁺ cells in a subject's circulating white blood cells, or (k) decrease the level of IL-4 or IL-10 that is expressed by or present in a subject's white blood cells or in a subject's plasma (or that is expressed after the subject's white cells are stimulated *in vitro*), (I) at least transiently increase the number of dendritic cell precursors or dendritic cells that are present in a subject's white blood cells or in a subject's plasma, or (m) enhance the capacity of an immune cell, e.g., macrophages, CD4⁺ T cells, CD8⁺ T cells to express IL-2, IL-12 or γIFN or to activate such cells, the method comprising administering to the subject an effective amount of a formula 1 compound, which is optionally present in a composition comprising a pharmaceutically acceptable excipient.

47C.The method of embodiment 46C wherein the formula 1 compound is administered to the subject daily over a period from one to about 15 days, e.g., for 1, 2, 3, 4, 5, 6, 7, 8, 9, 10, 11, 12, 13, 14, 15 or more days.

48C.The method of embodiment 46C or 47C wherein the expression of the immune cell antigen is detectable at least about 8-90 days after the last administration of the formula 1 compound, e.g., for at least about 8, 10, 12, 15, 20, 25, 28, 30, 35, 40, 42, 45, 49, 50, 55, 56, 60, 63, 65, 70, 75, 77, 80, 84, 85, 90, 91 95, 98, 100 or more days.

49C.The method of any of embodiments 46C-48C wherein the subject has an immunosuppression condition, a pathogen infection or a conditions associated with a deficient Th1 immune response or an excessive Th2 immune response.

50C.The method of embodiment 49C wherein the pathogen infection is a viral infection, a bacterial infection, a yeast infection, a fungal infection or a viroid infection, e.g., wherein the pathogen infection is a viral infection such as a DNA virus infection or an RNA virus infection (e.g., an infection caused by a Hepadnavirus, a Parvovirus, a Papovavirus, an Adenovirus, a Herpesvirus, Retrovirus, a Flavivirus, a Togavirus, a Rhabdovirus, a Picornavirus, a Bunyavirus, a Reovirus, an Orthomyxovirus or a Paramyxovirus, such as a HIV1, HIV2, SIV, SHIV or another virus described herein or in the cited references).

51C.The method of embodiment 50C wherein the subject has an immunosuppression condition that is associated with or caused by a pathogen infection.

52C.The method of any of embodiments 46C-51C wherein the subject is a mammal, a human, a primate or a rodent.

53C.The method of any of embodiments 46C-52C wherein about 0.05 mg/kg/day to about 20 mg/kg/day is administered parenterally (e.g., by intravenous, subcutaneous, intramuscular, or intramedullary injection), topically, orally, sublingually or bucally to the subject, e.g., about 0.1 mg/kg/day, about 0.2 mg/kg/day, about 0.5 mg/kg/day, about 1.0 mg/kg/day, about 1.5 mg/kg/day, about 2 mg/kg/day, about 2.5 mg/kg/day, about 3.0 mg/kg/day, about 4 mg/kg/day or about 6 mg/kg/day, i.e., about 0.1-10 mg/kg/day, typically about 0.2-7 mg/kg/day.

54C.A method to detect a biological response associated with the administration of a compound of formula 1 to a subject comprising (1) obtaining a sample from the subject, (2) administering the compound of formula 1 to the subject to obtain a treated subject (3) obtaining a second sample from the treated subject, (4) within 24 hours of obtaining the sample, analyzing the sample to obtain control information for detecting the biological response, (5) within 24 hours of obtaining the second sample, analyzing the second sample for the presence or absence of a biological response to obtain experimental information and (6) optionally comparing the control information with the experimental information to detect the presence, absence, relative magnitude or absolute magnitude of the biological response

55C.The method of embodiment 54C wherein the compound of formula 1 further comprises a pharmaceutically acceptable carrier.

56C.The method of embodiment 54C or 55C wherein the biological response associated with the administration of the compound of formula 1 to the subject is modulation of the expression of a cell surface antigen, an increased absolute or relative number of cells in an immune cell subset, a decreased absolute or relative number of cells in an immune cell subset or an unchanged absolute or relative number of cells in an immune cell subset.

57C.The method of embodiment 56C wherein the immune cell subset is CD8⁺ T cells, CD4⁺ T cells, CD8⁺ lymphokine activated killer cells, CD8⁻, CD16⁺ natural killer cells, circulating dendritic cell precursors, circulating dendritic cells, tissue dendritic cell precursors, tissue dendritic cells, CD8⁺ lymphokine activated killer cells, CD8- lymphokine activated killer cells, CD8⁻, CD16⁺ natural killer cells, CD8⁺, CD16⁺ natural killer cells, CD8⁻, CD16⁺ cells that mediate antibody-dependent cell-mediated cytotoxicity, CD8⁺, CD16⁺ cells that mediate antibody-dependent cell-mediated cytotoxicity, CD45RA⁺ T cells, CD45RA⁺, CD45RO⁺ T cells, CD45RO⁺ T cells, CD8⁺, CD62L T cells, CD4⁺, CD62L⁺ T cells or HLA-DR⁺, CD8⁺, CD38⁺ T cells, monocytes or macrophages.

58C.The method of embodiment 57C wherein the biological response is at least transient modulation of an immune cell antigen or an immune accessory cell antigen (e.g., an adhesion molecule at the surface of endothelial cells or a cytokine receptor at the surface of T cells ot B cells).

59C.The method of embodiment 58C wherein the immune cell antigen is a protein, glycoprotein or cell surface antigen usually or only expressed by lymphoid cells (lymphocytes or white blood cells or their precursors, e.g., T cells, B cells, monocytes, macrophage, LAK cells, NK cells, dendritic cells).

60C.The method of embodiment 59C wherein the immune cell antigen is a CD molecule, an interleukin or a cytokine, optionally selected from CD16, CD25, CD38, CD62L, CD69, CD45RA, CD45RO, IL-1, IL-2, IL-4, IL-6, IL-8, IL-10, IL-12, IL-13, IL-15, IL-17, IL-18, TNFα, IGF₁ and γIFN.

61C.The method of any of embodiments 46C-60C wherein the subject is a human, a primate, a canine, a feline or a rodent.

62C.A method to alter the Th1-Th2 balance in a subject comprising administering an effective amount a compound of formula 1 to a subject whereby the subject's expression or secretion of IL-4 or IL-10 is detectably modulated.

63C.The method of embodiment 62C wherein the subject's expression or secretion of IL-4 or IL-10 is decreased and the Th1-Th2 balance in the subject's Th1 immune responses to an infection or immunosuppression condition is detectably enhanced.

71C. The method of any of embodiments 1C-63C, wherein the formula 1 compound is a compound named in any of compound groups 1 through 54-53-52-51a6-50c27-49c27-48-47-46-45-44-43-42-41-40-39-38-37-36-35-34-33-32-31-30-29-28-27-39-38-37-36-35-34-33-32-31-30-29-28-27-26-25-23-21-17-10-8-6, or the formula 1 compound is a species in any genus described in any of compound groups 1 through 54-53-52-51 a6-50c27-49c27-48-47-46-45-44-43-42-41-40-39-38-37-36-35-34-33-32-31-30-29-28-27-39-38-37-36-35-34-33-32-31-30-29-28-27-26-25-23-21-17-10-8-6.

Invention embodiments include stimulation of hemopoiesis in a subject. The following embodiments exemplify various aspects if these embodiments

1 D. A method to enhance hemopoiesis in a subject in need thereof comprising administering to the subject, or delivering to the subject's tissues, an effective amount of a compound of formula 1

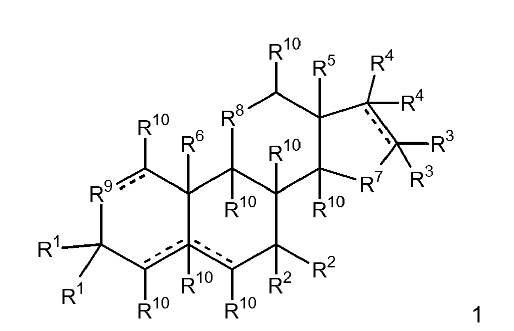

wherein, each R¹, R², R³, R⁴, R⁵, R⁶ and R¹⁰ independently are -H, -OR^{PR}, -SR^{PR}, - N(R^{PR})₂, -O-Si-(R¹³)₃, -CHO, -CHS, -CH=NH, -CN, -SCN, -NO₂, -OSO₃H, -OPO₃H, an ester, a thioester, a thionoester, a phosphoester, a phosphothioester, a phosphonoester, a phosphiniester, a sulfite ester, a sulfate ester, an amide, an amino acid, a peptide, an ether, a thioether, an acyl group, a thioacyl group, a carbonate, a carbamate, a thioacetal, a halogen, an optionally substituted alkyl group, an optionally substituted alkenyl group, an optionally substituted alkynyl group, an optionally substituted aryl moiety, an optionally substituted heteroaryl moiety, an optionally substituted heterocycle, an optionally substituted monosaccharide, an optionally substituted oligosaccharide, a nucleoside, a nucleotide, an oligonucleotide, a polymer, or,

one more of R¹, R², R³, R⁴, R⁵, R⁶, R¹⁰, R¹⁵, R¹⁷ and R¹⁸ are =O, =S , =N-OH or =CH₂ and the hydrogen atom or the second variable group that is bonded to the same carbon atom is absent, or,

all R³ and R⁴ together comprise a structure of formula 2

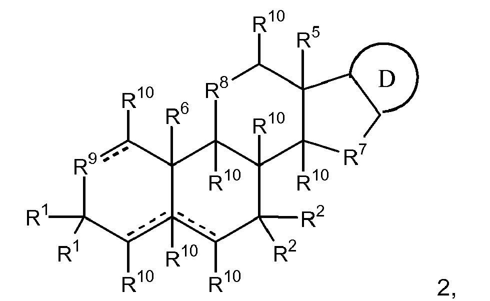

R⁷ is -C(R¹⁰)₂-, -C(R¹⁰)₂-C(R¹⁰)₂-, -C(R¹⁰)₂-C(R¹⁰)₂-C(R¹⁰)₂-, -C(R¹⁰)₂-O-C(R¹⁰)₂-, -C(R¹⁰)₂-S-C(R¹⁰)₂-, -C(R¹⁰)₂-NR^{PR}-C(R¹⁰)₂-, -O-, -O-C(R¹⁰)₂-, -S-, -S-C(R¹⁰)₂-, -NR^{PR}- or-NR^{PR}-C(R¹⁰)₂-, including -CHR¹⁰-, -CHR¹⁰-CHR¹⁰-, -CHR¹⁰-CHR¹⁰-CHR¹⁰-, -CHR¹⁰-O-CHR¹⁰-, -CHR¹⁰-S-CHR¹⁰-, -CHR¹⁰-NR^{PR}-CHR¹⁰-, -O-, -O-CHR¹⁰-, -S-, -S-CHR¹⁰-, -NR^{PR}- or -NR^{PR}-CHR¹⁰-

R⁸ and R⁹ independently are -C(R¹⁰)₂-, -C(R¹⁰)₂-C(R¹⁰)₂-, -O-, -O-C(R¹⁰)₂-, -S-, -S-C(R¹⁰)₂-, -NR^{PR}- or -NR^{PR}-C(R¹⁰)₂-, including -CHR¹⁰-, -CHR¹⁰-CHR¹⁰-, -O-, -O-CHR¹⁰-, -S-, -S-CHR¹⁰-, -NR^{PR}- or -NR^{PR}-CHR¹⁰-, or one or both of R⁸ or R⁹ independently are absent, leaving a 5-membered ring;

R¹³ independently is C₁₋₆ alkyl;

R¹⁶ independently are -CH₂-, -O-, -S- or -NH-;

D is a heterocycle or a 4-, 5-, 6- or 7-membered ring that comprises saturated carbon atoms, wherein 1, 2 or 3 ring carbon atoms of the 4-, 5-, 6- or 7-membered ring are optionally independently substituted with -O-, -S- or -NR^{PR}- or where 1, 2 or 3 hydrogen atoms of the heterocycle or where 1 or 2 hydrogen atoms of the 4-, 5-, 6- or 7-membered ring are substituted with -OR^{PR}, -SR^{PR}, -N(R^{PR})₂, -O-Si-(R¹³)₃, -CN, -NO₂, an ester, a thioester, a thionoester, a phosphoester, a phosphothioester, a sulfite ester, a sulfate ester, an amide, an amino acid, a peptide, an ether, a thioether, an acyl group, a thioacyl group, a carbonate, a carbamate, a thioacetal, a halogen, an optionally substituted alkyl group, an optionally substituted alkenyl group, an optionally substituted alkynyl group, an optionally substituted aryl moiety, an optionally substituted heteroaryl moiety, an optionally substituted monosaccharide, an optionally substituted oligosaccharide, a nucleoside, a nucleotide, an oligonucleotide or a polymer, or,

one more of the ring carbons are substituted with =O or =S,

or D comprises two 5- or 6-membered rings, wherein the rings are fused or are linked by 1 or 2 bonds, or a metabolic precursor or a biologically active metabolite thereof, optionally provided that the compound is not 5-androstene-3β-ol-17-one, 5-androstene-3β,17β-diol, 5-androstene-3β,7β,17β-triol or a derivative of any of these three compounds that can convert to these compounds by hydrolysis

2D. The method of embodiment 1 D wherein the subject's circulating platelets, red cells, mature myelomonocytic cells, or their precursor cells, in circulation or in tissue is detectably increased.

3D. The method of embodiment 2D wherein the subject's circulating platelets are detectably increased.

4D. The method of embodiment 3D wherein the method optionally further comprises administration of an effective amount of G-CSF, GM-CSF, IL-3, IL-6, IL-11, erythropoietin or thrombopoietin.

5D. The method of embodiment 2D wherein the subject's circulating myelomonocytic cells are detectably increased.

6D. The method of embodiment 2D wherein the circulating myelomonocytic cells are neutrophils.

7D. The method of embodiment 2D wherein the method further comprises administration of an effective amount of G-CSF, GM-CSF, M-CSF, IL-3, IL-5 or IL-6.

8D. The method of embodiment 2D wherein the myelomonocytic cells are basophils, neutrophils or eosinophils.

9D. The method of embodiment 2D wherein the subject's circulating red cells are detectably increased.

10D.The method of embodiment 9D wherein the subject is has renal failure.

11D.The method of embodiment 9D wherein the method further comprises administration of an effective amount of G-CSF, GM-CSF, IL-3, IL-6 or erythropoietin.

12D.The method of embodiment 2D wherein the formula 1 compound is present in a composition comprising an acceptable carrier and the method optionally further comprises administration of a neutrophil or monocyte stimulator.

13D.The method of embodiment 12D wherein neutrophil or monocyte stimulator is a TNF, a lithium salt, deuterium oxide, levamisole, lactoferrin, thyroxine, triiodothyromine, anthrax toxin, ascorbic acid, I-palmitoyl-lysophosphatidic acid, a calcium ionophore, cytochalasin B, sodium butyrate, piracetamine, micronized L-arginine, hydroxyurea or a bacterial lipopolysaccharide.

14D.The method of embodiment 2D further comprising the steps of obtaining blood from the subject before administration of the formula 1 compound and measuring the subject's white or red cell counts and optionally, on one, two, three or more occasions, measuring the subject's circulating white cell counts after administration of the formula 1 compound.

15D.The method of embodiment 2D wherein the formula 1 compound is a compound named in any of the compound groups disclosed herein.

16D.The method of embodiment 1 D wherein the subject has, or is subject to developing, thrombocytopenia or neutropenia.

17D.The method of embodiment 16D wherein the subject has thrombocytopenia or neutropenia.

18D.The method of embodiment 1 D wherein about 0.05 mg to about 30 mg of the formula 1 compound is administered per kg of the subject's weight per day.

19D.The method of any of embodiments 1 D-18D wherein the compound of formula 1 has formula 3

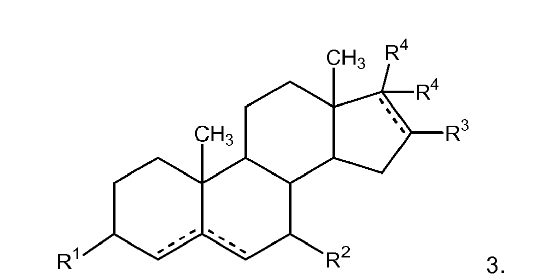

20D.The method of embodiment 19D wherein R¹ is -OH, alkoxy or an ester, R² is - OH, =O, alkoxy or an ester, R³ is -H, -OH, alkoxy, an ester or a halogen, one R⁴ is -H or it is absent and the other R⁴ is -OH, =O, -SH, -C(O)-CH₃, alkoxy or an ester and wherein R¹, R² and R³ are independently in the α or the β configuration when they are not =O.

21 D.The method of embodiment 20D wherein there is no double bond present in the molecule.

22D.A method to enhance thrombopoiesis, myelopoiesis or erythropoiesis in a subject comprising administering to the subject, or delivering to the subject's tissues, an effective amount of the compound of embodiment 1.

23D.The method of embodiment 22D wherein the subject has or is subject to thrombocytopenia or neutropenia.

24D.The method of embodiment 23D wherein the subject has thrombocytopenia or neutropenia.

25D.The method of embodiment 22D wherein the subject is a human.

26D.The method of embodiment 22D wherein the formula 1 compound is a compound named in any of the compound groups disclosed herein.

27D.The method of embodiment 22D wherein about 0.05 mg to about 30 mg of the formula 1 compound is administered per kg of the subject's weight per day.

28D.The method of embodiment 27D wherein the formula 1 compound is present in a composition that comprises a pharmaceutically acceptable carrier.

29D.The method of embodiment 28D wherein the pharmaceutically acceptable carrier is deuterium oxide, which comprises at least about 20% v/v of the water in the composition.

30D.The method of embodiment 19D wherein the subject's myeloperoxidase index is enhanced.

31 D.The method of embodiment 30D wherein the formula 1 compound is present in a composition that comprises one or more pharmaceutically acceptable carriers, which optionally include a halogen salt.

32D.A method to treat a blood cell deficiency in a subject comprising administering to the subject, or delivering to the subject's tissues, an effective amount of a compound of formula 1.

33D.The method of embodiment 32D wherein the formula 1 compound has the structure

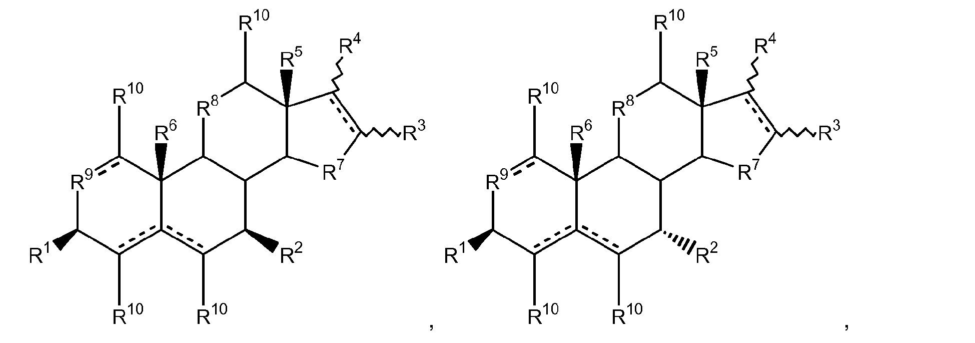

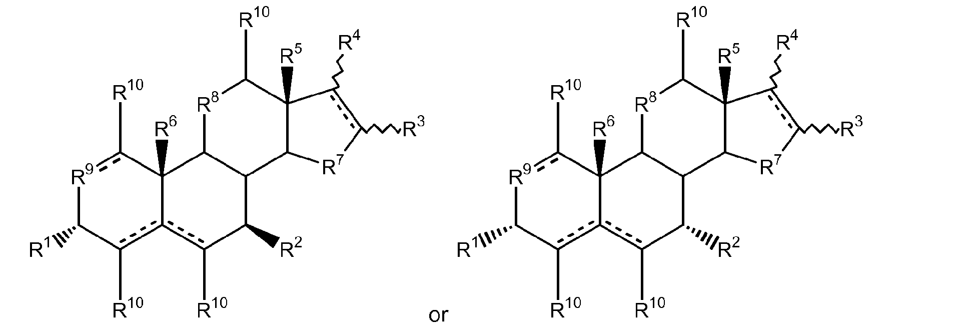

wherein one, two or three of R⁷, R⁸ and R⁹ are -CH₂- or -CH= and wherein the configuration of hydrogen atoms at the 5 (if present), 8, 9 and 14 positions respectively are α.α.α.α, α.α.α.β, α.α.β.α, α.β.α.α, β.α.α.α, α.α.β.β, α.β.α.β, β.α.α.β, β.α.β.α, β.β.α.α, α.β.β.α, α.β.β.β, β.α.β.β, β.β.α.β, β.β.β.α or β.β.β.β, typically α.α.β.α or β.α.β.α.

34D.The method of embodiment 32D wherein the formula 1 method has the structure 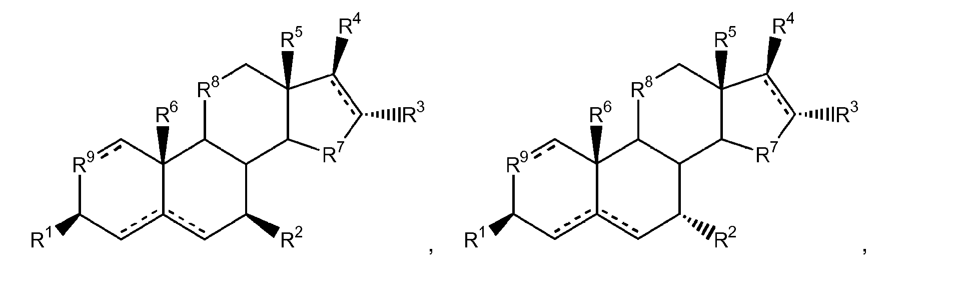 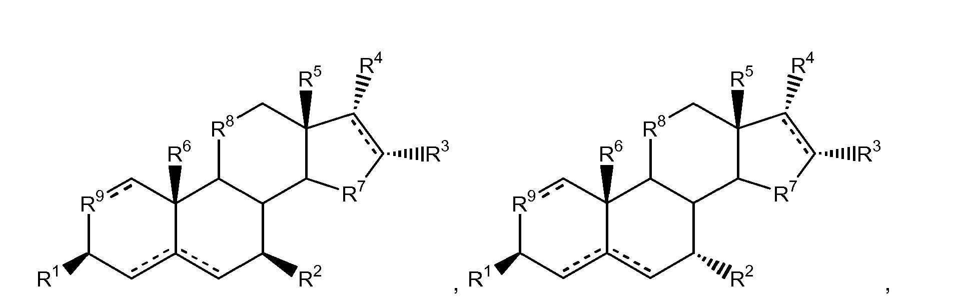 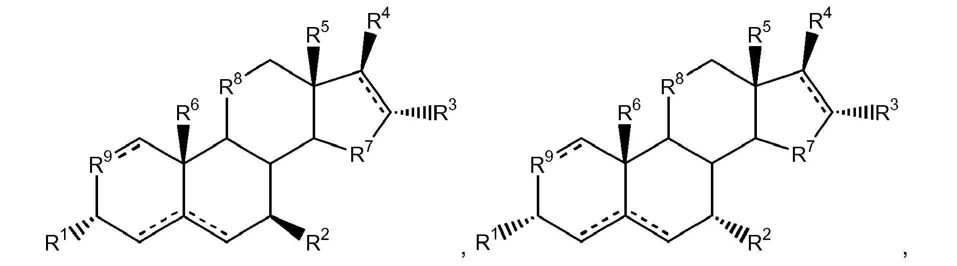 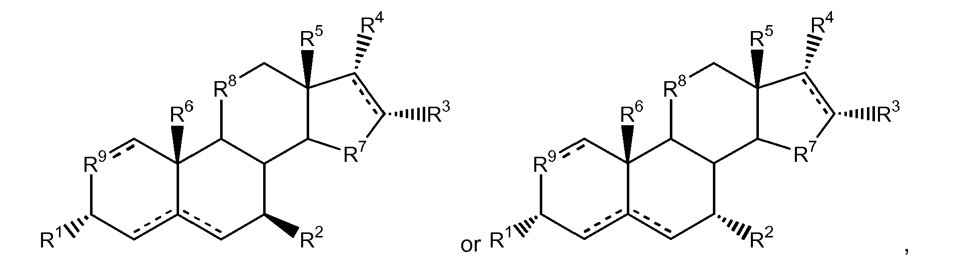 wherein independently selected R¹⁰ are present at the 1, 4, 6, and 12 positions, hydrogen atoms at the 5 (if present), 8, 9 and 14 positions respectively are in the α.α.α.α, α.α.α.β, α.α.β.α, α.β.α.α, β.α.α.α, α.α.β.β, α.β.α.β, β.α.α.β, β.α.β.α, β.β.α.α, α.β.β.α, α.β.β.β, β.α.β.β, β.β.α.β, β.β.β.α or β.β.β.β configurations, typically α.α.β.α or β.α.β.α, and the R¹⁰ are optionally selected from -H, -OH, =O, -F, -Cl, -Br, I, optionally substituted alkyl, e.g., -CH₃ or -C₂H₅, optionally substituted alkoxy and an ester.

35D.The method of embodiment 34D wherein R⁴ is -OH, =O, -SH, -SCN, a C₁₋₃₀ ester or C₁₋₃₀ alkoxy, wherein the ester or alkoxy moiety is optionally substituted with one, two or more independently selected substituents, which are optionally selected from -F, -Cl, - Br, -I, -O-, =O, -S-, -NH-, -R^{PR}, -OR^{PR}, -SR^{PR} or -NHR^{PR}.

36D.The method of embodiment 34D or 35D wherein R¹ is -OH, =O, -SH, -SCN, a C₁₋₃₀ ester or C₁₋₃₀ alkoxy, wherein the ester or alkoxy moiety is optionally substituted with one, two or more independently selected substituents, which are optionally selected from -F, -Cl, -Br, -I, -O-, =O, -S-, -NH-, -R^{PR}, -OR^{PR}, -SR^{PR} or -NHR^{PR}.

37D. Use of a compound of formula 1, e.g., a compound in any compound group or embodiment disclosed herein, to manufacture a medicament for the treatment of a blood cell deficiency, e.g., NP of TP, in a subject, e.g., a mammal or a human.

38D.A product produced by the process of contacting a formula 1 compound, e.g., a compound in any compound group or embodiment disclosed herein, and an excipient.

39D.A kit comprising a formulation that comprises a unit dosage or a multiple dosage comprising a formula 1 compound, e.g., a compound in any compound group or embodiment disclosed herein, and one or more excipients wherein the formulation is dispensed in a suitable container (e.g., a closed container), wherein the kit optionally further comprises a label that provides information about one or more of (1) the formula 1 compound's chemical structure, (2) any recommended dosing regimen, (3) any adverse effects of administering the formula 1 compound to a subject that are required to be disclosed and (4) the amount of the formula 1 compound that is present in each unit dose or in the entire container.

In other embodiments, the formula 1 compounds have the structures shown in embodiment 43D wherein hydrogen atoms at the 5 (if present), 8, 9 and 14 positions respectively are in the α.α.α.α, α.α.α.β, α.α.β.α, α.β.α.α, β.α.α.α, α.α.β.β, α.β.α.β, β.α.α.β, β.α.β.α, β.β.α.α, α.β.β.α, α.β.β.β, β.α.β.β, β.β.α.β, β.β.β.α or β.β.β.β configurations, typically α.α.β.α or β.α.β.α., R¹, R² and R³ independently are a moiety as defined herein such as -H, - OH, =O, an ester, an ether, a lipid moiety or -O-S(O)(O)-N(R³⁵)₂, where each R³⁵ independently is -H, optionally substituted alkyl (e.g., C1-8 alkyl, methyl, ethyl, propyl, butyl or hexyl optionally substituted with one or more -OH, =O or -O-), optionally substituted alkenyl, a heterocycle, phenyl or benzyl, R⁴ is a moiety as defined herein such as -H, -OH, =O, an ester, an ether, -C(O)-CH₂OH, -C(O)-CH₂-COOH, -C(O)-CH(O), -C(O)-CH₃, -C(O)-CH₂(halogen), -C(O)-CH₂F, -C(O)-CHF₂, -C(O)-CF₃, -C(O)-CH₂Cl, -C(O)-CHCl₂, -C(O)-CCl₃, -C(O)- CH₂CH₂OH, -C(O)- CH₂CH₃, -C(O)-CH₂CH₂F, -C(O)-CH₂CHF₂, -C(O)-CH₂CF₃, -C(O)-CH₂CH₂Cl, - C(O)-CH₂CHCl₂, -C(O)-CH₂CCl₃, -C(O)-O-CH₂OH, -C(O)-O-CH₃, -C(O)-O-CH₂(halogen), -C(O)-O-CH₂F, -C(O)-O-CHF₂, -C(O)-O-CF₃, -C(O)-O-CH₂Cl, -C(O)-CHCl₂, - C(O)-O-CCl₃, -C(O)-O-CH₂CH₂OH, -C(O)-O-CH₂CH₃, -C(O)-O-CH₂CH₂F, -C(O)-O-CH₂CHF₂, -C(O)-O-CH₂CF₃, -C(O)-O-CH₂CH₂Cl, - C(O)-O-CH₂CHCl₂, -C(O)-O-CH₂CCl₃, -CH(OH)-CH₂OH, -CH(OH)-CH(O), -CH(OH)-CH₃, -CH(OH)-CH₂(halogen), -CH(OH)-CH₂F, -CH(OH)-CHF₂, -CH(OH)-CF₃, -CH(OH)-CH₂Cl, -CH(OH)-CHCl₂, -CH(OH)-CCl₃, -CH(OH)-CH₂CH₂OH, -CH(OH)- CH₂CH₃, -CH(OH)-CH₂CH₂F, -CH(OH)-CH₂CHF₂, -CH(OH)-CH₂CF₃, -CH(OH)-CH₂CH₂Cl, - C(O)-CH₂CHCl₂, -CH(OH)-CH₂CCl₃, -CH(OH)-O-CH₂OH, -CH(OH)-O-CH₃, -CH(OH)-O-CH₂(halogen), -CH(OH)-O-CH₂F, -CH(OH)-O-CHF₂, -CH(OH)-O-CF₃, - CH(OH)-O-CH₂Cl, -CH(OH)-CHCl₂, -CH(OH)-O-CCl₃, -CH(OH)-O-CH₂CH₂OH, -CH(OH)-O-CH₂CH₃, -CH(OH)-O-CH₂CH₂F, -CH(OH)-O-CH₂CHF2_{,} -CH(OH)-O-CH₂CF₃, -CH(OH)-O-CH₂CH₂Cl, - C(O)-O-CH₂CHCl₂, -CH(OH)-O-CH₂CCl₃ or -CH(CH₃)CH₂CH₂CH(C₂H₅)-CH(CH₃)₃, R⁷ and R⁹ independently are a moiety as defined herein such as -CH₂-, -O-, - CHOH- with the -OH moiety in the α or β configuration or -C(O)-O and R⁸ is a moiety as defined herein such as -CH₂-, -O-, -CHOH- with the -OH moiety in the α or β configuration, - C(O)-O, -CH(hydroxyl ester, e.g., a C1-8 ester)-, -CH(alkyl, e.g., a C1-8 alkyl)-, -CH(alkenyl, e.g., C1-8)-, -CH(ether, e.g., C1-8) or -C(OH)₂-. Related formula 1 compounds are optionally substituted at the 3, 7 or 17 positions with a second R¹, R² or R⁴ that is not hydrogen, e.g., - CCH, halogen, -OH, alkyl such as methyl, ethyl or butyl or alkynyl such as ethynyl, 1-propynyl or 1-butynyl. For these compounds, double bonds may be absent, or they may be present at, e.g., the 1-2 and 4-5 positions or at a single position such as the 4-5 or the 5-6 positions. For some of these compounds, R⁵ and R⁶ independently are -H, -CH₃, -CH₂OH, -CH(O) or -C₂H₅. Also, 1, 2 or 3 of R¹⁰ at the 1, 4 or 6 positions may independently be a moiety other than hydrogen, e.g., alkyl such as methyl, a halogen =CH₂, -CN, -OH or an ester. All of these formula 1 compounds are suitable for the uses and compositions disclosed herein.

Variations and modifications of these embodiments, the claims and the remaining portions of this disclosure will be apparent to the skilled artisan after a reading thereof. Such variations and modifications are within the scope and spirit of this invention. All citations herein are incorporated herein by reference in their entirety. All citations herein are incorporated herein by reference with specificity.

Examples. The following examples further illustrate the invention and they are not intended to limit it in any way.

**Example 1.** 16α-bromoepiandrosterone (BrEA) formulation. Two lots of a non-aqueous BrEA formulation were made at a BrEA concentration of 50 mg/mL in 25% polyethylene glycol 300, 12.5% dehydrated ethyl alcohol, 5% benzyl benzoate, and 57.5% propylene glycol as follows. BrEA was obtained from Procyte, Inc. The remaining excipients are shown below.

| **Excipient** | **Specification** | **Supplier Lot No.** | **Final Product Concentration** |
|---|---|---|---|
| Propylene glycol | USP | Arco Chemical HOC-61220-01104 | 57.5% (v:v) |
| Polyethylene glycol 300 | NF | Union Carbide 695752 | 25% (v:v) |
| Dehydrated alcohol | USP | McCormick Distilling 97K10 | 12.5% (v:v) |
| Benzyl benzoate | USP | Spectrum Pharmaceuticals MG025 | 5% (v:v) |

The formulation was prepared by suspending BrEA in polyethylene glycol 300, and sequentially adding propylene glycol, benzyl benzoate, and dehydrated ethyl alcohol to form a solution, which was diluted to the final desired volume with additional propylene glycol. The procedure is described below.

The calculated amount of polyethylene glycol 300 was added to a compounding vessel. Then , while mixing, the calculated amount of BrEA was added to the vessel, and mixed for at least 5 minutes to form a smooth, creamy liquid propylene glycol was added to the vessel, and mixed for a minimum of 5 minutes to form a uniform suspension. The calculated amount of benzyl benzoate is added to the vessel, and mixed for approximately 5 minutes to form a translucent liquid suspension. Dehydrated alcohol was added to the vessel, and mixed for approximately 5 minutes to form a clear, colorless solution. Propylene glycol was then added to achieve the desired final formulation, and mixed for approximately 5 minutes. The drug solution was transferred to a volume dispensing device set to deliver 1.2 mL per vial. Under nitrogen pressure, the solution was filtered through two 0.2 µm polyvinylidene fluoride filters in series into 2 cc amber glass vials. The vials were capped with Teflon-coated, butyl-rubber stoppers and crimp sealed. Materials used in the product vials are listed below.

| **Material** | **Source** | **Product Code** | **Description** |
|---|---|---|---|
| Vial | Wheaton | 2702-B51BA | Tubing vial, 2 mL/13 mm, glass, type 1 amber |
| Stopper | Omniflex | V9239 FM257/2 | 13 mm, Teflon coated, butyl rubber stopper |
| Seal | West | 4107 | Flip seal, 13 mm, mist gray bridge |

Product specifications were examined by one or more of the following assays.

| **Test** | **Specification** | **Method** |
|---|---|---|
| Physical Examination | Clear colorless solution with slight alcoholic odor | |
| Volume recovery | NLT* 1.0 mL | USP23<1> |
| Specific gravity | TBD | USP23<841> |
| Assay for active component | 90-110% of label | HPLC |
| Sterility | sterile | USP23<71> |
| Endotoxin | <0.1 EU/mg | USP23<85> |
| Particulate matter | ≥ 10 µm NMT** 6000/cnt ≥ 25 µm NMT 600/cnt | USP23<788> |

| | | |
|---|---|---|
| * NLT- no less than **NMT - no more than | | |

| **Lot Analysis** | | | |
|---|---|---|---|
| **Test** | **Specification** | **Lot 1** | **Lot 2** |
| Physical Examination | Clear colorless solution with slight alcoholic odor | Positive | Positive |
| Volume recovery | NLT 1.0 mL | 1.15 mL | -- |
| Specific gravity | TBD | 1.0411 | -- |
| Assay for active component | 90-110% of label | 103.10 % | 104.25% |
| Sterility | sterile | sterile | -- |
| Endotoxin | <0.1 EU/mg | 0.024 EU/mg | -- |
| Particulate matter | ≥ 10 µm NMT 6000/cnt ≥ 25 µm NMT 600/cnt | 26 15 | -- |

**Example 3.** Primate intermittent dosing protocol. Pig-tail Macaque monkeys infected with the SHIV₂₂₉ retrovirus were treated with a BrEA formulation as described in example 1. SHIV₂₂₉ is a recombinant retrovirus containing HIV and SIV sequences. J. Thompson et al., abstract #75, 16th Annual Symposium on Nonhuman Primate Models for AIDS, October 7-10, 1998, Atlanta, GA, M. Agy et al., abstract #67, 16th Annual Symposium on Nonhuman Primate Models for AIDS, October 7-10, 1998, Atlanta, GA. In monkeys, it establishes an aggressive infection that leads to severe symptoms of end-stage disease in infected untreated animals at about 180-210 days after infection. Four pig-tail macaques (2/group) received subcutaneous injections of the formulation at 1 or 2 mg/kg body weight for 10 consecutive days (Protocol 1). On week 8, 3 of the 4 monkeys were retreated and 2 treatment naïve monkeys were treated with 5 mg/kg of the formulation on an every other day basis for a period of 20 days (Protocol 2). On week 19, all primates receiving treatment began a 3 course treatment regimen with 3 mg/kg of the BrEA formulation once daily for 10 consecutive days, repeated every four weeks for a total of 3 treatment courses (Protocol 3).

The animals were infected with 1-100 TCID₅₀ units administered intravenously or intrarectally. Viral titers in the first group of animals ranged from 10⁶ to 10⁸ before dosing began. All animals demonstrated an initial rise in plasma viral SHIV RNA. After a period of 2 to 3 weeks, titers began to decline and 3 of the 4 animals showed a response to therapy with average viral titers of 0.76 log below baseline at weeks 4 to 5 after initiation of treatment. By week 8, titers in all animals had returned to baseline values. Blood glucose levels dropped significantly, alkaline phosphatase levels were elevated and SGOT/GGT values trended towards the high end of normal. No other significant changes were observed in any of the parameters monitored. The CD4 levels in all monkeys remained less than 100 cells/mm³ at the end of the first protocol.

Three of the five monkeys on the second regimen (Protocol 2) responded to the BrEA therapy with a greater depth and duration of response than observed at the lower dose levels. In the responding animals, the average decline below baseline was 1.47 log. The non-responding animal from Protocol 1 responded when administered the BrEA formulation in Protocol 2. Two animals did not respond, one each from the treatment experienced and treatment naïve groups.

The monkeys on this study were salvaged from an infectivity study and the first cohort of four monkeys on study (Protocol 1) were expected to live only a few weeks past the initiation of these experiments as they were beginning to deteriorate due to disease related causes. One animal died at day 356 from a toxic reaction to the anesthetic used during acquisition of a blood sample for analysis. Survival was greater than 380 days from the time of infection. Treatment by intermittent dosing of the BrEA formulation was used. Three control monkeys were infected with 1-10,000 SHIV₂₂₉ TCID₅₀ units and did not receive treatment. These animals are considered the no treatment arm of a survival study. The mean time to death for pig-tailed macaques infected with SHIV₂₂₉ was 193 days. Monkeys receiving therapy remained in good clinical health for over 350 days with CD4 levels less than 20 cells/mm³ and without opportunistic infections or disease-related symptoms, other than a mild anemia in one animal.

These results show completely unexpected therapeutic responses by the primates infected with the SHIV retrovirus, which is quite virulent. The results show that the majority of subjects in these treatment protocols not only had significantly prolonged survival compared to untreated controls, but also the clinical symptoms associated with retroviral infection improved dramatically, despite the fact that CD4 counts remained low, i.e., less than about 100 CD4 cells/mm³ initially and less than about 20 CD4 cells/mm³ later in the treatment protocols. To date, results such as this, i.e., (1) good clinical health in a majority of subjects having low CD4 levels (less than about 150 cells/mm³, especially less than about 75 cells/mm³) and (2) no clinical sign of viral resistance to treatment despite intermittent dosing over a prolonged time period, are unprecedented in primates, humans or any other animal. The SHIV₂₂₉ model is extremely pathogenic in pig-tailed macaques. Events that occur in this model over several weeks would typically take several years in humans infected with HIV. Treatment of monkeys infected with this virus and treated with commonly used antiretrovirals, e.g., AZT, 3TC or a protease inhibitor, are not expected to significantly affect the course of disease progression. The clinical condition of the animals continues to improve, e.g., weight gain is about 8-15% per animal. These results show that the treatment using the intermittent dosing protocol is highly effective despite the apparent impairment of the subject's specific immunity, as shown by the low CD4 counts. Increased CD4 counts may be attained using immune stimulators such as IL2 or they may increase spontaneously in some subjects such as humans, depending on the treatment protocol, the duration of dosing or the subject's initial medical condition. The antiviral effects shown here appear to function at least in part by enhancing the subject's immune responses, e.g., enhanced immune response by phagocytic cells (NK cells, monocytes and/or macrophages), and/or enhancing any residual specific immune responses, if any, that the subject may be able to muster.

**Example 4.** Human treatment protocol. A dose escalation clinical trial is performed using a nonaqueous formulation containing BrEA or another formula 1 compound(s) that is prepared essentially as described in example 1. The patients are treatment naïve or treatment experienced and about 3-10 patients are examined at each dose level. The initial dose is 25 mg of BrEA or another formula 1 compound(s) that is administered parenterally , e.g., s.c. or i.m. The dose is administered once or once or twice per day for 1-12 days, followed by no dosing for at least 7 days (e.g., 7 to 90 days). Subsequent doses are administered once or once per day for 1-12 days, followed by no dosing for at least 7 days (e.g., 7 to 90 days). Other dose levels tested are 20 mg, 50 mg, 75 mg, 100 mg, 150 mg, 200 mg, 250 mg and 300 mg with each dose administered once per day as a single dose or as two, three or more subdivided doses. An efficacy dosing trial is performed using the same dosing protocol as the dose escalation trial or it may alternatively comprise dosing once or twice every other day for 3-17 days, followed by no dosing for 7-90 days and then repeating the dosing once or twice every other day for 3-17 days. This protocol is repeated indefinitely (e.g., at least about 3-18 months) using the optimal dose(s) obtained from the dose escalation trial, e.g., about 10-200 mg/day of a formula 1 compound.

**Example 27.** Inhibition of inflammation. The capacity of formula 1 compounds to limit or inhibit inflammation or symptoms of inflammation was shown using animal models for inflammatory bowel disease.

Groups of 3 male Wistar rats (180 ± 20 grams) fasted for 24 hours before 2,4-dinitrobenzene sulfonic acid (DNBS) or saline challenge were used. Distal colitis was induced by intra-colonic instillation of 0.5 mL of an ethanolic solution of DNBS (30 mg in 0.5 mL of a 30% ethanol in saline solution) after which 2 mL of air was injected through the cannula to ensure that the solution remained in the colon. The volume used was 0.1 mL per injection of 2 and 20 mg/mL of 7-oxodehydroepiandrosterone in a liquid formulation, was administered by subcutaneous injection once a day for 6 days. The formulation contained 100 mg/mL of 7-oxodehydroepiandrosterone in a non-aqueous suspension that contained 2% benzyl alcohol w/v, 0.1 % Brij 96 w/v and equal volumes of PEG 300 and propylene glycol. Concentrations of 2 mg/mL and 20 mg/mL were obtained by diluting the 20 mg/mL formulation with vehicle that lacked the active substance.

The first dose was given 30 minutes after DNBS challenge. Sulfasalazine (30 mg/mL in 2% Tween 80 in distilled water) was administered orally (PO) once a day (10 mL/kg/day) for 7 days, the first two doses beginning 24 hours and 2 hours before DNBS challenge. The presence of diarrhea was recorded daily by examining the anal area. Animals were fasted for 24 hours prior to being sacrificed. Animals were sacrificed on day 7 or day 8 and their colons were removed and weighed. Before removal of the colon, signs of adhesion between the colon and other organs were recorded. Also, the presence of ulcerations was noted after weighing of each colon. The "net" change of colon-to-body weight (BW) ratio was normalized relative to saline-challenged baseline group. A 30% decrease in "net" colon-to-body weight ratio was considered significant.

A total of five studies were conducted. Data from several studies are combined. The 7-oxodehydroepiandrosterone at 2 and 20 mg/mL, decreased the net colon-to-body weight ratio by 19 and by 14% relative to vehicle-treated group, respectively. Adhesions were absent in all three tested animals. Colonic ulceration was noted in 1/3 animals. All animals had diarrhea. Similarly, all animals treated with Sulfazalazine had diarrhea. Sulfasalazine exhibited significant protection from inflammation (-33% change in net colon-to-body weight (BW) ratio relative to EE-1 treated group) and no animals exhibited adhesions or colonic ulceration. The formula 1 compound 3β,7β,17β-trihydroxyandrost-5-ene (AET) was administered by subcutaneous injections of 0.2 and 2.0 mg/day for 5 or 6 days, with the first dose injected 30 minutes post-DNBS challenge. The AET formulation was the same as the 7-oxodehydroepiandrosterone formulation, except that AET replaced 7-oxodehydroepiandrosterone. AET decreased the net colon-to-body weight ratio by 15% and 21 % relative to controls (see the table below). Animals treated with AET (2 mg) had no diarrhea. Adhesion was absent and fewer animals had colonic ulcerations (33% relative to untreated animals, N=9, pooled data from two studies). The same formulations containing BrEA (100 mg/mL) or dehydroepiandrosterone (20 mg/mL) decreased the net colon-to-body weight ratio by 21 and 3%, respectively relative to controls. In four subsequent studies, AET variably reduced the net colon-to-body weight ratio, ulcers and diarrhea.

Severe acute inflammation, measured 7 days after DNBS challenge, was observed in 3 of 10 immunosteroid vehicle control animals, while AET, dehydroepiandrosterone, sulfasalazine, and sulfasalazine vehicle (polysorbate 80) resulted in severe inflammation in 4, 10, 3, and 8 animals respectively. Moderate acute inflammation, measured 7 days after DNBS challenge, was observed in 5 of 10 immunosteroid vehicle control animals, while AET, dehydroepiandrosterone, sulfasalazine, and sulfasalazine vehicle (polysorbate 80) resulted in moderate inflammation in 6, 0, 6, and 2 animals respectively. Severe inflammation at day 7 was observed in 3, 4, 10 and 3 animals that were treated with vehicle, AET, dehydroepiandrosterone and sulfasalazine respectively. Severe chronic inflammation, measured 14 days after DNBS challenge, was observed in 8 of 10 immunosteroid vehicle control animals, while AET, dehydroepiandrosterone, sulfasalazine, and sulfasalazine vehicle (polysorbate 80) resulted in severe inflammation in 3, 3, 2, and 6 animals respectively.

At day 7 (acute inflammation) after DNBS challenge, 8 of 10 animals treated with vehicle control had severe ulceration, while 2 of 10 animals treated with AET had severe ulceration. Such ulceration was observed in 3 of 10 sulfasalazine control and in 10 of 10 animals treated with dehydroepiandrosterone. Delaying the initiation of AET treatment until 2 days after DNBS challenge resulted in increased chronic ulceration, with 5 of 10 animals showing ulcers 14 days after challenge, compared to only 1 animal with ulcers when treatment began the same day as challenge. This indicated that for acute inflammation in this model, AET is most effective early in the initiation of a flare of inflammatory cellular responses. At day 14 after DNBS challenge (chronic inflammation) 5 of 10 vehicle controls showed severe ulceration, 1 of 10 AET treated animals had severe ulceration, 0 of 10 animals treated with dehydroepiandrosterone had severe ulceration and 4 of 10 sulfasalazine treated animals (positive control) has severe ulceration. The results showed that the formula 1 compounds reduced inflammation or its symptoms compared to untreated control animals.

In another protocol formula 1 compounds were used in a similar animal model. Groups of 10 male Sprague Dawley rats (250-300 grams) were fasted for 24 hours before 2,4,6-trinitrobenzene sulfonic acid (TNBS) or saline challenge. This model presents a severe challenge to the animal's immune system. On Day 0, the rats were lightly anesthetized and a catheter inserted rectally into the colon such that the tip was 8 cm proximal to the anus. Distal colitis was induced by intra-colonic instillation of 0.5 mL of an ethanolic solution of TNBS (60 mg/mL ethanol 50% in water). The test substance, 7-oxodehydroepiandrosterone 20 mg/mL in vehicle or vehicle control was administered by subcutaneous injection (0.1 mL/injection) once a day for 6 days, the first dose being given 30 minutes after TNBS challenge. Sulfasalazine (30 mg/mL in 2% Tween 80 in distilled water) or vehicle control was administered orally (PO) once a day (10mL/kg/day) for 7 days, the first two doses beginning 24 hours and 2 hours before DNBS challenge. Signs of diarrhea were recorded daily by examining the anal area. Animals were fasted for 24 hours prior to sacrifice on day 14 and the colon was removed and weighed. After gross observations of colonic tissues, 3 samples were taken from regions approximately 1, 3, and 8 cm proximal to the anus. If gross ulcers or inflammation were present, at least one sample was taken from the affected region. Before removal of the colon, signs of adhesion between the colon and other organs were recorded. Also, the presence of colonic ulcerations and intestinal adhesions was noted after weighing of each colon. Body weight, colon-to-body-weight ratio, and damage scores were assessed. Visible damage was scored on a 1-5 scale as follows: 0 = No damage; 1 = Localized hyperemia but no ulcers; 2= linear ulcers with no significant inflammation; 3 = Linear ulcers with inflammation at one site; 4 = Two or more sites of ulceration and/or inflammation; 5 = Two or more major sites of ulceration and inflammation or one major site of ulceration and inflammation extending more than 1 cm along the length of the colon. Inflammation is defined as regions of hyperemia and bowel wall thickening.

Ulcerations were present more frequently at multiple colonic levels following 7-oxodehydroepiandrosterone treatment compared to controls. Moderate to severe ulcerative lesions associated with moderate to severe fibrosis of the mucosa and/or submucosa occurred in 8/9 (89%) of 7-oxodehydroepiandrosterone treated animals compared to 5/9 (56%) of controls. The severity and frequency of muscular, peritoneal and/or mesenteric inflammation appeared slightly more than in controls. These histopathological data suggests that 7-oxodehydroepiandrosterone exacerbated the inflammation. Moderate to severe ulcerative lesions occurred in 6/10 (60%) and 5/10 (50%) of sulfazalazine and vehicle treated animals, respectively. The severity and frequency of muscular, peritoneal and/or mesenteric inflammation appeared to be similar to that occurring in vehicle groups. The frequency of moderate to severe lesions and their severity based on their occurrence at multiple colonic levels appeared to be similar between the negative control (vehicle), reference control (sulfazalazine), BrEA and dehydroepiandrosterone, indicating that for these compounds the degree of inflammation was similar for the control compound and the tested compounds.

**Example 28.** Modulation of delayed type hypersensitivity. 3β,7β,17β-trihydroxyandrost-5-ene (AET) was examined for its capacity to modulate delayed type hypersensitivity (DTH). Groups of five female BALB/cByJ mice (20-25 grams) were anesthetized and 100 µL of a 3% solution of oxazolone was applied on day 0 to the shaved abdomen and dried. Seven days later, on day 7, the mice were challenged by applying 5 µL of oxazolone topically to each side of the right ear. The compound AET (40 mg/mL) in vehicle was administered by subcutaneous injection (2 mg/day, 50 µL/injection) one time on day 6, 24 hours before the oxazolone challenge. The vehicle as a non-aqueous suspension of AET in 2% benzyl alcohol w/v, 0.1 % Brij 96 w/v and equal volumes of PEG 300 and propylene glycol.

Dexamethasone in saline (0.2 mg/mL) was administered daily for 9 days (day -1 to 7), first dose 24 hours before sensitization, last dose at challenge by subcutaneous injection (0.01 mg/dose, 50 µL/injection). On Day 8, 24 hours following the oxazolone challenge, both the right and left ear thicknesses were measured using a micrometer caliper and the differences determined. The differential ear thickness is measured as an indicator of the DTH response to topical oxazolone challenge. The DTH response was expressed as the difference in the thickness (mm) between the right and the left ears for each animal.

The differential ear thickness in animals receiving vehicle alone was 0.225 mm and treatment with dexamethasone (high dose) or cyclophosphamide reduced the DTH response (0.144 mm and 0.092 mm, respectively). AET administered subcutaneously had only a slight effect on the DTH response to oxazolone. When administered 24 hours before challenge or at challenge (2 mg/day), the effect was a 22-24 % reduction in the response. When administered daily for 8 days (2 mg/day), first dose 24 hours before sensitization and last dose 24 hours prior to challenge, the effect was an enhancement of the response (23%). This result shows that if the compound was delivered to the animals during sensitization, the DTH response increased. This is consistent with an enhanced Th1 immune response. If the compound was delivered to the animals after sensitization, the DTH response was decreased. This is consistent with a decreased inflammatory response.

**Example 31.** Modulation of monocyte or macrophage activation or survival. The capacity of the formula 1 compounds to activate monocytes and/or increase monocyte or macrophage activity or survival is determined using methods known in the art. The formula 1 compounds are assayed using, e.g., the assays described below. For these assays, peripheral blood mononuclear cells (PBMC) are purified from a subject, e.g., a human leukopack (American Red Cross, Baltimore, MD) by centrifugation through a histopaque gradient (Sigma). Monocytes are isolated from PBMC by counterflow centrifugal elutriation. In each of the assays, the activity of a given formula 1 compound is optionally compared to the response associated with AET or BrEA.

Modulation of monocyte survival is determined essentially as follows. Human peripheral blood monocytes progressively lose viability when cultured in absence of serum or other stimuli. Their death results from internally regulated process such as apoptosis. Addition to the culture of activating factors, such as TNF-α improves cell survival. Propidium iodide (PI) staining is used to measure apoptosis as follows. Monocytes are cultured for 48 hours in polypropylene tubes in serum-free medium (positive control), in the presence of about 100 ng/mL of TNF-α (negative control), and in the presence of varying concentrations of the formula 1 compound. Cells are suspended at a concentration of 2 x 10⁶/mL in PBS containing PI at a final 5 concentration of about 5 µg/mL, and then incubated at room temperature for 5 minutes before FACScan analysis. PI uptake has been demonstrated to correlate with DNA fragmentation in this experimental paradigm. The activity of formula 1 compounds such as AET or BrEA can be used as a comparison standard for other formula 1 compounds.

The effect of formula 1 compounds on monocyte or macrophage cytokine release is determined essentially as follows. An important function of monocytes and macrophages is their regulatory activity on other cellular populations of the immune system through the cytokines release after stimulation. An ELISA to measure cytokine release is performed using, e.g., human, monocytes, which are incubated at a density of about 5x10⁵ cells/mL. A range of formula 1 compound concentrations is used, e.g., 1 nm, 10 nm, 100 nm, 1 µm, 10 µm and 50 µm. To determine IL-12 production, the cells are primed overnight with IFN (100 U/mL) in presence of a formula 1 compound. LPS (10 ng/mL) is then added. Conditioned media are collected after 24h and kept frozen until use. Measurement of TNF-alpha, IL-10, MCP- I and IL-8 is then performed using a commercially available ELISA kit (e.g, R & D Systems (Minneapolis, MN)) and applying the standard protocols provided with the kit.

Activation of monocytes or macrophages by formula 1 compound is measured by assaying the oxidative burst after stimulation of the cells. Purified monocytes are plated in 96-well plate at about 2x10⁵ cells/well. A range of formula 1 compound concentrations, e.g., 1 nm, 10 nm, 100 nm, 1 µm, 10 µm and 50 µm, are added to the wells in a total volume of 0.2 mL culture medium (RPMI 1640 + 10% FCS, glutamine and antibiotics). After 3 days incubation, the plates are centrifuged and the medium is removed from the wells. To the macrophage monolayers, 0.2 mL per well of phenol red solution (140 mM NaCl, 10 mM potassium phosphate buffer pH 7.0, 5.5 MM dextrose, 0.56 mM phenol red and 19 U/ml of HRPO) is added, together with the stimulant (200 nM PMA). The plates are incubated at 37°C for 2 hours and the reaction is stopped by adding 20 pL 1 N NaOH per well. The absorbance is read at 610 nm. To calculate the amount of H₂0₂ produced by the macrophages, a standard curve of a H₂O₂ solution of known molarity is performed for each experiment.

**Example 32.** Modulation of central nervous system cell growth, differentiation or activity. The formula 1 compounds are used to modulate astrocyte or neuron survival, neurite outgrowth, phenotypic differentiation of cortical neuronal cells or for inducing the proliferation of glial fibrillary acidic protein immunopositive cells (astrocytes). A thymidine incorporation assay, for example, can be used to measure cell proliferation or survival. The biological effects of FGF-2 (basic FGF) on cortical or hippocampal neurons *in vitro* included increases in both neuron survival and neurite outgrowth (Walicke et al., Proc. Natl. Acad. Sci. U.S.A. 1986 83:3012-3016. Using primary cortical neuronal culture, the effect of a formula 1 compound to induce neurite outgrowth can be compared to the response achieved with FGF-2 using, for example, a thymidine incorporation assay.

The formula 1 compounds are used in a model for Parkinson disease essentially as follows. The loss of motor function in Parkinson's disease is attributed to a deficiency of striatal dopamine resulting from the degeneration of the nigrostriatal dopaminergic projection neurons. An animal model for Parkinson's that has been extensively characterized involves the systemic administration of 1-methyl-4 phenyl 1,2,3,6-tetrahydropyridine (MPTP). In the CNS, MPTP is taken-up by astrocytes and catabolized by monoamine oxidase B to 1-methyl-4-phenyl pyridine (MPP) and released. Subsequently, MPP is actively accumulated in dopaminergic neurons by the high-affinity reuptake transporter for dopamine. MPP is then concentrated in mitochondria by the electrochemical gradient, which selectively inhibits nicotidamide adenine disphosphate; ubiquinone oxidoreductionase (complex 1), thereby interfering with electron transport and eventually generating oxygen radicals. It has been demonstrated in tissue culture and *in vivo* that FGF-2 (basic FGF) has trophic activity for nigral dopaminergic neurons. FGF-2 in gel foam implants in the striatum results in the near complete protection of nigral dopaminergic neurons from the toxicity associated with MPTP exposure. The formula 1 compounds are administered with or without FGF-2 to measure their capacity to enhance survival of dopaminergic neurons *in vitro*, or they are delivered *in vivo* to enhance protection of dopaminergic neurons in the striatum from the damage associated with MPTP treatment.

*In vitro* dopaminergic neuronal cell cultures are prepared by dissecting the midbrain floor plate from gestation day 14 Wistar rat embryos. The tissue is dissociated with trypsin and seeded at a density of about 2 x 10⁵ cells/cm² on polyorthinine-laminin coated glass coverslips. The cells are maintained in Dulbecco's Modified Eagle's medium and F12 medium containing hormonal supplements (N1). The cultures are fixed with paraformaldehyde after 8 days and are processed for tyrosine hydroxylase, a specific marker for dopminergic neurons, immunohistochemical staining. Dissociated cell cultures are prepared from embryonic rats. The culture medium is changed every third day and the factors are also added at that time. Since the dopaminergic neurons are isolated from animals at gestation day 14, a developmental time which is past the stage when the dopaminergic precursor cells are proliferating, an increase in the number of tyrosine hydroxylase immunopositive neurons represents an increase in the number of dopaminergic neurons surviving *in vitro*.

**Example 33.** Supression of TNF-α induced adhesion molecule expression. The recruitment of lymphocytes to areas of inflammation and angiogenesis involves specific receptor-ligand interactions between cell surface adhesion molecules (CAMs) on lymphocytes and the vascular endothelium. The adhesion process, in both normal and pathological settings, follows a multi-step cascade that involves intercellular adhesion molecule-1 (ICAM-1), vascular cell adhesion molecule-1 (VCAM-1), and endothelial leukocyte adhesion molecule-1 (E-selectin) expression on endothelial cells (EC). The expression of these molecules and others on the vascular endothelium determines the efficiency with which leukocytes may adhere to the local vasculature and extravasate into the local tissue during the development of an inflammatory response. The local concentration of cytokines and growth factor participate in the modulation of the expression of these CAMs.

Tumor necrosis factor alpha (TNF-α), is a proinflammatory cytokine and stimulates all three CAMs on endothelial cells. It may be involved in a wide variety of inflammatory responses, often resulting in a pathological outcome. The capacity of a formula 1 compound to mediate a suppression of TNF-α induced CAM expression can be examined. A modified ELISA assay which uses ECs as a solid phase absorbent is employed to measure the amount of CAM expression on TNF-α treated ECs when co-stimulated with a member of the FGF family of proteins. To perform the experiment, human umbilical vein endothelial cell (HUVEC) cultures are obtained from pooled cord harvests and maintained in growth medium (EGM-2, Clonetics, San Diego, CA) supplemented with 10% FCS and 1% penicillin/streptomycin in a 37°C humidified incubator containing 5% CO₂. HUVECs are seeded in 96-well plates at concentrations of about 1 x 10⁴ cells/well in EGM medium at 37°C for 18-24 hrs or until confluent. The monolayers are subsequently washed 3 times with a serum-free solution of RPMI-1640 optionally supplemented with 100 U/mL penicillin and 100 mg/mL streptomycin, and treated with a given cytokine and/or growth 5 factor(s) for 24 h at 37°C. Following incubation, the cells are then evaluated for CAM expression.

HUVECs are grown in a standard 96 well plate to confluence. Growth medium is removed from the cells and replaced with 90 µL of 199 Medium (10% FBS). Samples for testing and positive or negative controls are added to the plate in triplicate (in 10 µL volumes). Plates are incubated at 37°C for either 5 h (selectin and integrin expression) or 24 h (integrin expression). Plates are aspirated to remove medium and 100 pL of 0.1% paraformaldehyde-PBS (with Ca⁺⁺ and Mg⁺⁺) is added to each well. Plates are held at 4°C for 30 min. Fixative is then removed from the wells and wells are washed 1X with PBS(+Ca,Mg)+0.5% BSA and drained. Do not allow the wells to dry. 10 pL of diluted primary antibody is added to the test and control wells. Anti-ICAM-1-Biotin, Anti-VCAM-1-Biotin and Anti-E-selectin-Biotin are used at a concentration of 10 pg/ml (1:10 dilution of 0.1 mg/ml stock antibody). Cells are incubated at 37°C for 30 min. in a humidified environment. Wells are washed X3 with PBS ( with Ca, Mg) and 0.5% BSA. Then add 20 pL of diluted ExtrAvidin- Alkaline Phosphotase (1:5,000 dilution) to each well and incubate at 37°C for 30 min. Wells are washed X3 with PBS (with Ca, Mg) and 0.5% BSA. 1 tablet of p-Nitrophenol Phosphate pNPP is dissolved in 5 mL of glycine buffer (pH 10.4). 100 pl of pNPP substrate in glycine buffer is added to each test well. Standard wells in triplicate are prepared from the working dilution of the ExtrAvidin-Alkaline Phosphotase in glycine buffer: 5 pL of each dilution is added to triplicate wells and the resulting AP content in each well is 5.50 ng, 1.74 ng, 0.55 ng, 0.18 ng. 100 pl of pNNP reagent is then be added to each of the standard wells. The plate is incubated at 37°C for 4h. A volume of 50 pL of 3M NaOH is added to all wells. The results are quantified on a plate reader at 405 nm. The background subtraction option is used on blank wells filled with glycine buffer only. The template is set up to indicate the concentration of AP-conjugate in each standard well. Results are indicated as amount of bound APconjugate in each sample.

**Example 34.** Inhibition of a mixed lymphocyte reaction. This assay can be used to evaluate inhibition of a Mixed Lymphocyte Reaction (MLR) by formula 1 compounds. Inhibition of a MLR may be due to a direct effect on cell proliferation and viability, modulation of costimulatory molecules on interacting cells, modulation of adhesiveness between lymphocytes and accessory cells, or modulation of cytokine production by accessory cells. Multiple cells may be targeted by the compounds since the peripheral blood mononuclear fraction used in this assay includes T, B and natural killer lymphocytes, as well as monocytes and dendritic cells. Compounds that inhibit the MLR are useful in treating, preventing or ameliorating diseases associated with lymphocyte and monocyte activation or proliferation. These include, e.g., inflammatory or autoimmune conditions such as asthma, arthritis, diabetes, inflammatory skin conditions, psoriasis, eczema, systemic lupus erythematosus, multiple sclerosis, glomerulonephritis, inflammatory bowel disease, Crohn's disease, ulcerative colitis, arteriosclerosis, cirrhosis, graft vs. host disease, host vs. graft disease and hepatitis.

To perform the assay, PBMCs from, e.g., human, donors are purified by density gradient centrifugation using Lymphocyte Separation Medium (LSM^{™}, density 1.0770 g/mL, Organon Teknika Corporation, West Chester, PA). PBMCs from two donors are adjusted to 2 x 10⁶ cells/mL in RPMI-1640 (Life Technologies, Grand Island, NY) supplemented with 10% FCS and 2 mM glutamine. PBMCs from a third donor is adjusted to 2 x 10⁵ cells/ml. Fifty µL of PBMCs from each donor is added to wells of a 96-well round bottom microtiter plate. Several concentrations of the formula 1 compound in the wells is used in triplicate, e.g., 1 nm, 10 nm, 100 nm, 1 µm and 10 µm. Recombinant human IL-2 (R&D Systems, Minneapolis, MN, catalog number 202-IL) is added to a final concentration of about 0.1 to 1 µg/mL and anti-CD4 monoclonal antibody (e.g., R&D Systems, clone 34930.11, catalog number MAB379) is added to a final concentration of about 1 to 10 µg/mL. Cells are cultured for 7-8 days at 37°C in 5% CO₂, and 1 µCi of [³H] thymidine is added to wells for the last 16 hrs of culture. Cells are harvested and thynudine incorporation determined using a suitable scintillation counter, e.g., a Packard TopCount. Data is expressed as the mean and standard deviation of triplicate determinations. Samples of the protein of interest are screened in separate experiments and compared to the negative control treatment, anti-CD4 mAb, which inhibits proliferation of lymphocytes and the positive control treatment, IL-2 (either as recombinant material or supernatant), which enhances proliferation of lymphocytes.

Previously described MLR protocols, sources of cells for use in the MLR and assay parameters can be used to evaluate the effects of the formula 1 compounds. See, e.g., K.V. Bromelow et al., J. Immunol. Methods 2001 247:1-8, Z. Amirghofran et al., J. Ethnopharmacology 2000 721:167-172, T. Itoh et al., J. Antibiot (Tokyo) 1993 46:1575-1581, P. Van Vlasselaer et al., Cell. Immunol. 1991 138:326-340, and A. Shaked et al., Transplantation 1991 52:1068-1072.

**Example 35.** Effects on the CNS. The effects of the formula 1 compounds on memory, learning, motor function or the status of a neurological condition or neurodegeneration condition are assayed using standard methods. For example, aged, two year old mice are tested in the Morris water maze procedure by training the mice to locate a pedestal in less than 15 seconds in three consecutive trials. Immediately upon completion of training one group of mice is treated with a formula 1 compound (5-30 mg/kg) and a second group is treated with a placebo. The treatment comprises one, two or three intraperitoneal, subcutaneous, intramuscular or intravenous injections of the formula 1 compound and the vehicle placebo. The injections are given once per day. Two weeks after treatment, the time to rescue is timed in the Morris water maze procedure and the control result is compared to the placebo control. The use of Morris water maze and other procedures to measure the effect of various conditions or compounds on learning, memory or neurological conditions have been described, see e.g., R. Gerlai Trends Neurosci. 1996, 19:177-181, J.L.W. Lau et al., Proc. Nat'l. Acad. Sci. 2001, 98:4716-4721, U.S. patent Nos. 6348489, 6251942 and 6277886.

Scopolamine induced amnesia is examined essentially as follows. Groups of 13 to 16 C57BL76 mice (about 35 gm) are trained in the Morris water maze procedure to locate a pedestal in less than 15 seconds in three consecutive trials. Immediately upon completion of training the mice in each of three groups are treated with scopolamine (1 mg/kg), scopolamine plus a formula 1 compound at one or more dosages (e.g., about 5-50 mg/kg), and scopolamine plus a placebo. The treatment comprises one, two or three intraperitoneal, subcutaneous, intramuscular or intravenous injections of the formula 1 compound and the vehicle placebo. The injections are given once per day. Six days after treatment the average time (sec) to rescue is timed using the Morris water maze procedure and the results from each group are compared. Results for a formula 1 compound such as 16α-fluoroandrost-5-ene-17-one or 7-hydroxy-16α-fluoroandrost-5-ene-17-one are optionally compared to the results that are obtained in these protocols using another control compound, e.g., (S)-(-)-N-propargyl-1-aminoindan or nefiracetam, or another formula 1 compound such as BrEA or AET to determine the relative potency of the formula 1 compounds with respect to each other.

**Example 36.** The capacity of formula 1 compounds to limit injury associated with ischemia and reperfusion is determined in an animal model essentially as follows. Male Sprague-Dawley rats weighing 130-170 g are randomly assigned to no pre-treatment, vehicle pre-treatment or formula 1 compound pre-treatment using one or more dosages, e.g., about 1-10 mg/kg. Animals are treated with vehicle or DHEA the day before and the day of surgery. Anesthesia is induced with intraperitoneal pentobarbital (60-70 mg/kg). The rats are placed on a heating pad, and body temperature is maintained at about 36°C. Detection of the cremaster muscle on its neurovascular pedicle is performed essentially according to conventional techniques, e.g., Anderson, G. L. et al., Microvascular Res. 1988 36:56-63, Siemionow, M. et al., Microcirc. Endoth. Lymphatics 1991 7:183-197, Siemionow, M. et al., J. Hand Surgery 1993 18A:963-971.

Briefly, a skin incision is made from the anterior iliac spine to the tip of the scrotum. The testis with cremaster muscle intact is then dissected away from the scrotum. An opening of 1 cm is made on the ventral surface of the cremaster, and the testis and spermatic cord are removed. Under a microscope, the neurovascular pedicle, consisting of the pubic-epigastric arteries, vein, and genitofemoral nerve, is then completely isolated by dissecting to the origin of the vessels from the external iliac artery and vein. The front wall of the cremaster muscle sac is opened and the island cremaster muscle flap is prepared for intravital videomicroscopy. The rat is secured on a tissue bath, and the cremaster muscle flap is spread over the coverglass in the opening at the bottom of the bath and fixed with 5-0 silk sutures. It is then transilluminated from below, using a fiber optic tungsten lamp. The muscle is kept moist and covered with impermeable plastic film. The tissue bath, designed specifically for temperature control, is filled with 0.9% saline and the temperature maintained at between 35-36°C. The microscope is equipped with a color video camera. The video image of the microcirculation is displayed on a 19" monitor, where the final magnification is 1800X. Measurement of microvascular activity is recorded after isolation of the muscle to establish the pre-ischemia baseline. After proper positioning of clamps to completely shut down blood flow to the muscle flap, the duration of the ischemic period is six hours. Following removal of clamps to induce reperfusion injury, activity in the microvasculature is measured at e.g., 30, 60 and 90 minutes post-reperfusion. In all experimental subjects, ischemia is followed by reflow and then by an initial period of flow of blood through the microcirculation. This burst of circulatory activity is followed by marked reperfusion injury that induces loss of flow.

One or more of the following parameters are used to evaluate the state of the cremaster muscle microvasculatory system prior to ischemia and after reperfusion. The density of perfused capillaries in each of three flap regions is measured by counting the number of flowing capillaries in proximity to the preselected post-capillary venule. Nine visual fields of capillaries are counted at each postcapillary venule site, for a total of 27 fields per cremaster muscle flap.

A leukocyte count in postcapillary venules is taken using video scans of three preselected post-capillary venules in proximal, middle and distal flap regions. For each venule, the number of leukocytes rolling through the lumen, the number adhering to the endothelium and the number migrating across the endothelium over a two-minute period are recorded. Results are optionally obtained for rollers, strikers and diapedesis.

Red blood cell velocities in first order and second order arterioles are measured. Red blood cell velocities are recorded in the main arterioles of the cremaster flap using an optical Doppler velocimeter. Results are obtained for velocity of venous and arterial blood.

In an exemplary protocol, six rats are untreated and six rats are pre-treated with vehicle. Under conditions of six hours of ischemia and 90 minutes of reperfusion, the absolute number of rolling, sticking and transmigrated leukocytes is determined within 60 minutes of reperfusion and at 90 minutes. Rats are pre-treated with a formula 1 compound by subcutaneous injection the day before and the day of surgery to measure any protective effect of the therapy. One or more of the three parameters are determined and are compared to normal values. The endothelial-adherent properties compared to baseline values are optionally determined, using numbers of rolling, sticking and transmigrating leukocytes. Red cell velocities in second order arterioles are compared to normal rates of flow at, e.g., 90 minutes post-reperfusion.

**Example 37.** Pulmonary vasoconstriction. The capacity of formula 1 compounds to limit hypoxia induced pulmonary vasoconstriction is demonstrated using an animal model essentially as follows. Isolated perfused ferret lungs are an established animal model to study secondary pulmonary hypertension. In brief, male ferrets are anesthetized with intraperitoneal pentobarbital sodium and the chest is opened. Stainless steel cannulae are secured in the left atrium and pulmonary artery, and the pulmonary artery and the aorta are ligated. The lungs are perfused with a mixture of autologous blood and Krebs-Henseleit buffer in a circulating manner at a constant rate of about 85 mL/min. The perfusion circuit includes a perfusate reservoir, a roller perfusion pump, filter, and a heat exchanger. The perfusion system is made of, e.g., tygon tubing, which is used for connections and for passage through the perfusion pump. The temperature of the perfusate is kept about 37-38°C. and the pH is maintained at 7.35 to 7.40 by adding sodium bicarbonate to the reservoir as needed. The venous reservoir is placed below the lowermost portion of the lung.

The lungs are ventilated with a hypoxic gas mixture of 5% CO₂, 4% O₂, and 91% N₂ by a tracheotomy with a Harvard animal respirator for 30 minutes. The animals are ventilated with a tidal volume of 30 mL, at a rate of 18 breaths/min. and with 2 cm of H₂O positive end-expiatory pressure. For measurements, pulmonary arterial, left atrial and tracheal pressures are monitored using Gould Statha P231D pressure transducers or an equivalent connected to the inflow circulation and recorded on, e.g., a Grass polygraph. After 30 minutes of ventilation with hypoxic gas mixture, a formula 1 compound in a dose between about 5-25 mg/kg body weight is added to the reservoir, and perfusate is allowed to perfuse the ferret lungs for 1.5 hours. Pulmonary artery pressure is measured until the end of the experiment, i.e., a total of two hours. Pressure that remains at or near basal level indicates the vasodilatory effect of the formula 1 compound in pulmonary circulation that is otherwise constricted in response to hypoxia. The effects of the formula 1 compounds can be compared to the effects and duration of nitric oxide, a therapeutic agent that is optionally used in this model as a control.

**Example 48.** Antiglucocorticoid effects of formula 1 compounds. Effects of formula 1 compounds and Hydrocortisone on Proliferation in the Absence of a Mitogen. A series of tests is run in triplicate using BALB/c mouse spleen cells to demonstrate the effect of the formula 1 compounds and hydrocortisone ("Hycort") on cellular proliferation in the absence of a mitogen. Cultures of spleen cells are prepared and steroids are added at, e.g., 0.1, 0.5, 1, 5 µM. Suitable controls are used. Twenty four hours after setup, about 50 µCi [³H]-thymidine is added to each cell. Four to six hours later, the cells are harvested and counted on a scintillation counter.

Spleen cells are obtained from normal BALB/c mice and prepared as a single cell suspension at a concentration of about 1x10⁷ cells/ml in RPMI 1640 supplemented with 2 mM L-glutamine, 5x10⁻⁵ M 2-mercaptoethanol, 20 µg/ml gentamycin-sulfate, and 1% Nutridona-NS (Boehringer-Mannheim). Individual aliquots of cells are then pulsed for 30 minutes at 37° C with selected concentrations of formula 1 compounds. After pulsing, the cells are washed several times in balanced salt solution, resuspended in fresh medium, and then dispensed into 24-well culture plates with a stimulatory concentration of anti-CD3 (e.g., Leo et al. Proc. Natl. Acad. Sci. U.S.A., 84:1374 (1987)). After a 24-hour incubation period, culture supernatants are harvested for assessment of proliferation or cytokine production, e.g., IL-2, IL-3 or IL-4 using, e.g., the method of Mossman (J. Immunol. Meth. 65:55 (1983)). 100% control titers of IL-3, IL-2 or IL-4 from normal stimulated splenocytes are obtained, exemplary values may be about 640 units/mL or IL-2 and 160 units/mL for IL-4.

Effects of formula 1 compounds and Hydrocortisone on Proliferation in the Presence of a Mitogen. A series of spleen cell cultures is run using a formula 1 compound and/or hydrocortisone with cell cultures to which concanavalin A is added. Preliminary tests on cultures to which concanavalin A is added at concentrations of 10.0, 5.0, 2.5 and 1.0 ng/mL. All tests on the effects of invention compounds on cultures stimulated with concanavalin A are performed with concanavalin A at, e.g., about 2.5 ng/mL. A mitogen such as ConA generally increases cell proliferation and the GCS can decrease proliferation. Detectable partial or complete reversal of the inhibitor effects of hydrocortisone indicate an anti-glucorticoid effect by the formula 1 compounds.

Effect of formula 1 compounds on IL-3 production. Exemplary formula 1 compounds are tested to determine whether their effect on the level of the cytokine IL-3 expresion by spleen cells in tissue culture and for their capacity to reverse the effects of a GCS in IL-3 expression. The spleen cell cultures are prepared in accordance with the general method above. After 30 hours the level of IL-3 in the supernatants of the cultures was measured using the IL-3 ELISA kit manufactured by EndoGen Inc., Boston, Mass. A GCS such as hydrocortisone will generally suppress the production of IL-3 and the invention compounds are examined for their capacity to modify this effect. The IL-3 expressed by cells in culture may be recovered from the media containing IL-3 by known methods such as single or sequential reverse-phase HPLC steps on a preparative HPLC column. (See Urdal, et al., J. Chromatog. 296:171 (1984) and U.S. Pat. No. 5,128,450).

**Example 49.** Activity of formula 1 compounds in the treatment of symptoms or progression of a skin condition. Conditions such as psoriasis are examined using formula 1 compounds in a suitable animal model or in human patients. The compounds are administered to the subject by topical administration or by systemic administration, e.g., oral, buccal or subcutaneous injection. In one animal model of psoriasis, varying numbers of naïve and memory CD4⁺ T cells from minor histocompatibility mismatched mice are used to reconstitute scid/scid mice. Skin lesions with characteristics of human psoriasis arises when no memory T cells are coinjected, and coinjection of memory T cells diminishes the symptoms (M.P. Schon et al., Nature Medicine 1999 3:183-188). Varying amounts of formula 1 compounds, e.g., 0 to 100 mg/kg/day, are used in the absence of memory T cells and with various numbers of memory T cells to characterize their effects on both types of T cells to cause disease or to reduce it.

Groups of the mice (e.g., about 4-15 animals per group) are injected subcutaneously with one or more dosage of about 1, 2.5, 5, 10 or 20 mg/kg of a formula 1 compound such as 16α-fluoroandrost-5-ene-17-one, 7β-hydroxy-16α-fluoroandrost-5-ene-17-one, 7α-hydroxy-16α-fluoroandrost-5-ene-17-one, 17α-hydroxy-16α-fluoroandrost-5-ene or 17β-hydroxy-16α-fluoroandrost-5-ene. The compound is administered once daily or once every 2, 3 or 4 days for 1, 2, 3, 4, 5 or 6 weeks beginning either before or at about the time when untreated control animals have developed symptoms or skin lesions characteristic of the disease. The formula 1 compounds can be used with other animal models and markers of psoriasis would be measured essentially as described. See, e.g., B.J. Nickoloff, J. Invest. Dermatol. Symp. Proc. 2000 5:67-73, M.P. Schon J. Invest. Dermatol. 1999 112:405-410, B.J. Nickoloff et al., Arch. Dermatol. 1999 135:546-552 and M.P. Schon et al., Nature Medicine 1997 3:183-188.

In a clinical dose ranging protocol, human patients suffering from psoriasis are treated topically or systemically with a formula 1 compound. Topical formulations would comprise about 1-20% w/w of a formula 1 compound in a suitable cream, gel or other topical formulation, e.g., as disclosed herein. Treatment is initiated at the onset of symptoms or is administered to patients with preexisting psoriatic lesions. The compound is administered twice per day, once per day or it is administered once or twice per day every other day, every third day, every fourth day or every seventh day for about 2-24 weeks, e.g., about 4, 8 or 12 weeks. The clinical response to treatment is monitored on one, two or more occasions during dosing and optionally again once or twice at 1 day to 6 weeks after dosing is completed. Treatment is optionally combined with another treatment(s), e.g., corticosteroid, calcipotriol or salicylic acid. Patient response is measured using standard assessment methods, e.g., the Psoriasis Scalp Severity Index for psoriasis of the scalp, the Psoriasis Disability Index or by assessment of erythema, scaling, induration or size of psoriatic lesions at various times before, during and/or after treatment.

The response of animals or humans is optionally examined by histopathological observation or other analysis, e.g., detectably reduced inflammation or epidermal thickening, erythema, induration or for a detectable modulation or reduction of inflammatory or pathology-associated cells, cytokines or markers, e.g., CD94⁺ T cells, CD69⁺ T cells, CD45RO⁺ T cells, CD25⁺ T cells, IgE, IL-1α, IL-6, IL-8, IL-13, keratinocyte growth factor or transforming growth factor-α in skin or skin lesions from treated animals as compared to suitable control animals. Modulation, e.g., reduction, of one or more markers or cell types associated with the severity or progression of disease is monitored.

**Example 50.** Treatment of neurodegenerative conditions. Experimental allergic encephalomyelitis (EAE), is demyelinating disease of the central nervous system with many pathological and clinical similarities with multiple sclerosis (MS). EAE is thus a recognized animal model for human autoimmune conditions such as MS. Formula 1 compounds 16α-fluoroandrost-5-ene-17-one, 3β,7β,17β,-trihydroxyandrost-5-ene and 16α-bromoepiandrosterone were tested for their capacity to delay the onset of EAE or to reduce its symptoms. Female SJL mice (5 animals per group) were immunized with 150 to 200 µg of PLP-139-151 peptide/CFA to induce EAE. Starting 7 days before injection of the peptide, the animals were given daily injections (s.c.) of the compounds (3.0 mg) in 0.1 mL vehicle, or vehicle alone for 33 days. The vehicle consisted of a suspension of the formula 1 compound in saline and carboxymethylcellulose. 16α-Fluoroandrost-5-ene-17-one and 3β,7β,17β,-trihydroxyandrost-5-ene significantly delayed the onset, reduced peak clinical score (from 5.2 ± 0.6 to 2.8 ± 1.8) and cumulative disease index (>60%) of EAE, and prevented or significantly attenuated relapses. A lower dose (0.3 mg/mouse) or other routes (p.o.) of administration had less effect. The effect of 16α-bromoepiandrosterone was not significant under these assay conditions. T cells from mice treated with 16α-fluoroandrost-5-ene-17-one and 3β,7β,17β,-trihydroxyandrost-5-ene had significantly reduced PLP-139-151 specific T cell proliferation responses and reduced numbers of TNF-α producing cells in the CNS. These compounds limited the production of autoimmune Th-1 associated cytokines, which is consistent with restoration of a more normal Th-1/Th-2 immune balance and/or with reduction of inflammation in this model.

The efficacy of the formula 1 compounds to treat other autoimmune conditions can be determined by incorporating their use with suitable animal models or assay methods, e.g., collagen-induced arthritis as a model for human autoimmune polyarthritis (e.g., L.K. Myers et al., Clin. Immunol. 2002, 102:185-191, A. Matejuk et al., Endocrinology 2002, 143:313-319, S. Thornton et al., J. Immunol. 165:1557-1563). The effect of the compounds on markers of inflammation such as TNFα, MIP-1β, IL-13, IL-15, IL-17 or IL-18, e.g., reduced expression or activity, is optionally observed in any autoimmune or inflammatory condition.

**Example 53.** Synthesis of compounds useful to treat the conditions or diseases described herein is exempliied in the following examples. Melting points were determined in open capillaries in an electrically heated and stirred Thiel-type bath and are uncorrected. Solvents were removed on a rotary evaporator under water pump vacuum at 30 °C or less. Nuclear magnetic resonance (NMR) spectra were taken on Bruker WP-200SY and AM-300 MHz spectrometers. Spectra were measured in deuterated chloroform (CDCl₃) using tetramethylsilane (δ 0) as reference for ¹H NMR spectra and the CDCl₃ triplet (δ 77.0) for ¹³C spectra. Abbreviations are s (singlet), d (doublet), t (triplet), q (quartet), m (multiplet). A Kratos MS-80RFA mass spectrometer was employed to determine the high resolution mass spectra (HRMS) of the compounds, and a Hewlett-Packard LC-MS, 1100 series was employed to determine the liquid chromatograph/mass spectra (LCMS) of the compounds. Spectral data were included for some known compounds where such data were not previously reported. Reagents and solvents used were purchased from Aldrich Chemical Co., Milwaukee, Wisconsin. For column chromatography, Aldrich brand silica gel of 70-230 and 200-400 mesh size was used.

The glucuronides of 7-oxygenated androgens were synthesized by reacting the appropriately protected 7-oxygenated derivative with bromo glucuronates followed by selective hydrolysis to remove acetate protection on the glucose hydroxyls. Good yields were obtained when acetylated glucuronides of steroids were prepared first, acetates were hydrolyzed selectively and the resulting Δ-5 steroid glucuronates were oxygenated at the allylic 7-position.

Synthesis of β-pyranoside forms of steroid glucuronides (as their tri-O-acetyl methyl esters) was carried out by condensation of a specific, open hydroxyl group of the steroid, with 2, 3, 4,-tri-O-acetyl-1-bromo-1-deoxy-αD-glucopyranuronate utilizing the well known Loenigs-Knorr reaction. Acid acceptors such as silver carbonate or cadmium carbonate in anhydrous benzene were used to promote the reaction and freshly pulverized dry calcium sulfate was included as internal desiccant. Reactions were run in the dark (when silver carbonate was used), at room temperature for 23 to 48 hours, during which time ∼40-60% conversion took place (identified by the ¹H-NMR spectrum of the reaction mixture). The acetylated glucuronide methyl esters were isolated either by crystallization or by column chromatography on silica gel. Fractional crystallization was the major technique employed for isolating the Koenigs-Knorr product from the reaction mixture and it was found to be quite satisfactory in providing high purity glucuronides. In cases where isomeric ortho ester or other side products were formed in larger quantities, the desired products were purified by column chromatography on silica gel.

The structures of acetylated glucuronide methyl esters were confirmed by ¹H-NMR, ¹³C-NMR, and mass spectrometry. The anomeric proton signal (1 H) of the glucose moiety appeared as a doublet at 4.58-4.69 ppm (J=8-8.4 Hz) indicating β-glucuronoside linkage. The position of the olefinic 6-H of the 7-keto steroids, usually occurred at 5.65-5.70 ppm and showed a down field shift of 0.35-0.4 ppm from 6-H of non 7-keto steroids; this was another significant point in the characterization of 7-keto glucuronides. β-Anomers of these steroid glucuronopyranosides were generally obtained in good to excellent yield when corrected for recovered unreacted steroids.

Deacetylation of acetylated glucuronide methyl esters, with or without a 7-keto group was performed with freshly prepared sodium methoxide in anhydrous methanol at ambient temperature yielding methyl esters of steroid glucuronides as the major product in generally good to excellent yield. ¹H-NMR spectra of deacetylated steroid glucuronides exhibited the anomeric proton as a doublet at 4.4-4.48 ppm (J=7.4-8.3 Hz) thereby confirming β-linkage. High resolution mass spectra (EI-MS) of steroid glucuronides helped confirm the structure of the products, but the expected molecular ion was absent. The molecular ion was obtained as a sodium adduct base peak in the FAB-MS spectra of the steroid glucuronides. Finally, allylic oxidation at the 7 position of Δ5-steroid glucuronides 8 and 12 was performed utilizing the aerobic oxidation procedure of described in Marwah and Lardy, U.S. Patent 5,869,709 (1999), herein incorporated by reference in its entirety, in the presence of N-hydroxy phthalimide and a radical initiator to afford the corresponding 7-oxo derivatives of steroid glucuronides 9 and 13.

Molecules with small and long chain alkyl ethers were also synthesized. The methyl ether (20) of 7-oxo DHEA (2) was prepared in 67% yield by allylic oxidation of 3µ-methoxy-DHEA (19) which was obtained by heating 3β-tosyl-DHEA to reflux in anhydrous methanol in 94% yield. Further reduction of the product 20 with sodium borohydride in methanol-dichloromethane (usually 90:10) at 0-5 °C in presence of cerium (III) chloride heptahydrate afforded 3β-methoxyandrost-5-en-7β,17β-diol (24). The 17-keto group of 3β-methoxy DHEA (19) was protected as ethylene ketal, and subsequent oxidation of the ketal derivative afforded the 7-oxo compound (21). The 7-oxo group of product 21 was further subjected to reduction with sodium borohydride in methanol at room temperature and a mixture of isomeric 7α- and 7β-hydroxy derivatives (22, 23) were formed, and were separated easily by column chromatography. Reduction of 3β-Methoxy DHEA (19) at position 17, at 0-5 °C afforded 3β-methoxy-17β-hydroxyandrost-5-ene (27) in 86% yield, which in turn was oxidized at the 7 position using N-hydroxy phthalimide, oxygen and a radical initiator in refluxing acetone to afford product 28 (53% yield).

3β-t-Butyl ether of 7-oxoDHEA (31) was prepared in 87% yield by oxidizing 3β-t-butyl-DHEA (30) using pyridinium dichromate and N-hydroxyphthalimide, a new, simple and high yielding procedure. 3β-t-ButylDHEA was synthesized in 67% yield by the procedure of Armstrong et al. (Armstrong et al., Tet. Lett., 29:2483-2486 (1988), incorporated herein by reference in its entirety,) using DHEA and t-butyl trichloroacetimidate in a mixture of dichloromethane and cyclohexane.

According to the methods of the present invention, a mixture of diastereomeric 7-methoxy ethers (26) was prepared in a simple, short procedure, which unexpectedly resulted in very good yields (73%) by stirring 7α-bromo-DHEA (25) in methanol and silica gel at room temperature for 2 hours. No attempt was made to synthesize these derivatives in pure α and β forms.

A long, straight chain alkyl ether (39) was prepared in 83% yield from 3β-dodecanoxyDHEA by allylic oxidation of 3β-dodecanosyl-DHEA (38) using pyridinium dichromate and N-hydroxyphthalimide as mentioned above. 3β-Dodecanoxy DHEA (38) was synthesized using a standard procedure of refluxing 1-bromododecane and DHEA solution in tetrahydrofuran in presence of sodium hydride.

Etherification of DHEA utilizing ethyl vinyl ether in dichloromethane and in presence of p-toluene sulfonic acid, at room temperature for 6 hours afforded 3β-ethoxy ethyl ether of DHEA (40) in 84% yield based on 55% conversion. The product 40 was subjected to aerobic oxidation in presence of N-hydroxyphthalimide and a radical initiator in refluxing acetone to obtain 3β-(1'-ethoxy) ethoxyandrost-5-en-7,17-dione (41).

3β-tetrahydropyranyl ether of 7-oxo-DHEA (37) was made to test the effect of a saturated pyran ring having two stereogenic centers thus generated, on the induction of thermogenic enzymes. This compound was easily formed utilizing dihydropyran and pyridinium p-toluene sulfonate in anhydrous dichloromethane and was stable to most non-acidic reagents.

Silyl protected ethers especially t-butyldimethylsilyl (TBDMS) ether, (32, 33, 35 and 36) substituted at hydroxyls 3, 3 and 17, 7, and 7 and 17 positions of the steroid molecule were also synthesized. TBDMS ethers are quite stable to a variety of organic reactions and have good stability toward base. Their synthesis involved use of t-butyl dimethylsilyl chloride and imidazole in dimethyl formamide. Dichloromethane was not appropriate for the synthesis of these specific 7-substituted derivatives because its acidic character caused the formation of dienes.

In another embodiment of the present invention, carbonate substitution on hydroxyls at 3 and 17 position of the corresponding sterols with 7-position oxygenated (keto or hydroxyl) in the steroid molecule was performed and its effect investigated. Various alkyl carbonates such as methyl (42), ethyl (44), allyl (43), isobutyl (45), and octyl (47) as well as tricyclic fluorenyl carbonates of the enzyme activator were made by standard procedures which involved a slow addition of respective chloroformates to the ice cold solution of steroid substrates in pyridine. Conversion was total in most of the cases and product yields were generally high. Carbonates 42 and 43 were isolated by column chromatography on silica gel. Some of the carbonate derivatives of the steroids (42, 44, 47) were subjected to borohydride reduction in methanol-dichloromethane with cerium (III) chloride heptahydrate at 0-5 °C and 7β- and 17β-hydroxy carbonate derivatives (49-51) were isolated in good yields.

The determination of the structure of the 7-oxygenated alkyl ethers as well as carbonates and sterochemical assignments were made on the basis of their ¹H NMR and ¹³C NMR spectroscopic data, high resolution mass spectroscopy and LC-MS studies. Reaction products, as well as some precursors, when known, were also characterized by comparison of spectroscopic data with those available from the literature. Proton NMR of 6-olefinic hydrogen of all 7-oxo derivatives showed a characteristic absorption at δ 5.7-5.75 with a corresponding ¹³C absorption at about 200-202 ppm. On the other hand, 7-hydroxyl derivative showed different chemical shift values for 6-olefinic protons. In case of 7β-hydroxy compound the olefinic 6α-proton appeared high field and around 5.3 ppm, whereas 7α-hydroxy compound showed a doublet for 6β-olefinic proton downfield and around 5.6 ppm.

Assays of compounds for their ability to induce liver mitochondrial glycerophosphate dehydrogenase and cytosolic malic enzyme when fed to rats have been described. Lardy et al., Steroids, 63:158-165 (1998); Su and Lardy, J Biochem, 110:207-213 (1991), which are incorporated herein by reference in their entirety,. In addition to the control rats that received no steroid supplement, each experiment included a group that received DHEA or 7-oxo-DHEA-acetate (usually at 0.04% - 0.06% of the diet). Because of variation in enzyme activity of both control and treated groups from one experiment to another the activities are reported relative to the enzyme activities of the control group. Compounds are considered active in this assay if they enhance the activity of these thermogenic enzymes to greater than about 150% of that in livers of control rats.

**Example 11:** General Procedure for Allylic Oxidation of Steroid Glucuronides. A solution of N-hydroxyphthalimide (5.0 mmol) and dibenzoyl peroxide (0.01 g) in acetone-ethyl acetate (1:0.5, 75.0 mL) was brought to reflux, and then a filtered hot solution of steroid methyl glucuronate (2.5 mmol) in acetone (20.0 mL) and dibenzoyl peroxide (0.01 g) were added. A slow stream of compressed air was passed into the solution and the mixture was heated to reflux for 12-16 h (checked with TLC). Solvent was removed completely and the resultant mass was taken up in toluene and the precipitated N-hydroxy phthalimide was filtered off. The organic layer was washed thoroughly with saturated sodium bicarbonate solution followed by water and brine and then dried over magnesium sulfate. The solvent was evaporated and the residue was chromatographed on silica gel using acetone-hexane (1:1, v/v) and crystallized from acetone-hexane.

Example 12: *Methy*/ *1-O-(7,17-diixoandrost-5-ene-3*β*-yl)*-β*-D-glucopyranosiduronate* (9). From compound 8, compound 9 was obtained, using the above general procedure, as white crystals, m.p. 203-5 °C (decomp.), yield 29%.

¹H NMR (CDCl₃, 200 MHz): δ 5.76 (1H, s, 6-H), 4.47 (1 H, d, J=7.9 Hz, 1-H (glu)), 3.83 (3H, s, OCH₃), 3.95-3.35 (5H, m, 2-5H (glu) and 3α-H), 1.22 (3H, s, 19-CH₃), 0.90 (3H, s, 18-(CH₃).

¹³C NMR (DMSO, 300 MHz): δ 219.12 (C-17) 200.48 (C-7), 169.54 (ester C=0), 166.3 (C-5), 125.23 (C-6), 101.3 (C-1 (glu)), 76.38, 75.79, 75,31, 73.0, 71.7 (C-2-5 (glu) and C-3), 16.93, 13.43 (C-18, 19), 51.83, 49.30, 47.23, 44.98, 43.69, 38.64, 38.14, 35.80, 35.0, 29.2, 24.0, 20.02 (C-1, 2, 4, 8-16, and OCH₃).

FAB m/z: 515.2 (M+23, 4%), 455.3.2 (M+23, -60, 9%), 413.2 (M, -60, -18, -1, 100%), 241.8 (M, -208, -28, -15, 2%).

Example 13: *Methyl 1-O-(3*β-*acetoxy-7-oxoandrost-5-ene-17*β*-yl)-*β*-D-glucopyranosiduronate* (13). White solid 13 was prepared from 12, using the above general procedure; yield 38%, m.p. 118-20 °C.

¹H NMR (CDCl₃, 200 MHz): δ 5.71 (1 H, s, 6-H), 4.72 (1 H, m, 3α-H), 4.39 (1 H, d, J=8.2 Hz, 1-H (glu)), 3.84 (3H, s, OCH₃), 3.88-3.27 (5H, m, 17α-H, and 2. 3, 4, 5-H (glu)), 2.06 (3H, s, OCOCH₃), 1.23 (3H, s, 19-CH₃), 0.83 (3H, s, 18-CH₃).

¹³C NMR (DMSO, 300 MHz): δ 200.5 (C-7), 169.0, 169.2 (C=O acetate, ester): 165.0 (C-5), 125.8 (C-6), 104.1 (C-1 (glu)), 87.5 (C-3), 76.0, 75.37, 73.6, 71.54 (C-17 and C-2, 3, 4, 5 (glu)), 21.0, 16.79, 11.30 (C-18, 19, acetate CH₃), 51.8, 48.8, 45.0, 44.6, 43.3, 38.0, 37.5, 35.5, 28.7, 27.0, 25.3, 20.0 (C-1, 2, 4, 8-16 and OCH₃).

Ether derivatives.

Example 14: *3*β*-Methoxyandrost-5-ene-7,17-dione* (20). A solution of 3β-tosyloxyandrost-5-ene-17-one (18) (4.0 g. 9.05 mmol) in anhydrous methanol (120 mL) was refluxed for 2 h. The reaction mixture was concentrated under vacuum. On cooling, a white solid 3β-methoxyandrost-5-ene-17-one (19) was obtained. Recrystallization from methanol afforded shining crystals (2.57 g. 94%), m.p. 133-5 °C (lit m.p. 118-120 °C aq. MeOH).

¹H NMR (CDCl₃, 200 MHz): δ 5.39 (1 H, d, J=5.3 Hz, 6-H), 3.37 (3H, s, OCH₃), 3.09 (1 h, M. 3α-H), 1.04 (3H, s, 19-CH₃), 0.90 (3H, s, 18-CH₃).

Air was passed through a refluxing solution of 19 (2.0 g; 6.62 mmol) and N-hydroxyphthalimide (1.5 g, 9.2 mmol) in a mixture of acetone-ethyl acetate (1:1, 50 mL). 1,1'-azobis (cyclohexanecarbonitrile) (0.08 g) was added to the reaction mixture (Foricher et al., U.S. Patent 5,030,739 (1991), incorporated herein by reference in its entirety) and the reaction continued for 10-12 h. After the usual work up as described in the general oxidation procedure, the crude product was dissolved in pyridine (5.0 mL) and stirred with acetic anhydride (3.0 mL) at room temperature for 3-4 h. The reaction mixture was poured into water and stirred for 2-3 h. The steroid was extracted with toluene and the organic layer washed with saturated bicarbonate solution and water. Toluene was distilled off and the residue was chromatographed on silica gel (ethyl acetate-hexane, 25:75, v/v) and crystallized from methanol. 3β-methoxyandrost-5-ene-7,17-dione (20) was obtained in 67% yield (1.4 g), m.p. 227-29 °C.

¹H NMR (CDCl₃, 200 MHz): δ 5.76 (1 H, d, J=1.8 Hz, 6-H), 3.39 (3H, s, OCH₃), 3.2 (1 H, m, 3α-H), 1.22 (3H, s, 19-CH₃), 0.90 (3H, s, 18-CH₃.

¹³C NMR (CDCl₃, 300 MHz): δ 220.27 (C-17), 200.99 (C-7), 166.22 (C-5), 125.85 (C-6), 78.79, 55.91, 50.05, 45.69, 44.26 (C-3, 8, 9, 14, OCH₃), 47.80, 38.72 (C-10, 13), 38.65, 36.13, 35.58, 30.67, 27.51, 24.12, 20.52 (C-1, 2, 4, 11, 12, 15, 16), 17.36, 13.71 (C-18, 19).

Example 15: 3β-Methoxy-17,17-ethylenedioxyandrost-5-ene-7β- and 7α-ol (22 and 23)

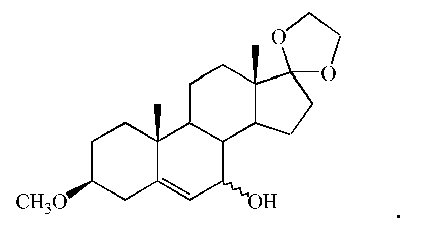

3β-Methoxyandrost-5-ene-17-one (19) was ketalized using ethylene glycol and p-toluene sulfonic acid in toluene, heated to reflux for 7 h; yield 95%, m.p. 138-40 °C.

¹H NMR (CDCl₃, 200 MHz): δ 5.35 (1H, d, J=4.88 Hz, 6-H), 3.9 (5H, m, O-CH₂-CH₂-O and 7α-H), 3.36 (3H, s, OCH₃), 3.05 (1 H, m, 3α-H), 1.0 (3H, s, 19-CH₃), 0.86 (3H, s, 18-CH₃).

The ketalized produce was oxidized at the allylic 7 position, using sodium periodate and t-butylhydroperoxide with sodium bicarbonate (Marwah and Lardy, U.S. Patent 5,869,709 (1999), incorporated herein by reference in its entirety,) to produce 3β-methoxy-17,17-ethylenedioxyandrost-5-ene-7-one (21) in 62% yield; m.p. 190-192 °C.

¹H NMR (CDCl₃, 200 MHz): δ 5.7 (1 H, d, J=1.96 Hz, 6-H), 3.89 (4H, m, -O-CH₂-CH₂-O-), 3.38 (3H, s, OCH₃), 3.2 (1 H, m, 3α-H), 1.19 (3H, s, 19-CH₃), 0.87 (3H, s, 18-CH₃).

Subsequent reduction of the carbonyl group at position 7 of product 21 with sodium borohydride in a mixture of methylene chloride-methanol (1:9) at room temperature afforded 7-hydroxy derivatives. ¹H NMR spectrum of the product showed 7α-and 7β- forms in 2:8 ratio. The diastereomers were separated by column chromatography on silica gel (eluent, ethyl acetate:hexane, 3:7). 3β-Methoxy-17,17-ethylenedioxyandrost-5-ene-7β-ol (22) melted at 173-75 °C.

¹H NMR (CDCl₃, 200 MHz): δ 5.29 (1 H, t, J=1.95, 6-H), 3.89 (5H, m, O-CH₂-CH₂-O and 7α-H), 3.36 (3H, s, OCH₃), 3.14 (1 H, m, 3α-H), 1.05 (3H, s, 19-CH₃), 0.88 (3H, s, 18-CH₃).

HRMS m/z: 362.2505 for C₂₂H₃₄O₄ requires 362.2457 (M⁺, 8.7%), 344.2338 for C₂₂H₃₂O₃ requires 344.2351 (M⁺, -H₂O, 2.4%), 329.2170 for C₂₁H₂₉O₃ requires 329.2117 (M⁺, H₂O, -CH₃, 7.2%), 261.1886 for C₁₇H₂₅O₂ requires 261.1854 (M⁺, -101, 100%), 229.1628 for C₁₆H₂₁O requires 229.1592 (M⁺, -101, CH₃OH, 8.3%), 211 for C₁₆H₁₉ (M⁺, - 101, CH₃OH,-H₂O, 2%).

3β-Methoxy-17,17-ethylenedioxyandrost-5-ene-7a-ol (23) melted at 183-84 °C.

¹H NMR (CDCl₃, 200 MHz): δ 5.62 (1 H, dd, J=1.96, 5.62 Hz, 6-H), 3.89 (4H, m, O-CH₂-Ch₂-O), 3.89 (1 H, 7β-H, merged in the ketal protons), 3.36 (3H, s, OCH₃), 3.14 (1 H, m, 3α-H), 0.99 (3H, s, 19-CH₃), 0.87 (3H, s, 18-CH₃).

HRMS m/z: 362.2447 for C₂₂H₃₄O₄ requires 362.2457 (M⁺, 7%), 344.2347 for C₂₂H₃₂O₃ requires 344.2351 (M⁺, -H₂O, 3.5%), 330.2228 for C₂₁H₃₀O₃ requires 330.2195 (M⁺, -CH₃OH, 1.6%), 329.2149 for C₂₁H₂₉O₃ requires 329.2117 (M⁺, -H₂O, -CH₃, 4.2%), 261.1838 for C₁₇H₂₅O₂ requires 261.1854 (M⁺, -101, 100%), 229.1594 for C₁₆H₂₁O requires 229.1592 (M⁺, -101, CH₃OH, 10%), 211 for C₁₆H₁₉ (M⁺, -101, CH₃OH, -H₂O, 2%).

Example 16: *3*β*-Methoxyandrost-5-ene-7*β*,17*β*-diol* (24). 24 was prepared by reduction of 3β-methoxyandrost-5-ene-7,17-dione 20, with sodium borohydride in a mixture of methylene dichloride-methanol at room temperature. The product was purified by column chromatography.

¹H NMR (CDCl₃, 200 MHz): δ 5.29 (1 H, t, J=1.95, 6-H), 3.84 (1 H, dt, J=8.3 Hz, 7α-H), 3.65 (1 H, t, J=8.3 Hz, 17α-H), 3.36 (3H, s, OCH₃), 3.09 (1 H, m, 3α-H), 1.06 (3H, s, 19-CH₃), 0.78 (3H, s, 18-CH₃).

Example 17: *3*β*-Acetoxy-7-methoxyandrost-5-ene-17-one* (26). A mixture of N-bromosuccimimide (2.15 g, 0.012 mol) and azobisisobutyronitrile (50 mg), a radical initiator, was added in one lot to refluxing solution of 3β-acetoxyandrost-5-ene-17-one (3.3 g, 0.01 mol) and azobisisobutyronitrile (50 mg) in carbon tetrachloride (70 mL). The mixture was refluxed for 20 min, cooled and solid succinimide was removed by filtration. The clear filtrate was concentrated at 20 °C and triturated with petroleum ether, cooled at 0 °C to obtain the white crystalline 7α-bromo derivative 25; yield 2.8 g (68.6%).

¹H NMR (CDCl₃, 200 MHz): δ 5.78 (1H, d, J=5.13 Hz, 6-H), 4.76 (1 H, m. 7β-H), 4.68 (1 H, m, 3α-H), 2.07 (3H, S, OCOCH₃), 1.07 (3H, s, 19-CH₃), 0.91 (3H, s. 18-CH₃).

To a clear solution of 3β-acetoxy-7α-bromoandrost-5-ene-17-one, 25, (0.5 g) in methanol (20 mL) was added silica gel (2.0 g, 70-230 mesh) and the mixture was stirred for 2 h at room temperature. The methanolic solution was cooled, neutralized with saturated sodium bicarbonate solution, and filtered on a thin bed of celite. The clear filtrate was cooled at 0 °C to obtain the white crystalline 7-methoxy derivative 26. Yield 0.32 g (72.7%), m.p. 175-78 °C. The NMR spectrum of the white solid showed 55% 7β-methoxy-and 45% 7α-methoxy-compound. Spectral data described below were deduced from the spectrum of the mixture. The biological activity was assayed as the mixture (Table 1).

7α-methoxy-derivative: ¹H NMR (CDCl₃, 200 MHz): δ 5.79 (1H, d, J=5.13 Hz, 6-H), 4.65 (1 H, m, 3α-H), 3.49 (1 H, m, 7β-H), 3.37 (3H, s, )CH₃), 2.05 (3H, s, OCOCH₃), 1.03 (3H, s, 19-CH₃), 0.87 (3H, s, 18-CH₃).

¹³C NMR (CDCl₃, 300 MHz): δ 223.5 (C-17), 171 (C=O acetate), 146.5 (C-5), 121.0 (C-6), 81.3 (C-3), 72.29 (C-7), 56.5 (OCH₃), 44.43, 42.86, 37.03 (C-methines), 38.14, 36.62, 35.71, 30.93, 27.44, 21.83, 20.09 (C-methylenes), 21.32 (OCOCH₃), 18.17 (C-19), 13.06 (C-18).

7β-methoxy-derivative: ¹H NMR (CDCl₃, 200 MHz): δ 5.53 (1H, s, 6-H), 4.65 (1H, m, 3α-H), 3.5 (1 H, m, 7α-H), 3.34 (3H, s, OCH₃), 2.05 (3H, s, OCOCH₃), 1.08 (3H, s, 19-CH₃), 0.89 (3H, s, 18-CH₃).

¹³C NMR (CDCl₃, 300 MHz): δ 223.5 (C-17), 171 (C=O acetate), 143.0 (C-5), 121.8 (C-6), 81.3 (C-3), 73.24 (C-7), 55.0 (OCH₃), 51.33, 48.18, 37.03 (C-methines), 37.83, 36.37, 35.92, 31.19, 27.6, 23.66, 20.39 (C-methylenes), 21.32 (OCOCH₃), 18.95 (C-19), 13.55 (C-18).

LCMS (APES, positive): m/z 383.3 (M+Na, 100%), 359.2 (M, -1, 2.5%), 299.3 (M, - 60, 47%), 269.2 (M, -60, -32, 4%).

Example 17: *3*β*-Methoxy-17*β*-hydroxyandrost-5-ene-7-one* (28). 3β-methoxyandrost-5-ene-17-one (19, 0.7 g, 2.3 mmol) was dissolved in dry tetrahydrofuran (20 mL) and the solution was cooled to 0 °C. Lithium tri-t-butoxyaluminum hydride (1.2 g) was added to the cooled solution and the mixture was stirred at the same temperature for 2.5 h. Excess base was neutralized with dilute acetic acid and the produce was extracted with methylene chloride. 3β-methoxyandrost-5-ene-17β-ol (27) was crystallized from methanol; yield 0.6 g (86%), m.p. 145-47 °C.

¹H NMR (CDCl₃, 200 MHz): δ 5.35 (1 H, d, J=5.3 Hz, 6-H), 3.65 (1 H, t, J=7.5 Hz, 17α-H), 3.36 (3H, s, OCH₃), 3.07 (1 H, m, 3α-H), 1.02 (3H, s, 19-CH₃), 0.76 (3H, s, 18-CH₃).

3β-methoxyandrost-5-ene-17β-ol (27) was subjected to air oxidation in the presence of N-hydroxyphthalimide and the producT 3β-methoxy-17β-hydroxyandrost-5-ene-7-one (28) was obtained in 53% yield. An analytical sample was obtained by recrystallization from acetone-hexane, m.p. 202-4 °C.

¹H NMR (CDCl₃, 200 MHz): δ 5.7 (1 H, d, J=1.47, 6-H), 3.36 (1 H, t, J=7.8, 17α-H), 3.38 (3H, s, OCH₃), 3.2 (1 H, m, 3α-H), 1.21 (3H, s, 19-CH₃), 0.77 (3H, s, 18-CH₃).

¹³C NMR (CDCl₃, 300 MHz): δ 202 (C-7), 165.9 (C-5), 125.8 (C-6), 80.8, 78.9 (C-3,17), 55.9, 50.03, 45.22, 44.9, 43.32, 38.72, 38.58,36.22, 35.63, 30.46, 27.56, 25.78, 20.85, 17.3, 11.1 (C-1,2,4,8-16,18,19,OCH₃).

LCMS (API-ES, positive ion mode): λmax 241.5, m/z: 659.5 ((2M+Na)⁺, 18%)), 341.2 ((M+Na)⁺, 100%)), 319.2 ((M+1)⁺, 22%)).

Example 18: *3*β*-Methoxy-17*β*-acetoxyandrost-5-ene-7-one* (29). 3β-methoxy-17β-hydroxyandrost-5-ene-7-one (28) was acetylated in pyridine-acetic anhydride mixture at room temperature to obtain product 29, m.p. 168-70 °C.

¹H NMR (CDCl₃, 200 MHz): δ 5.71 (1 H, d, J=1.73 Hz, 6-H), 4.63 (1 H, t, J=7.1 Hz, 17α-H), 3.41 (3H, s, OCH₃), 3.21 (1 H, m. 3α-H), 2.08 (3H,s, OCOCH₃), 1.2 (3H, s, 19-CH₃), 0.81 (3H, s, 18-CH₃).

¹³C NMR (CDCl₃, 300 MHz): δ 201.3 (C-7), 171 (C=O acetate), 165.7 (C-5), 125.8 (C-6), 81.9, 78.9 (C-3,17), 55.9, 49.9, 44.9, 44.6 (C-8,9,14 and OCH₃), 38.6, 36.2, 35.8, 30.46, 27.5, 25.8, 20.7 (C-1,2,4,11, 12, 15, 16), 21.1, 17.3, 12.0 (C-18,19, OCOCH₃).

HRMS m/z: 360.2311 for C₂₂H₃₂O₄ requires 360.23 (M⁺, 100%), 329.2152 for C₂₁H₂₉O₃ requires 329.2117 (M⁺ -CH₃, 42%), 328.2101 for C₂₁H₂₈O₃ requires 328.2038 (M⁺ - CHOH, 9.5%), 300.2061 for C2₂₀H2₂₈O₂ requires 300.2089 (M⁺ -CH₃COOH, 12%), 268.1840 for C₁₉H₂₄O requires 268.1827 (M⁺ -CH₃OH, -CH₃COOH, 6%), 253.1640 for C₁₈H₂₁O requires 253.1592 (M⁺ -CH₃OH, -CH₃COOH, -CH₃, 20%).

Example 19: *3*β*-t-Butoxyandrost-5-ene-17-one* (30). The product was prepared by the procedure of Armstrong et al. (Tet. Lett., 29:2483-2486 (1988), incorporated herein by reference in its entirety), utilizing t-butyl trichloroacetimidate and borontrifluoro etherate in a solution of dichloromethane and cyclohexane, in 67.2% yield; m.p. 185-87 °C.

¹H NMR (CDCl₃, 200 MHz): δ 5.34 (1 H, d, J=4.89 Hz, 6-H), 3.32 (1 H, m, 3α-H), 1.2 (9H, s, t-Bu), 1.02 (3H, s, 19-CH₃), 0.89 (3H, s, 18-CH₃).

Example 20: *3*β*-t-Butoxyandrost-5-ene-7,17-dione* (31). 3β-t-butoxyandrost-5-ene-17-one (30) was oxidized at the 7 position to obtain 3β-t-butoxyandrost-5-ene-7,17-dione (31) in 87% yield m.p. 189-91 °C.

¹H NMR (CDCl₃, 200 MHz): δ 5.71 (1 H, s, 6-H), 3.48 (1 H, m, 3α-H), 1.2 (9H, s, t-Bu), 1.2 (3H, s, 19-CH₃), 0.89 (3H, s, 18-CH₃).

HRMS m/z: 358.2502 for C₂₃H₃₄O₃ requires 358.2508 (M⁺, 2.4%), 302.1866 for C₁₉H₂₆O₃ requires 302.1882 (M⁺ -(CH₃)₂C=CH₂, 52%), 284.1798 for C₁₉H₂₄O₂ requires 284.1776 (M⁺ -(CH₃)₂C=CH₂, -H₂O, 17%), 274.1965 for C₁₈H₂₆O₂ requires 274.1933 (M⁺-CO, -(CH₃)₂C=CH₂, 21%), 256,1843 for C₁₈H₂₄O requires 256,1827 (M⁺-H₂O, -CO, - (CH₃)₂C=CH₂, 25%), 246.1563 for C₁₆H₂₂O₂ requires 246.1620 (M⁺-CO, -CH₂=CH₂, - (CH₃)₂C=CH₂, 100%), 231.1379 for C₁₅H₁₉O₂ requires 231.1385 (M⁺-CO, -CH₃, -CH₂=CH₂, -(CH₃)₂C=CH₂, 9%), 228,1551 for C₁₆H₂₀O requires 228.1514 (M⁺-CO, -CH₂=CH₂, -H₂O, - (CH₃)₂C=CH₂, 43%), 213.1383 for C₁₅H₁₇O requires 231.1446 (M⁺ -CO, -H₂O, -CH₂=CH₂, - CH₃, -(CH₃)₂C=CH₂, 8%).

Example 21: 3β-t-Butyldimethylsilyloxyandrost-5-ene-7,17-dione (32)

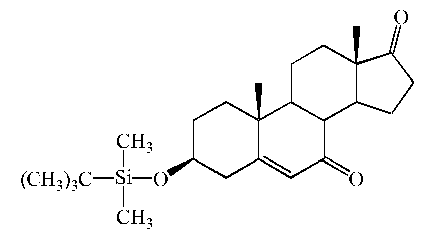

3β-hydroxyandrost-5-ene-7,17-dione (2) (0.3 g, 0.99 mmol) was dissolved in anhydrous dimethylformamide (10.0 mL). Imidazol (0.6 g, 8.82 mmol) and to-butyldimethysilyl chloride (0.3 g, 1.99 mmol) were added in sequence and the mixture was stirred at room temperature under argon for 3 h. The mixture was poured into cold water and the product was extracted with ether and washed with dilute acetic acid, saturated bicarbonate solution, then by water and brine. The solution was dried over MgSO₄ and the solvent was removed. The crude compound was crystallized from aqueous methanol to afford white crystals of 3β-t-butyldimethylsilyloxyandrost-5-ene-7,17-dione (32) in 92.6% yield (0.37 g), m.p. 135-6 °C.

¹H NMR (CDCl₃, 200 MHz): δ 5.73 (1 H, s, 6-H), 3.61 (1 H, m, 3α-H), 1.22 (3H, s, 19-CH₃), 0.89 (12H, s, 18-CH₃ and C(CH₃)₃), 0.07 (6H, s, Si(CH₃)₂).

¹³C NMR (CDCl₃, 300 MHz): δ 220.5 (C-17), 201 (C-7), 166.8 (C-5), 125.6 (C-6), 71.1 (C-3), 50.15, 46.0, 44.33 (C-8,9,14), 48.0, 38.5, 18.2 (C-18,19,C-Me₃); 42.62, 36.35, 35.63, 31.67, 30.73, 24.17, 20.57 (C-1,2,4,11,12,15,16), 25.82 ((CH₃)₃), 17.41, 13.74, (C-18,19), -4.0 (Si(CH₃)₂.

LCMS (AP, positive ion mode): λmax 240, m/z 417.3 ((M+1)⁺, 71%), 285.2 (M+1,-TBDMSiOH, 100%), 267.2 (M+1, -TBDMSiOH, -H₂O, 14%), 249.3 (M+1, -TBDMSiOH, - 2(H₂O), 1 %), 243.2 (M+1, -CH₂=C=O, 6%).

Example 22: *3*β*,17*β*-di(t-butyldimethylsilyloxy)androst-5-ene-7-one* (33). The product was prepared from 3β-17β-dihydroxyandrost-5-ene-7-one as described for compound 32. M.P. 99-101 °C.

¹H NMR (CDCl₃, 200 MHz): δ 5.65 (1H, s, 6-H), 3.60 (2H, m. 3α- and 17α-H), 1.18 (3H, s, 19-CH₃), 0.74 (3H, s, 18-CH₃), 0.87 (18H, s, Si-t-butyl), 0.04 (12H, s, Si-Me₂).

Example 23: *3*β*-Acetoxyandrost-5-ene-7*β*,17*β*-di(t-butyldimethylsilyl) ether* (35). Prepared from 3β-acetoxyandrost-5-ene-7β,17β-diol (34) and t-butyldimethylsilyl chloride in dichloromethane. M.p. 85-87 °C.

LCMS (APCI, positive ion mode): m/z 575.25 (M-1)⁺, 515.35 (M-1, -CH₃COOH, 40%), 385.25 (M+1, -CH₃COOH, -TBDMSiOH, 11%), 253.15 (M+1, -CH₃COOH, - 2(TBDMSiOH), 29%), 238.15 (M+1, -CH₃COOH, -2(TBDMSiOH), -CH₃, 7%).

Example 24: *3*β*-Acetoxy-17*β*-t-butyldimethylsilyloxyandrost-5-ene-7-one* (36). Prepared from 3β-acetoxy-17β-hydroxyandrost-5-ene-7-one (4). M.p. 202-4 °C.

¹H NMR (CDCl₃, 200 MHz): δ 5.7 (1H, d, J=1.3 Hz, 6-H), 4.72 (1H, m, 3α-H), 3.56 (1 H, dd, J=1.7 Hz, 17α-H), 2.07 (3H, s, OCOCH₃), 1.22 (3H, s, 19-CH₃), 0.88 (9H, s, Si-t-butyl), 0.72 (3H, s, 18-CH₃), 0.009 (6H, s. SiMe₂).

¹³C NMR (CDCl₃, 300 MHz): δ 201.5 (C-7), 170 (OCOCH₃), 164.1 (C-5), 126.5 (C-6), 80.9 (C-17), 72.1 (C-3), 50.1, 45.3, 44.5 (C-methines), 37.8, 36.0, 35.9, 30.9, 27.3, 26.2, 20.87 (C-methylenes), 25.9, 25.82, 21.2, 17.3, 11.3, -4.5, -4.9 (C-Methyls).

Example 25: 3β-(2-tetrahydropyranoxy)androst-5-ene-7,17-dione (37)

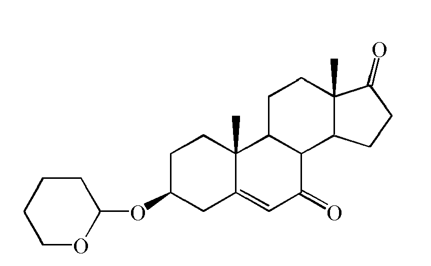

3β-hydroxyandrost-5-ene-7,17-dione (2) (0.5 g, 1.65 mmol) was placed in a dried, argon-flushed flask where it was dissolved in dry dichloromethane (20.0 mL). Pyridinium toluene-p-sulfonate (0.043 g. 0.17 mmol) was added followed by 3,4-dihydro-2H-pyran (0.23 mL, 2.5 mmol) and the solution was stirred at room temperature, under an atmosphere of argon, for 2-3 h. The reaction was quenched by adding water and the product was extracted thrice with ether (3 x 20 mL). The combined ether extracts were washed with dilute acetic acid, water and bicarbonate solution and dried (MgSO₄). Solvent was removed and the crude product was purified using chromatography on silica gel (ethyl acetate-hexane, 15:85, v/v). The compound first eluted was identified as androsta-3,5-diene-7,17-dione (0.150 g), m.p. 167-8° C.

Further elution with the same solvent gave 3β-(2-tetrahydropyranoxy)androst-5-ene-7,17-dione (37) as a white solid, which was crystallized from methanol (0.43 g, 68%), m.p. 137-39 °C.

¹H NMR (CDCl₃, 200 MHz): δ 5.76, 5.73* (1 H, dd, J=2 Hz, 6-H), 4.7 (1 H, m, 2-H Pyran), 3.91 (1 H, m, 6-H pyran), 3.67 (1 H, m, 3α-H), 3.51 (1 H, m, 6-H pyran), 1.23 (3H, s, 19-CH₃), 0.90 (3H, s, 18-CH₃).

¹³C NMR (CDCl₃, 300 MHz): δ 221 (C-17), 201 (C-7), 167, 166.4* (C-5), 125.8 (C-6), 97.4, 97.1*, 74.7, 74.53* (C-3, C-2 (Pyran)), 62.86 (C-6 (Pyran)), 50.05, 45.69, 44.26 (C-8, 9, 14), 47.82, 38.62 (C-10, 13), 40.10, 38.80, 36.41, 36.15, 35.59, 31.07, 30.68, 29.31, 27.64, 25.35, 24.14, 20.52, 19.83 (C-1, 2, 4, 11, 12, 15, 16, C-3, 4, 5 (Pyran))*, 17.35, 13.71 (C-18, 19).

LCMS (APES, positive): λmax 238; m/z 795.5 (2M+Na, 100%), 409.1 (M+Na, 85%), 285.1 (M, -101, 14%).

* Extra peaks are due to the diastereomers.

Example 26: 3β-dodecanoxyandrost-5-ene-7,17-dione (39)

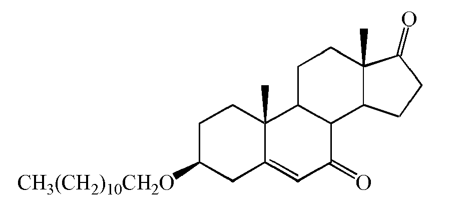

A solution of DHEA (1) (2.88 g. 0.01 mol) in anhydrous tetrahydrofuran (20 mL) was added to a mixture of sodium hydride (0.5 g, 60% in oil, washed twice with pentane) in anhydrous tetrahydrofuran (10 mL). The mixture was refluxed for 2 h, cooled to room temperature, and a solution of 1-bromododecane (2.74 g, 0.11 mol) was added slowly (10 min). The solution was refluxed for 4 h, cooled and poured into ice water and the product was extracted with ether-ethyl acetate mixture (1:1). 3β-Dodecanoxyandrost-5-ene-17-one (38) was purified by column chromatography on silica gel (eluent ethyl acetate-petroleum ether, 1:9), yield 0.6 g (43%, based on 30.5% conversion). Further elution with the same solvents at (4:6) afforded unreacted DHEA (2.0 g, 69.5%).

¹H NMR (CDCl₃, 200 MHz): δ 5.38 (1H, d, J=5.12 Hz, 6-H), 3.45 (2H, t, J=6.6, 6.83 Hz, -CH₂-O), 3.14 (1 H, m. 3α-H), 1.03 (3H, s, 19-CH₃), 0.89 (3H, s, 18-CH₃).

3β-dodecanoxyandrost-5-ene-17-one (38) was subjected to oxidation by a procedure to be published and the product, 3β-dodecanoxyandrost-5-ene-7,17-dione (39) was obtained in 82.8% yield, m.p. 70 °C.

¹H NMR (CDCl₃, 200 MHz): δ 5.74 (1 H, d, J=1.71 Hz, 6-H), 3.47 (2H, t, J=6.35, 6.83 Hz, -CH₂-O), 3.27 (1 H, m, 3α-H), 1.22 (3H, s, 19-CH₃), 0.9 (3H, s, 18-CH₃).

¹³C NMR (CDCl₃, 300 MHz): δ 220 (C-17), 200 (C-7), 166.0 (C-5), 125.8 (C-6), 77.45 (C-3), 68.58 (-CH₂-O), 50.14, 45.75, 44.3 (C-methines), 39.19, 36.31, 35.58, 31.9, 30.7, 30.1, 29.59, 29.46, 29.34, 28.07, 26.16, 24.15, 22.66, 20.55 (C-methylenes), 17.35, 14.07, 13.7 (C-methyls).

HRMS m/z: 470.3758 for C₃₁H₅₀O₃ requires 470.3760 (M⁺, 23%), 301.1783 for C₁₉H₂₅O₃ requires 301.1804 (M⁺ -C₁₂H₂₅, 10%), 285.1870 for C₁₉H₂₅O₂ requires 285.1854 (M⁺ -C₁₂H₁₅O, 12%), 284.1610 for C₁₉H₂₄O₂ requires 284.1776 (M⁺ -C₁₂H₁₅OH, 5.6%).

Example 27: 3β-(1'-ethoxy)ethoxyandrost-5-ene-7,17-dione (41)

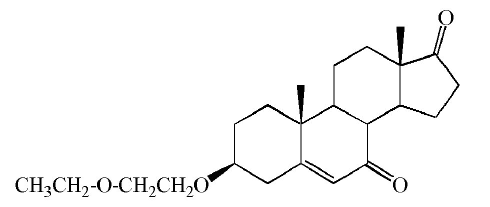

To a solution of DHEA (1) (1.5 g, 0.005 mol) and *p*-toluene sulfonic acid (35 mg) in methylene dichloride (20 mL), a solution of ethyl vinyl ether (1.17 g, 0.016 mol) in methylene dichloride was added slowly during 2 h at room temperature. The solution was stirred for an additional 6h and poured into cold water. The organic layer was separated and the product was subject to column chromatography on silica gel (eluent, ethyl acetate-petroleum ether, 1:9) to afford 3β-(1'-ethoxy)ethoxyandrost-5-ene-17-one (40) as a viscous mass (0.92 g, 84% based on 55% conversion).

¹H NMR (CDCl₃, 200 MHz): δ 5.37 (1H, s, 6-H), 4.78 (1H, q, J=5.37 Hz, O-CH-O), 3.68 (2H, m, -CH₂-O), 3.5 (1 H, m, 3α-H), 1.31 (3H, d, J=5.13, CH₃), 1.2 (3H, m, CH₃), 1.03 (3H, s, 19-CH₃), 0.89 (3H, s, 18-CH₃).

3β-(1'-ethoxy)ethoxyandrost-5-ene-17-one was oxidized at the allylic 7 position of the steroid using air and N-hydroxy phthalimide as described before to obtain 3β-(1'-ethoxy)ethoxyandrost-5-ene-7,17-dione (41) in 50% yield.

¹H NMR (CDCl₃, 200 MHz): δ 5.74 (1H, s, 6-H), 4.79 (1 H, m. O-CH-O), 3.58 (3H, m, -CH₂-O and 3α-H), 1.34 (3H, d, J=5.13 Hz, CH₃), 1.22 (3H, m, CH₃), 1.22 (3H, s, 19-CH₃), 0.9 (3H, s, 18-CH₃).

¹³C NMR (CDCl₃, 300 MHz): δ 220 (C-17), 200 (C-7), 166.0 (C-5), 125.77 (C-6), 98.42 (O-CH-O), 73.89 (C-3), 60.19 (O-CH₂), 50.06, 45.69, 44.27 (C-methines), 41.88, 40.03, 35.58, 30.67, 29.1, 24.1, 20.5 (C-methylenes), 20.7, 17.34, 15.28, 13.71 (C-methyls).

Carbonate Derivatives.

Example 28: 3β-Carbomethoxyandrost-5-ene-7,17-dione (42)

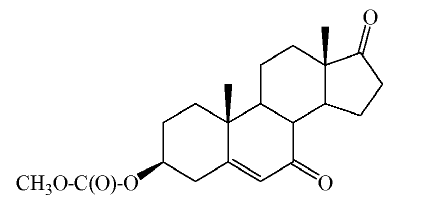

3β-Hydroxyandrost-5-ene-7,17-dione (2) (0.5 g, 0.0016 mol) was dissolved in dry pyridine (6 mL) and the solution was cooled to 0-5 °C. Methyl chlorofmate (0.19 g, 0.002 mol) was added dropwise during 15 min and the mixture was stirred at the same temperature for 3 h. The reaction mixture was quenched with water and the product was extracted with methylene dichloride. The organic layer was washed, dried and solvent was removed. The crude product was purified by column chromatography on silica gel (eluent: acetone-hexane, 2:8) to afford 0.42 g (89%, conversion 80%) of white solid which was crystallized from methanol, m.p. 168-70 °C.

¹H NMR (CDCL₃), 200 MHz): δ 5.77 (1 H, d, J = .7 Hz, 6-H), 3.78 (3H, s, OCH₃), 4.62 (1 H, m, 3α-H), 1.24 (3H, s, 19-CH₃), 0.9. (3H, s, 18-CH₃).

¹³C NMR (CDCl₃, 300 MHz): δ 220.1 (C-17), 200.2 (C-7), 164 (C-5), 155 (O-C-O), 126.7 (C-6), 75.7 (C-3), 54.8 (OCH₃), 49.9,45.7, 44.3, (CH-, 8, 9 and 14), 37.7, 35.8, 35.6, 30.7, 27.2, 24.1, 20.6 (CH₂-, 1, 2, 4, 11, 12, 15 and 16), 17.4, 13.7 (CH₃- 18, and 19).

HRMS m/z : 360.1929 for C₂₁H₂₈O₅ requires 360.1937 (M⁺, 1.7%), 284.1789 for C₁₉H₂₄O₂ requires 284.1776 CH₃OH, -CO₂, 100%), 269.1521 for C₁₈H₂₁O₂ requires 269.1541 (M⁺ -CH₃OH, -CO₂, -CH₃, 54.%), 256.1856 for C₁₈H₂₄O requires 256.1827 (M⁺ - CH₃OH, -CO₂, -CO, 25.6%), 241.1623 for C₁₇H₂₁O requires 241.1592 (M⁺ -CH₃OH, -CO₂, - CH₃, -CO, 11 %), 277.1461 for C₁₆H₁₉O requires 227.1463 (M⁺ -CH₃OH, -CO₂, -CH₃,-CO, - CH₂, 5.6%).

Example 29: 3β-Carboallyloxyandrost-5-ene-7,17-dione (43)

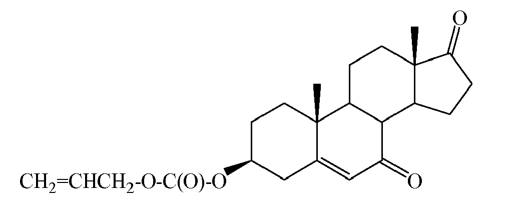

Compound 43 was prepared from 7-oxo-DHEA (2) (1.0 mmol) and allylchloroformate (4.0 mmol) in tetrahydrofuran-pyridine mixture at 0-5 °C. After stirring 6 hours only 30% conversion had occurred; from the mixture the product was isolated in 78% yield (based on 30% conversion) by column chromatography. M.p. 159-61 °C.

¹H NMR (CDCl₃, 200 MHz): δ 5.92 (1 H, m, =CH₂-), 5.74 (1 H, d, J = 1.7 Hz, 6-H), 5.38 (q), 5.29 (m), 5.23 (m) (2H, J = 18.5, 11.6, 1.47 Hz, -CH₂=), 4.62 (2H, dt, J = 5.86, 1.22, 1.47 Hz, -CH₂-O-), 4.62 (1 H, m, 3α-H) 1.21 (3H, s, 19-CH₃), 0.87 (3H, s, 18-CH₃).

¹³C NMR (DCCl₃, 300 MHz): δ 219.2 (C-17), 200 (c-7), 164 (c-5), 154 (O-C-O), 131.5 (=C-), 126.5 (C-6), 118.7 (-CH₂=), 75.7 (C-3), 68.24 (O-CH₂), 50, 45.8, 44.3, (CH-, 8, 9 and 14), 47.64, 38.33 (c-, 10, 13), 37.7, 35.8, 35.4, 30.7, 27.2, 24.02, 20.53, (CH₂-, 1, 2, 4, 11, 12, 15, and 16), 17.3, 13.7 (CH₃- 18, and 19).

LCMS (APES, positive): λmax 234nm; m/z 409.1 (M+Na, 100%), 307.1 (M, -102, +Na, 80%), 285 (M, -101, 52%), 256 (M, -102, -28).

Example 30: *3*β*-Carboethoxyandrost-5-ene-7,17-dione* (44). Prepared from (2) and ethylchloroformate in pyridine at 0-5 °C. Yield 90%, m.p. 187-8 °C.

¹H NMR (CDCl₃, 300 MHz): δ 5.77 (1 H, d, J = 1.65 Hz, 6-H), 4.6 (1 H, m, 3α-H), 4.2 (2H, q, J = 7.2 Hz, - CH₂-O), 1.32 (3H, t, J = 7.2 Hz, CH₃), 1.23 (3H, s, 19-CH₃), 0.9 (3H, s, 18-CH₃).

¹³C NMR (CDCl₃, 300 MHz): δ 220.2 (C-17), 200.7 (C-7), 164.4 (C-5), 154.3 (O-CO), 126.7 (C-6), 75.4 (C-3), 64.1 (O-CH₂), 49.9, 45.7, 44.3, (CH-, 8, 9 and 14), 47.8, 38.4 (C-, 10, 13), 37.7, 35.8, 35.6, 30.7, 27.2, 24.1, 20.5 (CH₂-, 1, 2, 4, 11, 12, 15, and 16), 14.4, 14.2, 13.8 (CH₃, CH₃- 18, 19).

Example 31: *3*β*-Carboisobutoxyandrost-5-ene-7,17-dione* (45). Prepared from (2) and isobutylchloroformate in pyridine at 0-5 °C. Yield 78%, m.p. 132-3 °C.

¹H NMR (CDCl₃, 300 (MHz): δ 5.77 (1H, d, J = 1.66 Hz, 6-H), 4.6 (1H, m, 3α-H), 3.92 (2H, d, J= 6.8 Hz, -CH₂-O), 1.24 (3H, s, 19-CH₃), 0.96 (6H, d, J = 6.6 Hz, ((CH₃)₂), 0.9 (3H, s, 18-CH₃).

¹³C NMR (CDCl₃, 300 MHz): δ 220.2 (C-17), 200.7 (C-7), 164.4 (C-5), 154.5 (O-CO), 126.6 (C-6), 75.5 (C-3), 74.14 (O-CH₂), 49.9, 45.7, 44.3, (CH-, 8, 9, and 14), 27.7 (-CH<), 37.8, 35.8, 35.6, 30.7, 27.2, 24.1, 20.6 (CH₂-, 1, 2, 4, 11, 12, 15, and 16), 18.91 (CH₃), 18.87 (CH₃), 17.35 (19-CH₃), 13.7 (18-CH₃).

Example 32: *3*β*,7*β*-dicarbomethoxyandrost-5-ene-7-one* (46). Prepared from 3β,17β-dihydroxyandrost-5-ene-7-one and methylchloroformate in pyridine at 0-5 °C. Yield 81%, m.p. 167-9 °C.

¹H NMR (CDCl₃, 300 MHz): δ 5.73 (1H, d, J = 1.66 Hz, 6-H), 4.57 (2H, m, 3α-H, 17α-H), 3.79 (3H, s, CH₃O), 3.77 (3H, s, CH₃O), 1.2 (3H, s, 19-CH₃), 0.84 (3H, s, 18-CH₃).

¹³C NMR (CDCl₃, 300 MHz): δ 200.97 (C-7), 163.89 (c-5), 155.7, 154.88 (O-C-O), 126.6 (C-6), 85.65 (C-17), 75.8 (C-3),54.72, 54.58 )OCH₃), 49.65, 44.91, 44.55, (CH-, 8, 9 and 14), 43.03, 38.32 (C-, 10, 13), 37.7, 35.82, 35.65, 27.43, 27.21, 25.68, 20.67 (CH₂-, 1, 2, 4, 11, 12, 15, and 16), 11.94 (CH₃- 18, and 19).

Example 33: *3*β*-Carbooctyloxyandrost-5-ene-7,17-dione* (47). Prepared from (2) and octylchloroformate in pyridine at 0-5 °C. The product was purified by column chromatography on silica gel. Yield 65%, m.p. 72-3 °C.

¹H NMR (CDCl₃, 300 MHz): δ 5.77 (1H, d, J = 1.66 Hz), 4.6 (1H, m, 3α-H), 4.13 (2H, t, J = 6.6 Hz, -CH₂-O), 1.23 (3H, s, 19-CH₃), 0.88 (3H, s, 18-CH₃).

¹³C NMR (CDCl₃, 300 MHz): δ 219.76 (C-17), 200.4 (C-7), 164.28 (C-5), 154.28 (O-C-O), 126.53 (C-6), 75.34 (C-3), 68.08 (O-CH₂), 49.81, 45.6, 44.2, (CH-, 8, 9 and 14), 47.69 (c-13), 38.31 (c-10), 37.67, 35.74, 35.46, 31.65, 30.61, 29.06, 29.04, 28.54, 27.15, 25.6, 24.05, 22.52, 20.47 (methylenes), 14.02, (CH₃), 17.25 (19-CH₃), 13.65 (18-CH₃).

LCMS (APES, positive): m/z 459.2 (M+1, 8%), 285.1 (M, -174, 100%).

Example 34: 3β-Carbo(9-fluorenyl)methoxyandrost-5-ene-7,17-dione (48).

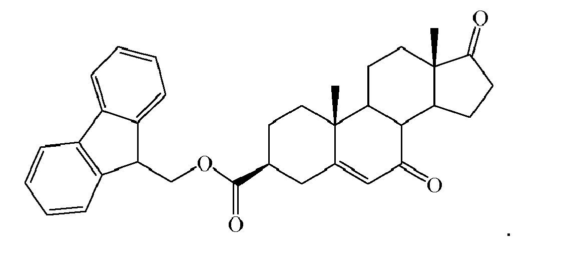

A mixture of 7-oxoDHEA (2) (0.3 g, mmol) and 9-fluorenylmethylchloroformate (0.285 g, 1.1 mmol) in pyridine (4.0 mL) was stirred at room temperature for 1 hour. The product was purified by column chromatography on silica gel, to yield 57% (0.3 g), m.p. 98-103 °C, λₘₐₓ 260 nm.

¹H NMR (CDCl₃, 200 MHz): δ 7.77 (2H, d, J=7.11 Hz, Ar-H), 7.62 (2H, d, J=6.83 Hz, Ar-H), 7.37 (4H, m, Ar-H), 5.77 (1 H, d, J=1.46 Hz, 6-H), 4.61 (1 H, m, 3-H), 4.43 (2H, d, J=6.96 Hz, AB system, OCH₂), 4.26 (1 H, t, J=7.08 Hz, AB system, Ar-H), 1.25 (3H, s, 19-CH₃), 0.90 (3H, s, 18-CH₃).

LC-MS (API-ES) m/z: 547 (M+23, 100%), 285 (M+1, -240, 3%).

Example 35: *3*β*-Carbomethoxyandrost-5-ene-7,17*β*-diol* (49). 3β-Carbomethoxyandrost-5-ene-7,17-dione (42) (0.5 g, 0.0014 mol) was dissolved in a mixture of dichloromethane (5 mL) and methanol (10 mL). The mixture was stirred at room temperature and finely powdered sodium borohydride (0.16 g, 0.004 mol) was added slowly. After 15 min the reaction mixture was quenched with water and the product was extracted with methylene dichloride. The organic layer was washed, dried and solvent removed. The product was crystallized from acetone-petroleum ether to afford 0.35 g (70%) of 3β-carbomethoxyandrost-5-ene-7,17β-diol (49) as a white crystalline solid, m.p. 150-52 °C. LC-MS and ¹H NMR analysis of the product showed a 8:2 ratio of isomeric 7β-hydroxy and 7α-hydroxy compound.

¹H NMR for 7β-hydroxy isomer (CDCl₃, 200 MHz): δ 5.33 (1H, s, 6-H), 4.49 (1H, m, 3α-H), 3.77 (3H, s, OCH₃), 3.84 (1 H, d, J=7.08 Hz, 7α-H), 3.64 (1 H, s, 6-H), 4.49 (1 H, m, 3α-H), 3.77 (3H, s, OCH₃), 3.84 (1 H, d, J=7.08 Hz, 7α-H), 3.64 (1 H, t, J=8.05 Hz, 17α-H), 1.07 (3H, s, 19-CH₃), 0.77 (3H, s, 18-CH₃).

7α-hydroxy isomer showed different chemical shifts for 6 and 19 protons, deduced from the spectrum of the mixture (1 H, d, J=4.89 Hz, 6-H), 1.02 (3H, s, 19-CH₃).

LC-MS (API-ES) m/z: 387 (M+23 100%), 311 (M+23, -76, 25%), 271 (M+1, -76, - 18, 5%), 253 (M+1, -76, -18, -18, 3%).

Example 36: *3*β*-Carboethoxyandrost-5-ene-7*β*,7*β*-diol* (50). 3β-Carbomethoxyandrost-5-ene-7,17-dione (44) (0.3 g, 0.8 mmol) in 5 mL dichloromethane and 10 mL methanol was treated with cerium (III) chloride heptahydrate (0.3 g, 0.8 mmol). To this cold solution, finely powdered sodium borohydride (0.09 g, 2.4 mmol) was added slowly. After 15 minutes the solvent was evaporated to complete dryness, the solid crude was taken up in dichloromethane, stirred for 30 minutes and filtered on a small bed of celite. The organic layer was washed once with water, dried and the solvent was removed. The product was crystallized from acetone-petroleum ether to afford 0.22 g (73%) of 3β-carboethoxyandrost-5-ene-7β,17β-diol (50) as a white solid, m.p. 170-71 °C.

¹H NMR (CDCl₃, 200 MHz): δ 5.33 (1H, s, 6-H), 4.48 (1H, m, 3α-H), 4.23 (2H, q, J=7.08, OCH₂), 3.84 (1 H, d, J=7.57 Hz, 7α-H), 3.64 (1 H, t, J=8.3 Hz, 17α-H), 1.31 (3H, t, J=7.08, CH₃), 1.07 (3H, s, 19-CH₃), 0.77 (3H, s, 18-CH₃).

LC-MS (API-ES) m/z: 401 (M+23, 100%), 311 (M+23, -90, 15%), 271 (M+1, -90, -18, 3%), 253 (M+1, -90, 18, -18, 5%).

Example 37: *3*β*-Carbooctyloxyandrost-5-ene-7*β*,17*β*-diol* (51).3β-Carbooctyloxyandrost-5-ene-7,17-dione (47) (0.3 g, 0.65 mmol) was converted to 51 as described for compound 49. The product was crystallized from petroleum ether to afford 0.23 g (77%) of 3β-carbooctyloxyandrost-5-ene-7β,17β-diol (51) as a white solid, m.p. 86-87 °C.

¹H NMR (CDCl₃, 200 MHz): δ 5.33 (1H, s, 6-H), 4.48 (1H, m, 3α-H), 4.12 (2H, t, J=6.6, OCH₂), 3.89 (1H, d, J=7.54 Hz, 7α-H), 3.65 (1H, t, J=8.3 Hx, 17α-H), 0.88 (3H, t, CH₃), 1.07 (3H, s, 19-CH₃), 0.77 (3H, s, 18-CH₃₃).

LC-MS (API-ES) m/z: 485 (M+23, 100%), 311 (M+23, -174, 3%), 293 (M+23, -174, -18, 2%).

| Induction of the Synthesis of Mitochondrial Glycerophosphate Dehydrogenase and Cytosolic Malic Enzymes in Rat Livers | | | | | | |
|---|---|---|---|---|---|---|
| 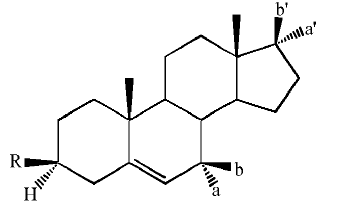 | | | | | | |
| # | R | b/a | b'/a' | Conc. & | Enzymes | |
| | | | | in diet | GDPH | Malic |
| | | | | | % of control | |
| * | AcO | O | O | 0.040 | 336 | 338 |
| 9 | Glu-ester | O | O | 0.085 | 166 | 306 |
| 10 | Glu-triAc-ester | O | O | 0.107 | 164 | 234 |
| 12 | AcO | H/H | Glu-ester | 0.083 | 128 | 229 |
| 13 | AcO | O | Glu-ester | 0.093 | 204 | 363 |
| 16 | AcO | Glu-ester/H | AcO | 0.071 | 241 | 367 |
| 17 | AcO | H/Glu-triAc-ester | O | 0.046 | 81 | 93 |
| 18 | OTs | H/H | O | | | |
| 19 | MeO | H/H | O | 0.085 | 241 | 312 |
| 20 | MeO | O | O | 0.055 | 323 | 424 |
| 21 | MeO | O | O-CH₂-CH₂-O | 0.042 | 194 | 311 |
| 22 | MeO | OH/H | O-CH₂-CH₂-O | 0.063 | 395 | 595 |
| 23 | MeO | H/OH | O-CH₂-CH₂-O | 0.063 | 262 | 324 |
| 24 | MeO | OH/H | OH/H | 0.055 | 403 | 444 |
| 25 | AcO | H/Br | O | | | |
| 26 | AcO | MeO.H** | O | 0.064 | 328 | 459 |
| 27 | MeO | H/H | OH/H | | | |
| 28 | MeO | O | OH/H | 0.055 | 322 | 307 |
| 29 | MeO | O | AcO/H | 0.06 | 188 | 166 |
| 30 | *tert*-BuO | H/H | O | | | |
| 31 | *tert*-BuO | O | O | 0.04 | 93 | 116 |
| 32 | TBDMSO | O | O | 0.036 | 241 | 313 |
| 33 | TBDMSO | O | TBDMSO/H | 0.085 | 168 | 380 |
| 34 | AcO | OH/H | OH/H | 0.046 | 497 | 508 |
| 35 | AcO | TBDMSO/H | TBDMSO/H | 0.082 | 123 | 100 |
| 36 | AcO | O | TBDMSO/H | 0.07 | 375 | 382 |
| 37 | Tetrahydropyran | O | O | 0.064 | 280 | 357 |
| 38 | Dodecanoxy | H/H | O | | | |
| 39 | Dodecanoxy | O | O | 0.068 | 156 | 189 |
| 40 | (Ethoxy)ethyl | H/H | O | | | |
| 41 | (Ethoxy)ethyl | O | O | 0.05 | 168 | 275 |
| 42 | Carbomethoxy | O | O | 0.1 | 500 | 786 |
| 43 | Carboallyloxy | O | O | 0.067 | 440 | 384 |
| 44 | Carboethoxy | O | O | 0.045 | 265 | 373 |
| 45 | Carbo,iso-butyloxy | O | O | 0.048 | 253 | 515 |
| 46 | Carbomethoxy | O | Carbomethoxy/H | 0.051 | 295 | 292 |
| 47 | Carbooctyloxy | O | O | 0.055 | 256 | 312 |
| 48 | Carbo(9-fluorene) | O | O | 0.09 | 218 | 310 |
| | methoxy | | | | | |
| 49 | Carbomethoxy | OH, H** | OH/H | 0.042 | 377 | 359 |
| 50 | Carboethoxy | OH/H | OH/H | 0.044 | 277 | 321 |
| 51 | Carbooctyloxy | OH/H | OH/H | 0.054 | 343 | 260 |

| | | | | | | |
|---|---|---|---|---|---|---|
| Each compound was tested in a group of two or three rats. Enzyme activity in the livers of rats fed the stock diet without supplementation is termed 100%. The abbreviation TBDMSO = tert-butyldimethylsilyl oxy. * Mean values from ten experiments (20 rats) in which 7-oxo-DHEA was the comparative standard. ** Products tested as diestereomeric mixture (% ratio of β and α, 26, 55:45, 49, 80:20) The following compounds did not induce the liver enzymes: 17-oxoA-3β-glucopyranoside Me ester (8), 3β-acetoxy-17-oxoA-7-glucopyranoside Me ester (17), 3β-*tert*-butoxyandrost-5-ene-17-one (30), 3β-acetoxyandrost-5-ene-7β,17β-di-*tert*-butyldimethylsilyl ether (35); A = androst-5-ene. | | | | | | |

It will be appreciated that the methods and compositions of the instant invention can be incorporated in the form of a variety of embodiments, some of which are disclosed herein. It will be apparent to the artisan that other embodiments exist and do not depart from the scope of the invention. Thus, the described embodiments are illustrative and should not be construed as restrictive.

For any of the uses of formula 1 compounds described herein, e.g., in any of the examples above, the results or biological effects that are obtained using individual formula 1 compounds are optionally compared to the results or biological effects that are obtained using a reference formula 1 compound such as AET, AED, BrEA, positive controls or negative controls or to other known modulators of the biological activity, symptom or clinical condition of interest. Such comparisons provide guidance for using the formula 1 compounds in the different methods or clinical conditions. Such comparison information allows, e.g., tailoring of dosages and dosing schedules, routes of administration or the like for individual applications for the formula 1 compounds.

To the extent not already indicated, it will be understood by those of ordinary skill in the art that any of the various specific embodiments, compounds or compositions described herein may be modified to incorporate other appropriate features, e.g., as shown in any other of the specific embodiments disclosed herein or in the cited references.

## Claims

1. A compound for use in the treatment of hyperglycemia or diabetes wherein the compound has the structure 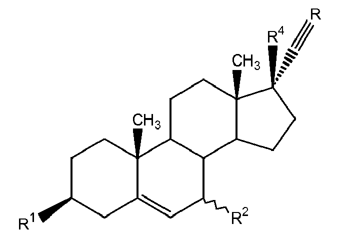 wherein R¹, R² and R⁴ independently are -OH or an ester, R is -CR^{A} and R^{A} is -H or -C₁₋₂₀ optionally substituted alkyl, -C₁₋₂₀ optionally substituted alkenyl, -C₁₋₂₀ optionally substituted alkynyl, -(CH₂)₀₋₆-optionally substituted phenyl or -(CH₂)₀₋₆-optionally substituted heterocycle.

2. Use according to claim 1 wherein the compound is for use in the treatment of hyperglycemia.

3. Use according to claim 2 wherein R^{A} is -H.

4. Use according to claim 1 wherein the compound has the structure

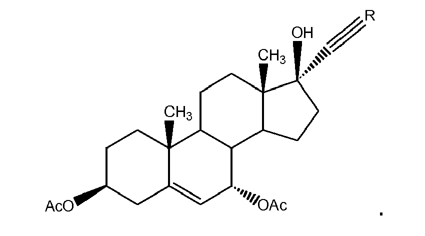

5. Use according to claim 4 wherein R^{A} is -H.

6. Use according to claim 1 wherein the compound has the structure

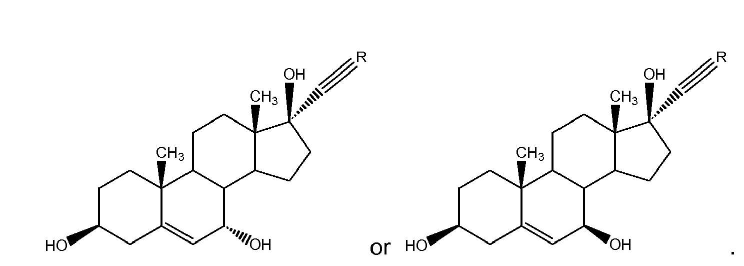

7. Use according to claim 6wherein R is -CH.

8. Use according to claim 7wherein the compound is for use in the treatment of hyperglycemia.

9. A compound for use in the treatment of hyperglycemia or diabetes wherein the compound has the structure 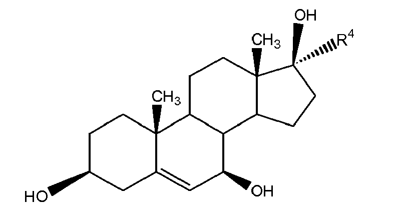 wherein R⁴ is -C=C-(CH₂)ₙH, wherein n is 0, 1, 2, 3, 4, 5, 6, 7 or 8.

10. Use according to claim 9 wherein R⁴ is -C≡CH or -C≡C-CH₃.

11. Use according to claim 10 wherein the compound is for use in the treatment of hyperglycemia.

12. A compound for use in the treatment of inflammation wherein the compound has the structure 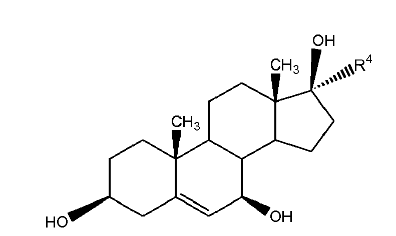 wherein R⁴ is -C≡C-(CH₂)ₙH, wherein n is 0, 1, 2, 3, 4, 5, 6, 7 or 8.

13. Use according to claim 12 wherein R⁴ is -C≡CH or -C≡C-CH₃.

14. Use according to claim 13 wherein the compound is for use in the treatment of inflammation associated with ischemia or oxygen reperfusion after ischemia.

15. Use according to claim 13 wherein the compound is for use in the treatment of inflammation associated with an autoimmune disease.

16. A compound for use in the treatment of a peripheral nerve injury, peripheral neuropathy, a localized neuropathy or a central nervous system disease, wherein the compound has the structure 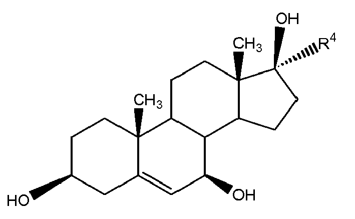 wherein R⁴ is -C≡C-(CH₂)ₙH, wherein n is 0, 1, 2, 3, 4, 5, 6, 7 or 8.

17. Use according to claim 12 wherein R⁴ is -C≡CH or -C≡C-CH₃.
